# EUROPEAN PATENT APPLICATION

(11) **EP 4 553 080 A1**
(43) Date of publication of application: **14.05.2025**
(21) Application number: 23208412.9
(22) Date of filing: 07.11.2023
(51) Int. Cl.: C07H 19/14, C07H 19/23, A61P 31/14

(54) **NUCLEOSIDE DERIVATIVES AS ANTIVIRAL AGENTS AGAINST CORONAVIRUSES**

(71) Applicant: Ustav Organicke Chemie a Biochemie AV CR, v.v.i., 16000 Praha 6 (CZ)
(72) Inventor: Nencka, Radim, 25263 Roztoky (CZ); Otava, Tomas, 28922 Lysa nad Labem (CZ); Dejmek, Milan, 16000 Praha 6 (CZ); Boura, Evzen, 10100 Praha 10 (CZ); Bobileva, Olga, 1014 Riga (LV)
(74) Representative: Harber IP s.r.o.

(57) **Abstract**

The invention relates to small molecule inhibitors of formula (I) targeting the nspl4 protein of SARS-CoV-2 and other coronaviruses, for use as antiviral agents directly targeting viral proteins.

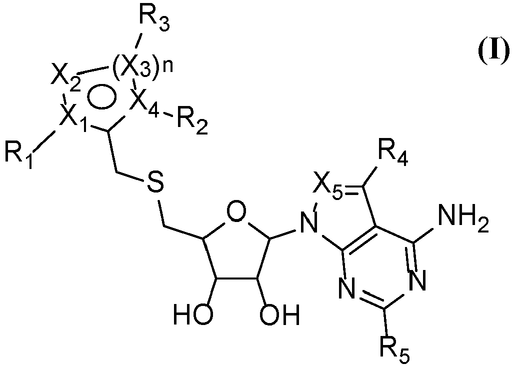

## Description

### Field of the Invention

The present invention pertains to the field of pharmaceutical compositions and therapeutics. More specifically, it relates to novel nsp14 inhibitors with antiviral activity against coronaviruses, including severe acute respiratory syndrome coronavirus (SARS-CoV), SARS-CoV-2 and Middle East respiratory syndrome coronavirus (MERS-CoV).

### Background Art

Coronaviruses have garnered significant attention due to their ability to cause severe respiratory infections in humans. Recent outbreaks of coronaviruses, such as the SARS-CoV in 2002-2003 and the ongoing COVID-19 pandemic caused by SARS-CoV-2, have highlighted the urgent need for effective antiviral treatments. The current standard of care primarily relies on repurposed drugs and vaccines, which have limitations such as variable efficacy and emerging drug resistance (Murakami, N., et al., Nat Rev Nephrol 2023/19, 38-52).

Coronavirus genomes are generally among the longest among RNA viruses with 27-31 thousands of nucleotides. The genome organization of coronaviruses is similar to other members of the Nidovirus order, with nonstructural proteins encoded in the 5'-proximal two-thirds and structural proteins encoded in the 3'-proximal one-third of the genome (Brian, D. A., Curr Top Microbiol Immunol 2005/287, 1-30). Important molecular targets of these viruses include viral polymerase (nsp12 protein) and viral proteases, which have been used to design new antiviral drugs. However, the SARS-CoV-2 genome, as well as the genomes of other coronaviruses, contains information for a number of other proteins that play key roles in viral replication and life cycle (Zhou, Y. W., et al., Signal Transduct Target Ther 2021/6 (1), 317). The bifunctional protein nsp14 is one of the viral proteins of this type. This protein has an exonuclease domain at its N-terminus, which mediates the repair activity when incorrect nucleotides are inserted into the newly inserted viral RNA strand. A methyltransferase domain is included at the C-terminus, which methylates the cap of the viral RNA at the guanine nucleobase (Tahir, M., J Med Virol 2021/93, 4258-4264; Nencka, R., et al., Nucleic Acids Res 2022/50, 635-650).

Cells install a so-called cap on their mRNA, the base of which is guanosine triphosphate (GTP) attached to the first nucleotide of the RNA via a hydroxy group at the 5' position. This structure is subsequently methylated at the guanine nucleobase of GTP and the 2' OH group of the first and sometimes second nucleotide of the mRNA. Such capped RNA is then not recognized by mechanisms that recognize the presence of foreign RNA in the cell cytoplasm and also appears to affect the efficiency of translation of mRNA into protein structures (Ramanathan, A., et al., Nucleic Acids Res 2016/44, 7511-7526). However, the capping process takes place in the nucleus to which the viral RNA does not have access and therefore these viruses cannot exploit the machinery in cells to install such caps on their RNA molecules (Nencka, R., et al., Nucleic Acids Res 2022/50, 635-650).

Different families of RNA viruses have therefore developed their own mechanisms by which they install and methylate caps on viral RNA. In the case of coronaviruses, a number of proteins are involved in this process, but the methyltransferase domain of the nsp 14 protein is crucial for the methylation of GTP cap on guanine nucleobase (Park, G. J., et al., Nature 2022/609, 793-800). Investigations of the role of nsp14 N7-methyltransferase suggest that it plays a particularly important role in the immune response to viral infection, and methylation of the N7 position of the guanine nucleobase cap GTP thus significantly interferes with the recognition of viral RNA in the cell cytoplasm and prevents the production of mediators of infection, namely interferon I. Mutation of nsp 14 in the methyltransferase domain preventing efficient methylation has been shown to significantly prevent the spread of infection in both human coronaviruses (SARS-CoV-2) and mouse models (mouse hepatitis virus (MHV)) (Pan, R., et al., mBio 2022/13*).*

Overall, this crucial involvement of nsp 14 in the coronavirus life cycle leads to significant suppression of viral infection by inhibition of nsp 14 N7 methyltransferase in vivo in both animal and human coronaviruses. Moreover, the conservation of this protein across pathogenic human coronaviruses is high and thus inhibitors of a nsp 14 from SARS-CoV-2 affect wide variety of different coronaviruses (Case, J. B., et al., J Virol 2016/90, 7248-7256).

### Disclosure of the Invention

One aspect of the present invention are compounds of the following general formula (**I**): 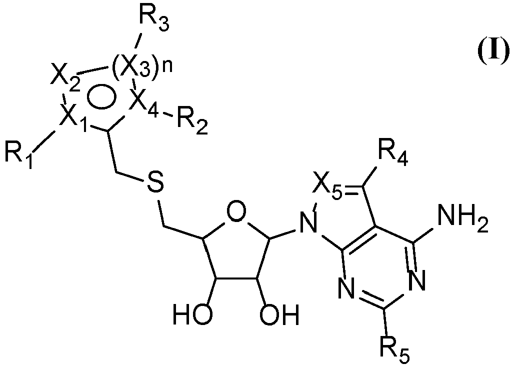 wherein:
**n** is 1 or 2,
**X₁** and **X₄** are independently selected from C and N; wherein one or both of **X₁** and **X₄** are carbon atoms;
and wherein when **X₁** and/or **X₄** is N, n is 1;
**X₂** and **X₃** are independently selected from C, CH, N, NH, O and S,
wherein preferably at least one of **X₂**, **X₃** is a heteroatom selected from N, O, S,
and wherein when **X₃** is oxygen or sulphur, **R₃** is not present;
**X₅** is C orN;
wherein **R₁** is selected from (C3-C6)cycloalkyl, benzyl, (C6-C12)aryl and heteroaryl containing 2-10 carbon atoms and at least one heteroatom selected from N, O, S;
wherein the cycloalkyl, benzyl, aryl and heteroaryl group in **R₁** may optionally be substituted by one or more substituents, which may be the same or different, selected from halogeno, nitro, amino, (C1-C8)alkyl, (C1-C8)alkoxy;
**R₂** is selected from hydrogen, (C1-C8)alkyl, (C3-C6)cycloalkyl, (C6-C12)aryl, (C1-C8)alkoxy,
**R₃** is selected from hydrogen and phenyl, wherein phenyl can be substituted by one or more substituents selected from (C1-C4)alkyl, (C1-C4)alkoxy and halogen;,
**R₄** is a substituent group of the formula: 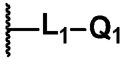
wherein **L₁** is selected from a bond, -(CH₂)_{1 to 4}-, -CH=CH-, -C≡C-, -S-, -O-, -NH-, (C6-C12)aryl and heteroaryl containing 2-10 carbon atoms and at least one heteroatom selected from N, O, S,
and **Q₁** is selected from hydrogen, halogen, trifluoromethyl, trifluoromethoxy, cyano, nitro, amino, (C 1-C8)alkyl, (C2-C8)alkenyl, (C2-C8)alkynyl, (C3-C6)cycloalkyl, (C7-C10)polycycloalkyl, (C5-C6)cycloalkenyl, benzyl, (C1-C8)alkoxy, (C6-C12)aryl, and heteroaryl containing 2-10 carbon atoms and at least one heteroatom selected from N, O, S,
and wherein **Q₁** is optionally substituted by one or more substituents, which may be the same or different, selected from cyano, cyano(C1-C4)alkyl, amino, carbamoyl, (C1-C8)alkoxy,
**R₅** is selected from hydrogen, halogeno, trifluoromethyl, (C1-C6)alkyl, and (C3-C6) cycloalkyl, and/or hydrates and/or solvates, and/or pharmaceutically accetable salts thereof.

An aspect of the present invention are compounds of the general formula (**I**) and/or hydrates and/or solvates, and/or pharmaceutically accetable salts thereof for use as medicaments.

In a preferred embodiment, **R₁** is benzo[d][1,3]dioxolyl, naphthyl or phenyl. More preferably, the phenyl substituent **R₁** is further substituted by one or more of amino, (C1-C8)alkyl and (C1-C8)alkoxy groups.

In a preferred embodiment, **R₂** is (C1-C3)alkyl or (C3-C6)cycloalkyl, more preferably methyl group or cyclopropyl group.

In a preferred embodiment, **R₃** is hydrogen, phenyl or 3,4-dimethoxyphenyl.

In a preferred embodiment, **R₄** is a substituent group of the formula: 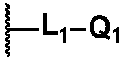 wherein **L₁** is preferably selected from a bond, -CH₂-, -CH₂-CH₂-, -CH₂-CH₂-CH₂-, -CH=CH-, -C≡C--S-, wherein the configuration of the -CH=CH- is "trans". More preferably, **L₁** is selected from a bond,

-CH₂-CH₂-CH₂- and -C≡C-.

**Q₁** is preferably selected from halogen, cyano, (C6-C10)aryl and heteroaryl containing 2-5 carbon atoms and at least one heteroatom selected from N, O, S.

Preferably, **Q₁** is optionally substituted by one or more substituents, which may be the same or different, selected from cyano, cyanomethyl, nitro, amino, carbamoyl, (C1-C8)alkyl, (C1-C8)alkoxy.

Even more preferably, **Q₁** is aryl or heteroaryl substituted by cyano, cyanomethyl, (C1-C8)alkyl and (C1-C8)alkoxy groups.

In a preferred embodiment, **R₅** is selected from hydrogen, halogen and (C1-C8)alkyl. More preferably, R₅ is halogen, most preferably, R₅ is chlorine.

In some preferred embodiments, both **X₁** and **X₄** are carbon atoms, n = 1, and **X₂** and **X₃** are N and O, or N and S, or C and S. Most preferably, **X₂** and **X₃** are N and O.

In some preferred embodiments, two of **X₁**, **X₂**, **X₃** and **X₄** are a nitrogen atom and the remaining instances of these substituents are carbon atoms, and n = 1.

In some preferred embodiments, three of **X₁**, **X₂**, **X₃** and **X₄** is a nitrogen atom and the remaining instances of these substituents are carbon atoms, and n = 1.

In some preferred embodiments, one of **X₂**, **X₃** is a nitrogen atom and the remaining instances of these substituents are carbon atoms, and n = 2.

In some preferred embodiments, two of **X₂**, **X₃** are a nitrogen atom and the remaining instances of these substituents are carbon atoms, and n = 2.

**X₅** is preferably carbon atom.

The present invention also provides a pharmaceutical composition comprising at least one compound of the general formula (**I**) and/or hydrates and/or solvates, and/or pharmaceutically accetable salts thereof as an active pharmaceutical ingredient and optionally at least one pharmaceutically acceptable carrier, filler, diluent and/or adjuvant.

An aspect of the present invention are compounds of the general formula (**I**) and/or hydrate, solvate, and/or pharmaceutically accetable salts, for use in the treatment or prevention of human or animal diseases caused by viruses from Orthocoronavirinae subfamily including SARS-CoV, MERS-CoV, SARS-CoV-2, and FIPV.

The compounds of general formula (I), optionally in the form of a hydrate, a solvate, and/or a pharmaceutically accetable salt, prevent viral RNA methylation by obstructing the binding of *S-*adenosyl-L-methionine (SAM), the methyl group donor, at its binding site. As viral RNA methylation is essential for evading innate immunity and impairs the ability of coronavirus-infected cells to be recognized, blocking this viral pathway results in the suppression of coronavirus replication in mammalian cells, including human cells. The novel inhibitors of general formula (I) possess a unique structure and effectively hinder the SAM binding to nsp 14 of SARS-CoV-2

Pharmaceutically acceptable salts may be formed with acetic acid, aspartic acid, benzenesulfonic acid, benzoic acid, besylic acid, bicarbonic acid, bitartaric acid, hydrobromic acid, camsylic acid, carbonic acid, hydrochloric acid, citric acid, decanoic acid, edetic acid, esylic acid, fumaric acid, gluceptic acid, gluconic acid, glutamic acid, glycolic acid, hexanoic acid, hydroxynaphthoic acid, hydroiodic acid, isothionic acid, lactic acid, lactobionic acid, malic acid, maleic acid, mandelic acid, mesylic acid, methylsulfonic acid, mucic acid, napsylic acid, nitric acid, octanoic acid, oleic acid, pamoic acid, pantothenic acid, phosphoric acid, polygalcturonic acid, propionic acid, salicylic acid, stearic acid, succinic acid, sulfonic acid, tartaric acid, teoclic acid, tosylic acid.

Pharmaceutically acceptable carriers, fillers, diluents and/or adjuvants are known to persons skilled in the art of pharmaceutical formulation, and are described in basic books, such as pharmacopoeias.

Unless defined otherwise, all technical and scientific terms used herein have the same meaning as commonly understood by one of ordinary skill in the art. A dash at the front or end of a chemical group is a matter of convenience to indicate the point of attachment to a parent moiety; chemical groups may be depicted with or without one or more dashes without losing their ordinary meaning. A prefix such as "(Cu-Cv)" indicates that the following group has from u to v carbon atoms, where u and v are integers.

For example "(C1-C6)alkyl" indicates that the alkyl group has from 1 to 6 carbon atoms.

The term "Halogen" as used herein refers to fluoro (-F), chloro (-Cl), bromo (-Br) and iodo (-I).

The term "Alkyl" as used herein is a linear or branched saturated monovalent hydrocarbon. For example, an alkyl group can have 1 to 6 carbon atoms (i.e, (C1-C6)alkyl). Examples of suitable alkyl groups include, but are not limited to, methyl (Me, -CH₃), ethyl (Et -CH₂CH₃), 1-propyl (n-Pr, n-propyl, - CH₂CH₂CH₃), 2-propyl (i-Pr, isopropyl, -CH(CH₃)₂), 1-butyl (*n*-Bu, *n*-butyl, -CH₂CH₂CH₂CH₃), 2-methyl-1-propyl *(*i-Bu, isobutyl, -CH₂CH(CH₃)₂), 2-butyl (*s*-Bu, *sec*-butyl, -CH₃), 2-methyl-2-propyl (*t-*Bu, *tert*-butyl -C(CH₃)₃), 1-pentyl (*n*-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (*n*-hexyl, - CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH3)₂), 2-methyl-3-pentyl (-CH(CH₂CH₃)CH(CH₃)₂), 2,3-dimethyl-2-butyl (-C(CH₃)₂CH(CH₃)₂) and 3,3-dimethyl-2-butyl (-CH(CH₃)C(CH₃)₃).

The term "Cycloalkyl" as used herein refers to a single saturated all carbon ring having 3 to 6 carbon atoms (i.e, (C3-C6)cycloalkyl). Examples of suitable cykloalkyl groups include, but are not limited to, cyclopropyl, cyclobutyl, cyclopentyl and cyclohexyl.

The term "Polycycloalkyl" as used herein refers to a bicyclic and tricyclic all carbon hydrocarbons having 7 to 10 carbon atoms (i.e, (C7-C10)polycycloalkyl). Examples of suitable polycykloalkyl groups include, but are not limited to, bicyclo[2.2.1]heptyl (norbornyl) and tricyclo[3.3.1.13,7]decyl (adamantyl).

The term "Alkenyl" as used herein is a linear or branched hydrocarbon with at least one site of unsaturation, i.e. a carbon-carbon, sp² double bond. For example, an alkenyl group can have 2 to 6 carbon atoms (i.e, (C2-C6)alkenyl). Examples of suitable alkenyl groups include, but are not limited to, ethylene or vinyl (-CH=CH₂), allyl (-CH₂CH=CH₂) and 3-hexenyl (-CH₂CH₂CH=CHCH₂CH₃).

The term "Cycloalkenyl" as used herein refers to a single all carbon ring with at least one site of unsaturation, i.e. a carbon-carbon, sp² double bond. For example cycloalkenyl group can have 5 to 6 carbon atoms (i.e, (C5-C6)cycloalkenyl). Examples of suitable cycloalkenyl groups include, but are not limited to, cyclopent-3-enyl, cyclopent-2-enyl and cyclohex-2-enyl.

The term "Alkynyl" as used herein is a linear or branched hydrocarbon with at least one site of unsaturation, i.e. a carbon-carbon, sp triple bond. For example, an alkenyl group can have 2 o 6 carbon atoms (i.e, (C2-C6)alkenyl). Examples of suitable alkynyl groups include, but are not limited to, ethynyl (-CH=CH), propargyl (-CH₂C≡CH) and the like.

The term "Aryl" as used herein refers to a single all carbon aromatic ring or a condensed all carbon ring system wherein at least one of the rings is aromatic. For example, an aryl group can have 5 to 10 carbon atoms. Aryl includes phenyl radical. Aryl also includes condensed ring systems (e.g., ring systems comprising 2 or more rings) having 9 to 10 carbon atoms in which at least one ring is aromatic and wherein the other rings may be aromatic or not aromatic (i.e., carbocycle). Such multiple condensed ring systems are optionally substituted with one or more (e.g., 1, 2) oxo groups on any carbocycle portion of the condensed ring system. Examples of suitable aryl groups include, but are not limited to, phenyl, indenyl, indanyl, naphthyl, 1,2,3,4- tetrahydronaphthyl, and the like.

The term "Heteroaryl" as used herein includes substituents having a single aromatic ring that has at least one atom other than carbon in the ring, wherein the atom is selected from the group consisting of oxygen, nitrogen and sulphur; "heteroaryl" also includes condensed ring system that have one such aromatic ring, which condensed ring systems are further described below. For example, an heteroaryl group can have 1 to 6 carbon atoms and about 1 to 4 heteroatoms selected from the group consisting of oxygen, nitrogen and sulphur."Heteroaryl" also includes condensed ring systems (e.g., ring systems comprising 2 rings) wherein a heteroaryl group, as defined above, is condensed with one more ring selected from heteroaryls (to form for example 1*H*-pyrrolo[2,3-*b*]pyridyl), heterocycles (to form for example 1,2,3,4-tetrahydro-1,8-naphthyridinyl), carbocycles (to form for example 5,6,7,8-tetrahydroquinolyl) and aryls (to form for example quinolyl) to form the condensed ring system. Thus, a heteroaryl (a single aromatic ring or condensed ring system) has about 1-10 carbon atoms and about 1-6 heteroatoms within the heteroaryl aring. Such multiple condensed ring systems may be optionally substituted with one or more (e.g, 1, or 2) oxo groups on the carbocycle or heterocycle protions of the condensed ring. The point of attachment for a heteroaryl or heteroaryl multiple condensed ring system can be at any suitable atom of the heteroaryl or heteroaryl multiple condensed ring system including a carbon atom and a heteroatom (e.g., a nitrogen). The atom range is for the total ring atoms of the heteroaryl and includes carbon atoms and heteroatoms (for example-5-membered heteroaryl would include a thiazolyl). Examples of suitable heteroaryl groups include, but are not limited to, thienyl, furanyl, pyrrolyl, pyridyl, thianaphthalenyl, indolyl, benzofuranyl, isobenzofuranyl, coumarinyl, quinolinyl, tetrahydroquinolyl, isoquinolinyl, tetrahydroisoquinolyl, chromanyl thiazolyl, isothiazolyl, oxazolyl, isoxazolyl, pyrazolyl, imidazolyl, pyrimidinyl, pyridazinyl, pyrazinyl, benzothiazolyl, benzoisothiazolyl, thienopyridyl, benzooxazolyl, benzoisoxazolyl, furopyridyl, indazolyl, benzimidazolyl, pyrrolopyridyl, quinaxolinyl, quinazolinyl, cinnolinyl, phthalazinyl, naphthyridinyl, thiadiazolyl, furazanyl, triazolyl, triazinyl, pyrrolopyrimidyl, pyrazolopyridyl, imidazopyridyl, tetrazolyl, purinyl, pteridinyl and the like.

The term "Alkoxy" as used herein alone or in combination, refers to a straight-chain or branched-chain alkyl ether radical, wherein the term alkyl is as defined above. For example (C1-C6)alkoxy refers to an -O-alkyl group having 1 to 6 carbon atoms. Examples of suitable alkoxy groups include, but are not limited to, methoxy (-OCH₃), ethoxy (-OCH₂CH₃), isopropoxy (-OCH(CH₃)₂) and *tert*-butoxy (-OC(CH₃)₃).

The term "Substituted" as used herein, and unless defined otherwise in the immediate context, refers to substitution with one or more substituents (e.g., 1, 2, 3, 4 or more), for example selected from the group consisting of halogen, amino, trifluoromethyl, trifluoromethoxy, cyano, cyanoalkyl, carbamoyl, nitro, alkyl, alkenyl, alkynyl, cycloalkyl, polycycloalkyl, cycloalkenyl, benzyl, alkoxy, aryl, heteroaryl or bivalent substituent -O-(CH₂)₁₋₂-O-.

The term "Treatment" or "Treat" or "Treating" as used herein includes one or more of the following: decreasing one or more symptoms resulting from the disease or condition, and/or diminishing the extent of the disease or condition: ameliorating the disease state, delaying the progression of the disease, increasing the quality of life, and/or prolonging survival.

The term "Delaying" as used herein means to defer, hinder, slow, retard, stabilize and/or postpone development of the disease or condition. As is evident to one skilled in the art, a sufficient or significant delay can, in effect, encompass prevention, in that the individual does not develop the disease or condition.

The term "Prevent" or "Prevention" as used herein refers to a regimen that protects against the onset of the disease or disorder such that the clinical symptoms of the disease do not develop.

### Examples

### List of Abbreviations

- FCC: Flash Column Chromatography
- TLC: Thin Layer Chromatography
- C18: Octadecyl
- NMR: Nuclear Magnetic Resonance
- HRMS: High Resolution Mass Spectrometry
- LCMS: Liquid Chromatography-Mass Spectrometry
- LRMS: Low Resolution Mass Spectrometry
- ESI: Electronspray Ionization
- DCA: Dichloroacetic acid
- DCM: Dichloromethane
- DME: Dimethoxyethane
- DMF: *N,N-*Dimethylformamide
- DMSO: Dimethylsulfoxide
- TBME: Methyl *tert*-butyl ether
- TFA: Trifluoracetic acid
- THF: Tetrahydrofuran
- Me: Methyl
- Et: Ethyl
- Pr: Propyl
- i-Pr: Isopropyl
- Bu: Butyl
- Ph: Phenyl
- Ac: Acetyl
- Bz: Benzoyl
- *t*-Bu: *tert*-Butyl
- Boc: *tert*-Butoxycarbonyl
- TBS: *tert*-Butyldimethylsilyl
- TIPS: Triisopropzlsilyl
- Tf: Trifluoromethylsulfonyl
- RT: Room Temperature
- BSA: bis(Trimethylsilyl)acetamide
- CuTC: Copper(I) thiophene-2-carboxylate
- DABCO: 1,4-Diazabicyclo [2.2.2] octane
- DBU: 1,8-Diazabicyclo[5.4.0]undec-7-ene
- DIAD: Diisopropyl azodicarboxylate
- DIBAL: Diisobutylaluminum Hydride
- DIPA: Diisopropylamine
- DIPEA: *N,N-*Diisopropylethylamine
- DMAP: Dimethylaminopyridine
- LAH: Lithiumaluminium Hydride
- LDA: Lithium diisopropylamide
- TBAF: Tetrabutylammonium Fluoride
- TEA: Triethylamine
- dppf: 1,1'-bis(Diphenylphosphino)ferrocene
- dba: Dibenzylidenacetone
- Xantphos: 4,5-bis(Diphenylphosphino)-9,9-dimethylxanthene
- XPhos: 2-Dicyclohexylphosphino-2',4',6'-triisopropylbiphenyl
- DPEphos: bis(2-Diphenylphosphinophenyl)ether
- TBTA: tris(Benzyltriazolylmethyl)amine
- SAH: *S*-Adenosylhomocysteine
- IC₅₀: The half maximal inhibitory concentration

Solvents were evaporated at 40 °C under reduced pressure and prepared compounds were dried at 45 °C in vacuo. Reagents were purchased from commercial suppliers (BLD Pharm, Enamine, Fluorochem, Sigma Aldrich, TCI) and used without further purification. DCM, 1,4- Dioxane and MeCN were dried by activated molecular sieves (3Å). Other dry solvents were purchased from commercial suppliers (Sigma-Aldrich, VWR). TLC was performed on silica gel 60 F₂₅₄ plates (Merck). Compounds were visualized on the TLC plates by irradiation with UV light. FCC (normal-VWR International Silica gel 60 from, particle size 40-63 µm or reverse-phase-C18 RediSep Rf columns) were performed using Combiflash^{®} Rf from Teledyne ISCO. Waters UPLC H-Class Core System (column Waters Acquity UPLC BEH C18 1.7 µm, 2.1 mm×100 mm), Waters Acquity UPLC PDA detector, mass spectrometer Waters SQ D2, and MassLynx mass spectrometry software were used for LCMS analysis of the reaction mixtures. NMR spectra were recorded on a Bruker Avance III^{™} HD 400 instrument (400.0 MHz for ¹H and 101 MHz for ¹³C) using inverse broadband probe with ATM module (5 mm BBO-¹H Z-GRD) or Bruker Avance III^{™} HD 400 instrument with broadband PRODIGY cryoprobe with ATM module (5 mm CPBBO BB-¹H/¹⁹F/D Z-GRD). Chemical shifts (δ) and coupling constants (*J*) are expressed in ppm and Hz, respectively. The NMR experiments were performed in DMSO-*d*₆ and referenced to the solvent signal (δ 2.50 for ¹H NMR and 39.70 for ¹³C NMR). All structures were confirmed by a combination of 1D NMR (¹H and ¹³C) and 2D NMR (H,H-COSY, H,C-HSQC, and H,C-HMBC) experiments. HRMS analyses were carried out on an LTQ XL Orbitrap XL (Thermo Fisher Scientific) using electrospray ionization (ESI).

### First generation

The starting material for preparation of key intermediates **5** and **6** was protected ribose **1**. The 6-chloro-7-deaza-7-iodo purine base was installed using the Vorbrüggen reaction with BSA and SnCl₄ (Seela, F., Tetrahedron 2007/63, 9850-9861). Removal of the benzoyl protection as well as ammonolysis of the 6 position was performed by treatment with NH₄OH (De Ornellas, S., et al., Org Biomol Chem 2015/13, 68-72). Persilylation of the resulting nucleoside (Bourderioux, A., et al., J Med Chem 2011/54, 5498-5507) followed by selective deprotection of the 5'-hydroxy group furnished derivative **4** (Otava, T., et al., ACS Infect Dis 2021/7, 2214-2220). Crucial intermediates **5** and **6** were obtained by introducing an acetylthio group into the 5' position using the Mitsunobu reaction and by subsequent hydrogenolysis **(Scheme 1).**

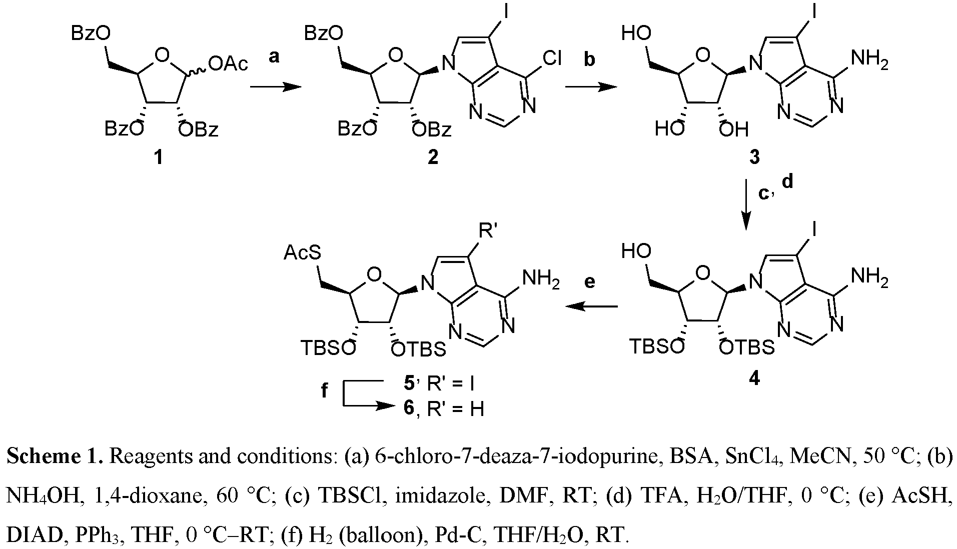

### Example 1

### (2R,3R,4R,5R)-2-((Benzoyloxy)methyl)-5-(4-chloro-5-iodo-7H-pyrrolo[2,3-d]pyrimidin-7-yl)tetrahydrofuran-3,4-diyl dibenzoate (2):

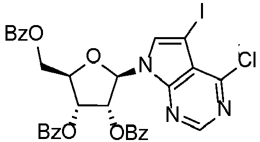

To a suspension of 4-chloro-5-iodo-7H-pyrrolo[2,3-d]pyrimidine (25 g, 89.46 mmol) in dry MeCN (600 mL) was added BSA (26.5 mL, 107.35 mmol). After 15 min the protected ribose 1 was added (49.6 g, 98.40 mmol) followed by SnCl₄ (11.5 mL, 98.40 mmol). Reaction mixture was heated to 50 °C overnight, poured in NaHCO₃ solution and product was extracted to EtOAc (2×700 mL). Pooled organic layers were washed with H₂O and brine dried over Na₂SO₄ and evaporated. FCC (0-50 % EtOAc in cyclohexane) afforded **2** (42 g, 58.02 mmol, 65 %). NMR spectra were consistent with published data (De Ornellas, S., et al., Org Biomol Chem 2015/13, 68-72).

### Example 2

### (2R,3R,4S,5R)-2-(4-Amino-5-iodo-7H-pyrrolo[2,3-d]pyrimidin-7-yl)-5-(hydroxymethyl)tetrahydrofuran-3,4-diol (3):

Nucleoside **2** (11.2 g, 15.47 mmol) was dissolved in aq. NH₃ (50 mL) and 1,4-dioxane (50 ml) and the reaction was stirred at 60 °C for 3 days. Volatiles were evaporated to afford the crude product **3** (6 g, 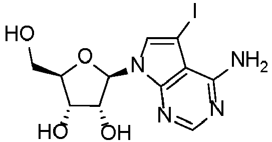 15.3 mmol, 99 %), which was used in the following reaction withour further purification. NMR spectra were consistent with published data (De Ornellas, S., et al., Org Biomol Chem 2015/13, 68-72).

### Example 3

### ((2R,3R,4R,SR)-5-(4-Amino-5-iodo-7H-pyrrolo[2,3-d]pyrimidin-7-yl)-3,4-bis((tert-butyldimethylsilyl)oxy)tetrahydrofuran-2-yl)methanol (4):

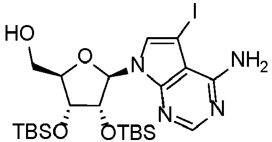

Nucleoside **3** (0.5 g, 1.28 mmol) was dissolved in DMF (4 mL), and imidazole (0.48 g, 6.89 mmol) and TBSCl (0.68 g, 4.48 mmol) were added consecutively. After stirring overnight, excess TBSCl was quenched by the addition of H₂O (50 mL) and the mixture was extracted with cyclohexane (3×100 mL). Combined organic phases were washed with brine, dried over Na₂SO₄, filtered and concentrated in vacuo. The crude mixture was dissolved in THF (9 mL), and mixture of TFA/H₂O (1:1, 8 mL) was added dropwise at 0 °C. After stirring for 5 h at 0 °C, the mixture was neutralized using solid Na₂CO₂, diluted with H₂O (100 mL) and extracted with EtOAc (3×100 mL). Organic layers were combined, washed with brine, dried over Na₂SO₄, and evaporated. FCC (5-50 % acetone in DCM) gave desired product **4** (0.58 g, 0.94 mmol, 72 %). NMR spectra were consistent with published data (Otava, T., et al., ACS Infect Dis 2021/7, 2214-2220).

### Example 4

### S-(((2S,3R,4R,5R)-5-(4-Amino-5-iodo-7H-pyrrolo[2,3-d]pyrimidin-7-yl)-3,4-bis((tert-butyldimethylsilyl)oxy)tetrahydrofuran-2-yl)methyl) thioacetate (5):

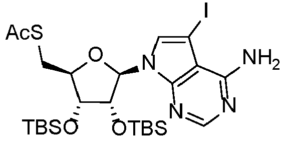

DIAD (3.5 mL, 17.72 mmol) was added to an ice-cold solution of PPh₃ (4.65 g, 17.72 mmol) in dry THF (80 mL) over 5 min. After stirring for 30 min, modified nucleoside **4** (5 g, 8.06 mmol) was added, and stirring at 0 °C was continued for 10 min. To the resulting suspension, a solution of AcSH (1.3 mL, 17.72 mmol) in dry THF (10 mL) was added dropwise and stirring was continued for another 30 min at RT. The solvent was removed under reduced pressure, and the product was purified by FCC (10-40 % acetone in cyclohexane) to afford **5** (4.3 g, 6.33 mmol, 79 %). **¹H NMR** (400 MHz, DMSO-*d₆*) δ 8.10 (1H, s), 7.70 (1H, s), 6.67 (2H, bs), 6.07 (1H, d, *J* = 7.3 Hz), 4.79 (1H, dd, *J =* 7.3, 4.5 Hz), 4.13 (1H, dd, *J* = 4.4, 1.1 Hz), 3.87 (1H, td, *J* = 7.1, 1.1 Hz), 3.41 (1H, dd, *J* = 13.9, 7.3 Hz), 3.18 (1H, dd, *J* = 14.3, 7.2 Hz), 2.35 (3H, s), 0.91, 0.66 (18H, 2×s), 0.12, 0.10, -0.10, -0.39 (12H, 4×s). **¹³C NMR** (101 MHz, DMSO-*d₆*) δ 195.1, 157.3, 152.2, 150.8, 127.5, 103.5, 86.3, 83.9, 74.5, 74.0, 52.6, 31.2, 30.6, 25.9, 25.6, 17.9, 17.6, -4.5, -4.60, -4.62, -5.5. **LRMS** Calculated for C₂₅H₄₄O₄N₄ISSi₂, [M+H]⁺ m/z: 679.2, [M+H]⁺, found 679.2.

### Example 5

### S-(((2S,3R,4R,5R)-5-(4-Amino-7H-pyrrolo[2,3-d]pyrimidin-7-yl)-3,4-bis((tert-butyldimethylsilyl)oxy) tetrahydrofuran-2-yl)methyl) thioacetate (6):

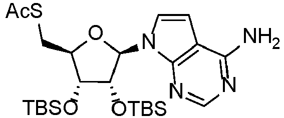

To a stirred solution of modfied nucleoside **5** (5 g, 7.37 mmol) in THF/H₂O mixture (3:1, 150 mL) was added Pd-C (10 % w/w, 0.3 g). The reaction vessel was backfilled several times with hydrogen and the mixture was stirred under a H₂ atmosphere for 12 h. Catalyst was filtered off on a pad of celite and the filter was washed with EtOH. FCC (2-5 % EtOH in DCM) gave thioacetate **6** (4 g, 7.24 mmol, 98 %). **¹H NMR** (400 MHz, CDCl₃) δ 8.29 (1H, s), 7.10 (1H, d, *J=* 3.7 Hz), 6.39 (1H, d, *J=* 3.7 Hz), 6.00 (1H, *d, J=* 6.1 Hz), 5.26 (2H, bs), 4.97 (1H, dd, *J=* 6.1, 4.0 Hz), 4.15-4.10 (2H, m), 3.46 (1H, dd, *J=* 13.9, 4.0 Hz), 3.28 (1H, dd, *J=* 13.9, 6.8 Hz), 2.36 (3H, s), 0.94, 0.76 (18H, 2×s), 0.13, 0.11, -0.11, -0.34 (12H, 4×s). **¹³C NMR** (101 MHz, CDCl₃) δ 195.2, 156.5, 151.3, 150.8, 124.4, 104.5, 98.6, 90.0, 83.8, 74.9, 74.4, 31.8, 30.7, 26.0, 25.9, 18.3, 18.0, -4.3, -4.5, -4.6, -5.1. **LRMS** Calculated for C₂₅H₄₅O₄N₄SSi₂, [M+H]⁺ m/z: 553.3, [M+H]⁺, found 553.3.

First, we decided to study the 5' modification by introducing various heterocycles bearing the same modifications (phenyl and methyl) vicinal to the connection bridge. In order to achieve this, we prepared a series of substituted (het)arylmethanols similar to the commercially available isoxazole derivatives **7** and **8.** To prepare other five and six-membered (het)arylmethanols, a number of synthetic procedures were applied. Imidazole, pyridine and pyrazole analogues **9**, **10** and **11** were prepared according to the published procedures (Chucholowski, A. W., et al. US4906624, 1990-03-06; Luke, R. W. A., et al. WO2004013141, 2004-02-12; Tan, W., et al., *J of Organomet Chem* **2009**/*694*, 199-206). Thiophene derivative **16** was synthesized from compound **12** that was closed with NaSH (Castro, A. C., et al. WO2013012918, 2013-01-24). Hydroxy group of the obtained ester **13** was activated by Tf₂O (Castro, A., et al. US2013267521, 2013-10-10) and subjected to the Suzuki-Miyaura cross-coupling with a phenyl boronic acid to afford **15**. Final reduction of the ester group using LAH gave the desired product **16**. Thiophene analogue **20** with the inversed phenyl and methyl groups was synthesized from the ester **17** (Padwa, A. K., Heterocycles 1993/35, 367-383). After activation of the hydroxy group with Tf₂O, a methyl group was installed by the Pd-catalyzed reaction with AlMe₃ affording **19**. Subsequent reduction of the ester function with LAH led to the desired thiophene **20**. Iodination of compound **21** (Theoclitou, M.-E., et al., J Med Chem 2011/54, 6734-6750) followed by Suzuki-Miyaura cross coupling and reduction with LAH afforded the suitably substituted isothiazole derivative **24**. Lithium salt of 1-methyl-4-phenyl-triazole **25** was formylated with DMF providing aldehyde **26**, which was reduced by NaBH₄ to corresponding alcohol **27** (Hernandez, M.-C., et al. US2012115844, 2012-05-10). Pyrimidine and pyridine analogues **31** and **35** were prepared according to the same methodology from appropriate dihalogenated aldehydes **28** (Gaul, M. D., et al. US2006281764, 2003-08-06) and **32** (Stewart, A. O., et al., J Med Chem 2001/44, 988-1002), respectively. First, the phenyl substituent was installed using the Suzuki-Miyaura cross-coupling with phenylboronic acid affording **29** and **33**, respectively. Then, the second chlorine was exchanged for a methyl with AlMe₃ affording **30** and **34**, respectively. Resulting aldehydes were reduced with NaBH₄ to furnish key heterocycles **31** and **35**, respectively. Alcohols based on benzene **37**, isothiazole **39**, pyrrazole **41**, imidazole **43** and pyridine **45** core were prepared by reduction of their corresponding esters **36** (Huang, H., et al. CN109305934, 2019-02-05), **38** (Zhou, X., et al. WO2009089547, 2009-07-16), **40** (Qian, Y, et al., J Med Chem 2012/55, 7920-7939), 42 (Moldovan, R.-P., et al., ACS Med Chem Lett 2017/8, 566-571) and **44** (Palacios, F., et al., J Org Chem 2006/71, 6020-6030) with LAH (**Scheme 2**).

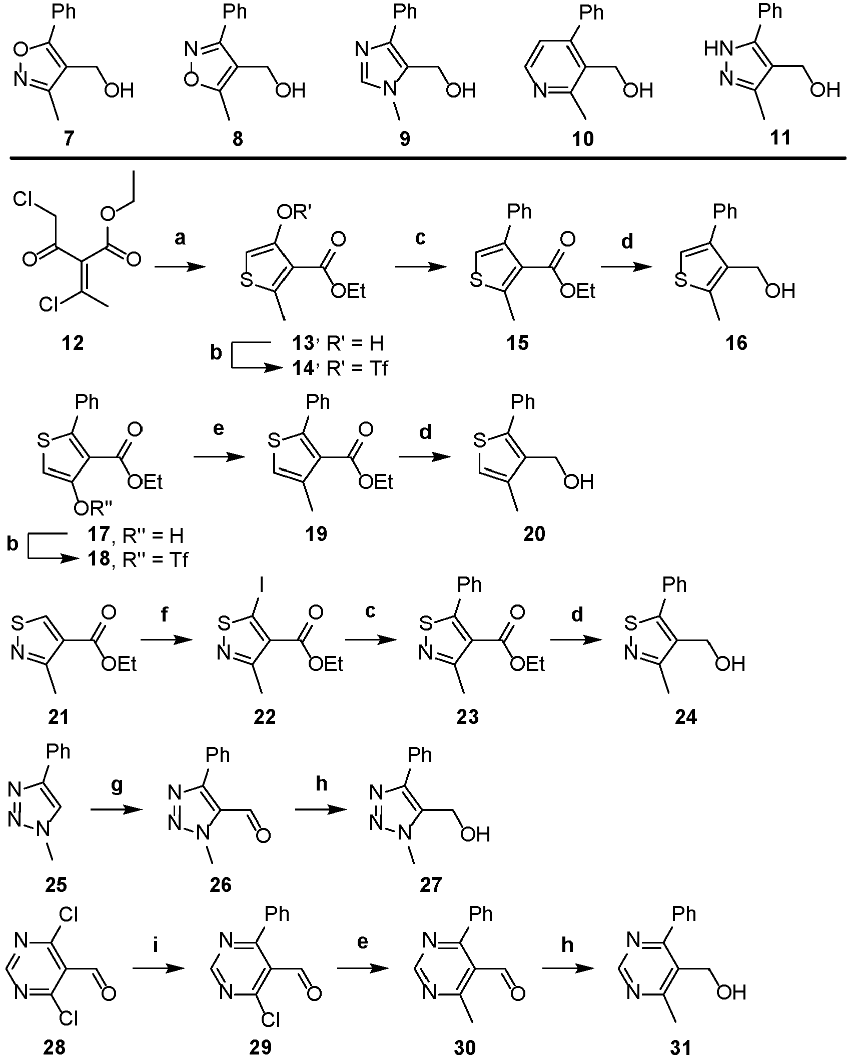

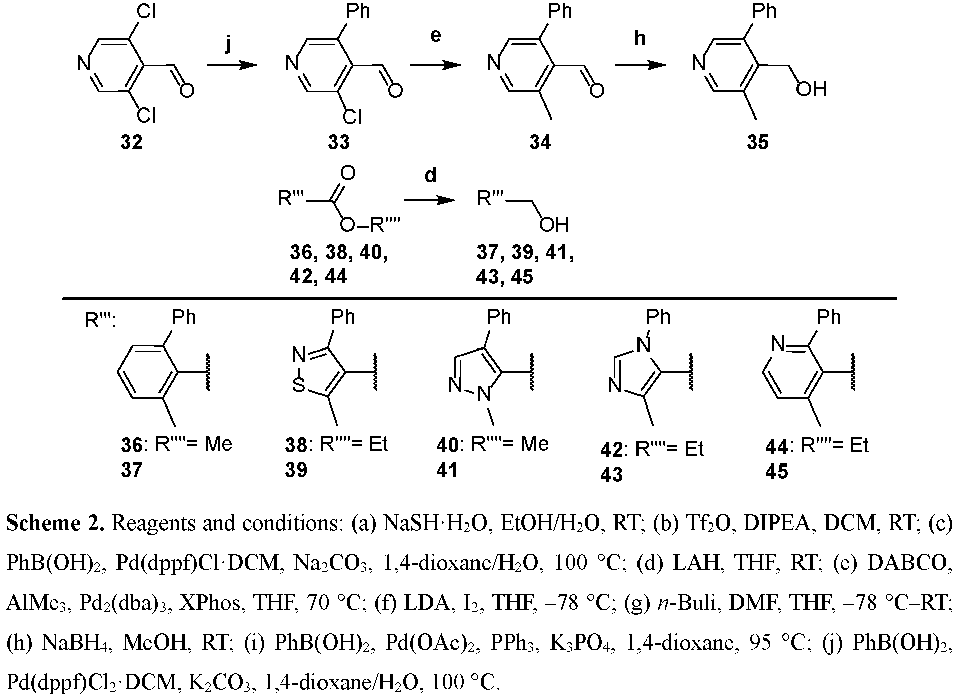

### Example 6

### Ethyl 4-hydroxy-2-methylthiophene-3-carboxylate (13):

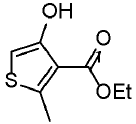

To a suspension of compound **12** (3 g, 14.59 mmol) in EtOH (25 mL), 30% NaSH solution in H₂O (3.89 g, 52.52 mmol) was added dropwise over 30 min and the resulting mixture was stirred at RT for 3 h. The mixture was poured into H₂O and stirred for another 30 min. The precipitate was collected by filtration and rinsed with H₂O. The collected solid was re-dissolved in ethyl acetate, dried over Na₂SO₄ and filtered. The filtrate was concentrated in vacuo to afford the product **13** (2 g, 10.74 mmol, 74 %). **¹H NMR** (400 MHz, DMSO-*d₆*) δ 9.28 (1H, s), 6.21 (1H, s), 4.28 (2H, q, *J=* 7.1 Hz), 2.55 (3H, s), 1.30 (3H, t, *J=* 7.1 Hz). **¹³C NMR** was consistent with published data (Banks, M. R., et al., J Chem Soc, Perkin Trans 1 1986, 2223-2232). **LRMS** Calculated for C₈H₁₁O₃S, [M+H]⁺ m/z: 187.0, [M+H]⁺, found 187.1.

### Example 7

### Ethyl 2-methyl-4-(((trifluoromethyl)sulfonyl)oxy)thiophene-3-carboxylate (14):

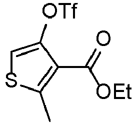

A mixture of ester **13** (1.9 g, 10.20 mmol) and DIPEA (4.1 mL, 23.47 mmol) in DCM (50 mL) was cooled to 15 °C. Tf₂O (3.3 mL, 19.39 mmol) was added and the resulting mixture was warmed up to RT and stirred for 3 h. The mixture was then quenched with H₂O, the aqueous layer was extracted with DCM and the combined organic layers were washed with brine, dried with Na₂SO₄ and concentrated to dryness. FCC (0-10 % EtOAc in cyclohexane) afforded ethyl ester 14 (2.6 g, 8.17 mmol, 80 %). **¹H NMR** (400 MHz, CDCl₃) δ 6.92 (1H, d, *J* = 0.6 Hz), 4.39 (2H, q, *J* = 7.1 Hz), 2.72 (3H, s), 1.39 (3H, t, *J* = 7.2 Hz). **¹⁹F NMR** (376 MHz, CDCl₃) δ -73.2. **LRMS** Calculated for C₉H₁₀F₃O₅S₂, [M+H]⁺ m/z: 319.0, [M+H]⁺, found 318.9.

### Example 8

### Ethyl 2-methyl-4-phenylthiophene-3-carboxylate (15):

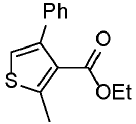

Triflate **14** (0.6 g, 1.89 mmol), PhB(OH)₂ (0.23 g, 1.89 mmol) and Na₃CO₃ (0.5 g, 4.71 mmol) were dissolved in the mixture of 1,4-dioxane and H₂O (10:1, 1.5 mL). Pd(dppf)Cl₂·DCM (0.08 g, 0.09 mmol) was added and the reaction mixture was stirred at 100 °C for 12 h under argon atmosphere. The crude mixture was adsorbed on silica and purified on FCC (0-10 % EtOAc in cyclohexane) to provide compound **15** (0.4 g, 1.62 mmol, 86 %). **¹H NMR** (400 MHz, CDCl₃) δ 7.38-7.28 (5H, m), 6.91 (1H, s), 4.12 (2H, q, *J* = 7.2 Hz), 2.68 (3H, s), 1.03 (3H, t, *J* = 7.1 Hz). **¹³C NMR** (101 MHz, CDCl₃) δ 164.9, 147.5, 143.8, 137.5, 128.9, 128.6, 128.0, 127.2, 120.0, 60.5, 15.7, 13.9. **LRMS** Calculated for C₁₄H₁₅O₂S, [M+H]⁺ m/z: 247.1, [M+H]⁺, found 247.2.

### Example 9

### (2-Methyl-4-phenylthiophen-3-yl)methanol (16):

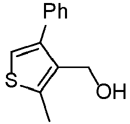

To an ice-cooled solution of ester **15** (0.5 g, 2.03 mmol) in THF (10 mL) was added 1M solution of LAH in THF (3 mL, 3.00 mmol) and the reaction mixture was stirred for 3 h. The reaction was quenched by addition of EtOAc, EtOH and H₂O and the product was extracted with EtOAc (2×100 mL). Combined organic phases were washed with brine, dried over Na₂SO₄ and the volatiles were evaporated under reduced pressure. FCC (10-40 % EtOAc in cyclohexane) furnished derivative **16** (0.38 g, 1.86 mmol, 92 %). **¹H NMR** (400 MHz, CDCl₃) δ 7.57-7.54 (2H, m), 7.44-7.39 (2H, m), 7.36-7.31 (1H, m), 7.23 (1H, s), 4.94 (1H, t, *J=* 5.0 Hz), 4.31 (3H, d, *J=* 4.9 Hz), 2.49 (3H, s). **¹³C NMR** (101 MHz, CDCl₃) δ 143.5, 138.3, 137.0, 136.2, 128.6, 128.5, 127.2, 119.1, 54.7, 13.5. **LRMS** Calculated for C₁₂H₁₃OS, [M+H]⁺ m/z: 205.1, [M+H]⁺, found 205.3.

### Example 10

### Ethyl 2-phenyl-4-(((trifluoromethyl)sulfonyl)oxy)thiophene-3-carboxylate (18):

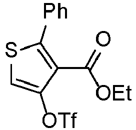

Triflate **18** was prepared from compound **17** (0.64 g, 2.58 mmol) in analogous fashion to triflate **14**. FCC (0-15 % EtOAc in cyclohexane) gave compound **18** (0.75 g, 1.97 mmol, 77 %). **¹H NMR** (400 MHz, CDCl₃) δ 9.38 (1H, s), 7.49-7.39 (5H, m), 7.19 (1H, q, *J* = 0.4 Hz), 4.25 (2H, q, *J* = 7.2 Hz), 1.19 (3H, t, *J* = 7.1 Hz). **¹⁹F NMR** (376 MHz, CDCl₃) δ -73.1. **LRMS** Calculated for C₁₄H₁₂F₃O₅S₂, [M+H]⁺ m/z: 381.0, [M+H]⁺, found 381.3.

### Example 11

### Ethyl 4-methyl-2-phenylthiophene-3-carboxylate (19):

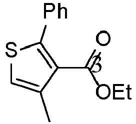

To a solution of DABCO (0.1 mL, 0.97 mmol) in THF (2 mL), AlMe₃ (0.25 g, 3.50 mmol) was added dropwise and the mixture was stirred at RT for 30 min. A solution of triflate **18** (0.74 g, 1.95 mmol), Pd₂(dba)₃ (0.1 g, 0.10 mmol) and XPhos (0.09 g, 0.20 mmol) in THF (20 mL) was added and the reaction mixture was stirred at 70 °C under argon atmosphere for 1 h. The mixture was then cooled to 0 °C and quenched with sat. NH₄Cl, diluted with EtOAc and filtered over celite. Solids were washed with EtOAc and solvents were evaporated in vacuo. FCC (0-10 % EtOAc in cyclohexane) gave **19** (0.45 g, 1.83 mmol, 94 %). **¹H NMR** (400 MHz, CDCl₃) δ 7.43-7.34 (5H, m), 6.90 (1H, q, *J* = 1.0 Hz), 4.15 (2H, q, *J* = 7.2 Hz), 2.40 (3H, s), 1.07 (3H, t, *J =* 7.1 Hz). **¹³C NMR** (101 MHz, CDCl₃) δ 165.2, 149.0, 139.1, 134.6, 129.3, 128.3, 128.2, 121.1, 117.3, 60.7, 16.5, 13.9. **LRMS** Calculated for C₁₄H₁₅O₂S, [M+H]⁺ m/z: 247.1, [M+H]⁺, found 247.2.

### Example 12

### (4-Methyl-2-phenylthiophen-3-yl)methanol (20):

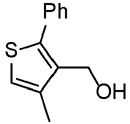

Substituted methanol **20** was synthesized from ester **19** (0.45 g, 1.83 mmol) according to the same methodology that was used for **16**. FCC (10-20 % EtOAc in cyclohexane) afforded derivative **20** (0.35 g, 1.69 mmol, 92 %). **¹H NMR** (400 MHz, CDCl₃) δ 7.54-7.49 (2H, m), 7.45-7.40 (2H, m), 7.39-7.34 (1H, m), 6.94 (1H, q, *J* = 1.1 Hz), 4.60 (2H, s), 2.36 (3H, d, *J* = 1.1 Hz). **¹³C NMR** (101 MHz, CDCl₃) δ 143.4, 138.6, 135.7, 134.4, 129.5, 128.8, 128.0, 120.6, 57.3, 15.0. **LRMS** Calculated for C₁₂H₁₃OS, [M+H]⁺ m/z: 205.1, [M+H]⁺, found 205.2.

### Example 13

### Ethyl 5-iodo-3-methylisothiazole-4-carboxylate (22):

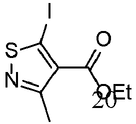

Solution of DIPA (2.5 mL, 13.67 mmol) in THF (30 mL) was cooled down to -78 °C. n-BuLi (8.3 mL, 13.21 mmol) was added dropwise under argon atmosphere and the LDA was formed over 40 min at -78 °C. Ester **21** (1.56 g, 9.11 mmol) in THF (1 mL) was added dropwise and the reaction mixture was stirred 40 min at -78 °C. A solution of I₂ (3.47 g, 13.67 mmol) in THF (1 mL) was added dropwise and the reaction mixture was stirred another 40 min at same temperature. Reaction was quenched by addition of H₂O, the crude mixture was extracted to EtOAc (2×200 mL), combined organic layers were washed with brine and dried over Na₂SO₄. Volatiles were evaporated and the product was purified on FCC (0-20 % EtOAc in cyclohexane) to provide **22** (2.10 g, 7.07 mmol, 78 %). **¹H NMR** (400 MHz, CDCl₃) δ 4.41 (2H, q, *J* = 7.1 Hz), 2.72 (3H, s), 1.43 (3H, t, *J* = 7.2 Hz). **¹³C NMR** (101 MHz, CDCl₃) δ 168.7, 161.9, 132.6, 107.6, 61.6, 20.6, 14.3. **LRMS** Calculated for C₇H₉INO₂S, [M+H]⁺ m/z: 297.9, [M+H]⁺, found 298.0.

### Example 14

### Ethyl 3-methyl-5-phenylisothiazole-4-carboxylate (23):

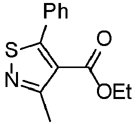

Ester **23** was prepared from compound **22** (0.5 g, 1.68 mmol) following the same coupling conditions as for **15**. FCC (0-10 % EtOAc in cyclohexane) furnished analogue **23** (0.37 g, 1,48 mmol, 88 %). NMR spectra were consistent with published data (Seo, B., et al., Org Lett 2016/18, 5050-5053).

### Example 15

### (3-Methyl-5-phenylisothiazol-4-yl)methanol (24):

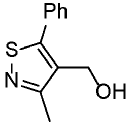

**24** was prepared from **23** (0.35 g, 1.42 mmol) using the same procedure as for **16**. FCC (10-50 % EtOAc in cyclohexane) gave **24** (0.27 g, 1.30 mmol, 92 %). **¹H NMR** (400 MHz, CDCl₃) δ 7.53-7.43 (5H, m), 4.64 (2H, d, *J* = 3.4 Hz), 2.59 (3H, s), 1.78 (1H, t, *J* = 3.6 Hz). **¹³C NMR** (101 MHz, CDCl₃) δ 168.0, 165.8, 131.9, 131.0, 129.5, 129.2, 129.0, 56.3, 18.3. **LRMS** Calculated for C₁₁H₁₂NOS, [M+H]⁺ m/z: 206.1, [M+H]⁺, found 206.3.

### Example 16

### 1-Methyl-4-phenyl-1H-1,2,3-triazole-5-carbaldehyde (26):

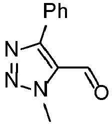

To a solution of aldehyde **25** (0.22 g, 1.38 mmol) in THF (7 mL) was added n-BuLi (1 mL, 1.66 mmol) dropwise at -75° C. under argon atmosphere. The resulting solution was stirred at -75 ° C for 15 min, then DMF (0.14 mL, 1.80 mmol) was added dropwise and the reaction mixture was allowed to warm up to RT over 1 h. The mixture was then poured into NH₄Cl solution and extracted with EtOAc (2×100 mL), organic extracts were washed with brine, dried over Na₂SO₄, filtered and evaporated. FCC (10-50 % EtOAc in cyclohexane) provided the **26** (0.25 g, 1.34 mmol, 97 %). **¹H NMR** (400 MHz, CDCl₃) δ 10.07 (1H, s), 7.76-7.71 (2H, m), 7.56-7.49 (3H, m), 4.38 (3H, s). **¹³C NMR** (101 MHz, CDCl₃) δ 180.4, 153.9, 130.0, 129.3, 129.1, 129.0, 38.4. **LRMS** Calculated for C₁₀H₁₀N₃O, [M+H]⁺ m/z: 188.1, [M+H]⁺, found 188.2.

### Example 17

### (1-Methyl-4-phenyl-1H-1,2,3-triazol-5-yl)methanol (27):

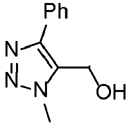

To a solution of **26** (0.25 g, 1.34 mmol) in MeOH (7 mL) was added NaBH₄ (0.1 g, 2.67 mmol) under argon atmosphere at 0 °C and the reaction mixture was allowed to warm up to RT over 1 h. The mixture was then diluted with H₂O (25 mL) and extracted with EtOAc (2×100 mL), combined organic extracts were washed with brine, dried over Na₂SO₄, filtered and evaporated. FCC (10-50 % EtOAc in cyclohexane) afforded 27 (0.23 g, 1.22 mmol, 91 %). **¹H NMR** (400 MHz, CDCl₃) δ 7.75-7.72 (2H, m), 7.51-7.46 (2H, m), 7.41-7.36 (1H, m), 5.57 (1H, t, *J* = 5.5 Hz), 4.66 (2H, d, *J* = 5.5 Hz), 4.08 (3H, s). **¹³C NMR** (101 MHz, CDCl₃) δ 144.5, 133.4, 131.4, 129.0, 128.0, 127.2, 51.3, 35.0. **LRMS** Calculated for C₁₀H₁₂N₃O, [M+H]⁺ m/z: 190.1, [M+H]⁺, found 190.2.

### Example 18

### 4-Chloro-6-phenylpyrimidine-5-carbaldehyde (29):

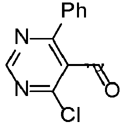

To a solution of **28** (1.3 g, 7.35 mmol) in dry 1,4-dioxane (150 mL) was added PhB(OH)₂ (0.9 g, 7.35 mmol), K₃PO₄ (6.24 g, 29.38 mmol) and Ph₃P (0.19 g, 0.74 mmol). Reaction flask was backfilled with argon and Pd(OAc)₂ (0.08 g, 0.37 mmol) was added. After 5 days of stirring the residue was adsorbed on silica and product was isolated by FCC (0-30 % EtOAc in cyclohexane) providing **29** (0.5 g, 2.29 mmol, 31 %). **¹H NMR** (400 MHz, CDCl₃) δ 10.11 (1H, s), 9.12 (1H, s), 7.64-7.51 (5H, m). **¹³C NMR** (101 MHz, CDCl₃) δ 188.5, 169.5, 159.4, 134.9, 131.7, 130.4, 129.1, 125.9. **LRMS** Calculated for C₁₁H₈ClN₂O, [M+H]⁺ m/z: 219.0, [M+H]⁺, found 219.1.

### Example 19

### 4-Methyl-6-phenylpyrimidine-5-carbaldehyde (30):

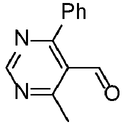

Aldehyde **30** was synthesized from analogue **29** (0.46 g, 2.10 mmol) in analogous fashion to **19**. FCC (0-50 % EtOAc in cyclohexane) afforded **30** (0.24 g, 1.21 mmol, 58 %). **¹H NMR** (400 MHz, CDCl₃) δ 10.06 (1H, s), 9.21 (1H, s), 7.63-7.51 (5H, m), 2.85 (3H, s). **¹³C NMR** (101 MHz, CDCl₃) δ 192.1, 169.1, 168.7, 159.3, 135.7, 131.1, 130.6, 129.0, 125.9, 24.1. **LRMS** Calculated for C₁₂H₁₁N₂O, [M+H]⁺ m/z: 199.1, [M+H]⁺, found 199.2.

### Example 20

### (4-Methyl-6-phenylpyrimidin-5-yl)methanol (31):

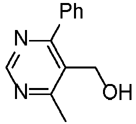

**31** was prepared from **30** (0.28 g, 1.41 mmol) in analogous fashion to **27**. FCC (10-50 % acetone in cyclohexane) furnished **31** (0.1 g, 0.50 mmol, 35 %). **¹H NMR** (400 MHz, CDCl₃) δ 9.03 (1H, s), 7.68-7.63 (2H, m), 7.50-7.45 (3H, m), 4.70 (2H, s), 2.72 (3H, s), 2.32 (1H, bs). **¹³C NMR** (101 MHz, CDCl₃) δ 168.7, 166.1, 157.2, 137.7, 129.7, 129.2, 128.6, 128.4, 58.9, 22.2. **LRMS** Calculated for C₁₂H₁₃N₂O, [M+H]⁺ m/z: 201.1, [M+H]⁺, found 201.2.

### Example 21

### 3 -Chloro-5 -phenylisonicotinaldehyde (33):

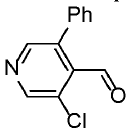

A mixture of **32** (1.5 g, 8.52 mmol), PhB(OH)₂ (0.83 g, 6.82 mmol) and K₂CO₃ (2.95 g, 21.31 mmol) in 1,4-dioxane/H₂O (10:1, 3.5 mL) was degassed, after which Pd(dppf)Cl₂·DCM (0.35 g, 0.43 mmol) was added and the reaction mixture was stirred at 100 °C for 12 h. The crude mixture was diluted with EtOAc and washed with H₂O and brine. FCC (0-30 % EtOAc in cyclohexane) afforded derivative **33** (1.32 g, 6.07 mmol, 71 %). **¹H NMR** (400 MHz, CDCl₃) δ 10.05 (1H, s), 8.74 (1H, s), 8.63 (1H,s), 7.52-7.47 (3H, m), 7.36-7.32 (2H, m). **¹³C NMR** (101 MHz, CDCl₃) δ 190.2, 150.2, 149.9, 138.7, 137.5, 133.8, 130.0, 129.7, 129.4, 129.1. **LRMS** Calculated for C₁₂H₉ClNO, [M+H]⁺ m/z: 218.0, [M+H]⁺, found 218.1.

### Example 22

### 3-Methyl-5-phenylisonicotinaldehyde (34):

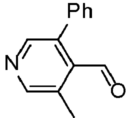

Aldehyde **34** was prepared from derivative **33** (0.6 g, 2.76 mmol) in analogous fashion to **19**. FCC (10-60 % EtOAc in cyclohexane) gave analogue **34** (0.44 g, 2.23 mmol, 81 %). **¹H NMR** (400 MHz, CDCl₃) δ 10.01 (1H, d, *J* = 0.6 Hz), 8.62 (1H, d, *J* = 0.7 Hz), 8.60 (1H, t, *J =* 0.7 Hz), 7.52-7.47 (3H, m), 7.39-7.34 (2H, m), 2.59 (3H, d, *J =* 0.7 Hz). **¹³C NMR** (101 MHz, CDCl₃) δ 194.0, 151.8, 149.6, 138.8, 137.9, 134.8, 131.9, 130.3, 129.0, 128.8. **LRMS** Calculated for C₁₃H₁₂NO, [M+H]⁺ m/z: 198.1, [M+H]⁺, found 198.2.

### Example 23

### (3-Methyl-5-phenylpyridin-4-yl)methanol (35):

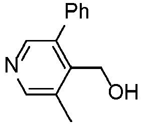

Substituted methanol **35** was synthesized from aldehyde **34** (0.32 g, 1.62 mmol) following the same procedure as for compound **27**. FCC (50-100 % EtOAc in cyclohexane) provided derivative **35** (0.12 g, 0.58 mmol, 36 %).**¹H NMR** (400 MHz, CDCl₃) δ 8.40 (1H, s), 8.35 (1H, s), 7.48-7.40 (3H, m), 7.39-7.35 (2H, m), 4.62 (2H, s), 2.50 (3H, s). **¹³C NMR** (101 MHz, CDCl₃) δ 1502, 148.6, 143.7, 137.6, 137.5, 133.0, 129.6, 128.6, 128.0, 59.2, 16.3. **LRMS** Calculated for C₁₃H₁₄NO, [M+H]⁺ m/z: 200.1, [M+H]⁺, found 200.2.

### Example 24

### (3-Methyl-[1,1'-biphenyl]-2-yl)methanol (37):

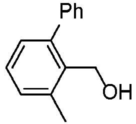

Derivative **37** was prepared from methyl ester **36** (0.75 g, 3.32 mmol) using the same methodology as for compound **16**. FCC (0-30 % EtOAc in cyclohexane) gave substituted methanol **38** (0.54 g, 2.74 mmol, 83 %).**¹H NMR** (400 MHz, CDCl₃) δ 7.46-7.36 (5H, m), 7.30-7.22 (2H, m), 7.15 (1H, dd, *J =* 7.0, 2.0 Hz), 4.61 (2H, s), 2.55 (3H, s). **¹³C NMR** (101 MHz, CDCl₃) δ 142.2, 141.3, 138.4, 136.4, 129.4, 129.3, 128.0, 127.5, 127.3, 126.9, 58.2, 19.2. **LRMS** Calculated for C₁₄H₁₅O, [M+H]⁺ m/z: 199.1, [M+H]⁺, found 199.1.

### Example 25

### (5-Methyl-3-phenylisothiazol-4-yl)methanol (39):

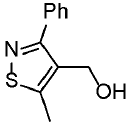

Substituted methanol **39** was prepared from ester **38** (0.25 g, 1.01 mmol) according to the same procedure as for compound **16**. FCC (25-50 % EtOAc in cyclohexane) furnished derivative **39** (0.11 g, 0.53 mmol, 52 %).**¹H NMR** (400 MHz, CDCl₃) δ 7.76-7.72 (2H, m), 7.49-7.42 (3H, m), 4.66 (2H, d, *J* = 4.5 Hz), 2.63 (3H, s), 1.71 (1H, t, *J* = 4.9 Hz). **¹³C NMR** (101 MHz, CDCl₃) δ 169.0, 163.5, 135.9, 132.7, 129.1, 128.7, 128.6, 56.1, 12.0. **LRMS** Calculated for C₁₁H₁₂NOS, [M+H]⁺ m/z: 206.1, [M+H]⁺, found 206.2.

### Example 26

### (1-Methyl-4-phenyl-1H-pyrazol-5-yl)methanol (41):

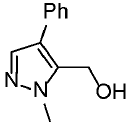

**41** was prepared from **40** (0.13 g, 0.60 mmol) in analogous fashion to **16**. FCC (10-50 % acetone in cyclohexane) provided **41** (0.1 g, 0.53 mmol, 88 %).**¹H NMR** (400 MHz, CDCl₃) δ 7.52 (1H, s), 7.42-7.34 (4H, m), 7.32-7.25 (1H, m), 4.74 (2H, d, *J* = 3.9 Hz), 3.94 (3H, s), 2.55 (1H, t, *J* = 4.0 Hz). **¹³C NMR** (101 MHz, CDCl₃) δ 137.3, 137.2, 132.9, 128.8, 128.1, 126.8, 122.5, 53.6, 36.9. **LRMS** Calculated for C₁₁H₁₃N₂O, [M+H]⁺ m/z: 189.1, [M+H]⁺, found 189.2.

### Example 27

### (4-Methyl-1-phenyl-1H-imidazol-5-yl)methanol (43):

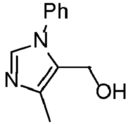

Derivative **43** was synthesized from ester **42** (0.4 g, 1.74 mmol) using the same methodology as for compound **16**. FCC (10-80 % acetone in cyclohexane) furnished substituted methanol **43** (0.27 g, 1.43 mmol, 83 %).**¹H NMR** (400 MHz, CDCl₃) δ 7.75 (1H, s), 7.58-7.50 (4H, m), 7.60-7.41 (1H, m), 5.06 (1H, t, *J* = 5.0 Hz), 4.32 (2H, d, *J* = 4.8 Hz), 2.19 (3H, s). **¹³C NMR** (101 MHz, CDCl₃) δ 136.79, 136.75, 134.3, 129.7, 127.8, 127.2, 124.9, 51.3, 13.0. **LRMS** Calculated for C₁₁H₁₃N₂O, [M+H]⁺ m/z: 189.1, [M+H]⁺, found 189.2.

### Example 28

### (4-Methyl-2-phenylpyridin-3-yl)methanol (45):

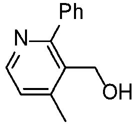

**45** was synthesized from ester **44** (1 g, 4.14 mmol) in analogous fashion to **16**. FCC (10-40 % acetone in cyclohexane) afforded **45** (0.63 g, 3.18 mmol, 77 %).**¹H NMR** (400 MHz, CDCl₃) δ 8.42 (1H, d, *J* = 4.8 Hz), 7.66-7.61 (2H, m), 7.48-7.39 (3H, m), 7.22 (1H, dd, *J* = 4.8, 0.8 H), 5.13 (1H, t, *J* = 4.5 Hz), 4.43 (2H, d, *J* = 4.5 Hz), 2.48 (3H, *d, J =* 0.7 Hz). **¹³C NMR** (101 MHz, CDCl₃) δ 158.6, 148.4, 148.1, 140.5, 132.5, 129.5, 128.01, 127.95, 124.6, 58.0, 18.8. **LRMS** Calculated for C₁₃H₁₄NO, [M+H]⁺ m/z: 200.1, [M+H]⁺, found 200.2.

Both synthesized and commercially available (het)arylmethanols were subsequently attached to the nucleosidic unit. Hydroxygroups of alcohols **7-11**, **16**, **20**, **24**, **27**, **31**, **35**, **37**, **39**, **41**, **43** and **45** were first activated by halogenation using PBr₃ (Huang, H., et al., ChemMedChem 2015/10, 1184-1199) or SOCl₂ and then condensed with the freshly prepared thiol **46.** Furnished protected modified nucleosides **47b-62b** were deprotected treating with HF-TEA to give the free SAH analogues **47c-62c (Scheme 3).**

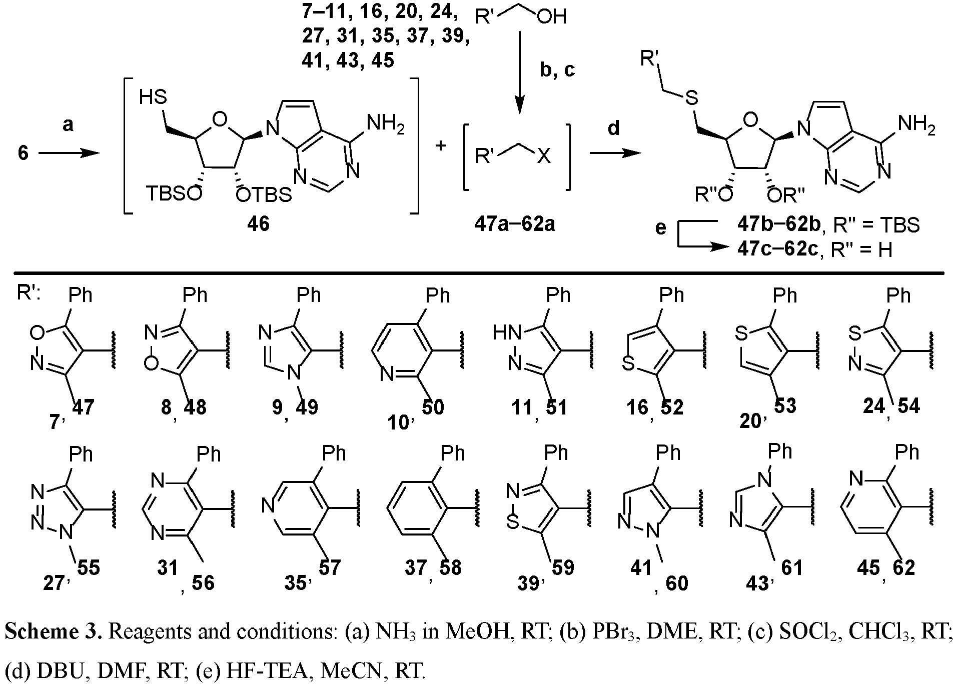

### Example 29

### ((2S,3R,4R,5R)-5-(4-Amino-7H-pyrrolo[2,3-d]pyrimidin-7-yl)-3,4-bis((tert-butyldimethylsilyl)oxy) tetrahydrofuran-2-yl)methanethiol (46):

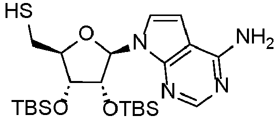

Thioacetate **6** (0.2 g, 0.36 mmol) was treated with 10M methanolic ammonia (5 mL) at RT for 2 h. Volatiles were evaporated and reaction mixture was co-evaporated with DMF and used as is. **LRMS** Calculated for C₂₃H₄₃N₄O₃SSi₂, [M+H]⁺ m/z: 511.3, [M+H]⁺, found 511.2.

### Example 30

### 7-((2R,3R,4R,5S)-3,4-bis((tert-Butyldimethylsilyl)oxy)-5-((((3-methyl-5-phenyhsoxazol-4-yl)methyl)thio)methyl)tetrahydrofuran-2-yl)-7H-pyrrolo[2,3-d]pyrimidin-4-amine (47b):

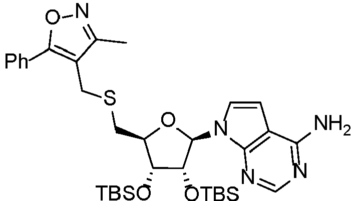

To an ice-cooled solution of **7** (0.08 g, 0.43 mmol) in dry DME (3 mL), PBr₃ (0.05 mL, 0.53 mmol) was added dropwise. After stirring for 1 h at RT, the mixture was diluted with EtOAc (50 mL), washed with saturated solution of NaHCO₃ and brine, dried over Na₂SO₄, filtered, concentrated and azeotroped with DMF. A solution of the obtained bromide **47a** (0.43 mmol) in dry DMF (1 mL) was added to a solution of the freshly prepared thiol **46** (0.36 mmol) in dry DMF (1 mL) at 0 °C. A solution of DBU (0.11 mL, 0.74 mmol) in dry DMF (1 mL) was added dropwise. After 1 h of stirring, volatiles were evaporated under reduced pressure, residue was adsorbed on silica and purified on FCC (10-80 % EtOAc in DCM) to afford SAH analogue **47b** (0.19 g, 0.27 mmol, 76 %).**¹H NMR** (400 MHz, CDCl₃) δ 8.26 (1H ,s), 7.76-7.70 (2H, m), 7.47-7.37 (3H, m), 7.07 (1H, d, *J =* 3.7 Hz), 6.35 (1H, d, *J* = 3.7 Hz), 6.01 (1H, d, *J* = 4.8 Hz), 5.41 (2H, bs), 4.83 (1H, t, *J* = 4.7 Hz), 4.26 (1H, t, *J* = 4.3 Hz), 4.24-4.19 (1H, m), 3.80 (1H, d, *J* = 13.1 Hz), 3.68 (1H, d, *J* = 13.1 Hz), 3.01 (1H, dd, *J* = 13.9, 5.3 Hz), 2.93 (1H, dd, *J* = 13.9, 6.1 Hz), 2.29 (3H, s), 0.92, 0.80 (18H, 2×s), 0.11, -0.09, -0.25 (12H, 3×s). **¹³C NMR** (101 MHz, CDCl₃) δ 166.1, 160.8, 156.9, 151.8, 150.7, 130.0, 129.0, 127.9, 127.2, 123.9, 110.5, 104.3, 98.7, 90.2, 83.9, 74.7, 74.6, 35.0, 26.0, 25.9, 25.9, 18.2, 18.0, 10.3, -4.2, -4.5, -4.6, -4.9. **LRMS** Calculated for C₃₄H₅₂N₅O₄SSi₂, [M+H]⁺ m/z: 682.3, [M+H]⁺, found 682.3.

### Example 31

### 7-((2R,3R,4R,5S)-3,4-bis((tert-Butyldimethylsilyl)oxy)-5-((((5-methyl-3-phenylisoxazol-4-yl)methyl)thio)methyl)tetrahydrofuran-2-yl)-7H-pyrrolo[2,3-d]pyrimidin-4-amine (48b):

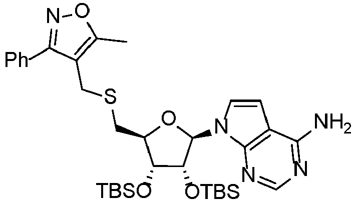

Derivative **48b** was prepared from bromide **48a** (obtained from **8** (0.08 g, 0.43 mmol)) and thiol **46** (provided from **6** (0.2 g, 0.36 mmol)) according to the same procedure as for **47b.** FCC (10-80 % EtOAc in DCM) furnished product **48b** (0.19 g, 0.28 mmol, 78 %). **¹H NMR** (400 MHz, DMSO-*d₆*) δ 8.04 (1H ,s), 7.73-7.68 (2H, m), 7.51-7.46 (3H, m), 7.30 (1H, d, *J =* 3.7 Hz), 7.00 (2H, bs), 6.60 (1H, d, *J* = 3.6 Hz), 6.06 (1H, d, *J* = 6.8 Hz), 4.76 (1H, dd, *J* = 6.8, 4.5 Hz), 4.15 (1H, dd, *J* = 4.4, 1.7 Hz), 3.95 (1H, td, *J =* 6.8, 1.6 Hz), 3.80 (1H, d, *J* = 13.8 Hz), 3.71 (1H, d, *J* = 13.8 Hz), 2.98 (1H, dd, *J* = 13.8, 7.1 Hz), 2.82 (1H, dd, *J* = 13.8, 6.5 Hz), 2.41 (3H, s), 0.90, 0.68 (18H, 2×s), 0.09, -0.14, -0.41 (12H, 3×s). **¹³C NMR** (101 MHz, DMSO-*d₆*) δ 168.1, 161.6, 157.6, 151.9, 150.7, 129.9, 129.1, 129.0, 128.0, 122.2, 110.3, 103.1, 100.3, 86.9, 84.2, 74.7, 74.1, 34.3, 25.9, 25.7, 24.1, 17.9, 17.7, 11.0, -4.4, -4.5, -4.6, -5.3. **LRMS** Calculated for C₃₄H₅₂N₅O₄SSi₂, [M+H]⁺ m/z: 682.3, [M+H]⁺, found 682.3.

### Example 32

### 7-((2R,3R,4R,5S)-3,4-bis((tert-Butyldlmethylsllyl)oxy)-5-((((1-methyl-4-phenyl-1H-imidazol-5-yl)methyl)thio)methyl)tetrahydrofuran-2-yl)-7H-pyrrolo[2,3-d]pyrimidin-4-amine (49b):

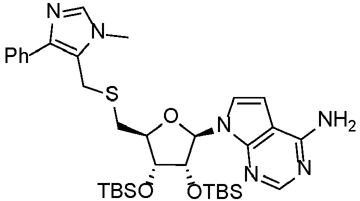

Alcohol **9** (0.08 g, 0.40 mmol) was refluxed in SOCl₂ (0.13 mL, 1.79 mmol) in chloroform (3 mL) for 2 h. Volatiles were evaporated under reduced pressure and obtained residue was co-evaporated with toluene. A solution of the obtained chloride **49a** (0.40 mmol) in dry DMF (1 mL) was added to a solution of the freshly prepared thiol **46** (0.36 mmol) in dry DMF (1 mL) at 0 °C. A solution of DBU (0.11 mL, 0.7 mmol) in dry DMF (1 mL) was added dropwise. After 1 h of stirring, volatiles were evaporated in vacuo and residue was subjected to FCC (2-30 % EtOH in DCM) to afford SAH analogue **49b** (0.11 g, 0.15 mmol, 43 %). **¹H NMR** (400 MHz, CDCl₃) δ 8.05 (1H, s), 7.67-7.63 (3H, m), 7.38-7.32 (2H, m), 7.30 (1H, d, *J =* 3.7 Hz), 7.26-7.20 (1H, m), 6.99 (2H, bs), 6.59 (1H, d, *J* = 3.7 Hz), 6.08 (1H, d, *J* = 7.0 Hz), 4.79 (1H, dd, *J* = 7.1, 4.5 Hz), 4.18 (1H, dd, *J* = 4.6, 1.6 Hz), 4.11 (1H, d, *J* = 13.5 Hz), 4.03 (1H, d, *J* = 13.5 Hz), 3.98 (1H, td, *J* = 6.9, 1.6 Hz), 3.63 (3H, s), 3.06 (1H, dd, *J* = 13.7, 6.9 Hz), 2.96 (1H, dd, *J* = 13.8, 6.9 Hz), 0.92, 0.68 (18H, 2×s), 0.13, 0.11, -0.12, -0.41 (12H, 4×s). **¹³C NMR** (101 MHz, CDCl₃) δ 157.5, 151.8, 150.6, 138.5, 138.3, 134.9, 128.4, 126.4, 126.3, 123.2, 122.0, 103.0, 100.2, 86.6, 84.5, 74.7, 74.0, 34.5, 31.1, 25.8, 25.52, 25.50, 17.8, 17.5, -4.6, -4.6, -4.8, -5.5. **LRMS** Calculated for C₃₄H₅₂N₆O₃SSi₂, [M+H]⁺ m/z: 681.3, [M+H]⁺, found 681.4.

### Example 33

### 7-((2R,3R,4R,5S)-3,4-bis((tert-Butyldimethylsilyl)oxy)-5-((((2-methyl-4-phenylpyridin-3-yl)methyl)thio)methyl)tetrahydrofuran-2-yl)-7H-pyrrolo[2,3-d]pyrimidin-4-amine (50b):

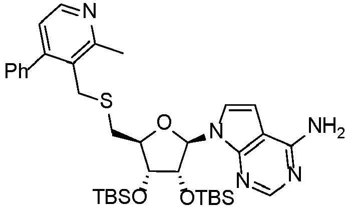

**50b** was prepared from chloride **50a** (obtained from **10** (0.06 g, 0.30 mmol)) and thiol **46** (provided from **6** (0.18 g, 0.33 mmol)) in analogous fashion to **49b.** FCC (10-100 % acetone in cyclohexane) gave **50b** (0.17 g, 0.24 mmol, 73 %). **¹H NMR** (400 MHz, CDCl₃) δ 8.38 (1H, d, *J* = 5.1 Hz), 8.24 (1H, s), 7.45-7.37 (5H, m), 7.03 (1H, d, *J =* 3.7 Hz), 6.99 (1H, d, *J* = 5.0 Hz), 6.37 (1H, d, *J =* 3.7 Hz), 5.97 (1H, d, *J* = 5.1 Hz), 5.33 (2H, bs), 4.76 (1H, t, *J* = 4.9 Hz), 4.18 (1H, dd, *J* = 4.7, 3.7 Hz), 4.08 (1H, td, *J* = 5.8, 3.8 Hz), 3.75 (1H, d, *J* = 12.0 Hz), 3.68 (1H, d, *J* = 12.0 Hz), 2.89 (1H, dd, *J* = 13.8, 5.8 Hz), 2.76 (1H, dd, *J* = 13.8, 5.9 Hz), 2.70 (3H, s), 0.92, 0.78 (18H, 2×s), 0.10, 0.08, -0.10, -0.29 (12H, 4×s). **¹³C NMR** (101 MHz, CDCl₃) δ 158.5, 156.4, 151.2, 150.6, 150.5, 147.2, 139.1, 129.3, 128.8, 128.5, 128.3, 124.0, 122.9, 104.3, 98.8, 89.9, 83.9, 74.7, 74.6, 36.0, 32.9, 26.0, 25.9, 22.9, 18.2, 18.1, -4.2, -4.4, -4.6, -4.9. **LRMS** Calculated for C₃₆H₅₄N₅O₃SSi₂, [M+H]⁺ m/z: 692.4, [M+H]⁺, found 692.4.

### Example 34

### 7-((2R,3R,4R,5S)-3,4-bis((tert-Butyldimethylsilyl)oxy)-5-((((3-methyl-5-phenyl-1H-pyrazol-4-yl) methyl)thio)methyl)tetrahydrofuran-2-yl)-7H-pyrrolo[2,3-d]pyrimidin-4-amine (51b):

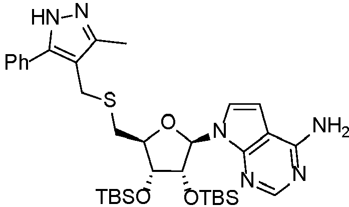

Modified nucleoside **51b** was synthesized from chloride **51a** (obtained from **11** (0.05 g, 0.27 mmol)) and thiol **46** (provided from **6** (0.14 g, 0.25 mmol)) according to the same procedure as for **49b.** FCC (2-30 % EtOH in DCM) furnished product **51b** (0.05 g, 0.07 mmol, 26 %). **¹H NMR** (400 MHz, CDCl₃) δ 8.26 (1H, s), 7.69-7.60 (2H, m), 7.45-7.38 (2H, m), 7.38-7.32 (1H, m), 7.10 (1H, d, *J* = 3.6 Hz), 6.33 (1H, d, *J* = 3.6 Hz), 6.03 (1H, d, *J* = 5.3 Hz), 5.28 (2H, bs), 4.81 (1H, t, *J* = 4.1 Hz), 4.25-4.16 (2H, m), 3.80 (1H, d, *J* = 13.0 Hz), 3.72 (1H, d, *J* = 13.0 Hz), 3.00 (1H, dd, *J* = 13.7, 6.1 Hz), 2.85 (1H, dd, *J* = 13.7, 5.5 Hz), 2.30 (3H, s), 0.93, 0.78 (18H, 2×s), 0.11, -0.10, -0.29 (12H, 3×s). **¹³C NMR(101** MHz, CDCl₃) δ 156.7, 151.7, 150.8, 146.5, 144.3, 131.8, 128.9, 128.3, 127.9, 124.0, 111.7, 104.4, 98.6, 89.8, 84.0, 74.7, 35.0, 29.8, 26.8, 26.1, 25.9, 18.2, 18.1, 11.2, -4.2, - 4.4, -4.6, -4.9. **LRMS** Calculated for C₃₄H₅₃N₆O₃SSi₂, [M+H]⁺ m/z: 681.3.1, [M+H]⁺, found 681.3.

### Example 35

### 7-((2R,3R,4R,5S)-3,4-bis((tert-Butyldimethylsilyl)oxy)-5-((((2-methyl-4-phenylthiophen-3-yl)methyl)thio)methyl)tetrahydrofuran-2-yl)-7H-pyrrolor2,3-d]pynmidin-4-amine (52b):

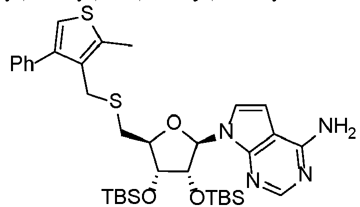

Derivative **52b** was prepared from bromide **52a** (obtained from **16** (0.06 g, 0.29 mmol)) and thiol **46** (provided from 6 (0.2 g, 0.35 mmol)) in analogous fashion to **47b**. FCC (20-60 % acetone in cyclohexane) afforded product **52b** (0.06 g, 0.08 mmol, 28 %). **¹H NMR** (400 MHz, CDCl₃) δ 8.25 (1H, s), 7.49-7.45 (2H, m), 7.42-7.36 (2H, m), 7.36-7.31 (1H, m), 7.10 (1H, d, *J* = 3.7 Hz), 6.94 (1H, s), 6.39 (1H, d, *J* = 3.7 Hz), 6.03 (1H, d, *J* = 5.4 Hz), 5.38 (2H, bs), 4.74 (1H, dd, *J* = 5.5, 4.4 Hz), 4.18 (1H, dd, *J* = 4.6, 3.5 Hz), 4.14 (1H, dd, *J =* 5.8, 3.6 Hz), 3.75 (1H, d, *J =* 12.6 Hz), 3.68 (1H, d, *J =* 12.6 Hz), 2.91 (1H, dd, *J* = 13.7, 6.2 Hz), 2.75 (1H, dd, *J* = 13.7, 5.6 Hz), 2.47 (3H, s), 0.92, 0.78 (18H, 2×s), 0.10, 0.09, -0.10, -0.30 (12H, 4×s). **¹³C NMR** (101 MHz, CDCl₃) δ 156.2, 150.8, 150.6, 143.7, 137.5, 137.4, 131.9, 129.0, 128.5, 127.4, 124.1, 119.5, 104.2, 98.9, 89.6, 84.1, 74.9, 74.7, 35.3, 29.5, 26.1, 25.9, 18.2, 18.1, 14.1, -4.2, -4.4, -4.5, -5.0. **LRMS** Calculated for C₃₅H₅₂N₄O₃S₂Si₂, [M+H]⁺ m/z: 697.3, [M+H]⁺, found 697.2.

### Example 36

### 7-((2R,3R,4R,5S)-3,4-bis((tert-Butyldimethylsilyl)oxy)-5-((((4-methyl-2-phenylthiophen-3-yl)methyl) thio)methyl)tetrahydrofuran-2-yl)-7H-pyrrolo[2,3-d]pyrimidin-4-amine (53b):

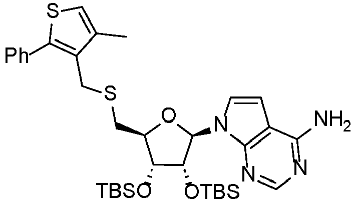

Title compound **53b** was prepared from bromide **53a** (obtained from **20** (0.06 g, 0.29 mmol)) and thiol **46** (provided from **6** (0.2 g, 0.35 mmol)) according to the same methodology as for **47b.** FCC (10-80 % acetone in cyclohexane) provided product **53b** (0.06 g, 0.08 mmol, 27 %). **¹H NMR** (400 MHz, CDCl₃) δ 8.24 (1H, s), 7.53-7.49 (2H, m), 7.42-7.32 (3H, m), 7.10 (1H, d, *J* = 3.7 Hz), 6.91 (1H, q, *J* = 1.0 Hz), 6.39 (1H, d, *J =* 3.7 Hz), 6.02 (1H, d, *J =* 5.5 Hz), 5.47 (2H, bs), 4.73 (1H, dd, *J* = 5.6, 4.5 Hz), 4.16 (1H, dd, *J* = 4.6, 3.3 Hz), 4.11 (1H, dd, *J* = 5.9, 3.4 Hz), 3.81 (1H, *d, J =* 12.3 Hz), 3.74 (1H, *d, J =* 12.3 Hz), 2.93 (1H, dd, *J* = 13.6, 6.2 Hz), 2.79 (1H, dd, *J* = 13.6, 5.6 Hz), 2.32 (3H, d*, J* = 1.1 Hz), 0.92, 0.77 (18H, 2×s), 0.10, 0.09, -0.11, -0.31 (12H, 4×s). **¹³C NMR** (101 MHz, CDCl₃) δ 156.0, 150.5, 150.4, 141.5, 138.4, 134.6, 132.4, 129.5, 128.8, 127.9, 124.1, 120.5, 104.2, 99.0, 89.5, 84.1, 75.0, 74.7, 35.4, 30.1, 26.0, 25.9, 18.2, 18.1, 15.2, -4.4, -4.5, -5.0. **LRMS** Calculated for C₃₅H₅₂N₄O₃S₂Si₂, [M+H]⁺ m/z: 697.3, [M+H]⁺, found 697.2.

### Example 37

### 7-((2R,3R,4R,5S)-3,4-bis((tert-Butyldimethylsilyl)oxy)-5-((((3-methyl-5-phenylisothiazol-4-yl)methyl)thio)methyl)tetrahydrofuran-2-yl)-7H-pyrrolo[2,3-d]pyrimidin-4-amine (54b):

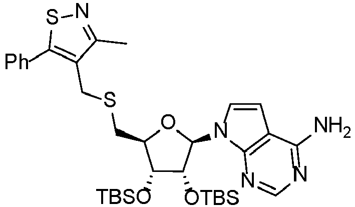

SAH analogue **54b** was prepared from bromide **54a** (obtained from **24** (0.07 g, 0.36 mmol)) and thiol **46** (provided from **6** (0.2 g, 0.36 mmol)) in analogous fashion to **47b.** FCC (10-60 % acetone in cyclohexane) gave modified nucleoside **54b** (0.19 g, 0.27 mmol, 75 %). **¹H NMR** (400 MHz, CDCl₃) δ 8.24 (1H, s), 7.51-7.47 (2H, m), 7.45-7.41 (3H, m), 7.09 (1H, d, *J* = 3.7 Hz), 6.39 (1H, d, *J* = 3.7 Hz), 5.99 (1H, d, *J* = 5.1 Hz), 5.46 (2H, bs), 4.78 (1H, t, *J=* 4.8 Hz), 4.21 (1H, t, *J* = 4.2 Hz), 4.14 (1H, td, *J* = 5.8, 3.8 Hz), 3.82 (1H, d, *J* = 12.5 Hz), 3.73 (1H, d, *J* = 12.5 Hz), 2.96 (1H, dd, *J* = 13.8, 5.7 Hz), 2.85 (1H, dd, *J* = 13.8, 5.7 Hz), 2.53 (3H, s), 0.92, 0.79 (18H, 2×s), 0.10, 0.10, -0.09, -0.28 (12H, 4×s). **¹³C NMR** (101 MHz, CDCl₃) δ 167.6, 163.9, 156.2, 150.6, 150.4, 131.2, 129.3, 129.2, 129.0, 128.9, 124.3, 104.2, 99.0, 90.0, 84.0, 74.8, 74.7, 35.4, 29.0, 26.0, 25.9, 18.4, 18.2, 18.1, -4.2, -4.4, -4.5, -4.9. **LRMS** Calculated for C₃₄H₅₁N₅O₃S₂Si₂, [M+H]⁺ m/z: 698.3, [M+H]⁺, found 698.2.

### Example 38

### 7-((2R,3R,4R,5S)-3,4-bis((tert-Butyldimethylsilyl)oxy)-5-((((1-methyl-4-phenyl-1H-1,2,3-triazol-5-yl)methyl)thio)methyl)tetrahydrofuran-2-yl)-7H-pyrrolo[2,3-d]pyrimidin-4-amine (55b):

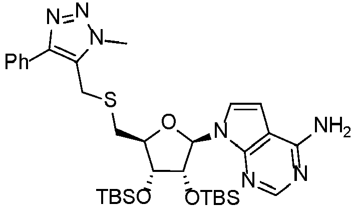

Derivative **55b** was prepared from **55a** (obtained from **27** (0.06 g, 0.32 mmol)) and thiol **46** (provided from **6** (0.23 g, 0.41 mmol) in analogous fashion to **47b.** FCC (20-90 % acetone in cyclohexane) provided **55b** (0.15 g, 0.22 mmol, 70 %). **¹H NMR** (400 MHz, CDCl₃) δ 8.24 (1H, s), 7.71-7.67 (2H, m), 7.44-7.39 (2H, m), 7.37-7.32 (1H, m), 7.03 (1H, d, *J =* 3.7 Hz), 6.39 (1H, d, *J =* 3.7 Hz), 5.96 (1H, d, *J* = 4.5 Hz), 5.45 (2H, bs), 4.84 (1H, t, *J* = 4.5 Hz), 4.26 (1H, t, *J* = 4.6 Hz), 4.21 (1H, dt, *J =* 6.5, 4.6 Hz), 4.02 (1H, d, *J* = 13.7 Hz), 3.97 (3H, s), 3.89 (1H, d, *J* = 13.6 Hz), 2.98 (1H, dd, *J* = 14.1, 6.5 Hz), 2.92 (1H, dd, *J* = 14.1, 4.7 Hz), 0.92, 0.81 (18H, 2×s), 0.10, 0.10, -0.07, -0.25 (12H, 4×s). **¹³C NMR** (101 MHz, CDCl₃) δ 156.4, 151.1, 150.4, 145.8, 131.0, 129.6, 128.9, 128.2, 127.6, 124.3, 104.3, 99.1, 90.7, 84.1, 74.7, 74.5, 35.0, 34.9, 26.0, 25.9, 25.2, 18.2, 18.1, -4.1, -4.5, -4.6, -4.8. Calculated for C₃₃H₅₂N₇O₃SSi₂, [M+H]⁺ m/z: 698.3, [M+H]⁺, found 698.3.

### Example 39

### 7-((2R,3R,4R,5S)-3,4-bis((tert-Butyldimethylsilyl)oxy)-5-((((4-methyl-6-phenylpyrimidin-5-yl)methyl)thio)methyl)tetrahydrofuran-2-yl)-7H-pyrrolo[2,3-d]pyrimidin-4-amine (56b):

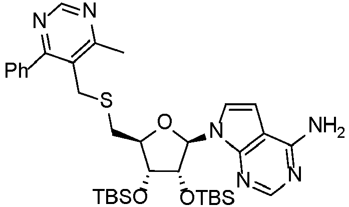

Analogue **56b** was prepared from bromide **56a** (obtained from **31** (0.07 g, 0.36 mmol)) and thiol **46** (provided from 6 (0.2 g, 0.36 mmol)) in analogous fashion to **47b.** FCC (10-100 % acetone in cyclohexane) gave derivative **56b** (0.2 g, 0.29 mmol, 80 %). **¹H NMR** (400 MHz, CDCl₃) δ 8.98 (1H, s), 8.21 (1H, s), 7.69-7.65 (2H, m), 7.48-7.44 (3H, m), 7.02 (1H, d, *J* = 3.7 Hz), 6.37 (1H, d, *J* = 3.7 Hz), 5.95 (1H, d, *J* = 4.8 Hz), 5.43 (2H, bs), 4.80 (1H, t, *J* = 4.7 Hz), 4.22 (1H, t, *J* = 4.4 Hz), 4.14 (1H, dt, *J* = 6.4, 5.0 Hz), 3.83 (1H, d, *J =* 12.2 Hz), 3.74 (1H, d, *J =* 12.2 Hz), 2.95 (1H, dd, *J =* 13.9, 5.2 Hz), 2.87 (1H, dd, *J* = 14.0, 6.3 Hz), 2.66 (3H, s), 0.92, 0.80 (18H, 2×s), 0.11, 0.10, -0.08, -0.27 (12H, 4×s). **¹³C NMR** (101 MHz, CDCl₃) δ 167.7, 165.8, 156.5, 156.3, 150.8, 150.4, 138.0, 129.5, 128.9, 128.6, 127.3, 124.3, 104.3, 98.9, 90.4, 83.9, 74.7, 74.6, 36.0, 32.2, 26.0, 25.9, 22.5, 18.2, 18.1, -4.1, -4.4, -4.5, -4.9. **LRMS** Calculated for C₃₅H₅₃N₆O₃SSi₂, [M+H]⁺ m/z: 693.3, [M+H]⁺, found 693.2.

### Example 40 7-((2R,3R,4R,5S)-3,4-bis((tert-Butyldimethylsilyl)oxy)-5-((((3-methyl-5-phenylpyridin-4-yl)methyl)thio)methyl)tetrahydrofuran-2-yl)-7H-pyrrolo[2,3-d]pyrimidin-4-amine (57b):

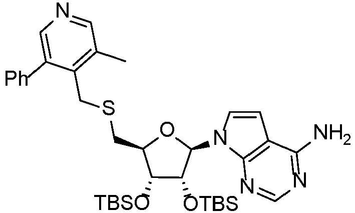

Analogue **57b** was prepared from chloride **57a** (obtained from **35** (0.02 g, 0.11 mmol)) and thiol **46** (provided from 6 (0.07 g, 0.13 mmol)) in analogous fashion to **49b.** FCC (2-15 % EtOH in DCM) afforded **57b** (0.07 g, 0.09 mmol, 85 %). **¹H NMR** (400 MHz, CDCl₃) δ 8.36 (1H, s), 8.30 (1H, s), 8.22 (1H, s), 7.46-7.37 (5H, m), 7.01 (1H, d, *J* = 3.7 Hz), 6.39 (1H, d, *J =* 3.6 Hz), 5.96 (1H, d, *J* = 5.1 Hz), 5.43 (2H, bs), 4.74 (1H, t, *J* = 4.8 Hz), 4.17 (1H, t, *J* = 4.2 Hz), 4.06 (1H, td, *J* = 5.8, 3.8 Hz), 3.72 (1H, d, *J* = 11.7 Hz), 3.64 (1H, d, *J* = 11.7 Hz), 2.87 (1H, dd, *J* = 13.8, 5.7 Hz), 2.75 (1H, dd, *J* = 13.8, 6.0 Hz), 2.42 (3H, s), 0.91, 0.78 (18H, 2×s), 0.09, 0.07, -0.10, -0.29 (12H, 4×s). **¹³C NMR** (101 MHz, CDCl₃) δ 156.2, 150.8, 150.5, 150.2, 148.6, 142.7, 137.7, 137.6, 132.4, 129.7, 128.5, 128.0, 124.1, 104.2, 99.0, 89.9, 83.9, 74.7, 74.6, 36.0, 32.3, 26.0, 25.9, 18.2, 18.1, 16.6, -4.2, -4.4, -4.6, -4.9. **LRMS** Calculated for C₃₆H₅₄N₅O₃SSi₂, [M+H]⁺ m/z: 692.4, [M+H]⁺, found 692.3.

### Example 41

### 7-((2R,3R,4R,5S)-3,4-bis((tert-Butyldimethylsilyl)oxy)-5-((((3-methyl-[1,1'-biphenyl]-2-yl)methyl)thio)methyl)tetrahydrofuran-2-yl)-7H-pyrrolo[2,3-d]pyrimidin-4-amine (58b):

Derivative **58b** was synthesized from **58a** (obtained from **37** (0.06 g, 0.30 mmol)) and thiol **46** (provided 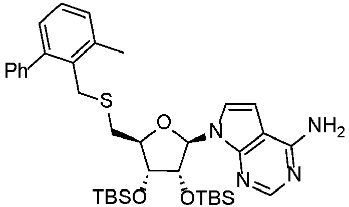 from **6** (0.2 g, 0.36 mmol)) using the same procedure as for **47b.** FCC (10-80 % acetone in cyclohexane) furnished **58b** (0.15 g, 0.22 mmol, 73 %). **¹H NMR** (400 MHz, CDCl₃) δ 8.25 (1H, s), 7.44-7.34 (5H, m), 7.22-7.15 (2H, m), 7.09-7.04 (2H, m), 6.37 (1H, d, *J* = 3.7 Hz), 6.01 (1H, d, *J* = 5.6 Hz), 5.35 (2H, bs), 4.71 (1H, dd, *J* = 5.6, 4.6 Hz), 4.14 (1H, dd, *J =* 4.6, 3.3 Hz), 4.06 (1H, td, *J* = 5.9, 3.4 Hz), 3.75 (1H, d, *J* = 11.9 Hz), 3.69 (1H, d, *J* = 11.9 Hz), 2.86 (1H, dd, *J* = 13.7, 6.2 Hz), 2.71 (1H, dd, *J =* 13.7, 5.5 Hz), 2.48 (3H, s), 0.91, 0.77 (18H, 2×s), 0.09, 0.07, -0.11, -0.32 (12H, 4×s). **¹³C NMR** (101 MHz, CDCl₃) δ 156.3, 151.0, 150.7, 143.0, 141.8, 137.9, 133.8, 130.0, 129.5, 128.22, 128.15, 127.2, 127.0, 124.0, 104.2, 98.8, 89.4, 84.1, 74.9, 74.6, 36.0, 33.6, 26.1, 25.9, 20.1, 18.2, 18.0, -4.2, -4.4, -4.6, -5.0. **LRMS** Calculated for C₃₇H₅₅N₄O₃SSi₂, [M+H]⁺ m/z: 691.4, [M+H]⁺, found 691.3.

### Example 42

### 7-((2R,3R,4R,5S)-3,4-bis((tert-Butyldlmethylsilyl)oxy)-5-((((5-methyl-3-phenylisothiazol-4-yl)methyl)thio)methyl)tetrahydrofuran-2-yl)-7H-pyrrolo[2,3-d]pyrimidin-4-amine (59b):

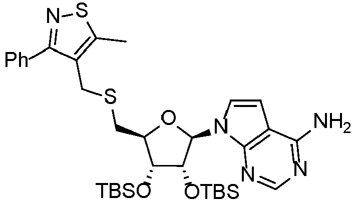

Modified nucleoside **59b** was prepared from bromide **59a** (obtained from **39** (0.02 g, 0.12 mmol)) and thiol **46** (provided from 6 (0.08 g, 0.15 mmol)) in analogous fashion to **47b.** FCC (10-80 % acetone in cyclohexane) gave title compound **59b** (0.08 g, 0.11 mmol, 92 %). **¹H NMR** (400 MHz, CDCl₃) δ 8.24 (1H, s), 7.73-7.69 (2H, m), 7.47-7.40 (3H, m), 7.08 (1H, d, *J* = 3.7 Hz), 6.36 (1H, d, *J* = 3.7 Hz), 5.98 (1H, d, *J =* 4.8 Hz), 5.33 (2H, bs), 4.81 (1H, t, *J =* 4.7 Hz), 4.23 (1H, t, *J =* 4.4 Hz), 4.17 (1H, ddd, *J =* 6.3, 5.2, 4.1 Hz), 3.94 (1H, d, *J =* 13.4 Hz), 3.86 (3H, s), 3.84 (1H, d, *J =* 13.4 Hz), 2.95 (1H, dd, *J =* 13.9, 5.3 Hz), 2.89 (1H, dd, *J =* 13.8, 6.2 Hz), 0.93, 0.80 (18H, 2×s), 0.11, 0.10, -0.08, -0.26 (12H, 4×s). **¹³C NMR** (101 MHz, CDCl₃) δ 156.5, 151.3, 150.6, 137.5, 134.6, 133,3, 128.9, 128.1, 126.8, 124.1, 122.4, 104.3, 98.9, 90.3, 83.9, 74.7, 74.7, 36.9, 35.0, 26.4, 26.0, 25.9, 18.2, 18.1, -4.2, -4.4, -4.6, -4.9. **LRMS** Calculated for C₃₄H₅₂N₅O₃S₂Si₂, [M+H]⁺ m/z: 698.3, [M+H]⁺, found 698.2.

### Exanmple 43 7-((2R,3R,4R,5S)-3,4-bis((tert-Butyldimethylsilyl)oxy)-5-((((1-methyl-4-phenyl-1H-pyrazol-5-yl) methyl)thio)methyl)tetrahydrofuran-2-yl)-7H-pyrrolo[2,3-d]pyrimidin-4-amine (60b):

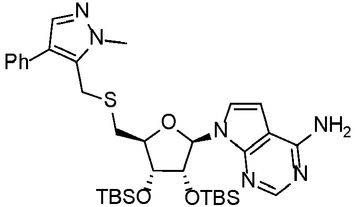

**60b** was prepared from chloride **60a** (obtained from **41** (0.06 g, 0.32 mmol)) and **46** (provided from **6** (0.19 g, 0.35 mmol)) in analogous fashion to **49b.** FCC (10-60 % acetone in cyclohexane) gave **60b** (0.15 g, 0.22 mmol, 70 %). **¹H NMR** (400 MHz, CDCl₃) δ 8.24 (1H, s), 7.52 (1h, s), 7.42-7.34 (4H, m), 7.29-7.25 (1H, m), 7.04 (1H, d, *J* = 3.7 Hz), 6.36 (1H, d, *J* = 3.7 Hz), 6.00 (1H, d, *J* = 5.0 Hz), 5.44 (2H, bs), 4.79 (1H, t, *J =* 4.7 Hz), 4.22 (1H, t, *J =* 4.2 Hz), 4.19 (1H, td, *J =* 5.7, 4.0 Hz), 3.84 (1H, d, *J* = 12.6 Hz), 3.74 (1H, d, *J =* 12.6 Hz), 2.96 (1H, dd, *J =* 13.8, 5.5 Hz), 2.85 (1H, dd, *J =* 13.8, 6.0 Hz), 2.52 (3H, s), 0.93, 0.80 (18H, 2×s), 0.11, 0.11, -0.09, -0.27 (12H, 4×s). **¹³C NMR** (101 MHz, CDCl₃) δ 168.7, 161.8, 156.2, 150.7, 150.5, 136.1, 129.8, 129.0, 128.6, 128.5, 124.2, 104.3, 99.0, 90.1, 84.0, 74.8, 74.7, 35.4, 29.0, 26.0, 25.9, 18.2, 18.1, 12.2, -4.2, -4.4, -4.5, -4.9. **LRMS** Calculated for C₃₄H₅₃N₆O₃SSi₂, [M+H]⁺ m/z: 681.3, [M+H]⁺, found 681.3.

### Example 44

### 7-((2R,3R,4R,5S)-3,4-bis((tert-Butyldimethylsilyl)oxy)-5-((((4-methyl-1-phenyl-1H-imidazol-5-yl)methyl)thio)methyl)tetrahydrofuran-2-yl)-7H-pyrrolo[2,3-d]pyrimidin-4-amine (61b):

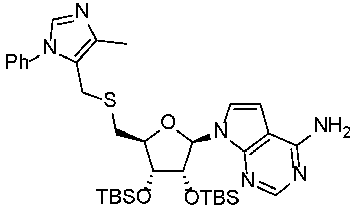

Derivative **61b** was synthesized from chloride **61a** (obtained from **43** (0.07 g, 0.37 mmol)) and thiol **46** (provided from **6** (0.24 g, 0.44 mmol)) in analogous fashion to **49b.** FCC (2-15 % EtOH in DCM) provided SAH analogue **61b** (0.23 g, 0.33 mmol, 90 %). **¹H NMR** (400 MHz, CDCl₃) δ 8.24 (1H, s), 7.51 (1H, s), 7.49-7.42 (3H, m), 7.39-7.35 (2H, m), 7.09 (1H, d, *J =* 3.7 Hz), 6.45 (1H, d, *J =* 3.7 Hz), 6.01 (1H, d, *J =* 4.8 Hz), 5.45 (2H, bs), 4.72 (1H, t, *J =* 4.7 Hz), 4.21 (1H, t, *J =* 4.3 Hz), 4.13 (1H, td, *J =* 5.6, 4.1 Hz), 3.71 (1H, d,J= 14.1 Hz), 3.63 (1H, d, *J* = 14.1 Hz), 2.86 (1H, dd, *J* = 14.0, 5.8 Hz), 2.71 (1H, dd, *J =* 14.0, 5.6 Hz), 2.22 (3H, s), 0.91, 0.79 (18H, 2×s), 0.09, 0.08, -0.10, -0.26 (12H, 4×s). **¹³C NMR** (101 MHz, CDCl₃) δ 155.8, 150.23, 150.16, 148.6, 142.7, 137.7, 132.4, 129.7, 128.5, 128.0, 124.3, 104.1, 99.4, 89.9, 83.9, 74.8, 74.6, 36.0, 32.3, 26.0, 25.9, 18.2, 18.0, 16.6, -4.2, -4.4, -4.6, -4.9. **LRMS** Calculated for C₃₄H₅₃N₆O₃SSi₂, [M+H]⁺ m/z: 681.3, [M+H]⁺, found 681.2.

### Example 45

### 7-((2R,3R,4R,5S)-3,4-bis((tert-Butyldimethylsilyl)oxy)-5-((((4-methyl-2-phenylpyridin-3-yl)methyl)thio)methyl)tetrahydrofuran-2-yl)-7H-pyrrolo[2,3-d]pyrimidin-4-amine (62b):

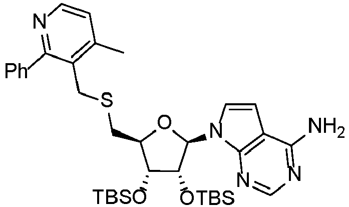

Modified nucleoside **62b** was prepared from chloride **62a** (obtained from **45** (0.06 g, 0.30 mmol)) and thiol **46** (provided from **6** (0.18 g, 0.33 mmol)) in analogous fashion to **49b.** FCC (20-100 % acetone in cyclohexane) provided derivative **62b** (0.17 g, 0.24 mmol, 80 %). **¹H NMR** (400 MHz, CDCl₃) δ 8.41 (1H, d, *J =* 4.9 Hz), 8.23 (1H, s), 7.62-7.58 (2H, m), 7.46-7.37 (3H, m), 7.07 (1H, d, *J =* 4.9 Hz), 7.01 (1H, *d, J =* 3.7 Hz), 6.35 (1H, d, *J* = 3.7 Hz), 5.98 (1H, *d, J =* 5.2 Hz), 5.35 (2H, bs), 4.76 (1H, dd, *J* = 5.2, 4.5 Hz), 4.18 (1H, dd, *J =* 4.7, 3.6 Hz), 4.10 (1H, td, *J* = 5.8, 3.8 Hz), 3.81 (1H, d, *J =* 12.1 Hz), 3.74 (1H, d, *J* = 12.0 Hz), 2.91 (1H, dd, *J* = 13.8, 5.7 Hz), 2.79 (1H, dd, *J* = 13.8, 6.1 Hz), 2.47 (3H, s), 0.92, 0.78 (18H, 2×s), 0.10, 0.08, -0.10, -0.29 (12H, 4×s). **¹³C NMR** (101 MHz, CDCl₃) δ 159.5, 156.4, 151.1, 150.6, 147.8, 147.7, 140.6, 130.2, 129.1, 128.30, 128.25, 124.8, 124.0, 104.3, 98.8, 89.9, 84.0, 74.7, 74.7, 36.0, 33.0, 26.0, 25.9, 19.6, 18.2, 18.1, -4.2, -4.4, -4.6, -4.9. **LRMS** Calculated for C₃₆H₅₄N₅O₃SSi₂, [M+H]⁺ m/z: 692.4, [M+H]⁺, found 692.4.

### Example 46

### (2R,3R,4S,5S)-2-(4-Amino-7H-pyrrolo[2,3-d]pyrimidin-7-yl)-5-((((3-methyl-5-phenylisoxazol-4-yl)methyl)thio)methyl)tetrahydrofuran-3,4-diol (47c):

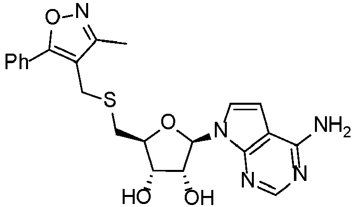

HF-TEA (0.25 mL, 1.53 mmol) was added to a solution of **47b** (0.18 g, 0.26 mmol) in dry MeCN (3 mL) at RT. After stirring for 2 days at RT, the mixture was concentrated under reduced pressure and the residue was co-evaporated twice with MeOH and the residue was purified by reverse-phase FCC (10-60 % MeCN in H₂O) affording **47c** (0.1 g, 0.22 mmol, 86 %). **¹H NMR** (400 MHz, DMSO-*d₆*) δ 8.06 (1H, s), 7.77-7.72 (2H, m), 7.55-7.47 (3H, m), 7.29 (1H, d, *J =* 3.7 Hz), 7.00 (2H, bs), 6.59 (1H, d, *J =* 3.5 Hz), 6.07 (1H, d, *J =* 5.7 Hz), 5.38 (1H, bs), 5.25 (1H, bs), 4.45 (1H, t, *J =* 5.5 Hz), 4.08-4.04 (1H, m), 4.04-3.99 (1H, m), 3.85 (1H, d, *J=* 13.0 Hz), 3.80 (1H, d, *J =* 13.0 Hz), 2.95 (1H, dd, *J =* 13.9, 5.4 Hz), 2.86 (1H, dd, *J* = 13.9, 6.9 Hz), 2.26 (3H, s). **¹³C NMR** (101 MHz, DMSO-*d₆*) δ 164.9, 160.8, 157.7, 152.0, 150.6, 130.3, 129.4, 127.3, 126.8, 121.8, 111.2, 103.0, 100.3, 87.2, 83.1, 73.3, 72.9, 34.9, 24.5, 9.7. **HRMS** (ESI) Calculated for C₂₂H₂₄N₅O₄S, [M+H]⁺ m/z: 454.1544, [M+H]⁺, found 454.1543.

### Example 47

### (2R,3R,4S,5S)-2-(4-Amino-7H-pyrrolo[2,3-d]pyrimidin-7-yl)-5-((((5-methyl-3-phenylisoxazol-4-yl)methyl)thio)methyl)tetrahydrofuran-3,4-diol (48c):

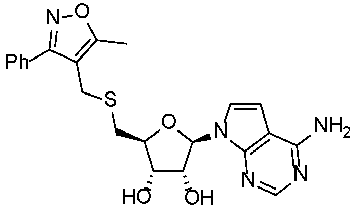

Derivative **48c** was prepared from protected analogue **48b** (0.18 g, 0.3 mmol) using the same procedure as for **47c.** FCC (10-60 % MeCN in H₂O) furnished modified nucleoside **48c** (0.11 g, 0.25 mmol, 93 %). **¹H NMR** (400 MHz, DMSO-*d₆*) δ 8.08 (1H, s), 7.75-7.68 (2H, m), 7.54-7.45 (3H, m), 7.28 (1H, d, *J =* 3.7 Hz), 7.11 (2H, bs), 6.61 (1H, d, *J =* 3.6 Hz), 6.06 (1H, d, *J =* 5.5 Hz), 5.38 (1H, br d, *J =* 5.1 Hz), 5.24 (1H, br d, *J =* 4.0 Hz), 4.43 (1H, br q, *J =* 4.4 Hz), 4. 03 (1H, br q, *J =* 4.0 Hz), 4.01-3.94 (1H, m), 3.74 (1H, *d, J =* 14.2 Hz), 3.70 (1H, d, *J* = 14.2 Hz), 2.87 (1H, dd, *J* = 13.9, 5.3 Hz), 2.74 (1H, dd, *J* = 13.9, 6.8 Hz), 2.39 (3H, s). **¹³C NMR** (101 MHz, DMSO-*d₆*) δ 168.2, 161.6, 157.4, 151.6, 150.5, 129.9, 129.1, 128.1, 121.9, 110.4, 102.9, 100.5, 87.3, 83.0, 73.4, 72.8, 34.4, 24.0, 11.0. **HRMS** (ESI) Calculated for C₂₂H₂₄N₅O₄S, [M+H]⁺ m/z: 454.1544, [M+H]⁺, found 454.1540.

### Example 48

### (2R,3R,4S,5S-2-(4-Amino-7H-pyrrolo[2,3-d]pyrimidin-7-yl)-5-((((1-methyl-4-phenyl-1H-imidazol-5-yl)methyl)thio)methyl)tetrahydrofuran-3,4-diol (49c):

**49c** was synthesized from protected derivative **49b** (0.11 g, 0.14 mmol) following the same methodology as for **47c.** FCC (0-60 % MeCN in H₂O) gave derivative **49c** (0.06 g, 0.13 mmol, 95 %). **¹H NMR** (400 MHz, DMSO-*d₆*) δ 8.14 (1H, s), 7.88 (1H, s), 7.67-7.64 (2H, m), 7.45-7.36 (5H, m), 7.30-7.24 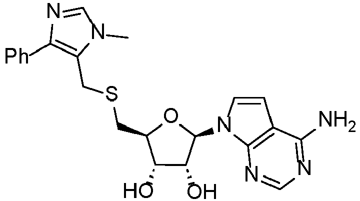 (1H, m), 6.67 (1H, d, *J =* 3.7 Hz), 6.08 (1H, d, *J =* 5.8 Hz), 5.42 (1H, bs), 5.29 (1H, bs), 4.47 (1H, t, *J =* 5.5 Hz), 4.09-3.99 (4H, m), 3.67 (3H, s), 2.98 (1H, dd, *J =* 13.9, 5.4 Hz), 2.90 (1H, dd, *J* = 13.9, 6.8 Hz). **¹³C NMR** (101 MHz, DMSO-*d₆*) δ 156.0, 149.9, 149.8, 137.9, 137.0, 133.5, 128.6, 126.8, 126.6, 123.9, 122.3, 102.6, 100.7, 87.0, 83.2, 73.2, 72.7, 34.7, 31.4, 25.4. **HRMS** (ESI) Calculated for C₂₂H₂₅N₆O₃S, [M+H]⁺ m/z: 453.1703, [M+H]⁺, found 453.1701.

### Example 49

### (2R,3R,4S,5S)-2-(4-Amino-7H-pyrrolo[2,3-d]pyrimidin-7-yl)-5-((((2-methyl-4-phenylpyridin-3-yl)methyl)thio)methyl)tetrahydrofuran-3,4-diol (50c):

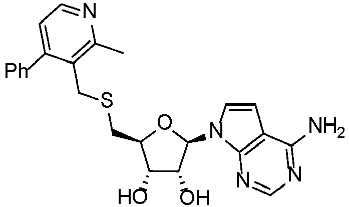

Derivative **50c** was prepared from protected analogue **50b** (0.16 g, 0.23 mmol) according to the same procedure as for **47c.** FCC (0-80 % MeCN in H₂O) gave modified nucleoside **50c** (0.09 g, 0.20 mmol, 87 %). **¹H NMR** (400 MHz, DMSO-*d₆*) δ 8.35 (1H, d, *J =* 5.0 Hz), 8.11 (1H, s), 7.51-7.42 (5H, m), 7.33 (2H, bs), 7.27 (1H, d, *J =* 3.7 Hz), 7.07 (1H, d, *J =* 5.0 Hz), 6.65 (1H, d, *J =* 3.7 Hz), 6.02 (1H, d, *J =* 5.8 Hz), 5.37 (1H, bs), 5.23 (1H, bs), 4.41 (1H, t, *J =* 5.6 Hz), 3.99 (1H, t, *J =* 4.8 Hz), 3.89 (1H, ddd, *J =* 6.8, 5.3, 3.9 Hz), 3.75 (1H, *d, J =* 11.9 Hz), 3.70 (1H, d, *J =* 11.9 Hz), 2.86 (1H, dd, *J =* 13.9, 5.3 Hz), 2.74 (1H, dd, *J=* 13.9, 6.9 Hz), 2.63 (3H, s). **¹³C NMR** (101 MHz, DMSO-*d₆*) δ 158.0, 156.3, 150.3, 149.9, 149.3, 147.0, 138.4, 128.5, 128.2, 122.7, 122.1, 102.7, 100.6, 86.9, 82.9, 73.2, 72.6, 35.7, 31.7, 22.4. **HRMS** (ESI) Calculated for C₂₄H₂₆N₅O₃S, [M+H]⁺ m/z: 464.1751, [M+H]⁺, found 464.1754.

### Example 50

### (2R,3R,4S,5S)-2-(4-Amino-7H-pyrrolo[2,3-d]pyrimidin-7-yl)-5-((((3-methyl-5-phenyl-1H-pyrazol-4-yl)methyl)thio)methyl)tetrahydrofuran-3,4-diol (51c):

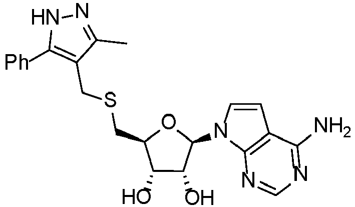

Title compound **51c** was synthesized **51b** (0.04 g, 0.06 mmol) in analogous fashion to **47c.** FCC (10-60 % MeCN in H₂O) furnished **51c** (0.02 g, 0.05 mmol, 87 %). **¹H NMR** (400 MHz, DMSO-*d₆*) δ 12.62 (1H, bs), 8.07 (1H, s), 7.75-7.61 (2H, m), 7.48-7.37 (2H, m), 7.36-7.27 (2H, m), 7.06 (2H, bs), 6.60 (1H, d, *J =* 3.6 Hz), 6.07 (1H, d, *J =* 5.8 Hz), 5.37 (1H,d, *J =* 5.9 Hz), 5.25 (1H, d, *J =* 4.8 Hz), 4.46 (1H, t, *J* = 5.5 Hz), 4.08-4.04 (1H, m), 4.04-3.99 (1H, m), 3.77 (1H, d, *J =* 12.6 Hz), 3.73 (1H, d, *J =* 12.7 Hz), 2.91 (1H, dd, *J =* 13.9, 5.6 Hz), 2.79 (1H, dd, *J =* 13.8, 6.6 Hz), 2.19 (3H, s). **¹³C NMR** (101 MHz, DMSO-*d₆*) δ 157.5, 151.9, 150.6, 128.8, 127.3, 121.8, 110.7, 103.0, 100.3, 87.1, 83.2, 73.4, 72.9, 34.9, 26.2, 9.3. **HRMS** (ESI) Calculated for C₂₂H₂₅N₆O₃S, [M+H]⁺ m/z: 453.1703, [M+H]⁺, found 453.1699.

### Example 51

### (2R,3R,4S,5S)-2-(4-Amino-7H-pyrrolo[2,3-d]pyrimidin-7-yl)-5-((((2-methyl-4-phenylthiophen-3-yl)methyl)thio)methyl)tetrahydrofuran-3,4-diol (52c):

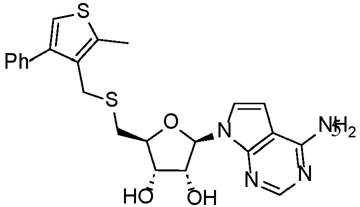

SAH analogue **52c** was synthesized from **52b** (0.06 g, 0.08 mmol) according to the same procedure as for **47c.** FCC (0-70 % MeCN in H₂O) provided modified nucleoside **52c** (0.04 g, 0.07 mmol, 91 %). **¹H NMR** (400 MHz, DMSO-*d₆*) δ 8.08 (1H, s), 7.53-7.47 (2H, m), 7.45-7.39 (2H, m), 7.37-7.31 (1H, m), 7.27 (1H, d, *J =* 3.7 Hz), 7.21 (1H, s), 7.10 (2H, bs), 6.62 (1H, d, *J =* 3.7 Hz), 6.06 (1H, d, *J =* 5.8 Hz), 5.37 (1H, bs), 5.23 (1H, bs), 4.44 (1H, t, *J =* 5.6 Hz), 4.03 (1H, t, *J =* 4.8 Hz), 4.00-3.92 (1H, m), 3.74 (1H, d, *J =* 12.2 Hz), 3.70 (1H, d, *J =* 12.2 Hz), 2.87 (1H, dd, *J* = 14.0, 5.5 Hz), 2.74 (1H, dd, *J* = 13.9, 6.7 Hz), 2.43 (3H, s). **¹³C NMR** (101 MHz, DMSO-*d₆*) δ 157.2, 151.5, 150.3, 142.7, 137.1, 136.7, 131.4, 128.5, 128.2, 127.7, 121.7, 119.8, 102.8, 100.2, 86.9, 82.9, 73.2, 72.7, 35.1, 28.5, 13.4. **HRMS** (ESI) Calculated for C₂₃H₂₅N₄O₃S₂, [M+H]⁺ m/z: 469.1363, [M+H]⁺, found 469.1368.

### Example 52

### (2R,3R,4S,5S)-2-(4-Amino-7H-pyrrolo[2,3-d]pyrimidin-7-yl)-5-((((4-methyl-2-phenylthiophen-3-yl)methyl)thio)methyl)tetrahydrofuran-3,4-diol (53c):

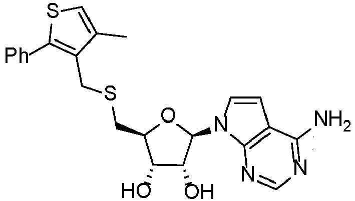

**53c** was prepared from **53b** (0.06 g, 0.08 mmol) according to the same procedure as for **47c.** FCC (0-70 % MeCN in H₂O) furnished **53c** (0.02 g, 0.04 mmol, 48 %). **¹H NMR** (400 MHz, DMSO-*d₆*) δ 8.06 (1H, s), 7.56-7.52 (2H, m), 7.47-7.43 (2H, m), 7.39-7.35 (1H, m), 7.26 (1H, d, *J* = 3.8 Hz), 7.16 (1H, q, *J* = 0.8 Hz), 7.00 (2H, bs), 6.59 (1H, *d, J =* 3.6 Hz), 6.065(1H, d, *J =* 5.9 Hz), 5.36 (1H, d, *J* = 6.2 Hz), 5.22 (1H, d, *J =* 5.2 Hz), 4.45 (1H, q, *J =* 5.4 Hz), 4.03 (1H, q, *J =* 4.2 Hz), 3.96 (1H, ddd, *J =* 6.9, 5.6, 3.9 Hz), 3.78 (1H, d,*J* = 11.8 Hz), 3.74 (1H, d,*J* = 11.8 Hz), 2.91 (1H, dd, *J* = 13.9, 5.5 Hz), 2.81 (1H, dd, *J* = 13.9, 6.9 Hz), 2.25 (3H, s). **¹³C NMR** (101 MHz, DMSO-*d₆*) δ 157.5, 151.8, 150.5, 140.0, 138.3, 133.9, 132.4, 129.0, 128.5, 127.8, 121.5, 120.7, 102.8, 100.1, 86.9, 82.9, 73.1, 72.7, 35.3, 29.3, 14.5. **HRMS** (ESI) Calculated for C₂₃H₂₅N₄O₃S₂, [M+H]⁺ m/z: 469.1363, [M+H]⁺, found 469.1367.

### Example 53

### (2R,3R,4S,5S)-2-(4-Amino-7H-pyrrolo[2,3-d]pyrimidin-7-yl)-5-((((3-methyl-5-phenylisothiazol-4-yl)methyl)thio)methyl)tetrahydrofuran-3,4-diol (54c):

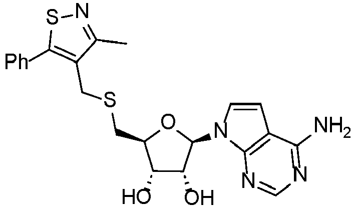

Modified nucleoside **54c** was synthesized from **54b** (0.18 g, 0.26 mmol) in analogous fashion to **47c.** FCC (0-70 % MeCN in H₂O) provided **54c** (0.09 g, 0.19 mmol, 74 %). **¹H NMR** (400 MHz, DMSO-*d₆*) δ 8.08 (1H, s), 7.58-7.47 (5H, m), 7.30 (1H, d, *J* = 3.7 Hz), 7.20 (2H, bs), 6.62 (1H, d, *J* = 3.6 Hz), 6.05 (1H, d, *J =* 5.8 Hz), 5.38 (1H, br d, *J =* 6.0 Hz), 5.25 (1H, br d, *J =* 5.1 Hz), 4.45 (1H, q, *J =* 5.2 Hz), 4.03 (1H, q, *J =* 4.2 Hz), 3.96 (1H, ddd, *J* = 7.0, 5.4, 3.8 Hz), 3.86 (1H, d, *J* = 12.4 Hz), 3.81 (1H, d, *J* = 12.4 Hz), 2.93 (1H, dd, *J* = 13.9, 5.5 Hz), 2.83 (1H, dd, *J* = 13.9, 7.0 Hz), 2.47 (3H, s). **¹³C NMR** (101 MHz, DMSO-*d₆*) δ 167.7, 162.5, 156.8, 151.0, 150.2, 130.3, 129.43, 129.38, 129.3, 128.2, 121.9, 102.7, 100.4, 86.9, 82.9, 73.1, 72.7, 35.2, 27.7, 17.9. **HRMS** (ESI) Calculated for C₂₂H₂₄N₅O₃S₂, [M+H]⁺ m/z: 470.1315, [M+H]⁺, found 470.1314.

### Example 54

### (2R,3R,4S,5S)-2-(4-Amino-7H-pyrrolo[2,3-d]pyrimidin-7-yl)-5-((((1-methyl-4-phenyl-1H-1,2,3-triazol-5-yl)methyl)thio)methyl)tetrahydrofuran-3,4-diol (55c):

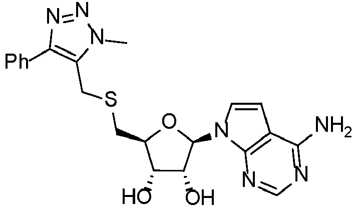

SAH analogue **55c** was prepared from protected derivative **55b** (0.14 g, 0.21 mmol) using the same procedure as for **47c**. FCC (0-50 % MeCN in H₂O) afforded modified nucleoside **55c** (0.08 g, 0.18 mmol, 85 %). **¹H NMR** (400 MHz, DMSO-*d₆*) δ 8.11 (1H, s), 7.73-7.69 (2H, m), 7.48-7.42 (2H, m), 7.39-7.34 (1H, m), 7.33 (1H, d, *J* = 3.7 Hz), 7.30 (2H, bs), 6.65 (1H, d, *J* = 3.7 Hz), 6.07 (1H, d, *J* = 5.7 Hz), 5.41 (1H, bs), 5.28 (1H, bs), 4.45 (1H, t, *J =* 5.7 Hz), 4.15 (1H, d, *J =* 13.7 Hz), 4.11 (1H, d, *J =* 13.8 Hz), 4.06-4.02 (1H, m), 4.02 (3H, s), 4.02-3.98 (1H, m), 2.97 (1H, dd, *J =* 13.9, 5.3 Hz), 2.89 (1H, dd, *J =* 13.9, 6.9 Hz). **¹³C NMR** (101 MHz, DMSO-*d₆*) δ 156.4, 150.5, 150.0, 143.9, 130.9, 130.2, 128.9, 127.8, 126.7, 122.1, 102.7, 100.6, 87.1, 82.9, 73.2, 72.7, 34.68, 34.66, 23.7. **HRMS** (ESI) Calculated for C₂₁H₂₄N₇O₃S, [M+H]⁺ m/z: 454.1656, [M+H]⁺, found 454.1660.

### Example 55

### (2R,3R,4S,5S)-2-(4-Amino-7H-pyrrolo[2,3-d]pyrimidin-7-yl)-5-((((4-methyl-6-phenylpyrimidin-5-yl)methyl)thio)methyl)tetrahydrofuran-3,4-diol (56c):

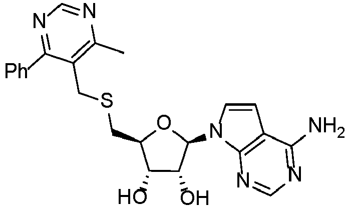

Derivative **56c** was synthesized from analogue **56b** (0.19 g, 0.27 mmol) in analogous fashion to **47c**. FCC (0-50 % MeCN in H₂O) gave **56c** (0.1 g, 0.21 mmol, 78 %). **¹H NMR** (400 MHz, DMSO-*d₆*) δ 8.95 (1H, s), 8.13 (1H, s), 7.71-7.65 (2H, m), 7.52-7.48 (3H, m), 7.43 (2H, bs), 7.31 (1H, d, *J* = 3.7 Hz), 6.67 (1H, d, *J =* 3.7 Hz), 6.04 (1H, d, *J =* 5.8 Hz), 5.40 (1H, bs), 5.26 (1H, bs), 4.43 (1H, t, *J =* 5.6 Hz), 4.01 (1H, t, *J* = 4.7 Hz), 3.93 (1H, ddd, *J =* 7.1, 5.2, 3.9 Hz), 3.85 (1H, d, *J =* 12.2 Hz), 3.80 (1H, d, *J =* 12.2 Hz), 2.93 (1H, dd, *J =* 14.0, 5.3 Hz), 2.82 (1H, dd, *J =* 14.0, 7.1 Hz), 2.63 (3H, s). **¹³C NMR** (101 MHz, DMSO-*d₆*) δ 167.4, 164.4, 156.1, 156.0, 149.8, 149.8, 137.5, 129.3, 128.7, 128.3, 126.9, 122.3, 102.6, 100.7, 87.0, 82.9, 73.2, 72.7, 35.6, 30.9, 22.0. **HRMS**
(ESI) Calculated for C₂₃H₂₅N₆O₃S, [M+H]⁺ m/z: 465.1703, [M+H]⁺, found 465.1702.

### Example 56

### (2R,3R,4S,5S)-2-(4-Amino-7H-pyrrolo[2,3-d]pyrimidin-7-yl)-5-((((3-methyl-5-phenylpyridin-4-yl)methyl)thio)methyl)tetrahydrofuran-3,4-diol (57c):

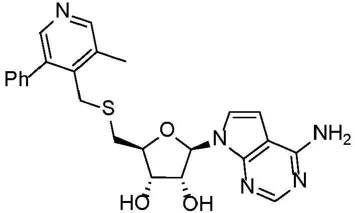

Modified nucleoside **57c** was prepared from protected derivative **57b** (0.06 g, 0.08 mmol) following the same methodology as for **47c**. FCC (0-60 % MeCN in H₂O) furnished SAH analogue **57c** (0.03 g, 0.05 mmol, 68 %). **¹H NMR** (400 MHz, DMSO-*d₆*) δ 8.37 (1H, d, *J =* 0.7 Hz), 8.22 (1H, d, *J* = 0.6 Hz), 8.06 (1H, s), 7.51-7.41 (5H, m), 7.20 (1H, d, *J =* 3.7 Hz), 7.07 (2H, bs), 6.59 (1H, d, *J =* 3.7 Hz), 6.01 (1H, d, *J =* 5.8 Hz), 5.35 (1H, d, *J =* 6.2 Hz), 5.20 (1H, d, *J =* 5.3 Hz), 4.40 (1H, q, *J =* 5.7 Hz), 3.97 (1H, q, *J =* 4.8 Hz), 3.86 (1H, ddd, *J =* 6.9, 5.3, 4.0 Hz), 3.71 (1H, d, *J =* 11.5 Hz), 3.67 (1H, d, *J =* 11.5 Hz), 2.84 (1H, dd, *J =* 13.9, 5.3 Hz), 2.73 (1H, dd, *J =* 13.9, 6.9 Hz), 2.41 (3H, s). **¹³C NMR** (101 MHz, DMSO-*d₆*) δ 157.3, 151.6, 149.8, 147.9, 142.1, 137.1, 136.8, 132.4, 129.3, 128.5, 127.9, 121.6, 102.8, 100.2, 86.9, 82.7, 73.1, 72.6, 35.7, 31.3, 15.9. **HRMS** (ESI) Calculated for C₂₄H₂₆N₅O₃S, [M+H]⁺ m/z: 464.1751, [M+H]⁺, found 464.1748.

### Example 57

### (2R,3R,4S,5S)-2-(4-Amino-7H-pyrrolo[2,3-d]pyrimidin-7-yl)-5-((((3-methyl-[1,1'-biphenyl]-2-yl)methyl)thio)methyl)tetrahydrofuran-3,4-diol (58c):

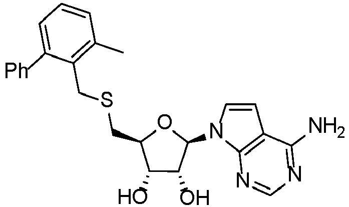

SAH analogue **58c** was synthesized from derivative **58b** (0.14 g, 0.21 mmol) in analogous fashion to **47c**. FCC (0-80 % MeCN in H₂O) afforded modified nucleoside **58c** (0.06 g, 0.12 mmol, 58 %). **¹H NMR** (400 MHz, DMSO-*d₆*) δ 8.07 (1H, s), 7.47-7.36 (5H, m), 7.23-7.17 (3H, m), 7.11 (2H, bs), 7.02 (1H, dd, *J* = 6.4, 2.7 Hz), 6.61 (1H, d, *J* = 3.6 Hz), 6.02 (1H, d, *J* = 5.8 Hz), 5.34 (1H, br d, *J* = 5.9 Hz), 5.19 (1H, br d, *J =* 4.9 Hz), 4.40 (1H, q, *J =* 5.5 Hz), 3.98 (1H, q, *J =* 4.4 Hz), 3.87 (1H, td, *J =* 5.9, 3.9 Hz), 3.69 (1H, d, *J* = 11.7 Hz), 3.66 (1H, d, *J* = 11.7 Hz), 2.83 (1H, dd, *J* = 13.9, 5.4 Hz), 2.69 (1H, dd, *J* = 13.9, 6.8 Hz), 2.44 (3H, s). **¹³C NMR** (101 MHz, DMSO-*d₆*) δ 157.1, 151.4, 150.3, 142.2, 141.1, 137.6, 133.3, 129.7, 128.9, 128.1, 127.8, 127.2, 126.9, 121.7, 102.7, 100.2, 86.8, 82.8, 73.1, 72.6, 35.8, 32.7, 19.4. **HRMS** (ESI) Calculated for C₂₅H₂₇N₄O₃S, [M+H]⁺ m/z: 463.1798, [M+H]⁺, found 463.1795.

### Example 58

### (2R,3R,4S,5S)-2-(4-Amino-7H-pyrrolo[2,3-d]pyrimidin-7-yl)-5-((((5-methyl-3-phenylisothiazol-4-yl)methyl)thio)methyl)tetrahydrofuran-3,4-diol (59c):

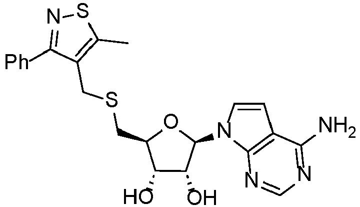

Derivative **59c** was prepared from **59b** (0.08 g, 0.11 mmol) in analogous fashion to **47c**. FCC (0-70 % MeCN in H₂O) provided **59c** (0.04 g, 0.07 mmol, 70 %). **¹H NMR** (400 MHz, DMSO-*d₆*) δ 8.07 (1H, s), 7.74-7.70 (2H, m), 7.51-7.42 (3H, m), 7.28 (1H, d, *J =* 3.7 Hz), 7.09 (2H, bs), 6.60 (1H, d, *J* = 3.6 Hz), 6.06 (1H, d, *J* = 5.7 Hz), 5.38 (1H, br d, *J* = 6.1 Hz), 5.25 (1H, br d, *J* = 5.2 Hz), 4.45 (1H, q, *J* = 5.5 Hz), 4.04 (1H, q, *J* = 4.5 Hz), 3.98 (1H, ddd, *J* = 6.8, 5.3, 4.0 Hz), 3.85 (1H, d, *J =* 12.5 Hz), 3.81 (1H, d, *J =* 12.5 Hz), 2.92 (1H, dd, *J =* 13.9, 5.4 Hz), 2.80 (1H, dd, *J =* 13.9, 6.9 Hz), 2.53 (3H, s). **¹³C NMR** (101 MHz, DMSO-*d₆*) δ 167. 6, 162.0, 157.2, 151.5, 150.3, 135.5, 129.9, 128.9, 128.6, 128.0, 121.7, 102.8, 100.2, 87.0, 82.8, 73.2, 72.7, 35.0, 27.7, 11.7. **HRMS** (ESI) Calculated for C₂₂H₂₄N₅O₃S₂, [M+H]⁺ m/z: 470.1315, [M+H]⁺, found 470.1313.

### Example 59

### (2R,3R,4S,5S)-2-(4-Amino-7H-pyrrolo[2,3-d]pyrimidin-7-yl)-5-((((1-methyl-4-phenyl-1H-pyrazol-5-yl)methyl)thio)methyl)tetrahydrofuran-3,4-diol (60c):

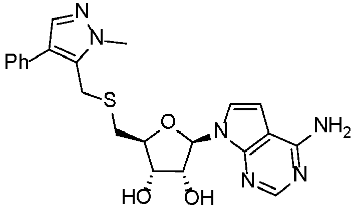

Modified nucleoside **60c** was synthesized from protected derivative **60b** (0.14 g, 0.21 mmol) following the same procedure as for **47c**. FCC (0-50 % MeCN in H₂O) furnished SAH analogue **60c** (0.09 g, 0.19 mmol, 90 %). **¹H NMR** (400 MHz, DMSO-*d₆*) δ 8.08 (1H, s), 7.59 (1H, s), 7.48-7.44 (2H, m), 7.41-7.36 (2H, m), 7.30 (1H, d, *J* = 3.7 Hz), 7.28-7.23 (1H, m), 7.14 (2H, bs), 6.62 (1H, d, *J* = 3.6 Hz), 6.07 (1H, d, *J =* 5.8 Hz), 5.39 (1H, br d, *J =* 6.2 Hz), 5.26 (1H, br d, *J =* 5.8 Hz), 4.46 (1H, q, *J =* 5.4 Hz), 4.07-3.96 (4H, m,), 3.82 (3H, s), 2.96 (1H, dd, *J =* 13.9, 5.5 Hz), 2.88 (1H, dd, *J =* 13.9, 6.9 Hz). **¹³C NMR** (101 MHz, DMSO-*d₆*) δ 157.0, 151.3, 150.3, 136.7, 134.5, 132.9, 128.8, 127.2, 126.3, 121.8, 102.7, 102.8, 100.3, 87.0, 83.0, 73.1, 72.7, 36.4, 34.9, 25.3. **HRMS** (ESI) Calculated for C₂₂H₂₅N₆O₃S, [M+H]⁺ m/z: 453.1703, [M+H]⁺, found 453.1701.

### Example 60

### (2R,3R,4S,5S)-2-(4-Amino-7H-pyrrolo[2,3-d]pyrimidin-7-yl)-5-((((4-methyl-1-phenyl-1H-imidazol-5-yl)methyl)thio)methyl)tetrahydrofuran-3,4-diol (61c):

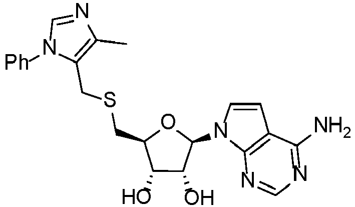

SAH analogue **61c** was prepared from modified nucleoside **61b** (0.22 g, 0.32 mmol) using the same methodology as for **47c**. FCC (0-60 % MeCN in H₂O) provided derivative **61c** (0.14 g, 0.31 mmol, 97 %). **¹H NMR** (400 MHz, DMSO-*d₆*) δ 8.13 (1H, s), 8.11 (1H, s), 7.57-7.47 (5H, m), 7.34 (2H, bs), 7.29 (1H, d, *J =* 3.7 Hz), 6.66 (1H, d, *J =* 3.7 Hz), 6.02 (1H, d, *J =* 5.6 Hz), 5.37 (1H, bs), 5.21 (1H, bs), 4.39 (1H, t, *J =* 5.5 Hz), 3.96 (1H, t, *J =* 4.8 Hz), 3.86 (1H, ddd, *J =* 6.6, 5.5, 4.1 Hz), 3.79 (1H, d, *J =* 14.5 Hz), 3.75 (1H, d, *J =* 14.5 Hz), 2.76 (1H, dd, *J =* 14.0, 5.5 Hz), 2.62 (1H, dd, *J =* 14.0, 6.6 Hz), 2.15 (3H, s). **¹³C NMR** (101 MHz, DMSO-*d₆*) δ 156.3, 150.3, 149.9, 136.4, 135.6, 133.9, 129.7, 128.8, 125.6, 124.1, 122.2, 102.7, 100.6, 87.0, 82.9, 73.2, 72.5, 33.9, 24.0, 11.8. **HRMS** (ESI) Calculated for C₂₂H₂₅N₆O₃S, [M+H]⁺ m/z: 453.1703, [M+H]⁺, found 453.1708.

### Example 61

### (2R,3R,4S,5S)-2-(4-Amino-7H-pyrrolo[2,3-d]pyrimidin-7-yl)-5-((((4-methyl-2-phenylpyridin-3-yl) methyl)thio)methyl)tetrahydrofuran-3,4-diol (62c):

Derivative **62c** was synthesized from analogue **62b** (0.16 g, 0.23 mmol) in analogous fashion to **47c**. 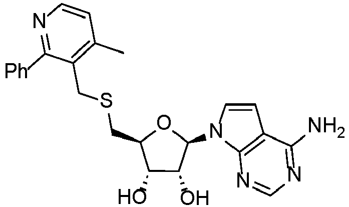 FCC (0-50 % MeCN in H₂O) gave **62c** (0.11 g, 0.23 mmol, 99 %). **¹H NMR** (400 MHz, DMSO-*d₆*) δ 8.37 (1H, d, *J =* 4.69Hz), 8.11 (1H, s), 7.62-7.57 (2H, m), 7.49-7.40 (2H, m), 7.34 (2H, bs), 7.27 (1H, d, *J* = 3.7 Hz), 7.21 (1H, d, *J =* 4.9 Hz), 6.65 (1H, d, *J =* 3.7 Hz), 6.03 (1H, d, *J =* 5.8 Hz), 5.38 (1H, bs), 5.24 (1H, bs), 4.42 (1H, t, *J =* 5.2 Hz), 4.00 (1H, t, *J =* 4.7 Hz), 3.91 (1H, ddd, *J* = 6.9, 5.3, 3.9 Hz), 3.79 (1H, d, *J* = 11.8 Hz), 3.74 (1H, d, *J* = 11.8 Hz), 2.89 (1H, dd, *J =* 13.9, 5.3 Hz), 2.77 (1H, dd, *J* = 13.9, 7.0 Hz), 2.46 (3H, s). **¹³C NMR** (101 MHz, DMSO-*d₆*) δ 158.3, 156.3, 150.2, 149.9, 147.6, 147.5, 140.1, 129.7, 128.8, 128.02, 127.97, 124.7, 122.1, 102.7, 100.6, 86.9, 82.9, 73.2, 72.6, 35.7, 32.0, 18.8. HRMS (ESI) Calculated for C₂₄H₂₆N₅O₃S, [M+H]⁺ m/z: 464.1751, [M+H]⁺, found 464.1750.

To evaluate the role of the phenyl in the 5'-residue, we used the best biologically performing heterocyclic residues and prepared a library of diversely substituted (het)arylmethanols. The 5-methylisooxazole core was selected for a study of substituents replacing phenyl moiety. As a general method, inspired by published procedure (Shi, W., et al., Bioorg & Med Chem Lett 2017/127, 147-151), ethyl acetoacetate **63** anion was reacted with a freshly prepared acylchloride, followed by a thermal closure of the heterocycle with NHzOH. Obtained isoxazole esters **64a-72a** were subsequently reduced with DIBAL to afford the suitably substituted (het)arenes **64b-72b**. One additional heterocycle **75,** containing a pyrimidine core, was synthesized using a similar protocol as for **31 (Scheme** 2). To verify the importance of the methyl substituent, we prepared several derivatives devoid of any modification at the relevant position. 5-phenylisothiazole analogue **79** was obtained from ester **76** (Aartis, D. R., et al. WO2022020244, 2022-01-27) by iodination, arylation using the Suzuki-Miyaura cross-coupling reaction and final reduction of methyl ester function. Suitable ethyl esters **80** and **82** were prepared according to published procedures (Dong, J., et al., Eur J Med Chem 2016/108, 605-615, Muraoka, M., et al., J Chem Soc, Perkin Trans 1 1989/7, 1241-1252) and their reduction either by DIBAL or LAH provided phenyl(het)arylmethanols **81** and **83** (Emmerich, J., et al., J Med Chem 2017/60, 5086-5098). To increase the bulkiness of the substituent replacing the methyl group, heterocycles with ethyl, isopropyl, cyclopropyl and phenyl group were synthesized either by reduction of commercially available carboxylic acids **84a-86a** or reaction of a suitable β-ketoester **87** with benzoylchlorid, followed by a closure of the heterocycle (Betti, Gazz Chim Ital 1915/45, 370) and subsequent reduction of ethyl ester group affording **89**. The derivative containing a methoxy group **92** was synthesized with modified synthetic strategy used for substituted pyrimidine **31**. First the methoxy group was installed via nucleophilic substitution followed by Suzuki cross coupling reaction and reduction of obtained aldehyde to afford **92** (**Scheme 4**).

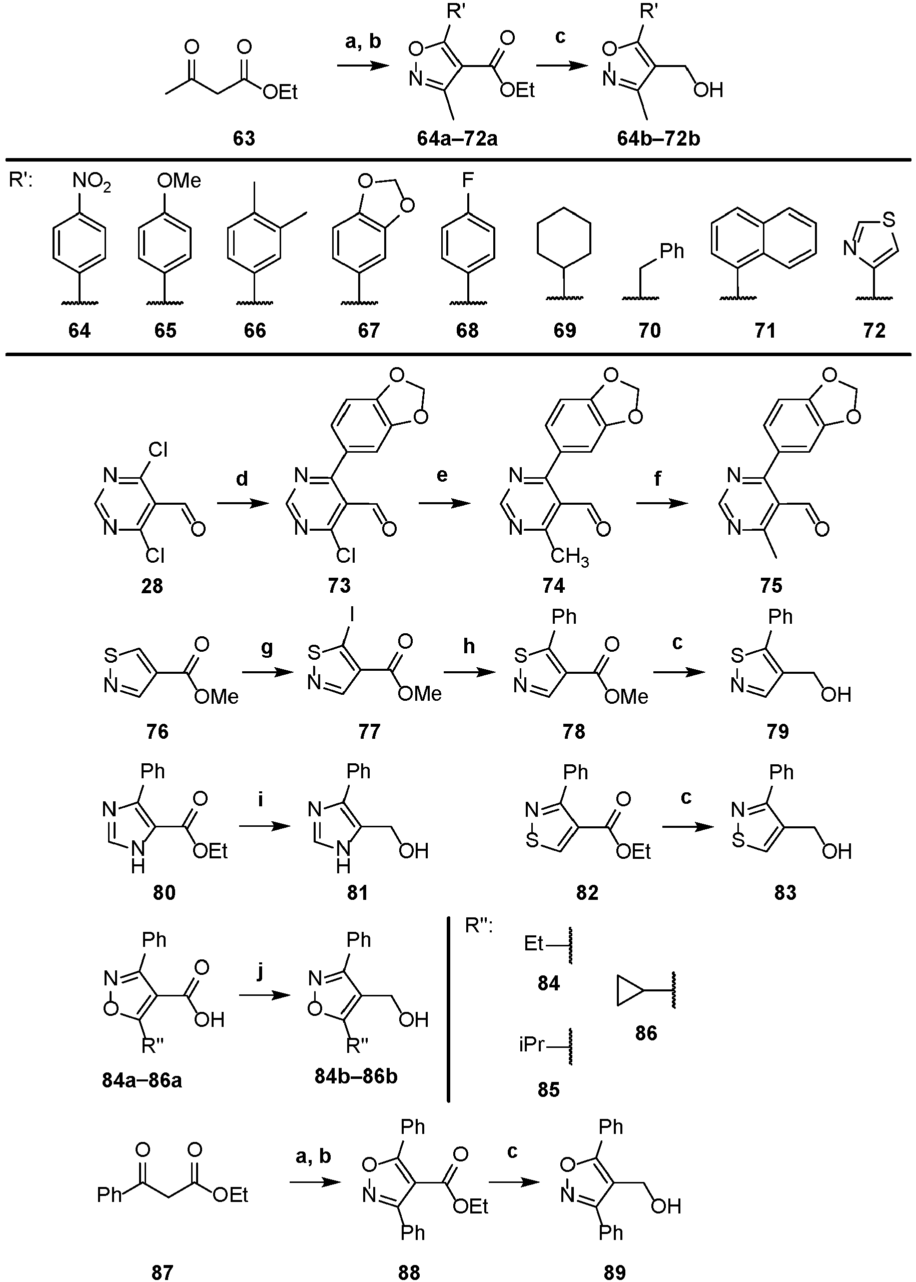

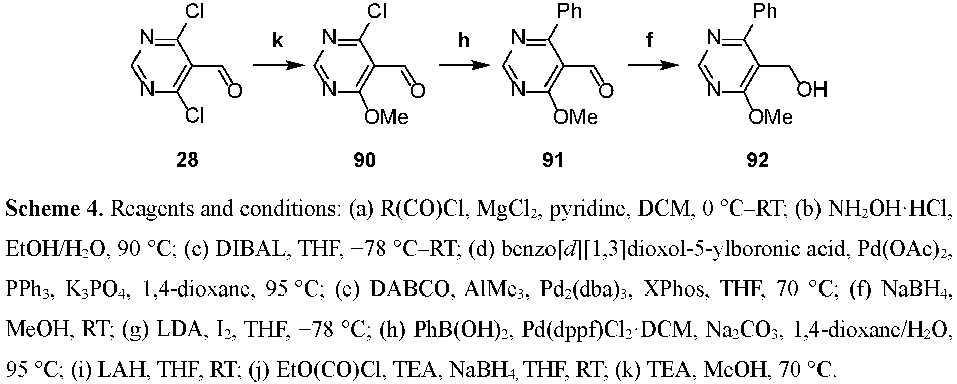

### Example 62

### Ethyl 3-methyl-5-(4-nitrophenyl)isoxazole-4-carboxylate (64a):

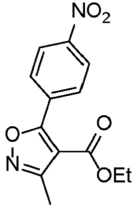

The mixture of **63** (0.75 mL, 5.91 mmol), MgCl₂ (0.68 g, 7.10 mmol) and pyridine (0.95 mL, 11.81 mmol) in dry DCM (12 mL) was cooled to 0 °C. After stirring for 30 min, a solution of 4-nitrobenzoyl chloride (1.43 g, 7.68 mmol) in dry DCM (2 mL) was added dropwise to the reaction and the reaction mixture was stirred at RT for 3 h. After cooling down to 0 °C the reaction was quenched with 6M HCl (30 mL), diluted with DCM (200 mL) and washed with H₂O, saturated NaHCO₃ solution and brine. Organic layer was dried with Na₂SO₄ and concentrated under reduced pressure to get crude intermediate that was added to asolution of NH₂OH.HCl (1.64 g, 23.63 mmol) in EtOH/H₂O (10:1, 10 mL). After stirring at reflux for 5 h, all volatiles were removed under reduced pressure, the residue was dissolved with DCM (200 mL), washed with H₂O and brine. Organic layer was dried with Na₂SO₄ and concentrated in vacuo. FCC (2-15 % EtOAc in cyclohexane) afforded **64a** (0.97 g, 3.50 mmol, 59 %). **¹H NMR** (400 MHz, DMSO-*d₆*) δ 8.39-8.36 (2H, m), 8.16-8.13 (2H, m), 4.25 (2H, q, *J* = 7.1 Hz), 2.46 (3H, s), 1.23 (3H, t, *J* = 7.1 Hz). **¹³C NMR** (101 MHz, DMSO-*d₆*) δ 169.9, 161.1, 160.9, 149.0, 132.3, 130.7, 123.7, 110.0, 61.3, 13.9, 11.8. **LRMS** Calculated for C₁₃H₁₃N₂O₅, [M+H]⁺ m/z: 277.1, [M+H]⁺, found 277.2.

### Example 63

### Ethyl 5-(4-methoxyphenyl)-3-methylisoxazole-4-carboxylate (65a):

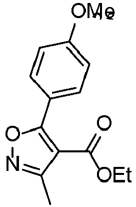

Derivative **65a** was synthesized from **63** (0.64 mL, 5.04 mmol) and 4-methoxybenzoyl chloride (0.75 mL, 5.54 mmol) in analogous fashion to **64a**. FCC (2-15 % EtOAc in cyclohexane) gave **65a** (0.94 g, 3.58 mmol, 71 %). **¹H NMR** (400 MHz, DMSO-*d₆*) δ 7.86 (1H, ddd, *J =* 9.0, 2.9, 2.1 Hz), 7.09 (3H, ddd, *J* = 9.0, 2.9, 2.2 Hz), 4.24 (2H, q, *J* = 7.1 Hz), 3.84 (3H, s), 2.41 (3H, s), 1.24 (3H, t, *J =* 7.1 Hz). **¹³C NMR** (101 MHz, DMSO-*d₆*) δ 172.1, 161.8, 161.7, 160.6, 130.9, 118.8, 114.2, 107.1, 60.8, 55.7, 14.0, 12.0. **LRMS** Calculated for C₁₄H₁₆NO₄, [M+H]⁺ m/z: 262.1, [M+H]⁺, found 262.2.

### Example 64

### Ethyl 5-(3,4-dimethylphenyl)-3-methylisoxazole-4-carboxylate (66a):

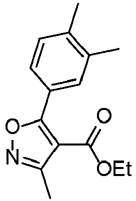

Ester **66a** was prepared from **63** (0.75 mL, 5.91 mmol) and 3,4-dimethylbenzoyl chloride (1.05 mL, 7.09 mmol) in analogous fashion to **64a**. FCC (2-15 % EtOAc in cyclohexane) provided **66a** (1.26 g, 4.86 mmol, 82 %). **¹H NMR** (400 MHz, DMSO-*d₆*) δ 7.63 (1H, s), 7.58 (1H, dd, *J* = 7.9, 1.8 Hz), 7.30 (1H, d, *J* = 7.9 Hz), 4.24 (2H, q, *J* = 7.1 Hz), 2.41 (3H, s), 2.30 (3H, s), 2.28 (3H, s), 1.23 (3H, t, *J* = 7.1 Hz). **¹³C NMR** (101 MHz, DMSO-*d₆*) δ 172.3, 161.6, 160.6, 140.6, 136.7, 129.8, 129.7, 126.5, 124.1, 107.6, 60.8, 19.6, 19.4, 13.9, 11.9. **LRMS** Calculated for C₁₅H₁₈NO₃, [M+H]⁺ m/z: 260.1, [M+H]⁺, found 260.2.

### Example 65

### Ethyl 5-(benzo[d][1,3]dioxol-5-yl)-3-methylisoxazole-4-carboxylate (67a):

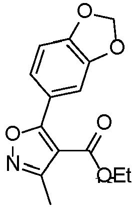

Analogue **67a** was synthesized from **63** (0.5 mL, 3.94 mmol) and piperonyloyl chloride (0.89 g, 4.82 mmol) in analogous fashion to **64a**. FCC (2-20 % EtOAc in cyclohexane) furnished **67a** (0.65 g, 2.36 mmol, 60 %). **¹H NMR** (400 MHz, DMSO-*d₆*) δ 7.45 (1H, s), 7.44 (1H, s, dd, *J =* 8.4, 1.8 Hz), 7.09 (1H, dd, *J =* 8.6, 1.0 Hz), 6.14 (2H, s), 4.24 (2H, *q, J =* 7.1 Hz), 2.40 (3H, s), 1.25 (3H, *t, J =* 7.1 Hz). **¹³C NMR** (101 MHz, DMSO-*d₆*) δ 171.7, 161.6, 160.6, 150.1, 147.5, 124.3, 120.0, 109.1, 108.6, 107.4, 102.1, 60.9, 14.0, 12.0. **LRMS** Calculated for C₁₄H₁₄NO₅, [M+H]⁺ m/z: 276.1, [M+H]⁺, found 276.2.

### Example 66

### Ethyl 5-(4-fluorophenyl)-3-methylisoxazole-4-carboxylate (68a):

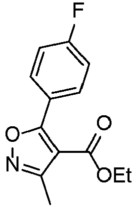

**68a** was prepared from **63** (0.25 mL, 1.97 mmol) and 4-fluorobenzoyl chloride (0.27 mL, 2.28 mmol) using the same methodology as for **64a**. FCC (2-20 % EtOAc in cyclohexane) provided ester **68a** (0.35 g, 1.40 mmol, 71 %). **¹H** NMR (400 MHz, DMSO-*d₆*) δ 7.97-7.91 (2H, m), 7.43-7.37 (2H, m), 4.23 (2H, q, *J =* 7.1 Hz), 2.43 (3H, s), 1.22 (3H, t, *J =* 7.1 Hz). **¹³C NMR** (101 MHz, DMSO-*d₆*) δ 171.3, 163.9 (d, *J =* 250.1 Hz), 161.4, 160.6, 131.9 (d, *J* = 9.1 Hz), 123.1 (d, *J* = 3.2 Hz), 115.8 (d, *J* = 22.1 Hz), 108.2, 60.9, 13.9, 11.9. **¹⁹F NMR** (377 MHz, DMSO-*d₆*) δ -110.28 (tt, *J =* 8.9, 5.4 Hz). **LRMS** Calculated for C₁₃H₁₃FNO₃, [M+H]⁺ m/z: 250.1, [M+H]⁺, found 250.2.

### Example 67

### Ethyl 5-cyclohexyl-3-methylisoxazole-4-carboxylate (69a):

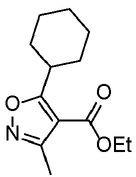

Ester **69a** was prepared from **63** (0.75 mL, 5.91 mmol) and cyclohexanecarbonyl chloride (0.92 mL, 6.88 mmol) following the same procedure as for **64a.** FCC (2-20 % EtOAc in cyclohexane) gave analogue **69a** (1.15 g, 4.86 mmol, 82 %). **¹H NMR** (400 MHz, DMSO-*d₆*) δ 4.25 (2H, q, *J* = 7.1 Hz), 2.34 (3H, s), 1.85 (2H, br d, *J =* 13.0 Hz), 1.82-1.75 (2H, m), 1.73-1.65 (1H, m), 1.52 (2H, qd, *J =* 12.0, 2.5 Hz), 1.39-1.21 (7H, m). **¹³C NMR** (101 MHz, DMSO-*d₆*) δ 181.3, 161.7, 159.4, 106.9, 60.5, 36.7, 29.9, 25.7, 25.3, 14.1, 11.5. **LRMS** Calculated for C₁₃H₂₀NO₃, [M+H]⁺ m/z: 238.1, [M+H]⁺, found 238.2.

### Example 68

### Ethyl 5-benzyl-3-methylisoxazole-4-carboxylate (70a):

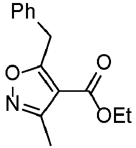

Ester **70a** was synthesized from **63** (0.75 mL, 5.91 mmol) and 2-phenylacetyl chloride (0.94 mL, 7.11 mmol) according to the same methodology as for **64a**. FCC (2-20 % EtOAc in cyclohexane) furnished isoxazole derivative **70a** (0.65 g, 2.65 mmol, 45 %). **¹H NMR** (400 MHz, DMSO-*d₆*) δ 7.36-7.29 (2H, m), 7.29-7.24 (2H, m), 4.42 (2H, s), 4.28 (2H, q,*J* = 7.1 Hz), 2.36 (3H, s), 1.29 (3H, t, *J* = 7.1 Hz). **¹³C NMR** (101 MHz, DMSO-*d₆*) δ 176.1, 161.6, 159.7, 135.7, 129.5, 128.9, 128.8, 127.2, 60.7, 32.4, 14.1, 11.6. **LRMS** Calculated for C₁₄H₁₆NO₃, [M+H]⁺ m/z: 246.1, [M+H]⁺, found 246.2.

### Example 69

### Ethyl 3-methyl-5-(naphthalen-1-yl)isoxazole-4-carboxylate (71a):

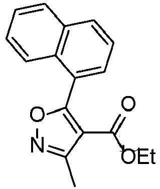

Derivative **71a** was prepared from **63** (0.75 mL, 5.91 mmol) and 1-naphthoyl chloride (1.04 mL, 6.90 mmol) using the same procedure as for **64a**. FCC (2-20 % EtOAc in cyclohexane) provided ester **71a** (0.62 g, 2.20 mmol, 37 %). **¹H NMR** (400 MHz, DMSO-*d₆*) δ 8.50 (1H, s), 8.05 (2H, d, *J =* 8.4 Hz), 8.00 (1H, d, *J=* 8.0 Hz), 7.89 (1H, dd, *J* = 8.6, 1.6 Hz), 7.68-7.57 (3H, m), 4.24 (2H, q, *J =* 7.1 Hz), 2.45 (3H, s), 1.20 (3H, t, *J =* 7.1 Hz). **¹³C NMR** (101 MHz, DMSO-*d₆*) δ 172.1, 161.5, 160.7, 134.0, 132.2, 129.7, 129.0, 128.3, 128.1, 127.9, 127.2, 125.4, 123.9, 108.4, 60.9, 13.9, 11.9. **LRMS** Calculated for C₁₇H₁₆NO₃, [M+H]⁺ m/z: 282.1, [M+H]⁺, found 282.2.

### Example 70

### Ethyl 3-methyl-5-(thiazol-4-yl)isoxazole-4-carboxylate (72a):

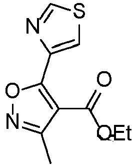

Ester **72a** was synthesized from **63** (0.75 mL, 5.91 mmol) and thiazole-4-carbonyl chloride (freshly prepared following the published procedure (Erlenmeyer, H., Helv Chim Acta 1945/28, 362-365) from thiazole-4-carboxylic acid (0.92 g, 7.09 mmol)) following the same methodology as for **64a**. FCC (2-60 % EtOAc in cyclohexane) furnished isoxazole derivative **72a** (0.56 g, 2.35 mmol, 40 %). **¹H NMR** (400 MHz, DMSO-*d₆*) δ 9.29 (1H, d, *J =* 1.8 Hz), 8.80 (1H, d, *J =* 1.9 Hz), 4.29 (2H, q, *J =* 7.2 Hz), 2.43 (3H, s), 1.28 (3H, t, *J =* 7.1 Hz). **¹³C NMR** (101 MHz, DMSO-*d₆*) δ 166.2, 161.5, 160.3, 155.7, 141.7, 126.2, 108.8, 61.1, 14.0, 11.8. **LRMS** Calculated for C₁₀H₁₁N₂O₃S, [M+H]⁺ m/z: 239.1, [M+H]⁺, found 239.2.

### Example 71

### (3-Methyl-5-(4-nitrophenyl)isoxazol-4-yl)methanol (64b):

1M solution of DIBAL in hexane (12 mL, 12.00 mmol) was added to a solution of ester **64a** (0.97 g, 3.50 mmol) in dry THF (25 mL) under argon atmosphere at -78 °C. After stirring at RT for 2 h, EtOH and H₂O (75 mL) were sequentially added at 0 °C to quench the reaction. The resulting suspension was extracted with EtOAc (2×200 mL), organic phase was dried over Na₂SO₄ and concentrated under 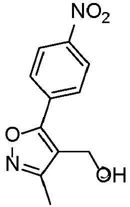 reduced pressure. FCC (5-35 % EtOAc in DCM) gave substituted methanol 64b (0.57 g, 2.44 mmol, 70 %). **¹H NMR** (400 MHz, DMSO-*d₆*) δ 8.39 (2H, d, *J =* 7.7 Hz), 8.08 (2H, d, *J =* 7.7 Hz), 5.39 (1H, t, *J =* 3.7 Hz), 4.43 (2H, d, *J =* 3.7 Hz), 2.34 (3H, s). **¹³C NMR** (101 MHz, DMSO-*d₆*) δ 163.4, 161.3, 148.1, 1332, 128.3, 124.5, 117.3, 51.9, 9.9. **LRMS** Calculated for C₁₁H₁₁N₂O₄, [M+H]⁺ m/z: 235.1, [M+H]⁺, found 235.2.

### Example 72

### (5-(4-Methoxyphenyl)-3-methylisoxazol-4-yl)methanol (65b):

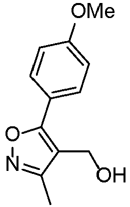

Derivative **65b** was synthesized from ester **65a** (0.5 g, 1.91 mmol) in analogous fashion to **64b**. FCC (5-40 % EtOAc in DCM) furnished **65b** (0.4 g, 1.84 mmol, 96%). **¹H NMR** (400 MHz, DMSO-*d₆*) δ 7.74 (1H, dt, *J =* 8.9, 2.0 Hz), 7.11 (1H, dt, *J =* 8.9, 2.0 Hz), 5.19 (1H, t, *J =* 5.0 Hz), 4.39 (2H, d, *J =* 4.7 Hz), 3.83 (3H, s), 2.28 (3H, s). **¹³C NMR** (101 MHz, DMSO-*d₆*) δ 165.9, 160.8, 160.7, 128.7, 120.2, 114.8, 113.6, 55.5, 52.2, 9.9. **LRMS** Calculated for C₁₂H₁₄NO₃, [M+H]⁺ m/z: 220.1, [M+H]⁺, found 220.2.

### Example 73

### (5-(3,4-Dimethylphenyl)-3-methylisoxazol-4-yl)methanol (66b):

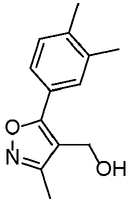

Isoxazole derivative **66b** was prepared from ester **66a** (1.26 g, 4.86 mmol) according to the same procedure as for **64b**. FCC (2-20 % EtOAc in DCM) afforded substituted methanol **66b** (0.83 g, 3.83 mmol, 79 %). **¹H NMR** (400 MHz, DMSO-*d₆*) δ 7.56 (1H, s), 7.51 (1H, dd, *J =* 7.8, 1.8 Hz), 7.31 (1H, d, *J =* 7.9 Hz), 5.19 (1H, t, *J =* 5.3 Hz), 4.40 (2H, d, *J =* 5.3 Hz), 2.29 (3H, s), 2.29 (3H, s), 2.28 (3H, s). **¹³C NMR** (101 MHz, DMSO-*d₆*) δ 167.0, 160.8, 139.0, 137.4, 130.3, 127.9, 125.3, 124.6, 114.2, 52.2, 19.6, 19.5, 9.9. **LRMS** Calculated for C₁₃H₁₆NO₂, [M+H]⁺ m/z: 218.1, [M+H]⁺, found 218.2.

### Example 74

### (5-(Benzo[d][1,3]dioxol-5-yl)-3-methylisoxazol-4-yl)methanol (67b):

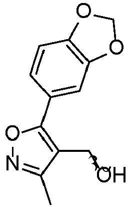

Derivative **67b** was synthesized from ester **67a** (0.5 g, 1.82 mmol) using the same methodology as for **64b**. FCC (5-40 % EtOAc in DCM) provided derivative **67b** (0.21 g, 0.91 mmol, 50 %). **¹H NMR** (400 MHz, DMSO-*d₆*) δ 7.34-7.31 (2H, m), 7.12-7.08 (1H, m), 6.12 (2H, s), 5.23 (1H, bs), 4.37 (2H, bs), 2.28 (3H, s). **¹³C NMR** (101 MHz, DMSO-*d₆*) δ 165.7, 160.8, 149.0, 148.1, 121.8, 121.5, 113.9, 109.2, 107.2, 101.9, 52.1, 9.9. **LRMS** Calculated for C₁₂H₁₂NO₄, [M+H]⁺ m/z: 234.1, [M+H]⁺, found 234.2.

### Example 75

### (5-(4-Fluorophenyl)-3-methylisoxazol-4-yl)methanol (68b):

Alcohol **68b** was prepared from ester **68a** (0.33 g, 1.33 mmol) in analogous fashion to **64b**. FCC (5-35 % EtOAc in DCM) furnished **68b** (0.11 g, 0.53 mmol, 40 %). **¹H NMR** (400 MHz, DMSO-*d₆*) δ 7.92-7.86 (2H, m), 7.36 (2H, tdd, *J* = 8.9, 3.0, 2.3 Hz), 5.24 (1H, t, *J =* 5.2 Hz), 4.34 (2H, *d, J =* 5.1 Hz), 2.45 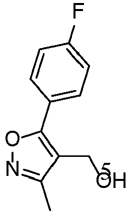 (3H, s). **¹³C NMR** (101 MHz, DMSO-*d₆*) δ 168.5, 163.2 (d, *J* = 246.9 Hz), 161.2, 130.5 (d, *J =* 8.5 Hz ), 125.8 (d, *J* = 3.1 Hz), 116.1 (d, *J* = 21.7 Hz ), 113.7, 51.9, 11.0. **¹⁹F NMR** (377 MHz, DMSO-*d₆*) δ -111.53 (tt, *J =* 9.0, 5.4 Hz). **LRMS** Calculated for C₁₁H₁₁FNO₂, [M+H]⁺ m/z: 208.1, [M+H]⁺, found 208.2.

### Example 76

### (5-Cyclohexyl-3-methylisoxazol-4-yl)methanol (69b):

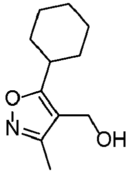

Analogue **69b** was synthesized from ester **69a** (0.15 g, 0.62 mmol) following the same methodology as for **64b**. FCC (5-45 % EtOAc in DCM) afforded analogue **69b** (0.1 g, 0.49 mmol, 78 %). **¹H NMR** (400 MHz, DMSO-*d₆*) δ 4.85 (1H, bs), 4.23 (2H, s), 2.88 (1H, t, *J =* 11.0 Hz), 2.18 (3H, s), 1.76 (4H, bs), 1.68 (1H, d, *J =* 11.0 Hz), 1.50 (1H, q,

*J =* 11.1 Hz), 1.42-1.11 (3H, m). **¹³C NMR** (101 MHz, DMSO-*d₆*) δ 172.4, 159.4, 113.1, 51.7, 35.2, 30.9, 25.7, 25.5, 9.8. **LRMS** Calculated for C₁₁H₁₈NO₂, [M+H]⁺ m/z: 196.1, [M+H]⁺, found 196.2.

### Example 77

### (5-Benzyl-3-methylisoxazol-4-yl)methanol (70b):

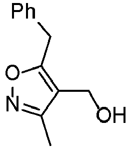

Isoxazole derivative **70b** was prepared from analogue **70a** (0.64 g, 2.61 mmol) following the same procedure as for **64b**. FCC (2-40 % EtOAc in DCM) afforded substituted methanol **70b** (0.39 g, 1.91 mmol, 73 %). **¹H NMR** (400 MHz, DMSO-*d₆*) δ 7.39-7.12 (5H, m), 4.99 (1H, t, *J =* 5.0 Hz), 4.35 (2H, d, *J =* 5.0 Hz), 4.10 (2H, s), 2.21 (3H, s). **¹³C NMR** (101 MHz, DMSO-*d₆*) δ 167.2, 159.6, 136.8, 128.8, 128.7, 126.8, 114.8, 51.9, 30.9, 9.7. **LRMS** Calculated for C₁₂H₁₄NO₂, [M+H]⁺ m/z: 204.1, [M+H]⁺, found 204.2.

### Example 78

### (3-Methyl-5-(naphthalen-1-yl)isoxazol-4-yl)methanol (71b):

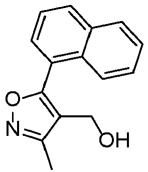

Analogue **71b** was synthesized from ester **71a** (0.36 g, 1.27 mmol) in analogous fashion to **64b**. FCC (2-35 % EtOAc in DCM) furnished **71b** (0.14 g, 0.60 mmol, 47 %). **¹H NMR** (400 MHz, DMSO-*d₆*) δ 8.39 (1H, d, *J =* 1.2 Hz), 8.09 (1H, d, *J =* 8.7 Hz), 8.08-8.04 (1H, m), 8.02-7.98 (1H, m), 7.91 (1H, dd, *J =* 8.6, 1.8 Hz), 7.65-7.57 (2H, m), 5.31 (1H, t, *J* = 5.3 Hz), 4.52 (2H, d, *J* = 5.3 Hz), 2.34 (3H, s). **¹³C NMR** (101 MHz, DMSO-*d₆*) δ 166.6, 161.0, 133.4, 132.9, 129.0, 128.8, 127.9, 127.7, 127.2, 126.9, 125.0, 124.1, 115.2, 52.2, 10.0. **LRMS** Calculated for C₁₅H₁₄NO₂, [M+H]⁺ m/z: 240.1, [M+H]⁺, found 240.2.

### Example 79

### (3-Methyl-5-(thiazol-4-yl)isoxazol-4-yl)methanol (72b):

Derivative **72b** was prepared from isoxazole derivative **72a** (0.44 g, 1.84 mmol) according to the same procedure as for **64b**. FCC (5-55 % EtOAc in DCM) afforded analogue **72b** (0.17 g, 0.87 mmol, 47 %). 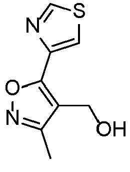 **¹H NMR** (400 MHz, DMSO-*d₆*) δ 9.32 (1H, d, *J =* 1.7 Hz), 8.30 (1H, d, *J =* 1.8 Hz), 5.04 (1H, t, *J =* 5.5 Hz), 4.70 (2H, d, *J =* 5.5 Hz), 2.32 (3H, s). **¹³C NMR** (101 MHz, DMSO-*d₆*) δ 161.0, 159.9, 156.3, 143.9, 120.4, 115.7,52.0, 10.1. **LRMS** Calculated for C₈H₉N₂O₂S, [M+H]⁺ m/z: 197.0, [M+H]⁺, found 197.1.

### Example 80

### 4-(Benzo[d][1,3]dioxol-5-yl)-6-chloropyrimidine-5-carbaldehyde (73):

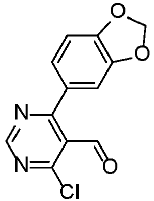

Pyrimidine derivative **73** was synthesized from aldehyde **28** (1 g, 5.65 mmol) and benzo[d][1,3]dioxol-5-ylboronic acid (0.94 g, 5.65 mmol) following the same procedure as for **29**. FCC (5-80 % EtOAc in cyclohexane) provided analogue **73** (0.91 g, 3.47 mmol, 61 %). **¹H NMR** (400 MHz, CDCl₃) δ 10.07 (1H, s), 9.05 (1H, s), 7.21 (1H, d, *J =* 1.8 Hz), 7.05 (1H, dd, *J =* 8.1, 1.8 Hz), 6.94 (1H, d, *J =* 8.1 Hz), 6.09 (2H, s). **¹³C NMR** (101 MHz, CDCl₃) δ 188.4, 168.5, 160.6, 159.2, 151.1, 148.8, 128.8, 126.5, 110.2, 108.8, 102.2. **LRMS** Calculated for C₁₂H₈ClN₂O₃, [M+H]⁺ m/z: 263.0, [M+H]⁺, found 263.0.

### Example 81

### 4-(Benzo[d][1,3]dloxol-5-yl)-6-methylpyrimidine-5-carbaldehyde (74):

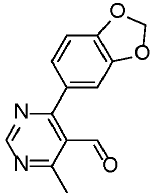

**74** was prepared from **73** (0.8 g, 3.05 mmol) in analogous fashion to **19**. FCC (5-70 % EtOAc in cyclohexane) gave **74** (0.56 g, 2.31 mmol, 76 %). **¹H NMR** (400 MHz, CDCl₃) δ 10.04 (1H, s), 9.15 (1H, s), 7.22 (1H, d, *J =* 1.7 Hz), 7.00 (1H, dd, *J =* 8.0, 1.8 Hz), 6.94 (1H, d, *J =* 8.0 Hz), 6.08 (2H, s), 2.82 (3H, s). **¹³C NMR** (101 MHz, CDCl₃) δ 191.9, 168.6, 168.1, 159.2, 150.6, 148.7, 129.8, 126.4, 126.0, 110.3, 108.7, 102.1, 24.0. **LRMS** Calculated for C₁₃H₁₁N₂O₃, [M+H]⁺ m/z: 243.1, [M+H]⁺, found 243.1.

### Example 82

### (4-(Benzo[d][1,3]dioxol-5-yl)-6-methylpyrimidin-5-yl)methanol (75):

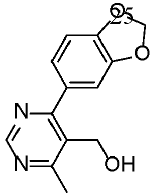

Substituted methanol **75** was synthesized from **74** (0.5 g, 2.06 mmol) using the same procedure as for **27**. FCC (2-10 % EtOH in DCM) afforded **75** (0.35 g, 1.43 mmol, 69 %). **¹H NMR** (400 MHz, CDCl₃) δ 9.02 (1H, s), 7.30-7.23 (2H, m), 6.93 (1H, d, *J* = 8.7 Hz), 6.06 (2H, s), 4.76 (2H, s), 2.74 (3H, s), 2.26 (1H, bs). **¹³C NMR** (101 MHz, CDCl₃) δ 168.6, 165.5, 157.1, 149.1, 148.0, 131.6, 128.0, 123.9, 109.8, 108.4, 101.6, 59.1, 22.2. **LRMS** Calculated for C₁₃H₁₃N₂O₃, [M+H]⁺ m/z: 245.1, [M+H]⁺, found 245.2.

### Example 83

### Methyl 5-iodoisothiazole-4-carboxylate (77):

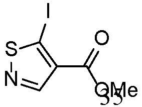

Derivative **77** was prepared from **76** (0.33 g, 2.32 mmol) in analogous fashion to **22**. FCC (2-15 % EtOAc in cyclohexane) gave **77** (0.09 g, 0.33 mmol, 14 %). **¹H NMR** (400 MHz, CDCl₃) δ 8.66 (1H, s), 3.93 (3H, s). **¹³C NMR** (101 MHz, CDCl₃) δ 161.4, 159.4, 133.6, 107.7, 52.4. **LRMS** Calculated for C₅H₅INO₂S, [M+H]⁺ m/z: 269.9, [M+H]⁺, found 269.8.

### Example 84

### Methyl 5-phenylisothiazole-4-carboxylate (78):

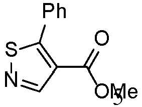

Ester **78** was synthesized from derivative **77** (0.12 g, 0.45 mmol) in analogous fashion to **15.** FCC (0-25 % EtOAc in cyclohexane) provided **78** (0.07 g, 0.30 mmol, 67 %). **¹H NMR** (400 MHz, CDCl₃) δ 8.87 (1H, s), 7.56-7.52 (2H, m), 7.49-7.45 (3H, m), 3.81 (3H, s). **¹³C NMR** (101 MHz, CDCl₃) δ 173.0, 162.4, 160.3, 130.2, 129.4, 129.3, 128.6, 126.0, 52.1. **LRMS** Calculated for C₁₁H₁₀NO₂S, [M+H]⁺ m/z: 220.0, [M+H]⁺, found 220.0.

### Example 85

### (5-Phenylisothiazol-4-yl)methanol (79):

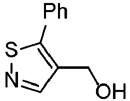

Substituted methanol **79** was prepared from analogue **78** (0.07 g, 0.30 mmol) using the same procedure as for **64b.** FCC (10-70 % EtOAc in cyclohexane) afforded isothiazole derivative **79** (0.03 g, 0.13 mmol, 44 %). **¹H NMR** (400 MHz, CDCl₃) δ 8.54 (1H, s), 7.55-7.44 (5H, m), 4.76 (2H, s). **¹³C NMR** (101 MHz, CDCl₃) δ 164.5, 159.6, 134.4, 130.3, 129.6, 129.3, 128.9, 56.8. **LRMS** Calculated for C₁₀H₁₀NOS, [M+H]⁺ m/z: 192.1, [M+H]⁺, found 192.1.

### Example 86

### (4-Phenyl-1H-imidazol-5-yl)methanol (81):

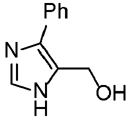

Derivative **81** was synthesized from ester **80** (0.5 g, 2.31 mmol) in analogous fashion to **16.** FCC (5-30 % EtOH in DCM) furnished alcohol **81** (0.33 g, 1.91 mmol, 83 %). **¹H NMR** (400 MHz, DMSO-*d₆*) δ 7.70-7.66 (2H, m), 7.63 (1H, s), 7.42-7.36 (2H, m), 7.27-7.21 (1H, m), 5.20 (1H, bs), 4.54 (2H, s). **¹³C NMR** (101 MHz, DMSO-*d₆*) δ 134.4, 134.0, 129.9, 128.4, 126.5, 126.2, 54.8. **LRMS** Calculated for C₁₀H₁₀NOS, [M+H]⁺ m/z: 175.1, [M+H]⁺, found 175.2.

### Example 87

### (3-Phenylisothiazol-4-yl)methanol (83):

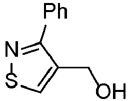

Substituted methanol **83** was prepared from ester **82** (0.85 g, 3.64 mmol) according to the same procedure as for **64b.** FCC (10-40 % EtOAc in cyclohexane) provided isothiazole derivative 83 (0.48 g, 2.51 mmol, 69 %). **¹H NMR** (400 MHz, DMSO-*d₆*) δ 8.97 (1H, t, *J* = 0.8 Hz), 7.78-7.75 (2H, m), 7.52-7.43 (3H, m), 5.45 (1H, t, *J* = 5.3 Hz), 4.60 (2H, dd, *J* = 5.3, 0.8 Hz). **¹³C NMR** (101 MHz, DMSO-*d₆*) δ 166.0, 149.1, 138.4, 135.1, 128.9, 128.5, 128.2, 57.0. **LRMS** Calculated for C₁₀H₁₀NOS, [M+H]⁺ m/z: 192.1, [M+H]⁺, found 192.1.

### Example 88

### (5-Ethyl-3-phenylisoxazol-4-yl)methanol (84b):

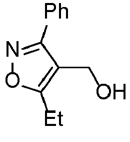

A solution of EtO(CO)Cl (0.27 µL, 2.82 mmol) in dry THF (3 mL) was added to a solution **of 84a** (0.5 g, 2.30 mmol) and TEA (0.39 µL, 2.80 mmol) in dry THF (15 mL) at RT. After 1 h the reaction suspension was filtered and the solid residues were washed with THF. The filtrate was added to a solution of NaBH4 (0.22 g, 5.79 mmol) in H₂O (3 mL). After stirring for 10 h at RT 1N aqueous NaOH solution (1.5 mL) was added. The resulting mixture was diluted with H₂O (50 mL) and extracted with EtOAc (2×200 mL). Pooled organic layers were dried with Na₂SO₄, the solvent was removed in vacuo and the crude product was subjected to FCC (5-25 % EtOAc in DCM) to afford **84b** (0.42 g, 2.06 mmol, 89 %). NMR spectra were consistent with published data (McKenna, J. I., et al., J Med Chem 1988/31, 473-476).

### Example 89

### (5-Isopropyl-3-phenylisoxazol-4-yl)methanol (85b):

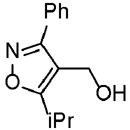

Isoxazole derivative **85b** was prepared from carboxylic acid **85a** (0.5 g, 2.16 mmol) using the same procedure as for **84b.** FCC (5-20 % EtOAc in DCM) furnished substituted methanol **85b** (0.4 g, 1.85 mmol, 85 %). NMR spectra were consistent with published data (Schierle, S., et al., J Med Chem 2020/63, 8369-8379).

### Example 90

### (5-Cyclopropyl-3-phenylisoxazol-4-yl)methanol (86b):

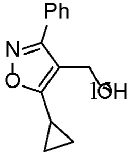

Alcohol **86b** was synthesized from **86a** (0.5 g, 2.18 mmol), in analogous fashion to **84b.** FCC (5-25 % EtOAc in DCM) provided analogue **86b** (0.43 g, 2.00 mmol, 92 %). **¹H NMR** (400 MHz, DMSO-*d₆*) δ 7.85-7.78 (2H, m), 7.54-7.48 (3H, m), 5.25 (1H, t, *J* = 5.0 Hz), 4.43 (2H, d, *J* = 4.9 Hz), 2.29 (1H, tt, *J* = 8.3, 5.2 Hz), 1.13-1.07 (3H, m), 1.07-1.01 (3H, m). **¹³C NMR** (101 MHz, DMSO-*d₆*) δ 171.7, 162.4, 129.9, 129.3, 129.1, 128.2, 113.4, 51.7, 7.8, 7.2. **LRMS** Calculated for C₁₃H₁₄NO₂, [M+H]⁺ m/z: 216.1, [M+H]⁺, found 216.2.

### Example 91

### Ethyl 3,5-diphenylisoxazole-4-carboxylate (88):

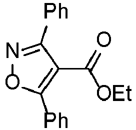

Ester **88** was prepared from 87 (0.75 mL, 4.36 mmol) and benzoyl chloride (0.61 mL, 5.25 mmol) according to the same methodology as for **64a.** FCC (2-25 % EtOAc in cyclohexane) furnished isoxazole derivative **88** (0.8 g, 2.74 mmol, 63 %). NMR spectra were consistent with published data (Feng, Q., et al., Org Lett 2021/23, 2431-2436).

### Example 92

### (3,5-Diphenylisoxazol-4-yl)methanol (89):

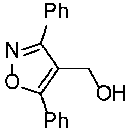

Derivative **89** was synthesized from ester **88** (0.78 g, 2.66 mmol) using the same procedure as for **64b.** FCC (5-20 % EtOAc in DCM) provided isoxazole derivative **89** (0.56 g, 2.21 mmol, 83 %). **¹H NMR** (400 MHz, DMSO-*d₆*) δ 7.94-7.90 (2H, m), 7.90-7.85 (2H, m), 7.65-7.55 (6H, m), 5.58 (1H, t, *J* = 4.4 Hz), 5.58 (2H, d, *J* = 4.4 Hz). **¹³C NMR** (101 MHz, DMSO-*d₆*) δ 168.1, 163.5, 130.7, 130.2, 129.4, 129.2, 128.8, 128.5, 127.4, 127.3, 113.6, 52.3. **LRMS** Calculated for C₁₆H₁₄NO₂, [M+H]⁺ m/z: 252.1, [M+H]⁺, found 252.2.

### Example 93

### 4-Chloro-6-methoxypyrimidine-5-carbaldehyde (90):

To a stirred suspension of **28** (2 g, 11.3 mmol) in MeOH (55 mL) was added TEA (2.4 mL, 16.95 mmol). 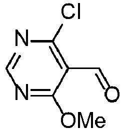 The reaction mixture was stirred at RT for 10 h, volatiles were evaporated, residue coevaporated with toluene and dried to afford **90** (1.76 g, 10.20 mmol, 90 %) that was used in the next step without further purification. All characterizations were consistent with published data (Kuragano, T., EP1333029, 2003-08-06).

### Example 94

### 4-Methoxy-6-phenylpyrimidine-5-carbaldehyde (91):

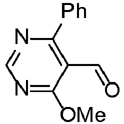

Aldehyde **91** was synthesized from analogue **90** (0.2 g, 1.16 mmol) in analogous fashion to **29.** FCC (5-20 % acetone in cyclohexane) furnished **91** (0.16 g, 0.74 mmol, 64 %). **¹H NMR** (400 MHz, CDCl₃) δ 10.06 (1H, s), 8.92 (1H, s), 7.61-7.57 (2H, m), 7.56-7.48 (3H, m), 4.16 (3H, s). **¹³C NMR** (101 MHz, CDCl₃) δ 189.1, 170.1, 168.4, 160.0, 135.7, 131.0, 130.4, 128.8, 115.1, 55.2. **LRMS** Calculated for C₁₂H₁₁N₂O₂, [M+H]⁺ m/z: 215.1, [M+H]⁺, found 215.1.

### Example 95

### (4-Methoxy-6-phenylpyrimidin-5-yl)methanol (92):

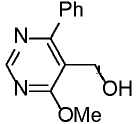

Derivative **92** was prepared from aldehyde **91** (0.16 g, 0.75 mmol) according to the same methodology as for **27.** FCC (10-30 % acetone in cyclohexane) provided substituted methanol **92** (0.12 g, 0.54 mmol, 72 %). **¹H NMR** (400 MHz, DMSO-*d₆*) δ 8.78 (1H, s), 7.84-7.73 (2H, m), 7.53-7.48 (3H, m), 5.23 (1H, t, *J* = 4.8 Hz), 4.39 (2H, d, *J* = 4.8 Hz), 4.00 (3H, s). **¹³C NMR** (101 MHz, DMSO-*d₆*) δ 168.8, 165.1, 156.8, 137.5, 129.6, 129.4, 128.3, 117.7, 54.6, 54.3. **LRMS** Calculated for C₁₂H₁₃N₂O₂, [M+H]⁺ m/z: 217.1, [M+H]⁺, found 217.1.

Provided (het)arylmethanols **64b-72b, 75, 79, 81, 83, 84b-86b, 89** and **92** were used to prepare SAH derivatives **93c-110c** using the same synthetic strategy as mentioned above **(Scheme 3).** One more SAH analogue **111** was obtained by the catalytic hydrogenation of nitro group of the modified nucleoside **93c** (**Scheme 5**).

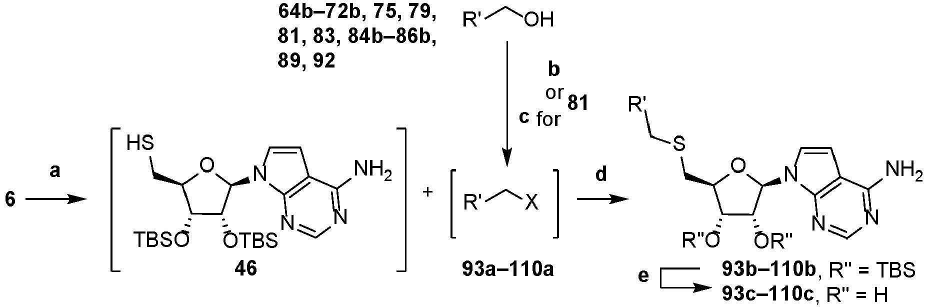

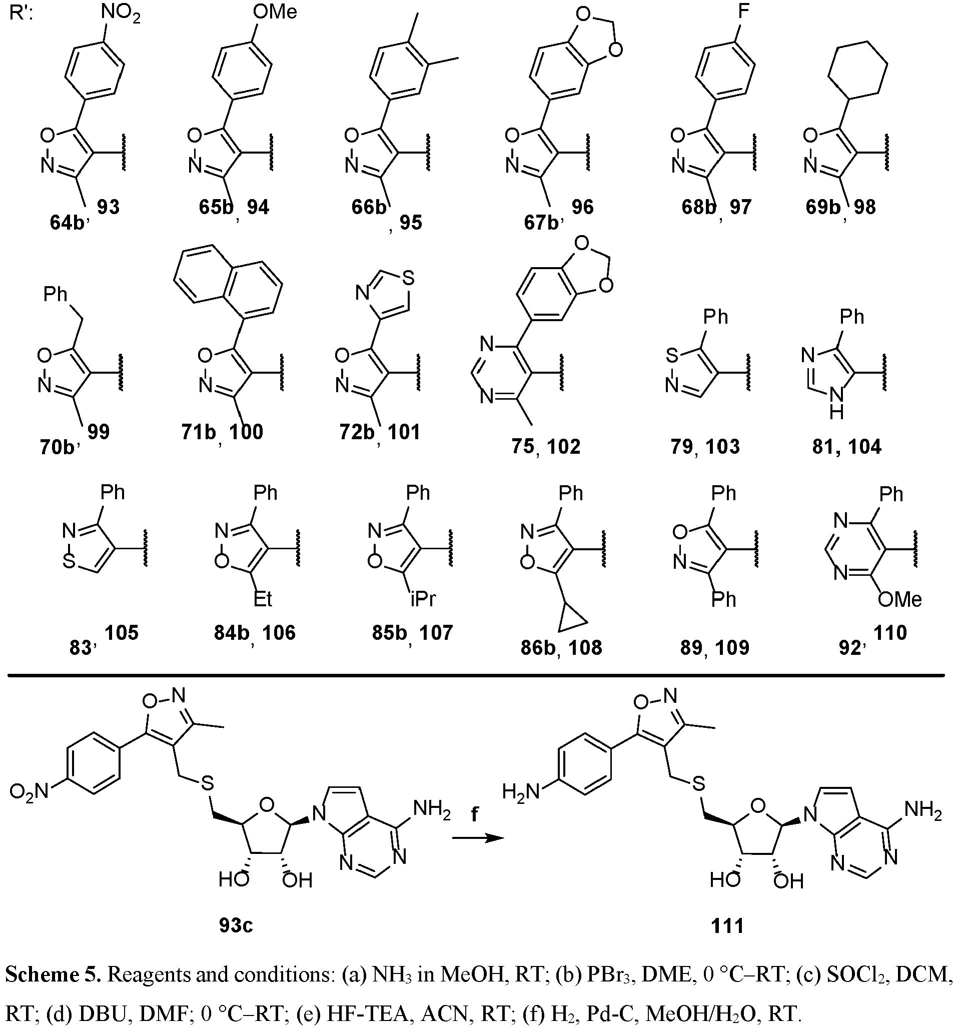

### Example 96

### 7-((2R,3R,4R,5S)-3,4-bis((tert-Butyldimethylsilyl)oxy)-5-((((3-methyl-5-(4-nitrophenyl)isoxazol-4-yl)methyl)thio)methyl)tetrahydrofuran-2-yl)-7H-pyrrolo[2,3-d]pyrimidin-4-amine (93b):

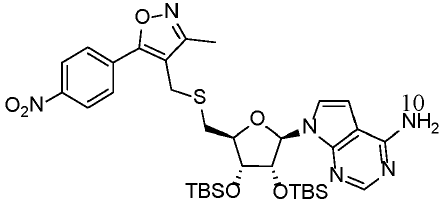

SAH analogue **93b** was prepared from bromide **93a** (obtained from **64b** (0.12 g, 0.53 mmol)) and thiol **46** (provided from **6** (0.25 g, 0.45 mmol)) using the same procedure as for **47b.** FCC (10-80 % EtOAc in DCM) furnished modified nucleoside **93b** (0.25 g, 0.35 mmol, 77 %). **¹H NMR** (400 MHz, DMSO-*d₆*) δ 8.32-8.27 (2H, m), 8.04-8.00 (3H, m), 7.31 (1H, d, *J* = 3.7 Hz), 6.98 (2H, bs), 6.58 (1H, *d, J* = 3.6 Hz), 6.06 (1H, d, *J* = 6.8 Hz), 4.78 (1H, dd, *J* = 6.7, 4.5 Hz), 4.17 (1H, dd, *J* = 4.4, 1.8 Hz), 4.00 (2H, m), 3.88 (1H, d, *J* = 13.5 Hz), 3.04 (1H, dd, *J* = 13.8, 6.5 Hz), 2.93 (1H, dd, *J* = 13.8, 7.0 Hz), 2.30 (3H, s), 0.89, 0.68 (18H, 2×s), 0.08, -0.14, -0.40 (12H, 3×s). **¹³C NMR** (101 MHz, DMSO-*d₆*) δ 162.7, 1612, 157.64, 157.57, 152.0, 150.7, 148.0, 132.9, 128.1, 124.4, 122.2, 113.9, 103.2, 100.3, 87.0, 84.3, 74.8, 74.1, 34.5, 25.9, 25.7, 24.2, 17.9, 17.7, 9.8, -4.4, -4.5, -4.6, -5.3. **LRMS** Calculated for C₃4H₅₁N₆O₆SSi₂, [M+H]⁺ m/z: 727.3, [M+H]⁺, found 727.3.

### Example 97

### 7-((2R,3R,4R,5S)-3,4-bis((tert-Butyldimethylsilyl)oxy)-5-((((5-(4-methoxyphenyl)-3-methylisoxazol-4-yl)methyl)thio)methyl)tetrahydrofuran-2-yl)-7H-pyrrolo[2,3-d]pyrimidin-4-amine (94b):

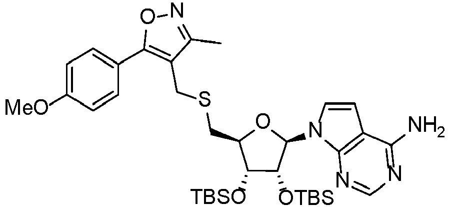

Derivative **94b** was synthesized from bromide **94a** (obtained from **65b** (0.07 g, 0.33 mmol)) and thiol **46** (provided from **6** (0.15 g, 0.27 mmol)) following the same methodology as for **47b.** FCC (10-80 % EtOAc in DCM) afforded analogue **94b** (0.13 g, 0.19 mmol, 68 %). **¹H NMR** (400 MHz, DMSO-*d₆*) δ 8.05 (1H, s), 7.68 (2H, d, *J* = 8.6 Hz), 7.32 (1H, d, *J* = 3.4 Hz), 7.09-6.94 (4H, m), 6.61 (1H, d, *J* = 3.4 Hz), 6.08 (1H, d, *J* = 6.8 Hz), 4.79 (1H, dd, *J* = 6.5, 4.5 Hz), 4.17 (1H, d, *J* = 3.2 Hz), 3.97 (1H, t, *J* = 6.4 Hz), 3.88 (1H, d, *J* = 13.2 Hz), 3.84-3.74 (4H, m), 3.04 (1H, dd, *J* = 13.6, 6.7 Hz), 2.93 (1H, dd, *J* = 13.7, 6.8 Hz), 2.25 (3H, s), 0.90, 0.68 (18H, 2×s), 0.10, -0.13, -0.41 (12H, 3×s). **¹³C NMR** (101 MHz, DMSO-*d₆*) δ 165.0, 160.7, 160.5, 157.6, 151.9, 150.8, 128.5, 122.2, 119.8, 114.7, 109.7, 103.2, 100.3, 86.9, 84.5, 74.8, 74.1, 55.5, 34.6, 25.9, 25.7, 24.7, 17.9, 17.7, 9.8, -4.45, -4.52, -4.6, -5.3. **LRMS** Calculated for C₃₅H₅₄N₅O₅SSi₂, [M+H]⁺ m/z: 712.3, [M+H]⁺, found 712.3.

### Example 98

### 7-((2R,3R,4R,5S)-3,4-bis((tert-Butyldimethylsilyl)oxy)-5-((((5-(3,4-dimethylphenyl)-3-methyl isoxazol-4-yl)methyl)thio)methyl)tetrahydrofuran-2-yl)-7H-pyrrolo[2,3-d]pyrimidin-4-amine (95b):

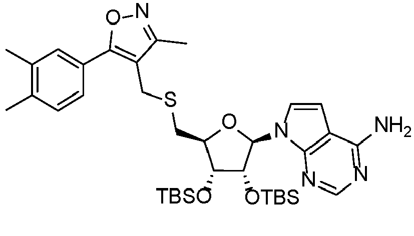

**95b** was prepared from bromide **95a** (obtained from **66b** (0.07 g, 0.33 mmol)) and thiol **46** (provided from **6** (0.15 g, 0.27 mmol)) according to the same procedure as for **47b.** FCC (10-80 % EtOAc in DCM) provided SAH analogue **95b** (0.15 g, 0.21 mmol, 76 %). **¹H NMR** (400 MHz, DMSO-*d₆*) δ 8.04 (1H, s), 7.52 (1H, d, *J* = 1.4 Hz), 7.45 (1H, dd, *J* = 7.8, 1.8 Hz), 7.29 (1H, d, *J* = 3.7 Hz), 7.22 (1H, d, *J* = 8.0 Hz), 6.99 (2H, bs), 6.59 (1H, d, *J* = 3.6 Hz), 6.07 (1H, d, *J* = 6.9 Hz), 4.76 (1H, dd, *J* = 6.9, 4.5 Hz), 4.16 (1H, dd, *J* = 4.4, 1.6 Hz), 3.97 (1H, td, *J* = 6.7, 1.4 Hz), 3.89 (1H, d, *J* = 13.3 Hz), 3.80 (1H, d, *J* = 13.3 Hz), 3.02 (1H, dd, *J* = 13.8, 6.8 Hz), 2.91 (1H, dd, *J* = 13.8, 6.9 Hz), 2.26 (3H, s), 2.26 (6H, s), 0.90, 0.68 (18H, 2×s), 0.09, -0.13, -0.41 (12H, 3×s). **¹³C NMR** (101 MHz, DMSO-*d₆*) δ 165.3, 160.5, 157.6, 152.0, 150.8, 139.0, 137.4, 130.3, 127.7, 124.9, 124.3, 122.1, 110.4, 103.1, 100.3, 86.8, 84.5, 74.8, 74.1, 34.6, 25.9, 25.7, 24.6, 19.5, 19.5, 17.9, 17.7, 9.8, -4.5, -4.6, -4.6, -5.3. **LRMS** Calculated for C₃₆H₅₆N₅O₄SSi₂, [M+H]⁺ m/z: 710.4, [M+H]⁺, found 710.4.

### Example 99

### 7-((2R,3R,4R,5S)-5-((((5-(Benzo[d][1,3]dloxol-5-yl)-3-methylisoxazol-4-yl)methyl)thio)methyl)-3,4-bis((tert-butyldimethylsilyl)oxy)tetrahydrofuran-2-yl)-7H-pyrrolo[2,3-d]pyrimidin-4-amine (96b):

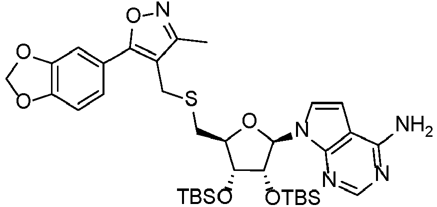

Title compound **96b** was synthesized from bromide **96a** (obtained from **67b** (0.8 g, 0.33 mmol)) and thiol **46** (provided from **6** (0.15 g, 0.27 mmol)) using the same methodology as for **47b.** FCC (10-80 % EtOAc in DCM) gave derivative **96b** (0.15 g, 0.21 mmol, 76 %). **¹H NMR** (400 MHz, DMSO-*d₆*) δ 8.04 (1H, s), 7.30 (1H, d, *J* = 3.7 Hz), 7.27-7.24 (2H, m), 7.03-7.01 (1H, m), 6.99 (2H, bs), 6.59 (1H, d, *J=* 3.6 Hz), 6.11 (2H, s), 6.07 (1H, d, *J* = 6.9 Hz), 4.78 (1H, dd, *J* = 6.9, 4.5 Hz), 4.17 (1H, dd, *J* = 4.4, 1.6 Hz), 3.97 (1H, td, *J* = 6.7, 1.5 Hz), 3.86 (1H, d, *J* = 13.3 Hz), 3.74 (1H, d, *J* = 13.3 Hz), 3.04 (1H, dd, *J* = 13.8, 6.9 Hz), 2.91 (1H, dd, *J* = 13.8, 6.8 Hz), 2.24 (3H, s), 0.91, 0.68 (18H, 2×s), 0.11, 0.10, -0.13, -0.41 (12H, 4×s). **¹³C NMR** (101 MHz, DMSO-*d₆)* δ 164.9, 160.6, 157.6, 151.9, 150.7, 149.0, 148.2, 122.2, 121.6, 121.1, 110.0, 109.1, 106.9, 103.2, 101.9, 100.3, 86.9, 84.5, 74.8, 74.1, 34.6, 25.9, 25.7, 24.6, 17.9, 17.7, 9.8, -4.5, -4.5, -4.6, -5.3. **LRMS** Calculated for C₃₅H₅₂N₅O₆SSi₂, [M+H]⁺ m/z: 726.3, [M+H]⁺, found 726.3.

### Example 100

7-((2*R*,3*R*,4*R*,5*S*)-3,4-bis((*tert*-Butyldimethylsilyl)oxy)-5-((((5-(4-fluorophenyl)-3-methylisoxazol-4- 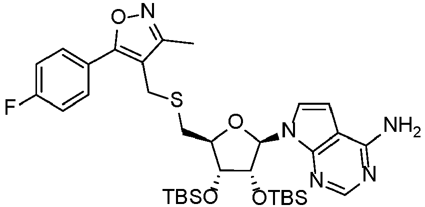 yl)methyl)thio)methyl)tetrahydrofuran-2-yl)-7H-pyrrolo[2,3-*d*]pyrimidin-4-amine (**97b**):
**97b** was prepared from bromide **97a** (obtained from **68b** (0.07 g, 0.34 mmol)) and thiol **46** (provided from **6** (0.15 g, 0.27 mmol)) in analogous fashion to **47b.** FCC (10-80 % EtOAc in DCM) afforded **97b** (0.15 g, 0.21 mmol, 77 %). **¹H NMR** (400 MHz, DMSO-*d₆*) δ 8.04 (1H, s), 7.79-7.73 (2H, m), 7.34-7.27 (3H, m), 7.00 (2H, bs), 6.60 (1H, d, *J* = 3.6 Hz), 6.06 (1H, d, *J* = 6.8 Hz), 4.75 (1H, dd, *J* = 6.7, 4.5 Hz), 4.15 (1H, dd, *J* = 4.3, 1.7 Hz), 3.94 (1H, td, *J* = 6.7, 1.5 Hz), 3.80 (1H, d, *J* = 13.8 Hz), 3.71 (1H, d, *J* = 13.8 Hz), 2.96 (1H, dd, *J* = 13.8, 7.0 Hz), 2.80 (1H, dd, *J* = 13.8, 6.5 Hz), 2.40 (3H, s), 0.90, 0.68 (18H, 2×s), 0.08, 0.08, -0.14, -0.41 (12H, 4×s). **¹³C NMR** (101 MHz, DMSO-*d₆*) δ 168.2, 163.1 (d, *J* = 247.2 Hz), 160.7, 157.6, 152.0, 150.7, 130.4 (d, *J* = 8.5 Hz), 125.5 (d, *J* = 3.2 Hz), 122.2, 116.1 (d, *J* = 21.7 Hz), 110.2, 103.2, 100.3, 87.0, 84.1, 74.7, 74.1, 34.2, 25.9, 25.7, 23.9, 17.9, 17.7, 11.0, -4.45, -4.51, -4.6, -5.3. **¹⁹F NMR** (377 MHz, DMSO-*d₆*) δ -111.32--111.47 (m). **LRMS** Calculated for C₃₄H₅₁FN₅O₄SSi₂, [M+H]⁺ m/z: 700.3, [M+H]⁺, found 700.3.

### Example 101 7-((2R,3R,4R,5S)-3,4-bis((tert-Butyldlmethylsllyl)oxy)-5-((((5-cyclohexyl-3-methyllsoxazol-4-yl) methyl)thio)methyl)tetrahydrofuran-2-yl)-7H-pyrrolo[2,3-d]pyrimidin-4-amine (98b):

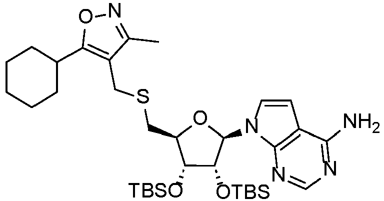

Derivative **98b** was synthesized from bromide **98a** (obtained from **69b** (0.07 g, 0.34 mmol)) and thiol **46** (provided from **6** (0.15 g, 0.27 mmol)) according to the same methodology as for **47b.** FCC (10-80 % EtOAc in DCM) furnished SAH analogue **98b** (0.14 g, 0.20 mmol, 72 %). **¹H NMR** (400 MHz, DMSO-*d₆*) δ 8.05 (1H, s), 7.31 (1H, d, *J* = 3.7 Hz), 7.01 (2H, bs), 6.61 (1H, d, *J* = 3.6 Hz), 6.08 (1H, d, *J* = 6.9 Hz), 4.80 (1H, dd, *J* = 6.9, 4.5 Hz), 4.20 (1H, dd, *J* = 4.4, 1.7 Hz), 3.99 (1H, td, *J* = 6.7, 1.6 Hz), 3.66 (1H, d, *J* = 13.3 Hz), 3.58 (1H, d, *J* = 13.5 Hz), 3.00 (1H, dd, *J* = 13.8, 7.5 Hz), 2.83-2.74 (2H, m), 2.16 (3H, s), 1.77-1.59 (5H, m), 1.53-1.40 (2H, m), 133-1.15 (3H, m), 0.93, 0.68 (18H, 2×s), 0.15, 0.12, -0.13, -0.40 (12H, 4×s). **¹³C NMR** (101 MHz, DMSO-*d₆*) δ 172.8, 159.2, 157.6, 152.0, 150.8, 122.0, 109.0, 103.1, 100.4, 86.8, 84.3, 74.7, 74.1, 35.1, 34.0, 30.8, 25.9, 25.7, 25.62, 25.57, 25.3, 23.5, 17.9, 17.7, 9.8, -4.4, -4.5, -4.6, -5.3. **LRMS** Calculated for C₃₄H₅₈N₅O₄SSi₂, [M+H]⁺ m/z: 688.4, [M+H]⁺, found 688.4.

### Example 102

### 7-((2R,3R,4R,5S)-5-((((5-Benzyl-3-methyllsoxazol-4-yl)methyl)thio)methyl)-3,4-bis((tertbutyldimethylsilyl)oxy)tetrahydrofuran-2-yl)-7H-pyrrolo[2,3-d]pyrimidin-4-amine (99b):

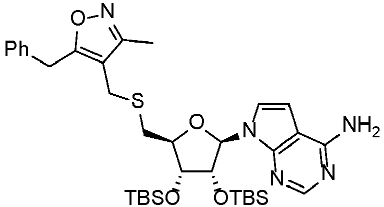

Analogue **99b** was synthesized from bromide **99a** (obtained from **70b** (0.07 g, 0.34 mmol)) and thiol **46** (provided from **6** (0.15 g, 0.27 mmol)) in analogous fashion to **47b.** FCC (10-80 % EtOAc in DCM) furnished **99b** (0.14 g, 0.21 mmol, 76 %). **¹H NMR** (400 MHz, DMSO-*d₆*) δ 8.05 (1H, s), 7.32 (1H, d, *J* = 3.7 Hz), 7.29-7.15 (5H, m), 7.00 (2H, bs), 6.62 (1H, d, *J* = 3.4 Hz), 6.08 (1H, d, *J* = 6.2 Hz), 4.78 (1H, dd, *J* = 6.7, 4.6 Hz), 4.19 (1H, dd, *J* = 4.3, 2.1 Hz), 4.04 (2H, s), 4.01 (1H, td, *J* = 6.1, 2.0 Hz), 3.70 (1H, d, *J* = 13.9 Hz), 3.64 (1H, d, *J* = 13.8 Hz), 2.95 (1H, dd, *J* = 14.0, 7.2 Hz), 2.77 (1H, dd, *J* = 13.8, 6.1 Hz), 2.19 (3H, s), 0.92, 0.69 (18H, 2×s), 0.13, 0.11, -0.12, -0.38 (12H, 4×s). **¹³C NMR** (101 MHz, DMSO-*d₆*) δ 167.6, 159.6, 157.6, 152.0, 150.8, 136.5, 128.8, 128.7, 126.9, 122.1, 111.2, 103.2, 100.4, 87.0, 84.2, 74.7, 74.2, 33.9, 30.8, 25.9, 25.7, 23.4, 17.9, 17.7, 9.9, -4.4, -4.5, -4.6, -4.9. **LRMS** Calculated for C₃₅H₅₄N₅O₄SSi₂, [M+H]⁺ m/z: 696.3, [M+H]⁺, found 696.3.

### Example 103

### 7-((2R,3R,4R,5S)-3,4-bis((tert-Butyldlmethylsllyl)oxy)-5-((((3-methyl-5-(naphthalen-1-yl)lsoxazol-4-yl)methyl)thio)methyl)tetrahydrofuran-2-yl)-7H-pyrrolo[2,3-d]pyrimidin-4-amine (100b):

**100b** was prepared from **100a** (obtained from **71b** (0.08 g, 0.34 mmol)) and thiol **46** (provided from **6** (0.15 g, 0.27 mmol)) in analogous fashion to **47b.** FCC (10-80 % EtOAc in DCM) afforded **100b** (0.16 g, 0.22 mmol, 82 %). **¹H NMR** (400 MHz, DMSO-*d₆*) δ 8.36 (1H, d, *J* = 1.1 Hz), 8.05 (1H, s), 7.99 (3H, q, *J* = 8.5 Hz), 7.84 (1H, dd, *J* = 8.5, 1.8 Hz), 7.64-7.53 (2H, m), 7.31 (1H, d, *J* = 3.7 Hz), 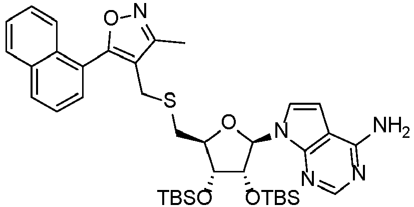 7.01 (2H, bs), 6.59 (1H, d, *J* = 3.6 Hz), 6.09 (1H, d, *J* = 7.0 Hz), 4.77 (1H, dd, *J* = 6.9, 4.5 Hz), 4.16 (1H, dd, *J* = 4.4, 1.6 Hz), 4.02 (1H, d, *J* = 13.3 Hz), 4.00 (1H, td, *J* = 6.9, 1.6 Hz), 3.93 (1H, d, *J* = 13.3 Hz), 3.07 (1H, dd, *J* = 13.7, 6.7 Hz), 2.97 (1H, dd, *J* = 13.8, 7.0 Hz), 2.31 (3H, s), 0.86, 0.67 (18H, 2×s), 0.07, 0.05, -0.14, -0.42 (12H, 4×s). **¹³C NMR** (101 MHz, DMSO-*d₆*) δ 164.9, 160.8, 157.6, 151.9, 150.8, 133.4, 132.8, 129.0, 128.8, 127.9, 127.8, 127.1, 126.8, 124.7, 123.7, 122.1, 111.4, 103.1, 100.4, 86.8, 84.4, 74.8, 74.1, 34.6, 25.9, 25.7, 24.5, 17.9, 17.7, 9.9, -4.5, -4.57, -4.62, -5.3. **LRMS** Calculated for C₃₈H₅₄N₅O₄SSi₂, [M+H]⁺ m/z: 732.3, [M+H]⁺, found 732.3.

### Example 104

### 7-((2R,3R,4R,5S)-3,4-bis((tert-Butyldimethylsilyl)oxy)-5-((((3-methyl-5-(thiazol-4-yl)isoxazol-4-yl)methyl)thio)methyl)tetrahydrofuran-2-yl)-7H-pyrrolo[2,3-d]pyrimidin-4-amine (101b):

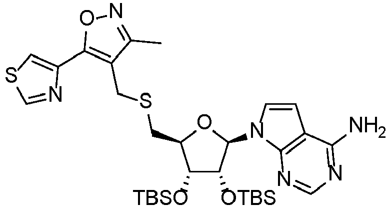

Derivative **101b** was synthesized from **101a** (obtained from **72b** (0.07 g, 0.35 mmol)) and thiol **46** (provided from **6** (0.15 g, 0.27 mmol)) in analogous fashion to **47b.** FCC (10-80 % EtOAc in DCM) provided **101b** (0.14 g, 0.21 mmol, 76 %). **¹H NMR** (400 MHz, DMSO-*d₆*) δ 9.28 (1H, d, *J* = 1.9 Hz), 8.29 (1H, d, *J* = 1.9 Hz), 8.02 (1H, s), 7.21 (1H, d, *J* = 3.7 Hz), 6.99 (2H, bs), 6.58 (1H, d, *J* = 3.6 Hz), 6.02 (1H, d, *J* = 7.1 Hz), 4.70 (1H, dd, *J* = 7.0, 4.5 Hz), 4.26 (1H, d, *J* = 13.7 Hz), 4.12 (1H, dd, *J* = 4.4, 1.2 Hz), 4.02 (1H, d, *J* = 13.8 Hz), 4.00-3.95 (1H, m), 2.97 (1H, dd, *J* = 13.7, 8.3 Hz), 2.70 (1H, dd, *J* = 13.8, 5.8 Hz), 2.32 (3H, s), 0.90, 0.66 (18H, 2×s), 0.08, -0.16, -0.45 (12H, 4×s). **¹³C NMR** (101 MHz, DMSO-*d₆*) δ 160.8, 160.0, 157.6, 156.4, 151.9, 150.7, 143.8, 121.9, 120.4, 112.0, 103.1, 100.3, 86.7, 84.0, 74.7, 74.1, 33.4, 25.9, 25.7, 23.2, 17.9, 17.7, 9.9, -4.5, -4.61, -4.64, -5.4. **LRMS** Calculated for C₃₁H₄₉N₆O₄S₂Si₂, [M+H]⁺ m/z: 689.3, [M+H]⁺, found 689.3.

### Example 105

### 7-((2R,3R,4R,5S)-5-((((4-(Benzo[d][1,3]dioxol-5-yl)-6-methylpyrimidin-5-yl)methyl)thio)methyl)-3,4 -bis((tert-butyldimethylsilyl)oxy)tetrahydrofuran-2-yl)-7H-pyrrolo[2,3-d]pyrimidin-4-amine (102b):

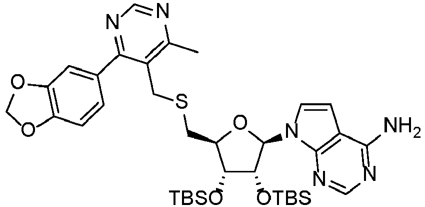

Analogue **102b** was prepared from **102a** (obtained from **75** (0.06 g, 0.25 mmol)) and thiol **46** (provided from **6** (0.16 g, 0.30 mmol)) in analogous fashion to **47b.** FCC (20-100 % acetone in cyclohexane) afforded **102b** (0.18 g, 0.24 mmol, 96 %). **¹H NMR** (400 MHz, CDCl₃) δ 8.93 (1H, s), 8.24 (1H, s), 7.23-7.20 (2H, m), 7.03 (1H, d, *J* = 3.7 Hz), 6.88-6.85 (1H, m), 6.37 (1H, d, *J* = 3.7 Hz), 6.02 (2H, s), 5.97 (1H, d, *J* = 4.8 Hz), 5.34 (2H, bs), 4.83 (1H, t, *J* = 4.7 Hz), 4.25 (1H, t, *J* = 4.4 Hz), 4.17 (1H, ddd, *J* = 6.4, 5.1, 4.2 Hz), 3.85 (1H, d, *J* = 12.1 Hz), 3.75 (1H, d, *J* = 12.1 Hz), 2.99 (1H, dd, *J* = 14.0, 5.2 Hz), 2.92 (1H, dd, *J* = 13.9, 6.4 Hz), 2.64 (3H, s), 0.93, 0.80 (18H, 2×s), 0.12, 0.11, -0.08, -0.26 (12H, 4×s). **¹³C NMR** (101 MHz, CDCl₃) δ 167.8, 165.1, 156.5, 156.4, 151.2, 150.5, 148.8, 148.0, 131.8, 127.0, 124.2, 123.4, 109.6, 108.4, 104.3, 101.6, 98.8, 90.4, 84.0, 74.7, 74.6, 36.0, 32.4, 26.0, 25.9, 22.5, 18.2, 18.1, -4.1, -4.4, -4.6, -4.9. **LRMS** Calculated for C₃₆H₅₃N₆O₅SSi₂, [M+H]⁺ m/z: 737.3, [M+H]⁺, found 737.3.

### Example 106

### 7-((2R,3R,4R,5S)-3,4-bis((tert-Butyldimethylsilyl)oxy)-5-((((5-phenylisothiazol-4-yl)methyl)thio)methyl)tetrahydrofuran-2-yl)-7H-pyrrolo[2,3-d]pyrimidin-4-amine (103b):

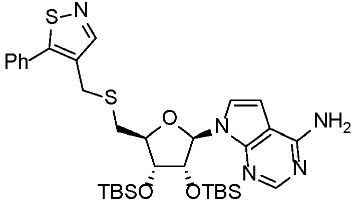

SAH analogue **103b** was synthesized from bromide **103a** (obtained from **79** (0.03 g, 0.13 mmol)) and thiol **46** (provided from **6** (0.09 g, 0.17 mmol)) using the same procedure as for **47b.** FCC (10-60 % acetone in cyclohexane) provided modified nucleoside **103b** (0.08 g, 0.12 mmol, 92 %). **¹H NMR** (400 MHz, CDCl₃) δ 8.44 (1H, s), 8.25 (1H, s), 7.49-7.40 (5H, m), 7.13 (1H, d, *J* = 3.8 Hz), 6.42 (1H, d, *J* = 3.7 Hz), 6.03 (1H, d, *J* = 4.6 Hz), 5.46 (2H, bs), 4.74 (1H, t, *J* = 4.6 Hz), 4.27 (1H, t, *J* = 4.5 Hz), 4.19 (1H, td, *J* = 5.6, 4.3 Hz), 3.84 (1H, d, *J* = 13.4 Hz), 3.81 (1H, d, *J* = 13.4 Hz), 2.99 (1H, dd, *J* = 14.1, 5.7 Hz), 2.84 (1H, dd, *J* = 14.1, 5.6 Hz), 0.92, 0.80 (18H, 2×s), 0.10, 0.10, -0.08, -0.24 (12H, 4×s). **¹³C NMR** (101 MHz, CDCl₃) δ 163.2, 159.8, 156.2, 150.8, 150.5, 131.4, 130.4, 129.4, 129.2, 128.9, 124.1, 104.2, 99.1, 90.0, 83.6, 74.9, 74.4, 34.4, 28.1, 26.0, 25.9, 18.2, 18.1, -4.1, -4.4, -4.5, -4.8. **LRMS** Calculated for C₃₃H₅₀N₅O₃S₂Si₂, [M+H]⁺ m/z: 684.3, [M+H]⁺, found 684.3.

### Example 107

### 7-((2R,3R,4R,5S)-3,4-bis((tert-Butyldimethylsilyl)oxy)-5-((((4-phenyl-1H-imidazol-5-yl)methyl)thio)methyl)tetrahydrofuran-2-yl)-7H-pyrrolo[2,3-d]pyrimidin-4-amine (104b):

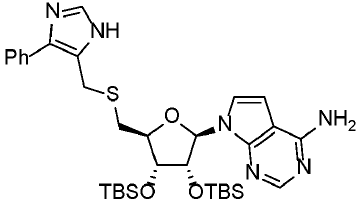

**104b** was prepared from chloride **104a** (obtained from **81** (0.06 g, 0.36 mmol)) and thiol **46** (provided from **6** (0.2 g, 0.36 mmol)) in analogous fashion to **49b.** Reverse-phase FCC (0-100 % MeCN in H₂O) afforded crude product **104b,** that was used in the next step without further purification. **LRMS** Calculated for C₃₃H₅₁N₆O₃SSi₂, [M+H]⁺ m/z: 667.3, [M+H]⁺, found 667.3.

### Example 108

### 7-((2R,3R,4R,5S)-3,4-bis((tert-Butyldimethylsilyl)oxy)-5-((((3-phenylisothiazol-4-yl)methyl)thio)methyl)tetrahydrofuran-2-yl)-7H-pyrrolo[2,3-d]pyrimidin-4-amine (105b):

Modified nucleoside **105b** was synthesized from bromide **105a** (obtained from **83** (0.07 g, 0.37 mmol)) and thiol **46** (provided from **6** (0.2 g, 0.37 mmol)) according to the same procedure as for **47b.** FCC 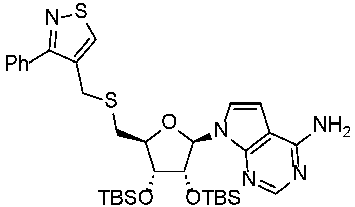 (10-60 % acetone in cyclohexane) furnished 105b (0.19 g, 0.27 mmol, 75 %). ¹H NMR (400 MHz, CDCl₃) δ 8.54 (1H, s), 8.26 (1H, s), 7.66-7.62 (2H, m), 7.43-7.39 (3H, m), 7.11 (1H, d, *J* = 3.7 Hz), 6.39 (1H, d, *J* = 3.7 Hz), 6.03 (1H, d, *J* = 4.5 Hz), 5.40 (2H, bs), 4.77 (1H, t, *J=* 4.5 Hz), 4.32 (1H, t, *J=* 4.6 Hz), 4.22 (1H, td, *J=* 5.6, 4.6 Hz), 3.84 (2H, s), 2.97 (1H, dd, *J=* 14.2, 5.4 Hz), 2.83 (1H, dd, *J=* 14.2, 5.8 Hz), 0.92, 0.81 (18H, 2×s), 0.11, 0.10, -0.07, -0.22 (12H, 4×s). **¹³C NMR** (101 MHz, CDCl₃) δ 167.6, 156.3, 151.0, 150.5, 148.0, 135.3, 133.4, 129.0, 128.64, 128.62, 124.1, 104.2, 99.0, 90.3, 83.8, 74.8, 74.5, 34.0, 29.6, 26.0, 25.9, 18.2, 18.1, -4.1, -4.4, -4.5, -4.8. **LRMS** Calculated for C₃₃H₅₀N₅O₃S₂Si₂, [M+H]⁺ m/z: 684.3, [M+H]⁺, found 684.3.

### Example 109

### 7-((2R,3R,4R,5S)-3,4-bis((tert-Butyldimethylsilyl)oxy)-5-((((5-ethyl-3-phenylisoxazol-4-yl)methyl)thio)methyl)tetrahydrofuran-2-yl)-7H-pyrrolo[2,3-d]pyrimidin-4-amine (106b):

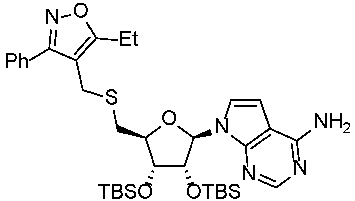

**106b** was prepared from **106a** (obtained from **84b** (0.07 g, 0.34 mmol)) and thiol **46** (provided from 6 (0.15 g, 0.27 mmol)) in analogous fashion to for **47b.** FCC (10-80 % EtOAc in DCM) afforded **106b** (0.15 g, 0.22 mmol, 80 %). **¹H NMR** (400 MHz, DMSO-*d₆*) δ 8.04 (1H, s), 7.73-7.66 (2H, m), 7.52-7.45 (3H, m), 7.30 (1H, d, *J* = 3.7 Hz), 7.01 (2H, bs), 6.60 (1H, d, *J* = 3.6 Hz), 6.06 (1H, d, *J* = 6.8 Hz), 4.76 (1H, dd, *J* = 6.8, 4.5 Hz), 4.16 (1H, dd, *J* = 4.4, 1.8 Hz), 3.96 (1H, td, *J* = 6.7, 1.7 Hz), 3.80 (1H, d, *J* = 13.6 Hz), 3.71 (1H, d, *J* = 13.6 Hz), 2.98 (1H, dd, *J* = 13.8, 6.9 Hz), 2.83 (1H, dd, *J* = 13.8, 6.7 Hz), 2.78 (2H, q, *J=* 7.6 Hz), 1.19 (3H, t, *J=* 7.6 Hz), 0.90, 0.68 (18H, 2×s), 0.09, 0.09, -0.13, -0.40 (12H, 4×s). **¹³C NMR** (101 MHz, DMSO-*d₆*) δ 172.4, 161.6, 157.6, 151.9, 150.7, 129.9, 129.0, 128.1, 122.2, 109.4, 103.2, 100.3, 86.9, 84.3, 74.8, 74.1, 34.5, 25.9, 25.7, 24.0, 18.7, 17.9, 17.7, 12.2, -4.45, -4.50, -4.6, -5.3. **LRMS** Calculated for C₃₅H₅₄N₅O₄SSi₂, [M+H]⁺ m/z: 696.3, [M+H]⁺, found 696.3.

### Example 110

### 7-((2R,3R,4R,5S)-3,4-bis((tert-Butyldimethylsiyl)oxy)-5-((((5-isopropyl-3-phenylisoxazol-4-yl)methyl)thio)methyl)tetrahydrofuran-2-yl)-7H-pyrrolo[2,3-d]pyrimidin-4-amine (107b):

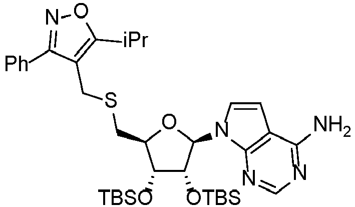

**107b** was synthesized from **107a** (obtained from **85b** (0.08 g, 0.35 mmol)) and thiol **46** (provided from **6** (0.15 g, 0.27 mmol)) in analogous fashion to **47b.** FCC (10-70 % EtOAc in DCM) provided **107b** (0.15 g, 0.22 mmol, 80 %). **¹H NMR** (400 MHz, DMSO-*d₆*) δ 8.04 (1H, s), 7.72-7.66 (2H, m), 7.52-7.45 (3H, m), 7.31 (1H, d, *J* = 3.7 Hz), 7.00 (2H, bs), 6.60 (1H, d, *J* = 3.6 Hz), 6.07 (1H, d, *J* = 6.9 Hz), 4.78 (1H, dd, *J* = 6.9, 4.5 Hz), 4.17 (1H, dd, *J* = 4.4, 1.8 Hz), 3.97 (1H, td, *J* = 6.7, 1.7 Hz), 3.80 (1H, d, *J* = 13.4 Hz), 3.70 (1H, d, *J* = 13.4 Hz), 3.22 (1H, p, *J* = 7.0 Hz), 3.00 (1H, dd, *J* = 13.8, 6.7 Hz), 2.86 (1H, dd, *J* = 13.8, 6.8 Hz), 1.25 (3H, d, *J* = 7.0 Hz), 1.23 (3H, d, *J* = 7.0 Hz), 0.91, 0.68 (18H, 2×s), 0.10, 0.10, -0.13, -0.40 (12H, 4×s). **¹³C NMR** (101 MHz, DMSO-*d₆*) δ 175.3, 161.7, 157.6, 152.0, 150.8, 129.9, 129.04, 129.00, 128.1, 122.2, 108.4, 103.2, 100.3, 86.9, 84.5, 74.8, 74.1, 34.7, 25.9, 25.8, 25.7, 24.1, 20.9, 20.9, 17.9, 17.7, -4.5, -4.5, -4.6, -5.3. **LRMS** Calculated for C₃₆H₅₆N₅O₄SSi₂, [M+H]⁺ m/z: 710.4, [M+H]⁺, found 710.4.

### Example 111

### 7-((2R,3R,4R,5S)-3,4-bis((tert-Butyldimethylsilyl)oxy)-5-((((5-cyclopropyl-3-phenylisoxazol-4-yl)methyl)thio)methyl)tetrahydrofuran-2-yl)-7H-pyrrolo[2,3-d]pyrimidin-4-amine (108b):

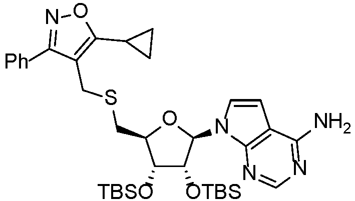

**108b** was prepared from bromide **108a** (obtained from **86b** (0.08 g, 0.35 mmol)) and thiol **46** (provided from **6** (0.15 g, 0.27 mmol)) following the same methodology as for **47b.** FCC (10-80 % EtOAc in DCM) gave (0.15 g, 0.21 mmol, 78 %). **¹H NMR** (400 MHz, DMSO-*d₆*) δ 8.04 (1H, s), 7.71-7.67 (2H, m), 7.52-7.46 (3H, m), 7.30 (1H, d, *J* = 3.7 Hz), 7.01 (2H, bs), 6.60 (1H, d, *J* = 3.6 Hz), 6.06 (1H, d, *J* = 6.8 Hz), 4.76 (1H, dd, *J* = 6.7, 4.5 Hz), 4.17 (1H, dd, *J* = 4.4, 1.9 Hz), 3.99 (1H, td, *J* = 6.7, 2.0 Hz), 3.85 (1H, d, *J* = 13.7 Hz), 3.79 (1H, d, *J* = 13.7 Hz), 3.01 (1H, dd, *J* = 13.8, 6.9 Hz), 2.87 (1H, dd, *J* = 13.8, 6.6 Hz), 2.23-2.14 (1H, m), 1.04-0.98 (4H, m), 0.90, 0.68 (18H, 2×s), 0.09, -0.13, -0.40 (12H, 4×s). **¹³C NMR** (101 MHz, DMSO-*d₆*) δ 171.3, 161.9, 157.6, 152.0, 150.7, 129.9, 129.03, 128.96, 128.1, 122.1, 109.9, 103.1, 100.3, 86.9, 84.3, 74.8, 74.1, 34.4, 25.9, 25.7, 24.0, 17.9, 17.7, 8.0, 7.2, -4.5, -4.5, -4.6, -5.3. **LRMS** Calculated for C₃₆H₅₄N₅O₄SSi₂, [M+H]⁺ m/z: 708.3, [M+H]⁺, found 708.3.

### Example 112

### 7-((2R,3R,4R,5S)-3,4-bis((tert-Butyldimethylsily)oxy)-5-((((3,5-diphenylisoxazol-4-yl)methyl)thio)methyl)tetrahydrofuran-2-yl)-7H-pyrrolo[2,3-d]pyrimidin-4-amine (109b):

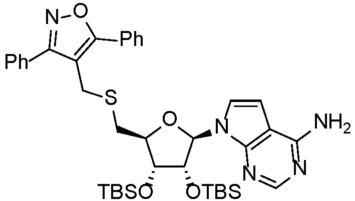

Modified nucleoside **109b** was synthesized from bromide **109a** (obtained from **89** (0.09 g, 0.34 mmol)) and thiol **46** (provided from **6** (0.15 g, 0.27 mmol)) in analogous fashion to **47b.** FCC (10-80 % EtOAc in DCM) provided **109b** (0.17 g, 0.23 mmol, 83 %). **¹H NMR** (400 MHz, DMSO-*d₆*) δ 8.02 (1H, s), 7.91-7.85 (2H, m), 7.83-7.77 (2H, m), 7.58-7.49 (6H, m), 7.29 (1H, d, *J* = 3.7 Hz), 6.98 (2H, bs), 6.57 (1H, d, *J* = 3.6 Hz), 6.07 (1H, d, *J* = 7.1 Hz), 4.76 (1H, dd, *J* = 7.0, 4.5 Hz), 4.13 (1H, dd, *J* = 4.4, 1.5 Hz), 4.00 (1H, d, *J* = 13.1 Hz), 3.96-3.91 (1H, m), 3.87 (1H, d, *J* = 13.0 Hz), 3.04 (1H, dd, *J* = 13.8, 6.2 Hz), 2.98 (1H, dd, *J* = 13.8, 7.7 Hz), 0.89, 0.67 (18H, 2×s), 0.08, 0.06, -0.13, -0.42 (12H, 4×s). **¹³C NMR** (101 MHz, DMSO-*d₆*) δ 166.8, 163.1, 157.6, 151.9, 150.8, 130.7, 130.2, 129.4, 129.1, 128.6, 128.3, 127.2, 127.1, 122.2, 110.2, 103.2, 100.3, 86.8, 84.5, 74.9, 74.0, 35.2, 25.9, 25.7, 25.3, 17.9, 17.7, -4.5, -4.5, -4.6, -5.3. **LRMS** Calculated for C₃₉H₅₄N₅O₄SSi₂, [M+H]⁺ m/z: 744.3, [M+H]⁺, found 744.3.

### Example 113

### 7-((2R,3R,4R,5S)-3,4-bis((tert-Butyldimethylsilyl)oxy)-5-((((4-methoxy-6-phenylpyrimidin-5-yl) methyl)thio)methyl)tetrahydrofuran-2-yl)-7H-pyrrolo[2,3-d]pyrimidin-4-amine (110b):

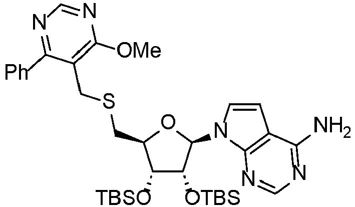

Title compound **110b** was prepared from bromide **110a** (obtained from **92** (0.04 g, 0.20 mmol)) and thiol **46** (provided from **6** (0.14 g, 0.24 mmol)) in analogous fashion to **47b.** FCC (10-70 % acetone in cyclohexane) afforded **110b** (0.13 g, 0.19 mmol, 92 %). **¹H NMR** (400 MHz, CDCl₃) δ 8.72 (1H, s), 8.24 (1H, s), 7.66-7.61 (2H, m), 7.47-7.42 (3H, m), 7.08 (1H, d, *J* = 3.7 Hz), 6.36 (1H, d, *J* = 3.7 Hz), 6.00 (1H, d, *J* = 5.2 Hz), 5.36 (2H, bs), 4.77 (1H, t, *J* = 4.9 Hz), 4.22 (1H, t, *J* = 4.1 Hz), 4.14 (1H, td, *J* = 6.0, 3.7 Hz), 4.07 (3H, s), 3.77 (1H, d, *J* = 12.6 Hz), 3.72 (1H, d, *J* = 12.6 Hz), 3.04 (1H, dd, *J* = 13.9, 6.3 Hz), 2.89 (1H, dd, *J* = 13.9, 5.6 Hz), 0.92, 0.78 (18H, 2×s), 0.10, 0.09, -0.10, -0.28 (12H, 4×s). **¹³C NMR** (101 MHz, CDCl₃) δ 168.4, 165.3, 156.3, 156.2, 151.0, 150.6, 137.7, 129.4, 129.0, 128.6, 124.1, 116.9, 104.3, 98.8, 89.9, 83.8, 74.8, 74.6, 54.6, 36.3, 29.0, 26.0, 25.9, 18.2, 18.1, -4.2, -4.4, -4.6, -4.9. **LRMS** Calculated for C₃₅H₅₃N₆O₄SSi₂, [M+H]⁺ m/z: 709.3, [M+H]⁺, found 709.3.

### Example 114

### (2R,3R,4S,5S)-2-(4-Amino-7H-pyrrolo[2,3-d]pyrimidin-7-yl)-5-((((3-methyl-5-(4-nitrophenyl)isoxazol-4-yl)methyl)thio)methyl)tetrahydrofuran-3,4-diol (93c):

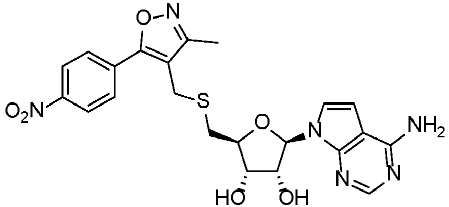

SAH analogue **93c** was prepared from derivative **93b** (0.24 g, 0.33 mmol) following the same procedure as for **47c.** Reverse-phase FCC (5-60 % MeCN in H₂O) provided final compound **93c** (0.14 g, 0.29 mmol, 88 %). **¹H NMR** (400 MHz, DMSO-*d₆*) δ 8.36-8.30 (2H, m), 8.05 (1H, s), 8.04-7.98 (2H, m), 7.30 (1H, d, *J* = 3.7 Hz), 7.00 (2H, bs), 6.57 (1H, d, *J* = 3.6 Hz), 6.07 (1H, d, *J* = 5.7 Hz), 5.40 (1H, d, *J* = 6.2 Hz), 5.28 (1H, d, *J=* 3.3 Hz), 4.47 (1H, q, *J* = 5.7 Hz), 4.08-3.99 (2H, m), 3.92 (1H, d, *J* = 13.2 Hz), 3.87 (1H, d, *J* = 13.2 Hz), 2.94 (1H, dd, *J* = 14.0, 5.3 Hz), 2.86 (1H, dd, *J* = 14.0, 6.8 Hz), 2.29 (3H, s). **¹³C NMR** (101 MHz, DMSO-*d₆*) δ 162.6, 161.3, 157.6, 152.1, 150.6, 148.0, 132.8, 128.0, 124.5, 121.8, 113.9, 103.0, 100.3, 87.2, 83.0, 73.3, 72.9, 34.7, 24.1, 9.7. **HRMS** (ESI) Calculated for C₂₂H₂₃N₆O₆S, [M+H]⁺ m/z: 499.1394, [M+H]⁺, found 499.1390.

### Example 115

### (2R,3R,4S,5S)-2-(4-Amino-7H-pyrrolo[2,3-d]pyrimidin-7-yl)-5-((((5-(4-methoxyphenyl)-3-methylisoxazol-4-yl)methyl)thio)methyl)tetrahydrofuran-3,4-diol (94c):

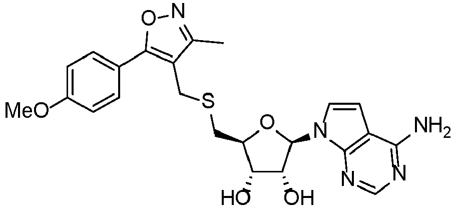

Title compound **94c** was prepared from protected analogue **94b** (0.11 g, 0.16 mmol) in analogous fashion to **47c.** FCC (10-60 % MeCN in H₂O) afforded **94c** (0.07 g, 0.15 mmol, 94 %). **¹H NMR** (400 MHz, DMSO-*d₆*) δ 8.06 (1H, s), 7.70 (2H, d, *J* = 8.2 Hz), 7.30 (1H, d, *J* = 3.3 Hz), 7.06 (2H, d, *J* = 8.4 Hz), 7.02 (2H, bs), 6.60 (1H, d, *J* = 3.3 Hz), 6.08 (1H, d, *J* = 5.3 Hz), 5.39 (1H, d, *J=* 5.4 Hz), 5.26 (1H, d, *J* = 4.2 Hz), 4.47 (1H, q, *J* = 5.8 Hz), 4.13-3.97 (2H, m), 3.82 (1H, d, *J* = 14.2 Hz), 3.78 (1H, d, *J* = 14.2 Hz), 2.94 (1H, dd, *J* = 13.3, 4.5 Hz), 2.86 (1H, dd, *J* = 13.7, 6.7 Hz), 2.24 (3H, s). **¹³C NMR** (101 MHz, DMSO-*d₆*) δ 165.0, 160.7, 160.6, 157.7, 152.0, 150.6, 128.4, 121.8, 119.8, 114.8, 109.7, 103.0, 100.3, 87.1, 83.1, 73.3, 72.9, 55.5, 34.8, 24.6, 9.8. **HRMS** (ESI) Calculated for C₂₃H₂₆N₅O₅S, [M+H]⁺ m/z: 484.1649, [M+H]⁺, found 484.1648.

### Example 116

### (2R,3R,4S,5S)-2-(4-Amino-7H-pyrrolo[2,3-d]pyrimidin-7-yl)-5-((((5-(3,4-dimethylphenyl)-3-methylisoxazol-4-yl)methyl)thio)methyl)tetrahydrofuran-3,4-diol (95c):

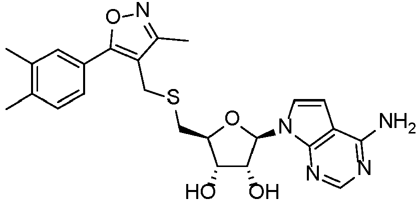

Derivative **95c** was synthesized from modified nucleoside **95b** (0.14 g, 0.19 mmol) using the same procedure as for **47c.** Reverse-phase FCC (5-60 % MeCN in H₂O) furnished SAH analogue **95c** (0.09 g, 0.18 mmol, 94 %). **¹H NMR** (400 MHz, DMSO-*d₆*) δ 8.06 (1H, s), 7.53 (1H, d, *J* = 1.5 Hz), 7.47 (1H, dd, *J* = 7.8, 1.5 Hz), 7.28 (1H, d, *J* = 3.7 Hz), 7.24 (1H, d, *J* = 7.9 Hz), 7.01 (2H, bs), 6.59 (1H, d, *J* = 3.6 Hz), 6.08 (1H, d, *J* = 5.7 Hz), 5.38 (1H, d, *J* = 6.2 Hz), 5.25 (1H, d, *J* = 5.1 Hz), 4.45 (1H, q, *J* = 5.7 Hz), 4.08-3.98 (2H, m), 3.84 (1H, d, *J* = 13.0 Hz), 3.79 (1H, d, *J* = 13.0 Hz), 2.94 (1H, dd, *J* = 13.9, 5.2 Hz), 2.85 (1H, dd, *J* = 13.9, 6.8 Hz), 2.26 (3H, s), 2.26 (3H, s), 2.25 (3H, s). **¹³C NMR** (101 MHz, DMSO-*d₆*) δ 165.2, 160.7, 157.7, 152.0, 150.6, 139.0, 137.5, 130.4, 127.6, 124.9, 124.3, 121.7, 110.5, 103.0, 100.3, 87.1, 83.2, 73.4, 72.9, 34.9, 24.6, 19.6, 19.5, 9.8. **HRMS** (ESI) Calculated for C₂₄H₂₈N₅O₄S, [M+H]⁺ m/z: 482.1857, [M+H]⁺, found 482.1853.

### Example 117

### (2R,3R,4S,5S)-2-(4-Amino-7H-pyrrolo[2,3-d]pyrimidin-7-yl)-5-((((5-(benzo[d][1,3]dioxol-5-yl)-3-methylisoxazol-4-yl)methyl)thio)methyl)tetrahydrofuran-3,4-diol (96c):

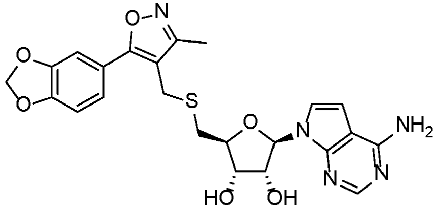

Modified nucleoside **96c** was prepared from protected analogue **96b** (0.13 g, 0.18 mmol) following the same methodology as for **47c.** Reverse-phase FCC (10-60 % MeCN in H₂O) provided **96c** (0.08 g, 0.15 mmol, 86 %). **¹H NMR** (400 MHz, DMSO-*d₆*) δ 8.06 (1H, s), 7.32-7.22 (3H, m), 7.05 (1H, d, *J* = 8.7 Hz), 7.02 (2H, bs), 6.59 (1H, *d, J=* 3.6 Hz), 6.12 (2H, s), 6.07 (1H, d, *J* = 5.6 Hz), 5.38 (1H, d, *J* = 6.1 Hz), 5.24 (1H, d, *J* = 5.2 Hz), 4.46 (1H, q, *J* = 5.7 Hz), 4.05 (1H, q, *J* = 4.9 Hz), 4.03- 3.98 (1H, m), 3.81 (1H, d, *J* = 13.1 Hz), 3.76 (1H, d, *J* = 13.1 Hz), 2.94 (1H, dd, *J* = 13.9, 5.3 Hz), 2.85 (1H, dd, *J* = 13.9, 6.9 Hz), 2.23 (3H, s). **¹³C NMR** (101 MHz, DMSO-*d₆*) δ 164.8, 160.7, 157.7, 152.0, 150.6, 149.0, 148.2, 121.8, 121.6, 121.1, 110.1, 109.2, 106.9, 103.0, 101.9, 100.3, 87.2, 83.1, 73.3, 72.9, 34.9, 24.5, 9.7. **HRMS** (ESI) Calculated for C₂₃H₂₄N₅O₆S, [M+H]⁺ m/z: 498.1442, [M+H]⁺, found 498.1438.

### Example 118

### (2R,3R,4S,5S)-2-(4-Amino-7H-pyrrolo[2,3-d]pyrimidin-7-yl)-5-((((5-(4-fluorophenyl)-3-methylisoxazol-4-yl)methyl)thio)methyl)tetrahydrofuran-3,4-diol (97c):

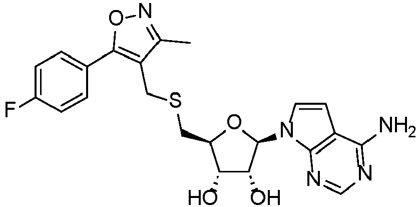

SAH analogue **97c** was synthesized from protected derivative **97b** (0.14 g, 0.19 mmol) according to the same procedure as for **47c.** Reverse-phase FCC (10-60 % MeCN in H₂O) afforded modified nucleoside **97c** (0.08 g, 0.17 mmol, 86 %). **¹H NMR** (400 MHz,

DMSO-*d₆*) δ 8.06 (1H, s), 7.77 (2H, dd, *J* = 8.7, 5.5 Hz), 7.33 (2H, t, *J* = 8.8 Hz), 7.27 (1H, d, *J* = 3.6 Hz), 7.04 (2H, bs), 6.60 (1H, d, *J* = 3.6 Hz), 6.05 (1H, d, *J* = 5.5 Hz), 5.38 (1H, d, *J* = 6.1 Hz), 5.23 (1H, d, *J* = 5.3 Hz), 4.43 (1H, q, *J* = 5.2 Hz), 4.03 (1H, q, *J* = 4.9 Hz), 3.97 (1H, q, *J* = 5.3 Hz), 3.73 (1H, d, *J* = 13.9 Hz), 3.70 (1H, d, *J* = 13.9 Hz), 2.86 (1H, dd, *J* = 13.9, 5.2 Hz), 2.73 (1H, dd, *J* = 14.0, 6.8 Hz), 2.38 (3H, s). **¹³C NMR** (101 MHz, DMSO-*d₆*) δ 168.3, 163.1 (d, *J* = 247.1 Hz), 160.8, 157.6, 151.9, 150.6, 130.4 (d, *J* = 8.6 Hz), 125.6 (d, *J* = 3.1 Hz), 121.8, 116.2 (d, *J* = 21.7 Hz), 110.3, 103.0, 100.4, 87.2, 82.9, 73.3, 72.9, 34.3, 23.8, 10.9. **¹⁹F NMR** (377 MHz, DMSO-*d₆*) δ -111.27-111.45 (m). **HRMS** (ESI) Calculated for C₂₂H₂₃FN₅O₄S, [M+H]⁺ m/z: 472.1449, [M+H]⁺, found 472.1447.

### Example 119

### (2R,3R,4S,5S)-2-(4-Amino-7H-pyrrolo[2,3-d]pyrimidin-7-yl)-5-((((5-cyclohexyl-3-methylisoxazol-4-yl)methyl)thio)methyl)tetrahydrofuran-3,4-diol (98c):

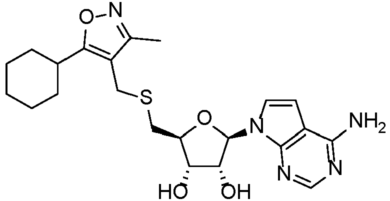

Title compound **98c** was prepared from modified nucleoside **98b** (0.12 g, 0.18 mmol) using the same methodology as for **47c.** Reverse-phase FCC (10-60 % MeCN in H₂O) gave SAH analogue **98c** (0.07 g, 0.15 mmol, 85 %). **¹H NMR** (400 MHz, DMSO-*d₆*) δ 8.07 (1H, s), 7.32 (1H, d, *J* = 3.7 Hz), 7.07 (2H, bs), 6.63 (1H, d, *J* = 3.7 Hz), 6.07 (1H, d, *J* = 4.5 Hz), 5.39 (1H, d, *J* = 6.3 Hz), 5.23 (1H, d, *J* = 5.3 Hz), 4.47 (1H, q, *J* = 4.1 Hz), 4.17-4.03 (1H, m), 4.03-3.92 (1H, m), 3.60 (1H, d, *J* = 13.5 Hz), 3.54 (1H, d, *J* = 13.5 Hz), 2.81 (1H, dd, *J* = 13.3, 4.8 Hz), 2.82-2.72 (1H, m), 2.81 (1H, dd, *J* = 13.9, 6.0 Hz), 2.15 (3H, s), 1.81-1.53 (5H, m), 1.53-1.35 (2H, m), 135-0.99 (3H, m). **¹³C NMR** (101 MHz, DMSO-*d₆*) δ 172.9, 159.3, 157.5, 151.8, 150.6, 121.7, 109.1, 103.0, 100.4, 87.1, 83.1, 73.4, 72.7, 34.9, 33.8, 30.88, 30.86, 25.6, 25.5, 25.4, 23.3, 9.8. **HRMS** (ESI) Calculated for C₂₂H₃₀N₅O₄S, [M+H]⁺ m/z: 460.2013, [M+H]⁺, found 460.2009.

### Example 120

### (2R,3R,4S,5S)-2-(4-Amino-7H-pyrrolo[2,3-d]pyrimidin-7H-yl)-5-((((5-benzyl-3-methylisoxazol-4-yl)methyl)thio)methyl)tetrahydrofuran-3,4-diol (99c):

SAH analogue **99c** was synthesized from derivative **99b** (0.13 g, 0.19 mmol) using the same procedure 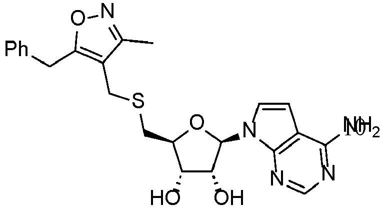 as for **47c.** Reverse-phase FCC (5-60 % MeCN in H₂O) furnished modified nucleoside **99c** (0.08 g, 0.16 mmol, 86 %). **¹H NMR** (400 MHz, DMSO-*d₆*) δ 8.08 (1H, s), 7.32 (1H, d, *J* = 3.7 Hz), 7.28-7.15 (5H, m), 7.13 (2H, bs), 6.64 (1H, d, *J* = 3.7 Hz), 6.08 (1H, d, *J* = 5.9 Hz), 5.41 (1H, bs), 5.26 (1H, bs), 4.47-4.39 (1H, m), 4.10-4.02 (2H, m), 4.01 (2H, s), 3.65 (1H, d, *J* = 13.8 Hz), 3.61 (1H, d, *J* = 13.8 Hz), 2.82 (1H, dd, *J* = 14.0, 5.1 Hz), 2.68 (1H, dd, *J* = 14.0, 6.5 Hz), 2.17 (3H, s). **¹³C NMR** (101 MHz, DMSO-*d₆*) δ 167.6, 159.7, 157.4, 151.7, 150.5, 136.6, 128.8, 128.7, 126.9, 121.9, 111.2, 103.0, 100.6, 87.3, 83.2, 73.4, 72.7, 33.8, 30.8, 23.3, 9.9. **HRMS** (ESI) Calculated for C₂₃H₂₆N₅O₄S, [M+H]⁺ m/z: 468.1700, [M+H]⁺, found 468.1696.

### Example 121

### (2R,3R,4S,5S)-2-(4-Amino-7H-pyrrolo[2,3-d]pyrimidin-7-yl)-5-((((3-methyl-5-(naphthalen-1-yl)isoxazol-4-yl)methyl)thio)methyl)tetrahydrofuran-3,4-diol (100c):

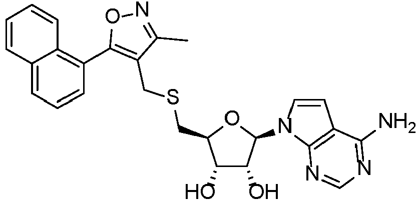

Title compound **100c** was prepared from **100b** (0.15 g, 0.21 mmol) according to the same methodology as for **47c.** Reverse-phase FCC (10-70 % MeCN in H₂O) afforded **100c** (0.09 g, 0.18 mmol, 87 %). **¹H NMR** (400 MHz, DMSO-*d₆*) δ 8.37 (1H, s), 8.07 (1H, s), 8.02 (2H, d, *J* = 8.3 Hz), 7.96 (1H, d, *J* = 7.8 Hz), 7.86 (1H, dd, *J* = 8.6, 1.4 Hz), 7.66-7.54 (2H, m), 7.30 (1H, d, *J* = 3.6 Hz), 7.02 (2H, bs), 6.59 (1H, d, *J* = 3.6 Hz), 6.11 (1H, d, *J* = 5.7 Hz), 5.40 (1H, d, *J* = 6.2 Hz), 5.27 (1H, d, *J* = 4.9 Hz), 4.48 (1H, q, *J* = 5.7 Hz), 4.13-4.03 (2H, m), 3.97 (1H, d, *J* = 13.0 Hz), 3.92 (1H, d, *J* = 13.0 Hz), 3.00 (1H, dd, *J* = 13.8, 4.8 Hz), 2.90 (1H, dd, *J* = 13.8, 6.6 Hz), 2.30 (3H, s). **¹³C NMR** (101 MHz, DMSO-*d₆*) δ 164.8, 161.0, 157.6, 152.0, 150.6, 133.4, 132.8, 129.1, 128.9, 127.84, 127.75, 127.2, 126.7, 124.7, 123.6, 121.7, 111.5, 103.0, 100.3, 87.2, 83.1, 73.4, 73.0, 34.9, 24.5, 9.8. **HRMS** (ESI) Calculated for C₂₆H₂₆N₅O₄S, [M+H]⁺ m/z: 504.1700, [M+H]⁺, found 504.1697.

### Example 122

### (2R,3R,4S,5S)-2-(4-Amino-7H-pyrrolo[2,3-d]pyrimidin-7-y1)-5-((((3-methyl-5-(thiazol-4-y1)isoxazo1-4-yl)methyl)thio)methyl)tetrahydrofuran-3,4-diol (101c):

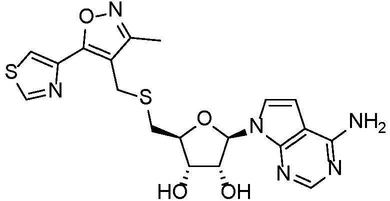

**101c** was synthesized from **101b** (0.13 g, 0.19 mmol) following the same procedure as for **47c.** Reverse-phase FCC (5-50 % MeCN in H₂O) provided **101c** (0.05 g, 0.10 mmol, 52 %). **¹H NMR** (400 MHz, DMSO-*d₆*) δ 9.31 (1H, d, *J* = 1.3 Hz), 8.29 (1H, d, *J* = 1.3 Hz), 8.04 (1H, s), 7.25 (1H, d, *J* = 3.4 Hz), 7.01 (2H, bs), 6.58 (1H, d, *J* = 3.3 Hz), 6.02 (1H, d, *J* = 5.8 Hz), 5.33 (1H, d, *J* = 6.1 Hz), 5.18 (1H, d, *J* = 4.4 Hz), 4.43 (1H, q, *J* = 5.4 Hz), 4.11 (1H, d, *J* = 13.5 Hz), 4.06 (1H, d, *J* = 13.5 Hz), 4.03-3.91 (2H, m), 2.85 (1H, dd, *J* = 13.6, 5.7 Hz), 2.71 (1H, dd, *J* = 13.6, 6.3 Hz), 2.30 (3H, s). **¹³C NMR** (101 MHz, DMSO-*d₆*) δ 160.8, 159.9, 157.7, 156.4, 152.0, 150.6, 143.8, 121.7, 120.3, 112.3, 103.0, 100.3, 86.9, 82.9, 73.2, 72.8, 34.2, 23.5, 9.9. **HRMS** (ESI) Calculated for C₁₉H₂₁N₆O₄S₂, [M+H]⁺ m/z: 461.1060, [M+H]⁺, found 461.1057.

### Example 123

### (2R,3R,4S,5S)-2-(4-Amino-7H-pyrrolo[2,3-d]pyrimidin-7-yl)-5-((((4-(benzo[d][1,3]dioxol-5-yl)-6-methylpyrimidin-5-yl)methyl)thio)methyl)tetrahydrofuran-3,4-diol (102c):

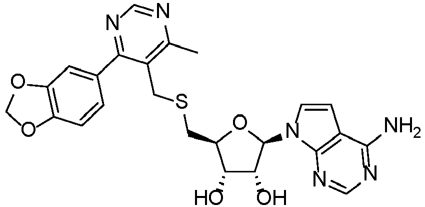

Derivative **102c** was prepared from modified nucleoside **102b** (0.17 g, 0.23 mmol) using the same methodology as for **47c.** Reverse-phase FCC (0-50 % MeCN in H₂O) gave SAH analogue **102c** (0.1 g, 0.20 mmol, 89 %). **¹H NMR** (400 MHz, DMSO-*d₆*) δ 8.90 (1H, s), 8.10 (1H, s), 7.30 (1H, d, *J* = 3.7 Hz), 7.28 (2H, bs), 7.26 (1H, dd, *J* = 8.0, 1.8 Hz), 7.23 (1H, d, *J* = 1.7 Hz), 7.02 (1H, d, *J* = 8.0 Hz), 6.64 (1H, d, *J* = 3.6 Hz), 6.12 (1H, d, *J* = 1.0 Hz), 6.11 (1H, d, *J* = 1.0 Hz), 6.05 (1H, d, *J* = 5.7 Hz), 5.39 (1H, br d, *J* = 6.0 Hz), 5.25 (1H, br d, *J* = 5.2 Hz), 4.43 (1H, q, *J* = 5.0 Hz), 4.02 (1H, q, *J* = 4.5 Hz), 3.94 (1H, ddd, *J* = 7.2, 5.2, 4.0 Hz), 3.88 (1H, d, *J* = 12.2 Hz), 3.82 (1H, d, *J* = 12.1 Hz), 2.95 (1H, dd, *J* = 14.0, 5.2 Hz), 2.84 (1H, dd, *J* = 14.0, 7.2 Hz), 2.61 (3H, s). **¹³C NMR** (101 MHz, DMSO-*d₆*) δ 167.4, 163.8, 156.5, 156.0, 150.5, 150.0, 148.3, 147.4, 126.6, 123.0, 122.0, 109.1, 108.1, 102.7, 101.5, 100.5, 87.0, 82.8, 73.2, 72.7, 35.5, 31.0, 22.0. **HRMS** (ESI) Calculated for C₂₄H₂₅N₆O₅S, [M+H]⁺ m/z: 509.1602, [M+H]⁺, found 509.1606.

### Example 124

### (2R,3R,4S,5S)-2-(4-Amino-7H-pyrrolo[2,3-d]pyrimidin-7-yl)-5-((((5-phenylisothlazol-4-yl)methyl)thio)methyl)tetrahydrofuran-3,4-diol (103c):

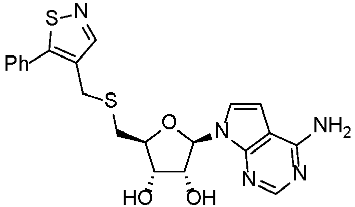

SAH analogue **103c** was synthesized from modified nucleoside **103b** (0.07 g, 0.11 mmol) according to the same procedure as for **47c.** Reverse-phase FCC (0-70 % MeCN in H₂O) furnished derivative **103c** (0.03 g, 0.06 mmol, 56 %). **¹H NMR** (400 MHz, DMSO-*d₆*) δ 8.54 (1H, s), 8.07 (1H, s), 7.58-7.53 (2H, m), 7.53-7.44 (3H, m), 7.28 (1H, d, *J* = 3.7 Hz), 7.07 (2H, bs), 6.61 (1H, d, *J* = 3.6 Hz), 6.05 (1H, d, *J* = 5.5 Hz), 5.38 (1H, bs), 5.24 (1H, bs), 4.43 (1H, t, *J i=* 5.7 Hz), 4.05 (1H, t, *J* = 5.1 Hz), 3.96 (1H, dt, *J* = 7.0, 5.0 Hz), 3.90 (2H, s), 2.92 (1H, dd, *J* = 13.9, 5.2 Hz), 2.80 (1H, dd, *J* = 13.9, 6.9 Hz). **¹³C NMR** (101 MHz, DMSO-*d₆*) δ 161.8, 160.5, 157.4, 151.7, 150.4, 132.0, 129.7, 129.5, 129.4, 128.4, 121.7, 102.8, 100.2, 87.1, 82.8, 73.2, 72.6, 34.6, 26.8. **HRMS** (ESI) Calculated for C₂₁H₂₂N₅O₃S, [M+H]⁺ m/z: 456.1159, [M+H]⁺, found 456.1163.

### Example 125

### (2R,3R,4S,5S)-2-(4-Amino-7H-pyrrolo[2,3-d]pyrimidin-7-yl)-5-((((4-phenyl-1H-imidazol-5-yl) methyl)thio)methyl)tetrahydrofuran-3,4-diol (104c):

**104c** was prepared from **104b** (0.06 g, 0.08 mmol) in analogous fashion to **47c.** Reverse-phase FCC (0-40 % MeCN in H₂O) afforded **104c** (0.02 g, 0.03 mmol, 41 %). **¹H NMR** (400 MHz, MeOD) δ 8.11 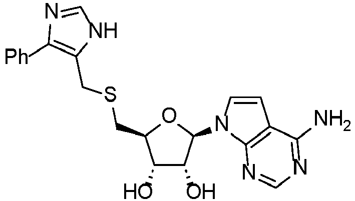 (1H, bs), 7.83 (1H, bs), 7.58-7.52 (2H, m), 7.39-7.32 (2H, m), 7.31-7.24 (2H, m), 6.64 (1H, d, *J* = 3.7 Hz), 6.13 (1H, d, *J* = 5.1 Hz), 4.47 (1H, t, *J* = 5.3 Hz), 4.19 (1H, t, *J* = 4.9 Hz), 4.13 (1H, q, *J* = 5.2 Hz), 3.98 (1H, d, *J* = 13.8 Hz), 3.93 (1H, d, *J* = 13.8 Hz), 2.96 (1H, dd, *J* = 14.2, 5.0 Hz), 2.87 (1H, dd, *J* = 14.1, 5.8 Hz). **¹³C NMR** (101 MHz, MeOD) δ 157.9, 151.0, 150.9, 136.0, 133.6, 132.6, 129.8, 129.7, 128.6, 128.4, 123.6, 104.6, 101.8, 89.3, 85.0, 75.4, 74.0, 35.4, 29.0. **HRMS** (ESI) Calculated for C₂₁H₂₃N₆O₃S, [M+H]⁺ m/z: 439.1547, [M+H]⁺, found 439.1545.

### Example 126

### (2R,3R,4S,5S)-2-(4-Amino-7H-pyrrolo[2,3-d]pyrimidin-7-yl)-5-((((3-phenylisothiazol-4-yl)methyl)thio)methyl)tetrahydrofuran-3,4-diol (105c):

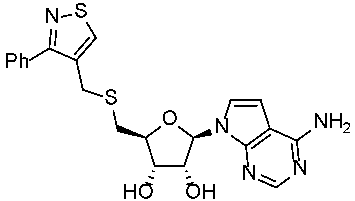

**105c** was synthesized from **105b** (0.18 g, 0.26 mmol) using the same procedure as for **47c.** Reverse-phase FCC (0-60 % MeCN in H₂O) provided derivative **105c** (0.11 g, 0.24 mmol, 94 %). **¹H NMR** (400 MHz, DMSO-*d₆*) δ 8.92 (1H, s), 8.09 (1H, s), 7.71-7.64 (2H, m), 7.49-7.41 (3H, m), 7.31 (1H, d, *J* = 3.7 Hz), 7.22 (2H, bs), 6.64 (1H, d, *J* = 3.7 Hz), 6.05 (1H, d, *J* = 5.5 Hz), 5.38 (1H, bs), 5.24 (1H, bs), 4.43 (1H, q, *J* = 4.6 Hz), 4.04 (1H, q, *J* = 4.0 Hz), 3.97 (1H, dt, *J* = 6.7, 4.8 Hz), 3.90 (2H, s), 2.89 (1H, dd, *J* = 13.9, 5.2 Hz), 2.71 (1H, dd, *J* = 13.6, 6.3 Hz). **¹³C NMR** (101 MHz, DMSO-*d₆*) δ 166.6, 156.8, 150.9, 150.1, 135.0, 133.4, 128.8, 128.5, 128.2, 121.9, 102.7, 100.4, 87.1, 83.0, 73.2, 72.7, 34.2, 28.5. **HRMS** (ESI) Calculated for C₂₁H₂₂N₅O₃S₂, [M+H]⁺ m/z: 456.1159, [M+H]⁺, found 456.1159.

### Example 127

### (2R,3R,4S,5S)-2-(4-Amino-7H-pyrrolo[2,3-d]pyrimidin-7-yl)-5-((((5-ethyl-3-phenyllsoxazol-4-yl)methyl)thio)methyl)tetrahydrofuran-3,4-diol (106c):

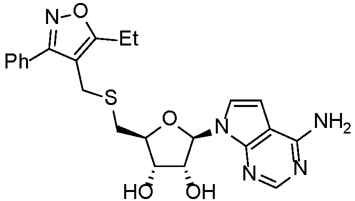

Derivative **106c** was prepared from **106b** (0.14 g, 0.20 mmol) according to the same methodology as for **47c.** Reverse-phase FCC (10-60 % MeCN in H₂O) furnished SAH analogue **106c** (0.08 g, 0.18 mmol, 90 %). **¹H NMR** (400 MHz, DMSO-*d₆*) δ 8.08 (1H, bs), 7.76-7.66 (2H, m), 7.54-7.44 (3H, m), 7.27 (1H, d, *J* = 3.5 Hz), 7.05 (2H, bs), 6.60 (1H, d, *J* = 3.4 Hz), 6.06 (1H, d, *J* = 5.4 Hz), 5.38 (1H, d, *J* = 5.9 Hz), 5.23 (1H, d, *J* = 5.2 Hz), 4.43 (1H, q, *J* = 5.4 Hz), 4.07-3.93 (2H, m), 3.73 (1H, d, *J* = 14.1 Hz), 3.70 (1H, d, *J* = 14.4 Hz), 2.87 (1H, dd, *J* = 13.9, 5.0 Hz), 2.81-2.69 (3H, m), 1.17 (3H, t, *J* = 7.5 Hz). **¹³C NMR** (101 MHz, DMSO-*d₆*) δ 172.4, 161.6, 157.6, 151.9, 150.6, 129.9, 129.1, 128.1, 121.8, 109.5, 100.4, 87.3, 83.1, 73.3, 72.8, 34.6, 23.9, 18.6, 12.2. **HRMS** (ESI) Calculated for C₂₃H₂₆N₅O₄S, [M+H]⁺ m/z: 468.1700, [M+H]⁺, found 468.1699.

### Example 128

### (2R,3R,4S,5S)-2-(4-Amino-7H-pyrrolo[2,3-d]pyrimidin-7-yl)-5-((((5-isopropyl-3-phenylisoxazol-4-yl)methyl)thio)methyl)tetrahydrofuran-3,4-diol (107c):

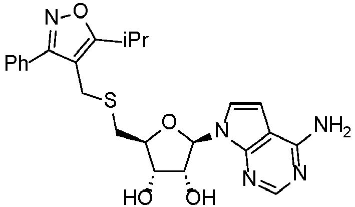

SAH analogue **107c** was synthesized from derivative **107b** (0.14 g, 0.20 mmol) following the same procedure as for **47c.** Reverse-phase FCC (10-70 % MeCN in H₂O) providided title compound **107c** (0.08 g, 0.17 mmol, 84%). **¹H NMR** (400 MHz, DMSO-*d₆*) δ 8.07 (1H, s), 7.76-7.66 (2H, m), 7.56-7.44 (3H, m), 7.27 (1H, d, *J* = 3.7 Hz), 7.05 (2H, bs), 6.60 (1H, d, *J* = 3.7 Hz), 6.06 (1H, d, *J* = 4.9 Hz), 5.39 (1H, d, *J* = 4.6 Hz), 5.23 (1H, d, *J* = 4.9 Hz), 4.45 (1H, q, *J* = 4.5 Hz), 4.07-4.01 (1H, m), 4.01-3.95 (1H, m), 3.73 (1H, d, *J* = 13.9 Hz), 3.70 (1H, d, *J* = 13.9 Hz), 3.19 (1H, p, *J* = 6.8 Hz), 2.88 (1H, dd, *J* = 13.7, 4.3 Hz), 2.78 (1H, dd, *J* = 13.6, 6.8 Hz), 1.23 (3H, d, *J* = 6.6 Hz), 1.21 (3H, d, *J* = 6.6 Hz). **¹³C NMR** (101 MHz, DMSO-*d₆*) δ 175.3, 161.7, 157.6, 151.9, 150.6, 129.9, 129.1, 129.0, 128.1, 121.8, 108.4, 103.0, 100.4, 87.3, 83.2, 73.3, 72.9, 34.8, 25.7, 24.0, 20.93, 20.89. **HRMS** (ESI) Calculated for C₂₄H₂₈N₅O₄S, [M+H]⁺ m/z: 482.1857, [M+H]⁺, found 482.1852.

### Example 129

### (2R,3R,4S,5S)-2-(4-Amino-7H-pyrrolo[2,3-d]pyrimidin-7-yl)-5-((((5-cyclopropyl-3-phenylisoxazol-4-yl)methyl)thio)methyl)tetrahydrofuran-3,4-diol (108c):

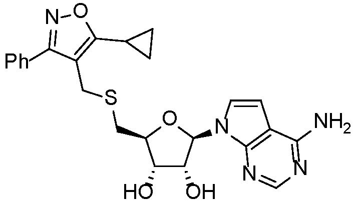

Title compound **108c** was prepared from protected analogue **108b** (0.14 g, 0.20 mmol) using the same methodology as for **47c.** Reverse-phase FCC (10-70 % MeCN in H₂O) to obtain **108c** (0.08 g, 0.16 mmol, 82 %). **¹H NMR** (400 MHz, DMSO-*d₆)* δ 8.06 (1H, s), 7.76-7.65 (2H, m), 7.57-7.43 (3H, m), 7.27 (1H, d, *J* = 3.6 Hz), 7.04 (2H, bs), 6.60 (1H, d, *J* = 3.5 Hz), 6.05 (1H, d, *J* = 5.4 Hz), 5.39 (1H, d, *J* = 6.0 Hz), 5.22 (1H, d, *J* = 5.3 Hz), 4.44 (1H, q, *J* = 5.5 Hz), 4.05 (1H, q, *J* = 5.0 Hz), 4.00 (1H, q, *J* = 5.1 Hz), 3.78 (2H, s), 2.91 (1H, dd, *J* = 14.0, 4.9 Hz), 2.79 (1H, dd, *J* = 14.0, 6.8 Hz), 2.15 (1H, p, *J* = 6.8 Hz), 1.05-0.94 (4H, m). **¹³C NMR** (101 MHz, DMSO-*d₆*) δ 171.3, 161.9, 157.6, 152.0, 150.6, 129.9, 129.1, 129.0, 128.1, 121.8, 110.0, 102.9, 100.3, 87.3, 83.1, 73.3, 72.9, 34.5, 23.9, 8.0, 7.2. **HRMS** (ESI) Calculated for C₂₄H₂₆N₅O₄S, [M+H]⁺ m/z: 480.1700, [M+H]⁺, found 480.1696.

### Example 130

### (2R,3R,4S,5S)-2-(4-Amino-7H-pyrrolo[2,3-d]pyrimidin-7-yl)-5-((((3,5-diphenylisoxazol-4-yl)methyl)thio)methyl)tetrahydrofuran-3,4-diol (109c):

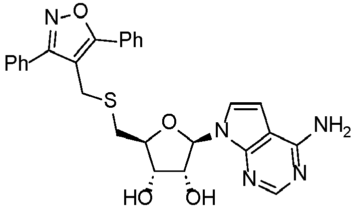

**109c** was synthesized from **109b** (0.16 g, 0.21 mmol) in analogous fashion to **47c.** Reverse-phase FCC (10-70 % MeCN in H₂O) furnished **109c** (0.09 g, 0.17 mmol, 81 %). **¹H NMR** (400 MHz, DMSO-*d₆*) δ 8.06 (1H, s), 7.96-7.88 (2H, m), 7.88-7.80 (2H, m), 7.64-7.48 (6H, m), 7.25 (1H, d, *J* = 3.4 Hz), 7.01 (2H, bs), 6.57 (1H, d, *J* = 3.4 Hz), 6.08 (1H, d, *J* = 5.7 Hz), 5.40 (1H, d, *J* = 6.0 Hz), 5.26 (1H, d, *J* = 4.5 Hz), 4.45 (1H, q, *J* = 5.1 Hz), 4.08-3.97 (2H, m), 3.93 (1H, d, *J* = 12.7 Hz), 3.86 (1H, d, *J* = 12.7 Hz), 2.99 (1H, dd, *J* = 13.8, 4.4 Hz), 2.90 (1H, dd, *J* = 13.8, 7.0 Hz). **¹³C NMR** (101 MHz, DMSO-*d₆*) δ 166.8, 163.1, 157.6, 152.0, 150.6, 130.7, 130.2, 129.5, 129.3, 128.5, 128.3, 127.12, 127.06, 121.7, 110.2, 103.0, 100.3, 87.1, 82.9, 73.3, 73.0, 34.5, 25.4. **HRMS** (ESI) Calculated for C₂₇H₂₆N₅O₄S, [M+H]⁺ m/z: 517.1700, [M+H]⁺, found 516.1695.

### Example 131

### (2R,3R,4S,5S)-2-(4-Amino-7H-pyrrolo[2,3-d]pyrimidin-7-yl)-5-((((4-methoxy-6-phenylpyrimidin-5-yl)methyl)thio)methyl)tetrahydrofuran-3,4-diol (110c):

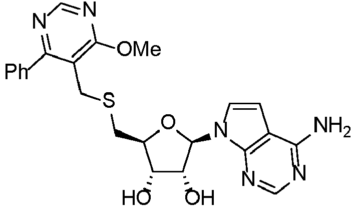

Derivative **110c** was prepared from SAH analogue **110b** (0.12 g, 0.17 mmol) following the same methodology as for **47c.** Reverse-phase FCC (0-50 % MeCN in H₂O) afforded modified nucleoside **110c** (0.07 g, 0.14 mmol, 79 %). **¹H NMR** (400 MHz, DMSO-*d₆*) δ 8.73 (1H, s), 8.06 (1H, s), 7.67-7.62 (2H, m), 7.52-7.47 (3H, m), 7.25 (1H, d, *J* = 3.7 Hz), 7.11 (2H, bs), 6.61 (1H, d, *J* = 3.7 Hz), 6.02 (1H, d, *J* = 5.7 Hz), 5.35 (1H, d, *J* = 6.2 Hz), 5.21 (1H, d, *J* = 5.2 Hz), 4.40 (1H, q, *J* = 5.4 Hz), 4.03-3.97 (4H, m), 3.90 (1H, ddd, *J* = 6.6, 5.4, 4.0 Hz), 3.71 (2H, s), 2.90 (1H, dd, *J* = 13.9, 5.5 Hz), 2.77 (1H, dd, *J* = 13.9, 6.7 Hz). **¹³C NMR** (101 MHz, DMSO-*d₆*) δ 167.7, 164.4, 157.2, 155.9, 151.4, 150.3, 137.3, 129.3, 128.7, 128.3, 121.7, 116.3, 102.8, 100.2, 86.9, 82.7, 73.2, 72.5, 54.4, 35.8, 27.8. **HRMS** (ESI) Calculated for C₂₃H₂₅N₆O₄S, [M+H]⁺ m/z: 481.1653, [M+H]⁺, found 481.1650.

### Example 132

### (2R,3R,4S,5S)-2-(4-Amino-7H-pyrrolo[2,3-d]pyrimidin-7-yl)-5-((((5-(4-aminophenyl)-3-methylisoxazol-4-yl)methyl)thio)methyl)tetrahydrofuran-3,4-diol (111):

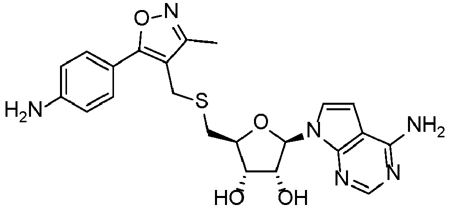

SAH analogue **111** was synthesized from nitro derivative **93c** (0.08 g, 0.15 mmol) according to the similar methodology as **6.** FCC (10-60 % MeCN in H₂O) furnished **111** (0.05 g, 0.11 mmol, 75 %). **¹H NMR** (400 MHz, DMSO-*d₆*) δ 8.07 (1H, s), 7.44 (2H, d, *J* = 8.6 Hz), 7.30 (1H, d, *J* = 3.6 Hz), 7.01 (2H, bs), 6.65 (2H, d, *J* = 8.6 Hz), 6.60 (1H, d, *J* = 3.6 Hz), 6.07 (1H, d, *J* = 5.7 Hz), 5.66 (2H, bs), 5.38 (1H, d, *J* = 5.7 Hz), 5.27 (1H, d, *J* = 4.1 Hz), 4.47 (1H, q, *J* = 5.2 Hz), 4.16-3.97 (2H, m), 3.78 (1H, d, *J* = 13.0 Hz), 3.74 (1H, d, *J* = 13.0 Hz), 2.95 (1H, dd, *J* = 13.8, 5.5 Hz), 2.85 (1H, dd, *J* = 13.8, 6.7 Hz), 2.19 (3H, s). **¹³C NMR** (101 MHz, DMSO-*d₆*) δ 166.1, 160.4, 157.7, 152.1, 150.8, 150.6, 128.0, 121.8, 114.5, 113.9, 107.7, 103.0, 100.3, 87.2, 83.2, 73.3, 72.9, 34.9, 25.0, 9.8. **HRMS** (ESI) Calculated for C₂₂H₂₅N₆O₄S, [M+H]⁺ m/z: 469.1653, [M+H]⁺, found 469.1648.

The effect of introducing a third substituent to the core of the 5'-substituent was studied on the pyrimidine and imidozole derivatives **119c** and **120c,** respectively. Hetarylmethanol **114** was prepared from ester **112** (Han, B., et al., Tetrahedron 2011/67, 5615-5620) by reduction with DIBAL and subsequent Liebeskind-Srogl coupling reaction with 3,4-dimethoxyboronic acid. Imidazole derivative **118** was prepared in three steps from a dibromo analogue **115** (Yamauchi, T., et al., J Org Chem 2014/79, 7185-7192). First, the ester function was installed by deprotonation with *n*-BuLi and subsequent reaction with EtO(CO)CN. Then, the second bromine was exchanged for a phenyl group using Suzuki-Miyaura cross coupling with phenylboronic acid affording **117.** Resulting ester was reduced with DIBAL to provide hetarylmethanol **118.** Obtained alcohols **114** and **118** were condensed with freshly prepared thiol **46** following the same conditions as mentioned above (**Scheme 3**). Final deprotection by the treatment with HF-TEA furnished trisubstituted derivatives **119c** and **120c** (**Scheme 6**).

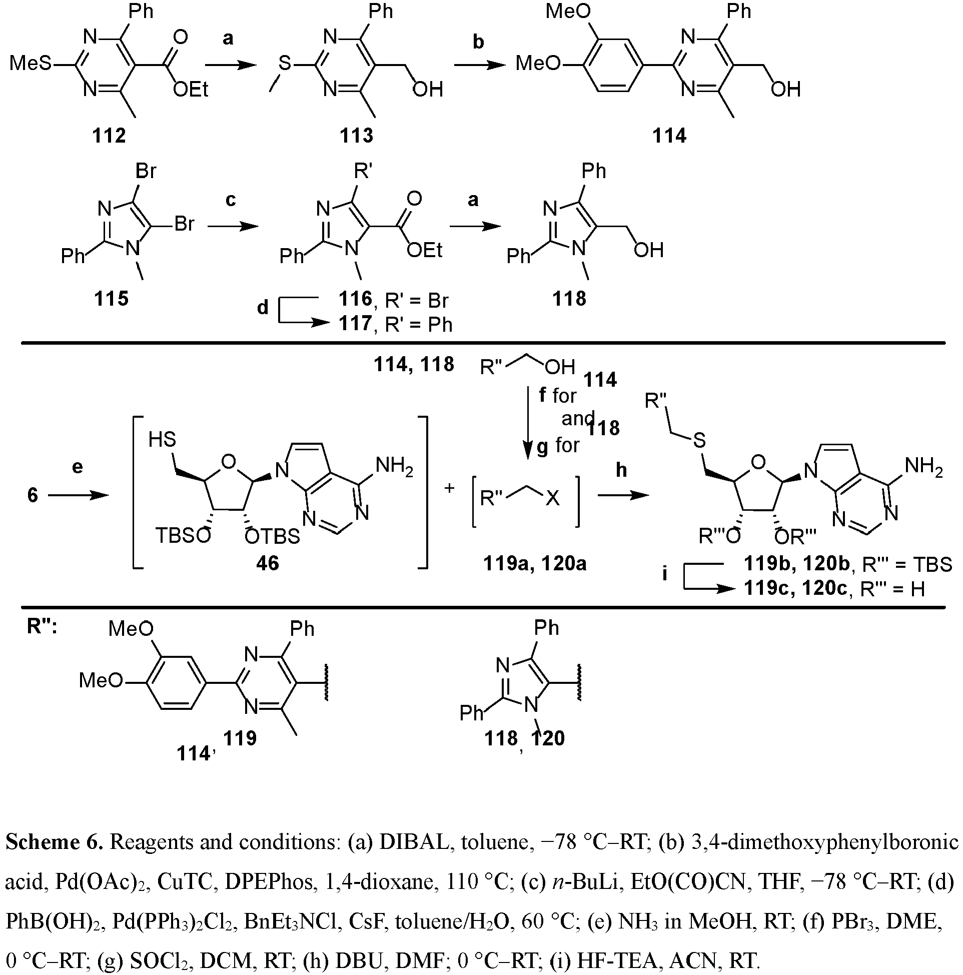

### Example 133

### (4-Methyl-2-(methylthio)-6-phenylpyrimidin-5-yl)methanol (113):

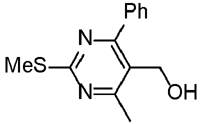

Pyrimidine derivative **113** was prepared from ester **112** (5 g, 17.34 mmol) in analogous fashion to **64b.** FCC (10-60 % EtOAc in hexane) gave title compound **113** (3.25 g, 13.19 mmol, 76 %). **¹H NMR** (400 MHz, DMSO-*d₆*) δ 7.77-7.71 (2H, m), 7.53-7.49 (3H, m), 5.31 (1H, t, *J* = 4.6 Hz), 4.41 (2H, d, *JJ* 4.6 Hz), 2.59 (3H, s), 2.52 (3H, s). **¹³C NMR** (101 MHz, DMSO-*d₆*) δ 169.0, 168.9, 164.8, 137.4, 129.5, 129.2, 128.2, 124.6, 56.8, 21.8, 13.5. **LRMS** Calculated for C₁₃H₁₅N₂OS, [M+H]⁺ m/z: 247.1, [M+H]⁺, found 247.2.

### Example 134

### (2-(3,4-Dimethoxyphenyl)-4-methyl-6-phenylpyrimidin-5-yl)methanol (114):

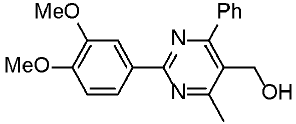

Under argon atmosphere, ester **113** (0.1 g, 0.41 mmol), 3,4-dimethoxyphenylboronic acid (0.15 g, 0.81 mmol), Pd(OAc)₂ (0.01 g, 0.02 mmol), CuTC (0.16 g, 0.81 mmol), DPEPhos (0.01 mL, 0.02 mmol) were added into a dried Schlenk tube. Then 1,4-dioxane (2 mL) was added and the reaction mixture was stirred under argon atmosphere at 110°C for 24 h. The mixture was cooled down to RT, adsorbed on silica and purified by FCC (20-80 % EtOAc in hexane) to give the pyrimidine derivative **114** (0.13 g, 0.38 mmol, 94 %). **¹H NMR** (400 MHz, DMSO-*d₆*) δ 8.05 (1H, dd, *J=* 8.4, 2.0 Hz), 7.98 (1H, d, *J* = 1.9 Hz), 7.84-7.80 (2H, m), 7.57-7.52 (3H, m), 7.08 (1H, d, *J* = 8.6 Hz), 5.33 (1H, t, *J* = 4.6 Hz), 4.49 (2H, d, *J* = 4.1 Hz), 3.84 (3H, s), 3.83 (3H, s), 2.71 (3H, s). **¹³C NMR** (101 MHz, DMSO-*d₆*) δ 168.5, 164.7, 161.0, 151.2, 148.6, 138.1, 129.9, 129.3, 128.2, 126.3, 121.2, 111.4, 110.7, 57.0, 55.6, 55.5, 22.2. **LRMS** Calculated for C₂₀H₂₁N₂O₃, [M+H]⁺ m/z: 337.2, [M+H]⁺, found 337.1.

### Example 135

### Ethyl 4-bromo-1-methyl-2-phenyl-1H-imidazole-5-carboxylate (116):

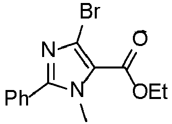

A solution of 1.6M *n*-BuLi in n-hexane (1.74 mL, 2.79 mmol) was added dropwise to a solution of dibromo derivative **115** (0.8 g, 2.53 mmol) in dry THF (5 mL) under an argon atmosphere at -78 °C. The mixture was stirred at -60 °C for 1 h, and EtO(CO)CN (0.5 mL, 5.06 mmol) was then added and the temperature was slowly increased to RT. After 2 h, the reaction was quenched with H₂O (100 mL) and extracted with EtOAc (2×200 mL). The organic layer was dried over Na₂SO₄, filtered, and concentrated in vacuo. FCC (0-20 % EtOAc in cyclohexane) provided title compound **116** (0.52 g, 1.68 mmol, 66 %). **¹H NMR** (400 MHz, CDCl₃) δ 7.64-7.59 (2H, m), 7.53-7.48 (3H, m), 4.42 (2H, q, *J* = 7.2 Hz), 3.94 (3H, s), 1.45 (3H, t, *J* = 7.1 Hz). **¹³C NMR** (101 MHz, DMSO-*d₆*) δ 160.1, 151.8, 130.2, 129.6, 128.8, 128.7, 124.2, 121.9, 61.2, 35.6, 14.4. **LRMS** Calculated for C₁₃H₁₄BrN₂O₂, [M+H]⁺ m/z: 309.0, [M+H]⁺, found 309.2.

### Example 136

### Ethyl 1-methyl-2,4-diphenyl-1H-imidazole-5-carboxylate (117):

A deaerated mixture of **116** (0.1 g, 0.32 mmol), PhB(OH)₂ (0.08 g, 0.65 mmol), Pd(PPh₃)₂Cl₂ (0.01 g, 0.02 mmol), BnEt₃NCl (0.01 g, 0.02 mmol), and CsF (0.15 g, 0.97 mmol) in toluene (1.5 mL) and H₂O 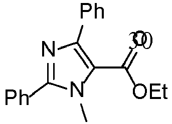 (1.5 mL) was vigorously stirred at 60°C under an atmosphere of argon for 48 h. The reaction mixture was then cooled to RT, diluted with H₂O (20 mL) and extracted with EtOAc (2×50 mL). Pooled organic layers were washed with brine, dried over Na₂SO₄, and concentrated. FCC (0-30 % EtOAc in cyclohexane) gave the **117** (0.09 g, 0.31 mmol, 95 %). **¹H NMR** (400 MHz, CDCl₃) δ 7.72-7.65 (4H, m), 7.52-7.47 (3H, m), 7.41-7.33 (3H, m), 4.26 (2H, q, *J* = 7.1 Hz), 3.94 (3H, s), 1.20 (3H, t, *J* = 7.1 Hz). **¹³C NMR** (101 MHz, DMSO-*d₆*) δ 161.4, 151.5, 149.0, 134.6, 129.9, 129.82, 129.76, 129.7, 128.8, 128.2, 127.7, 120.3, 60.7, 35.2, 14.1. **LRMS** Calculated for C₁₉H₁₉N₂O₂, [M+H]⁺ m/z: 307.1, [M+H]⁺, found 307.2.

### Example 137

### (1-Methyl-2,4-diphenyl-1H-imidazol-5-yl)methanol (118):

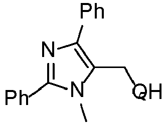

**118** was synthesized from **117** (0.09 g, 0.28 mmol) in analogous fashion to **64b.** FCC (20-100 % EtOAc in cyclohexane) furnished **118** (0.03 g, 0.11 mmol, 41 %). **¹H NMR** (400 MHz, CDCl₃) δ 7.74-7.68 (4H, m), 7.55-7.46 (3H, m), 7.45-7.40 (2H, m), 7.32-7.27 (1H, m), 5.32 (1H, t, *J* = 5.0 Hz), 4.62 (2H, d, *J* = 5.0 Hz), 3.75 (3H, s). **¹³C NMR** (101 MHz, DMSO-*d₆*) δ 146.6, 138.2, 134.7, 130.5, 129.4, 129.3, 128.8, 128.7, 128.6, 128.4, 127.2, 126.6, 52.6, 52.5, 32.0. **LRMS** Calculated for C₁₇H₁₇N₂O, [M+H]⁺ m/z: 265.3, [M+H]⁺, found 265.4.

### Example 138

### 7-((2R,3R,4R,5S)-3,4-bis((tert-Butyldimethylsilyl)oxy)-5-((((2-(3,4-dimethoxyphenyl)-4-methyl-6-phenylpyrimidin-5-yl)methyl)thio)methyl)tetrahydrofuran-2-yl)-7H-pyrrolo[2,3-d]pyrimidin-4-amine (119b):

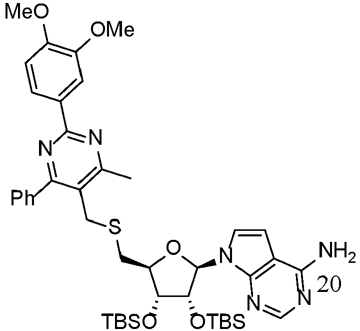

**119b** was prepared from bromide **119a** (obtained from **114** (0.06 g, 0.18 mmol)) and thiol **46** (provided from **6** (0.12 g, 0.21 mmol)) in analogous fashion to **47b.** FCC (10-80 % acetone in cyclohexane) afforded **119b** (0.14 g, 0.16 mmol, 91 %). **¹H NMR** (400 MHz, CDCl₃) δ 8.23 (1H, s), 8.11 (1H, dd, *J* = 8.4, 2.0 Hz), 8.04 (1H, d, *J* = 2.0 Hz), 7.78-7.74 (2H, m), 7.50-7.45 (3H, m), 7.05 (1H, d, *J* = 3.7 Hz), 6.93 (1H, d, *J* = 8.5 Hz), 6.37 (1H, d, *J* = 3.7 Hz), 5.98 (1H, d, *J* = 5.0 Hz), 5.40 (2H, bs), 4.79 (1H, t, *J* = 4.8 Hz), 4.22 (1H, t, *J* = 4.3 Hz), 4.14 (1H, td, *J* = 5.7, 3.9 Hz), 3.98 (3H, s), 3.94 (3H, s), 3.86 (1H, d, *J* = 12.2 Hz), 3.78 (1H, d, *J* = 12.2 Hz), 2.96 (1H, dd, *J* = 13.8, 5.5 Hz), 2.85 (1H, dd, *J* = 13.8, 5.9 Hz). 2.72 (3H, s). 0.92, 0.79 (18H, 2×s), 0.11, 0.10, -0.09, -0.28 (12H, 4×s). **¹³C NMR** (101 MHz, CDCl₃) δ 167.7, 165.8, 161.9, 156.2, 151.3, 150.8, 150.5, 149.0, 138.7, 130.8, 129.3, 129.2, 128.4, 124.2, 123.9, 121.8, 111.1, 110.8, 104.3, 98.9, 90.1, 84.0, 74.7, 56.13, 56.07, 35.9, 32.3, 26.0, 25.9, 22.8, 18.2, 18.1, -4.2, -4.4, -4.5, -4.8. **LRMS** Calculated for C₄₃H₆₁N₆O₅SSi₂, [M+H]⁺ m/z: 829.4, [M+H]⁺, found 829.4.

### Example 139

### 7-((2R,3R,4R,5S)-3,4-bis((tert-Butyldimethylsilyl)oxy)-5-((((1-methyl-2,4-diphenyl-1H-imidazol-5-yl)methyl)thio)methyl)tetrahydrofuran-2-yl)-7H-pyrrolo[2,3-d]pyrimidin-4-amine (120b):

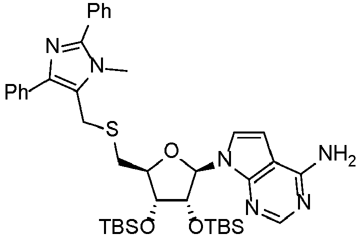

SAH analogue **120b** was prepared from chloride **120a** (obtained from 118 (0.03 g, 0.11 mmol)) and thiol **46** (provided from **6** (0.08 g, 0.14 mmol)) following the same methodology as for **47b.** FCC (2-10 % EtOH in DCM) provided derivative **120b** (0.05 g, 0.06 mmol, 58 %). **¹H NMR** (400 MHz, CDCl₃) δ 8.25 (1H, s), 7.78-7.70 (2H, m), 7.64-7.58 (2H, m), 7.48-7.42 (3H, m), 7.42-7.36 (3H, m), 7.05 (1H, d, *J* = 3.7 Hz), 6.35 (1H, d, *J* = 3.7 Hz), 6.01 (1H, d, *J* = 4.9 Hz), 5.35 (2H, bs), 4.80 (1H, t, *J* = 4.4 Hz), 4.26-4.20 (2H, m), 3.72 (1H, d, *J* = 14.1 Hz), 3.71 (1H, d, *J* = 14.1 Hz), 3.04-2.93 (2H, m), 0.93, 0.80 (18H, 2×s), 0.11, -0.08, -0.26 (12H, 3×s). **LRMS** Calculated for C₄₀H₅₇N₆O₃SSi₂, [M+H]⁺ m/z: 757.4, [M+H]⁺, found 757.4.

### Example 140

### (2R,3R,4S,5S)-2-(4-Amino-7H-pyrrolo[2,3-d]pyrimidin-7-yl)-5-((((2-(3,4-dimethoxyphenyl)-4-methyl-6-phenylpyrimidin-5-yl)methyl)thio)methyl)tetrahydrofuran-3,4-diol (119c):

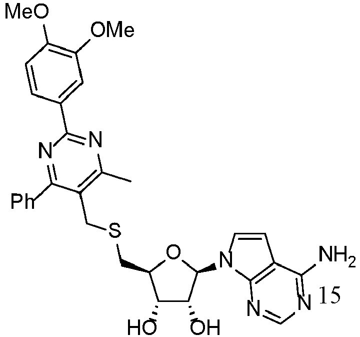

Derivative **119c** was synthesized from **119b** (0.13 g, 0.15 mmol) according to the same procedure as for **47c.** FCC (0-70 % MeCN in H₂O) provided modified nucleoside **119c** (0.07 g, 0.11 mmol, 72 %). **¹H NMR** (400 MHz, DMSO-*d₆*) δ 8.09 (1H, s), 8.00 (1H, dd, *J* = 8.5, 2.0 Hz), 7.93 (1H, d, *J* = 2.0 Hz), 7.79-7.74 (2H, m), 7.56-7.51 (3H, m), 7.26 (1H, d, *J* = 3.7 Hz), 7.16 (2H, bs), 7.07 (1H, d, *J* = 8.6 Hz), 6.62 (1H, d, *J* = 3.7 Hz), 6.04 (1H, d, *J* = 5.7 Hz), 5.38 (1H, d, *J* = 6.1 Hz), 5.25 (1H, d, *J* = 5.2 Hz), 4.44 (1H, q, *J* = 5.4 Hz), 4.03 (1H, q, *J* = 4.6 Hz), 3.94 (1H, dt, *J* = 7.0, 4.5 Hz), 3.87 (1H, d, *J* = 12.2 Hz), 3.82 (3H, s), 3.82 (1H, d, *J* = 12.2 Hz), 2.94 (1H, dd, *J* = 13.9, 5.3 Hz), 2.83 (1H, dd, *J* = 14.0, 7.0 Hz). 2.70 (3H, s). **¹³C NMR** (101 MHz, DMSO-*d₆*) δ 167.7, 164.8, 160.7, 157.0, 151.20, 151.17, 150.2, 148.6, 138.0, 129.6, 129.3, 128.7, 128.4, 124.0, 121.8, 121.2, 111.4, 110.6, 102.8, 100.3, 86.4, 82.8, 73.1, 72.7, 55.6, 55.5, 35.6, 31.0, 22.3. **HRMS** (ESI) Calculated for C₃₁H₃₃N₆O₅S, [M+H]⁺ m/z: 601.2228, [M+H]⁺, found 601.2231.

### Example 141

### (2R,3R,4S,5S)-2-(4-Amino-7H-pyrrolo[2,3-d]pyrimidin-7-yl)-5-((((1-methyl-2,4-diphenyl-1H-imidazol-5-yl)methyl)thio)methyl)tetrahydrofuran-3,4-diol (120c):

Title compound **120c** was prepapred from derivative **120b** (0.04 g, 0.05 mmol) according to the same procedure as for **47c.** Reverse-phase FCC (0-70 % MeCN in H₂O) furnished derivative **120c** (0.02 g, 0.04 mmol, 81 %). **¹H NMR** (400 MHz, MeOD) δ 8.09 (1H, s), 7.68-7.63 (2H, m), 7.62-7.57 (2H, m), 7.53-7.46 (3H, m), 7.40-7.34 (2H, m), 7.30-7.25 (1H, m), 7.23 (1H, d, *J* = 3.8 Hz), 6.61 (1H, d, *J* = 3.8 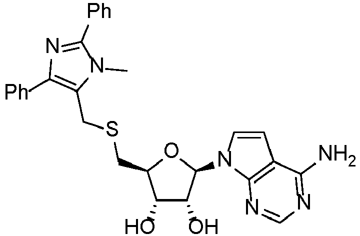 3.8 Hz), 6.14 (1H, d, *J* = 5.2 Hz), 4.49 (1H, t, *J* = 5.4 Hz), 4.17 (1H, t, *J* = 5.1 Hz), 4.12 (1H, dt, *J* = 6.2, 4.9 Hz), 4.10 (1H, d, *J* = 13.6 Hz), 4.01 (1H, d, *J* = 13.6 Hz), 3.68 (3H, s), 2.96 (1H, dd, *J* = 14.3, 5.1 Hz), 2.90 (1H, dd, *J* = 14.3, 6.2 Hz). **¹³C NMR** (101 MHz, MeOD) δ 158.7, 152.0, 151.3, 149.5, 140.1, 135.0, 131.1, 130.5, 130.4, 129.8, 129.6, 129.0, 128.4, 126.5, 123.3, 104.8, 101.5, 89.4, 84.9, 75.3, 74.1, 35.8, 32.5, 27.8. **HRMS** (ESI) Calculated for C₂₈H₂₉N₆O₃S, [M+H]⁺ m/z: 529.2016, [M+H]⁺, found 529.2020.

### Second generation

For a study of the nucleobase modification in the C-7 position, SAH analogue with the 3-methyl-5-phenylisoxazolylmethyl substituent was selected as the most suitable (good biological activity, commercially available). The key intermediates **122** and **123** that were prepared from isoxazole derivative **47a** according to the same methodology as described for the 7-deaza analogues. (**Scheme 7**). Next, the 7-fluoro analogue **128** was prepared. The starting material for this derivative was the protected nucleoside 124 (Nauš, P., et al., J Med Chem 2010/53, 460-470). Amonolysis and subsequent deprotection of the 5' hydroxyl group provided **125** that was subj ected to Mitsunobu reaction with AcSH. Furnished thioacetate **126** was converted to desired 7-fluoro SAH analogue **128** using the same conditions as for 7-iodo derivative **123.**

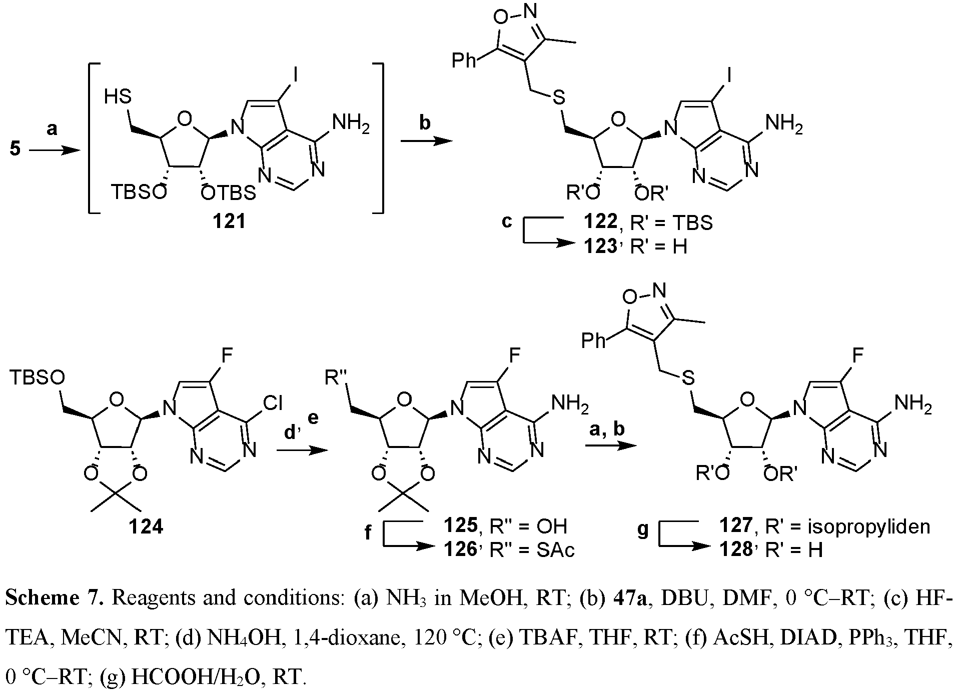

### Example 142

### ((2S,3R,4R,SR)-5-(4-Amino-5-iodo-7H-pyrrolo[2,3-d]pyrimidin-7-yl)-3,4-bis((tertbutyldimethylsilyl)oxy)tetrahydrofuran-2-yl)methanethiol (121):

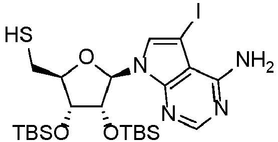

Thiol **121** was prepared from protected analogue **5** (2.5 g, 3.68 mmol) according to the same methodology as for **46.** The crude thiol **121** was after co-evaporation with DMF used as is. **LRMS** Calculated for C₂₃H₄₂IN₄O₃SSi₂, [M+H]⁺ m/z: 637.2, [M+H]⁺, found 637.1.

### Example 143

### 7-((2R,3R,4R,5S)-3,4-bis((tert-Butyldimethylsilyl)oxy)-5-((((3-methyl-5-phenyhsoxazol-4-yl)methyl)thio)methyl)tetrahydrofuran-2-yl)-5-iodo-7H-pyrrolo[2,3-d]pyrimidin-4-amine (122):

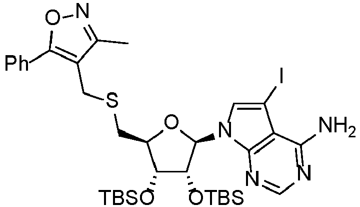

Iodinated intermediate **122** was synthesized from bromide **47a** (obtained from 7 (0.77 g, 4.05 mmol)) and thiol **121** (provided from **5** (2.5 g, 3.68 mmol)) following the same procedure as for **47b.** FCC (15-60 % EtOAc in DCM) afforded modified nucleoside **122** (2.8 g, 3.21 mmol, 94 %). **¹H NMR(400** MHz, DMSO-*d₆*) δ 8.09 (1H, s), 7.78-7.71 (2H, m), 7.66 (1H, s), 7.54-7.47 (3H, m), 6.66 (2H, bs), 6.08 (1H, d, *J* = 7.1 Hz), 4.74 (1H, dd, *J* = 7.0, 4.5 Hz), 4.15 (1H, dd, *J* = 4.3, 1.3 Hz), 3.96 (1H, td, *J* = 6.9, 1.2 Hz), 3.91 (1H, d, *J* = 13.2 Hz), 3.83 (1H, d, *J* = 13.3 Hz), 3.08 (1H, dd, *J* = 13.8, 7.3 Hz), 2.94 (1H, dd, *J* = 13.7, 6.8 Hz), 2.29 (3H, s), 0.90, 0.67 (18H, 2×s), 0.10, 0.09, -0.11, -0.39 (12H, 4×s). **¹³C NMR** (101 MHz, DMSO-*d₆*) δ 165.0, 160.7, 157.3, 152.2, 150.8, 130.3, 129.3, 127.4, 127.3, 126.9, 111.0, 103.5, 86.4, 84.7, 74.7, 74.1, 34.6, 25.9, 25.6, 24.6, 17.9, 17.6, 9.8, -4.5, -4.5, -4.6, -5.4. **LRMS** Calculated for C₃₄H₅₁N₅O₄ISSi₂, [M+H]⁺ m/z: 808.2, [M+H]⁺, found 808.2.

### Example 144

### (2R,3R,4S,5S)-2-(4-Amino-5-iodo-7H-pyrrolo[2,3-d]pyrimidin-7-yl)-5-((((3-methyl-5-phenylisoxazol-4-yl)methyl)thio)methyl)tetrahydrofuran-3,4-diol (123):

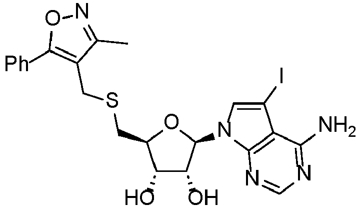

Analogue **123** was prepared from derivative **122** (0.35 g, 0.43 mmol) using the same procedure as for **47c.** reverse-phase FCC (15-80 % MeCN in H₂O) furnished **123** (0.24 g, 0.41 mmol, 96 %). **¹H NMR** (400 MHz, DMSO-*d₆*) δ 8.11 (1H, s), 7.79-7.72 (2H, m), 7.65 (1H, s), 7.57-7.45 (3H, m), 6.67 (2H, bs), 6.07 (1H, d, *J* = 6.1 Hz), 5.42 (1H, d, *J* = 6.2 Hz), 5.28 (1H, d, *J* = 4.8 Hz), 4.48 (1H, q, *J* = 6.0), 4.07-3.96 (2H, m), 3.86 (1H, d, *J* = 13.1 Hz), 3.81 (1H, d, *J* = 13.0), 2.95 (1H, dd, *J* = 13.9, 5.7 Hz), 2.88 (1H, dd, *J* = 13.8, 6.8 Hz), 2.28 (3H, s). **¹³C NMR** (101 MHz, DMSO-*d₆*) δ 164.9, 160.8, 157.4, 152.3, 150.7, 130.3, 129.4, 127.3, 127.1, 126.8, 111.1, 103.4, 86.8, 83.4, 73.1, 72.8, 52.7, 34.8, 24.4, 9.8. **HRMS** (ESI) Calculated for C₂₂H₂₃N₅O₄IS, [M+H]⁺ m/z: 580.0510, [M+H]⁺, found 580.0509.

### Example 145

### ((3aR,4R,6R,6aR)-6-(4-Amino-5-fluoro-7H-pyrrolo[2,3-d]pyrimidin-7-yl)-2,2-dimethyltetrahydrofuro[3,4-d]1,3]dioxol-4-yl)methanol (125):

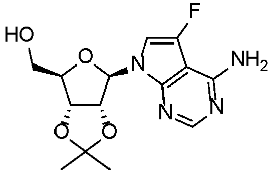

**124** (0.67 g, 1.46 mmol) was dissolved in aqueous NH₃ (5 mL) and 1,4-dioxane (5 mL). The reaction was stirred in a pressure vessel at 120 °C for 10 h. Volatiles were evaporated, the crude product was dissolved in THF (10 mL) and treated with 1M solution of TBAF in THF (1.5 mL, 1.5 mmol). After stirring at RT for 10 h, the crude mixture was adsorbed on silica and the product was purified by FCC (30-90 % acetone in cyclohexane) to afford **125** (0.4 g, 1.23 mmol, 84 %). **¹H NMR** (400 MHz, DMSO-*d₆*) δ 8.08 (1H, s), 7.37 (1H, d, *J* = 2.1 Hz), 7.06 (2H, bs), 6.21 (1H, dd, *J* = 3.7, 1.5 Hz), 5.14 (1H, t, *J* = 5.4 Hz), 5.07 (1H, dd, *J* = 6.3, 3.5 Hz), 4.87 (1H, dd, *J* = 6.3, 2.9), 4.10 (1H, td, *J* = 4.7, 2.9), 3.56-3.51 (2H, m), 1.53 (3H, s), 1.30 (3H, s). **¹³C NMR** (101 MHz, DMSO-*d₆*) δ 155.9 (d, *J* = 2.6 Hz), 153.0, 146.0 (d, *J* = 2.7 Hz), 142.8 (d, *J* = 245.7 Hz), 113.2, 104.6 (d, *J* = 26.9 Hz), 92.5 (d, *J* = 15.7 Hz), 88.4, 85.2, 83.4, 81.0, 61.6, 27.2, 25.3. **¹⁹F NMR** (377 MHz, DMSO-*d₆*) δ -167.16 (t, *J* = 2.0 Hz). **LRMS** Calculated for C₁₄H₁₈FN₄O₄, [M+H]⁺ m/z: 325.1, [M+H]⁺, found 325.1.

### Example 146

### S-(((3aS,4S,6R,6aR)-6-(4-Amino-5-fluoro-7H-pyrrolo[2,3-d]pyrimidin-7-yl)-2,2-dimethyltetrahydrofuro[3.4-d][1,3]dioxol-4-yl)methyl)thioacetate (126):

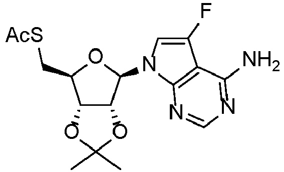

**126** was prepared from **125** (0.1 g, 0.28 mmol) in analogous fashion to 5. FCC (20-80 % EtOAc in cyclohexane) gave **126** (0.1 g, 2.51 mmol, 91 %). **¹H NMR** (400 MHz, CDCl₃) δ 8.23 (1H, s), 6.85 (1H, d, *J* = 2.4 Hz), 6.18 (2H, bs), 6.13 (1H, dd, *J* = 2.8, 0.9 Hz), 5.21 (1H, dd, *J* = 6.6, 2.7 Hz), 4.79 (1H, dd, *J* = 6.5, 3.5), 4.28 (1H, ddd, *J* = 6.9, 5.9, 3.6), 3.25 (1H, dd, *J* = 13.8, 6.8 Hz), 3.18 (1H, dd, *J* = 13.9, 6.0 Hz), 2.37 (3H, s), 1.59 (3H, s), 1.36 (3H, s). **¹³C NMR** (101 MHz, CDCl₃) δ 194.8, 155.2 (d, *J* = 2.9 Hz ), 151.4, 145.6, 143.8 (d, *J* = 247.9 Hz), 114.3, 105.8 (d, *J* = 26.4 Hz), 94.2 (d, *J* = 14.8 Hz), 90.5, 84.8, 84.7, 83.2, 31.4, 27.3, 25.5. **¹⁹F NMR** (377 MHz, CDCl₃) δ -167.08 (d, *J* = 1.5 Hz). **LRMS** Calculated for C₁₆H₂₀FN₄O₄S, [M+H]⁺ m/z: 383.1, [M+H]⁺, found 383.1.

### Example 147

### 7-((3aR,4R,6S,6aS)-2,2-dimethyl-6-((((3-methyl-5-phenylisoxazol-4-yl)methyl)thio)methyl) tetrahydrofuro[3,4-d][1,3]dioxol-4-yl)-5-fluoro-7H-pyrrolo[2,3-d]pyrimidin-4-amine (127):

**127** was synthesized from **47a** (obtained from **7** (0.05 g, 0.28 mmol)) and thiol (provided from **126** 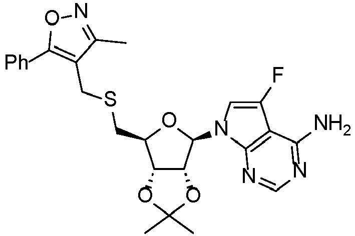 (0.09 g, 0.24 mmol)) following the same procedure as for **47b.** FCC (10-80 % acetone in cyclohexane) afforded **122** (0.08 g, 0.16 mmol, 70 %). **¹H NMR (400** MHz, CDCl₃) δ 8.24 (1H, s), 7.75-7.71 (2H, m), 7.49-7.43 (3H, m), 6.78 (1H, d, *J* = 2.4 Hz), 6.09 (1H, d, *J* = 2.5 Hz), 5.56 (2H, bs), 5.25 (1H, dd, *J* = 6.6, 2.7 Hz), 4.91 (1H, dd, *J* = 6.7, 3.8 Hz), 4.29 (1H, td, *J* = 6.4, 3.8 Hz), 3.75 (1H, d, *J* = 13.0 Hz), 3.65 (1H, d, *J* = 12.9 Hz), 2.83 (1H, dd, *J* = 13.9, 6.6 Hz), 2.79 (1H, dd, *J* = 13.8, 6.1 Hz), 2.34 (3H, s), 1.59 (3H, s), 1.37 (3H, s). **¹³C NMR** (101 MHz, CDCl₃) δ 166.3, 160.7, 155.3 (d, *J* = 2.9 Hz), 152.6, 145.9 (d, *J* = 2.3 Hz), 143.6 (d, *J* = 247.4 Hz), 130.1, 129.1, 127.9, 127.3, 114.9, 110.2, 105.6 (d, *J* = 26.5 Hz), 94.3 (d, *J* = 15.0 Hz), 90.5, 85.5, 84.6, 83.5, 34.7, 27.3, 25.5, 25.3, 10.3. **¹⁹F NMR** (377 MHz, CDCl₃) δ -167.39. **LRMS** Calculated for C₂₅H₂₇FN₅O₄S, [M+H]⁺ m/z: 512.2, [M+H]⁺, found 512.2.

### Example 148

### (2R,3R,4S,5S)-2-(4-Amino-5-fluoro-7H-pyrrolo[2,3-d]pyrimidin-7-yl)-5-((((3-methyl-5-phenylisoxazol-4-yl)methyl)thio)methyl)tetrahydrofuran-3,4-diol (128):

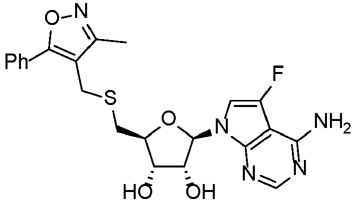

**127** (0.07 g, 0.14 mmol) was dissolved in 80% HCOOH (2 mL) and the reaction mixture was stirred at RT for 2 h. The reaction mixture was evaporated and coevaporated with twice with EtOH, methanolic ammonia and one more time with EtOH. Reverse phase FCC (0-100 % MeCN in H₂O) gave **128** (0.06 g, 0.13 mmol, 90 %). **¹H NMR** (400 MHz, DMSO-*d₆*) δ 8.08 (1H, s), 7.78-7.74 (2H, m), 7.55-7.46 (3H, m), 7.32 (1H, d, *J* = 2.0 Hz), 7.00 (2H, bs), 6.13 (1H, dd, *J* = 5.9, 1.8 Hz), 5.44 (1H, d, *J* = 6.1 Hz), 5.30 (1H, d, *J* = 4.6 Hz), 4.38 (1H, q, *J* = 5.7), 4.05-3.96 (2H, m), 3.86 (1H, *d, J=* 13.0 Hz), 3.81 (1H, d, *J* = 13.0 Hz), 2.93 (1H, dd, *J* = 13.9, 5.4 Hz), 2.85 (1H, dd, *J* = 13.9, 6.8 Hz), 2.27 (3H, s). **¹³C NMR** (101 MHz, DMSO-*d₆*) δ 164.9, 160.8, 156.0 (d, *J* = 2.6 Hz), 153.2, 146.9 (d, *J* = 2.8 Hz ), 143.1 (d, *J =* 245.7 Hz), 130.3, 129.4, 127.3, 126.8, 111.1, 104.3 (d, *J* = 26.5 Hz), 92.7 (d, *J* = 15.7 Hz), 86.5, 83.3, 73.2, 72.8, 34.9, 24.5, 9.8. **¹⁹F NMR** (377 MHz, DMSO-*d₆*) δ -167.18. **HRMS** (ESI) Calculated for C₂₂H₂₃FN₅O₄S, [M+H]⁺ m/z: 472.1449, [M+H]⁺, found 472.1452.

Iodinated intermediates **122** and **123** were than subjected to a number of various cross-coupling reactions in order to prepare compounds bearing aliphatic or aromatic substitution in the 7-position of nucleobase. Negishi cross-coupling reaction with commercially available zinc bromides together with Pd-catalyzed cyanation with Zn(CN)₂ were used for preparation of analogues with alkylated nucleobase. The combination of zinc powder and a catalytic system consisting of Pd₂dba₃ and ligand dppf (Tan, H., et al., Eur J Med Chem 2021/223, 113670) provided a library of substances with diverse aliphatic substituents **129a-138a** Removal of protecting groups by HF-TEA provided SAH analogues **129b-138b.** Stille and Sonogashira cross-coupling reactions, deprotection and subsequent catalytic hydrogenation were used to prepare analogues **139b, 140b** and **142,** bearing vinyl, ethynyl and ethyl, respectively. Suzuki-Miyaura cross-coupling followed by deprotection with HF-TEA afforded **141b** with a functionalized cyclohexenyl (**Scheme 8**).

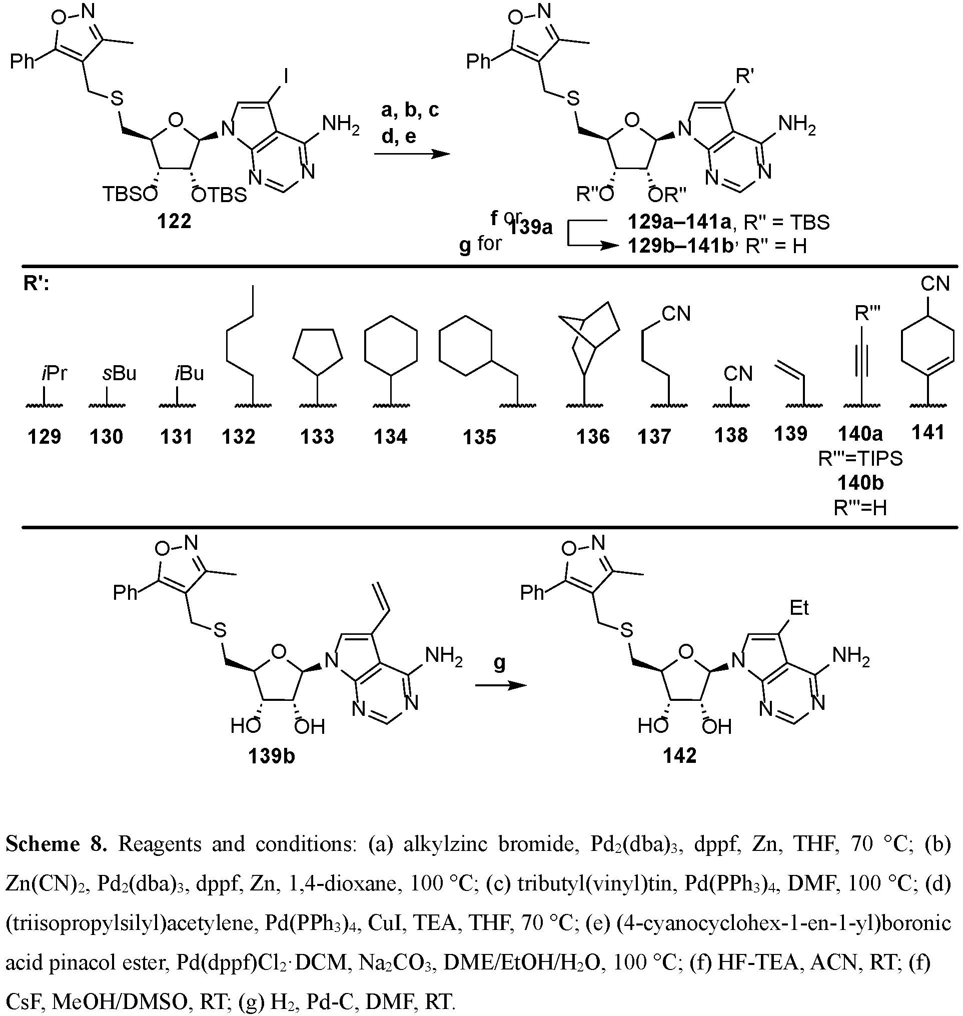

### Example 149

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-isopropyl-7H-pyrrolo[2,3-d]pyrimidin-4-amine (129a):

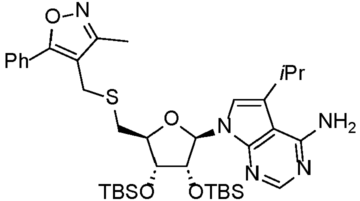

To a solution of intermediate **122** (0.2 g, 0.25 mmol), Pd₂(dba)₃ (0.01 g, 0.01 mmol), dppf (0.01 g, 0.03 mmol) and zinc (0.01 g, 0.17 mmol) in dry THF (2 mL) was added 0.5M solution of *i*PrZnBr in THF (1.5 mL, 0.75 mmol) and the reaction mixture was stirred at 70 °C under argon atmosphere. After stirring for 10 h, H₂O (50 mL) was added, and the reaction mixture was extracted with EtOAc (2×100 mL). The collected organic layers were dried over Na₂SO₄, filtrated and concentrated. FCC (5-70 % acetone in cyclohexane) furnished desired product **129a** (0.16 g, 0.22 mmol, 87 %). **¹H NMR** (400 MHz, DMSO-*d₆*) δ 8.01 (1H, s), 7.69 (2H, dd, , *J* = 7.3, 1.8 Hz), 7.52-7.41 (3H, m), 7.07 (1H, s), 6.51 (2H, bs), 6.08 (1H, d, *J* = 7.4 Hz), 4.74 (1H, dd, *J* = 7.4, 4.5 Hz), 4.11 (1H, dd, *J* = 4.5, 1.0 Hz), 3.97-3.91 (1H, m), 3.82 (1H, d, *J=* 13.7 Hz), 3.72 (1H, d, *J=* 13.7 Hz), 3.27 (1H, sext, *J* = 6.6), 3.00 (1H, dd, *J* = 13.8, 7.3 Hz), 2.83 (1H, dd, *J* = 13.8, 6.4 Hz), 2.41 (3H, s), 1.18 (1H, d, *J* = 6.8 Hz), 1.17 (1H, d, *J* = 6.7 Hz), 0.90, 0.65 (18H, 2×s), 0.09, 0.08, -0.14, -0.40 (12H, 4×s). **¹³C NMR** (101 MHz, DMSO-*d₆*) δ 168.7, 162.2, 157.0, 151.6, 151.0, 130.4, 129.5, 129.5, 128.4, 124.2, 117.9, 110.7, 102.1, 86.5, 85.1, 75.1, 74.3, 34.8, 26.3, 26.1, 25.6, 24.6, 24.4, 24.1, 18.3, 18.1, 11.4, -4.1, -5.0. **LRMS** Calculated for C₃₇H₅₈N₅O₄SSi₂, [M+H]⁺ m/z: 724.4, [M+H]⁺, found 724.4.

### Example 150

### 7-((2R,3R,4R,5S)-3,4-bis((tert-Butyldlmethylsilyl)oxy)-5-((((3-methyl-5-phenylisoxazol-4-yl)methyl) thio)methyl)tetrahydrofuran-2-yl)-5-(sec-butyl)-7H-pyrrolo[2,3-d]pyrimidin-4-amine (130a):

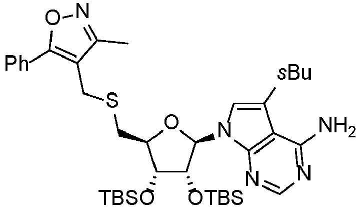

**130a** was synthesized from **122** (0.2 g, 0.25 mmol) and 0.5M solution of sBuZnBr in THF (1.5 mL, 0.75 mmol) in analogous fashion to **129a.** FCC (5-70 % acetone in cyclohexane) provided **130a** (0.15 g, 0.20 mmol, 82 %) as a mixture of diastereomers. **¹H NMR** (400 MHz, DMSO-*d₆*) δ 8.01 (1H, s), 8.01 (1H, s), 7.72-7.66 (4H, m), 7.51-7.42 (6H, m), 7.08 (1H, s), 7.08 (1H, s), 6.47 (4H, bs), 6.10 (1H, d, *J* = 7.3 Hz), 6.07 (1H, d, *J* = 7.4 Hz), 4.79 (1H, dd, *J* = 7.4, 4.5 Hz), 4.74 (1H, dd, *J* = 7.3, 4.5 Hz), 4.13-4.10 (2H, m), 3.97-3.92 (2H, m), 3.82 (2H, d, *J* = 13.7 Hz), 3.72 (1H, d, *J* = 13.8 Hz), 3.71 (1H, d, *J* = 13.7 Hz), 3.08 (2H, br q, *J* = 6.9), 3.00 (1H, dd, *J* = 13.9, 7.3 Hz), 3.00 (1H, dd, *J* = 13.6, 6.8 Hz), 2.86 (1H, dd, *J* = 13.4, 6.6 Hz), 2.84 (1H, dd, *J* = 13.7, 6.4 Hz), 2.40 (3H, s), 2.40 (3H, s), 1.70-1.57 (2H, m), 1.52-1.39 (2H, m), 1.16 (3H, d, *J* = 6.8 Hz), 1.12 (3H, d, *J* = 6.8 Hz), 0.90 (18H, s), 0.88 (3H, t, *J* = 7.4 Hz), 0.81 (3H, t, *J* = 7.4 Hz) 0.66, 0.65 (18H, 2×s), 0.09 (12H, s), -0.13, -0.14, -0.39, -0.40 (12H, 4×s). **¹³C NMR** (101 MHz, DMSO-*d₆*) δ 168.1, 161.58, 161.57, 157.6, 157.5, 151.4, 151.3, 127.8, 129.01, 129.00, 128.0, 121.9, 121.7, 117.9, 117.6, 110.27, 110.26, 102.21, 102.18, 86.3, 86.1, 84.6, 84.4, 74.8, 73.7, 73.6, 33.4, 31.5, 30.5, 30.1, 25.9, 25.7, 24.2, 21.6, 21.4, 17.9, 17.7, 11.6, 11.5, 11.0, -4.4, -4.5, -4.5, -5.3. Calculated for C₃₈H₆₀N₅O₄SSi₂, [M+H]⁺ m/z: 738.4, [M+H]⁺, found 738.4.

### Example 151

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-isobutyl-7H-pyrrolo[2,3-d]pyrimidin-4-amine (131a):

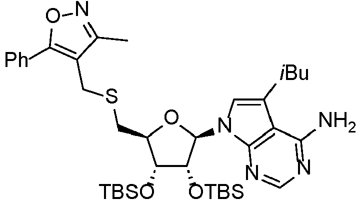

**131a** was prepared from **122** (0.2 g, 0.25 mmol) and 0.5M solution of iBuZnBr in THF (1.5 mL, 0.75 mmol) in analogous fashion to **129a.** FCC (5-70 % acetone in cyclohexane) afforded **131a** (0.15 g, 0.21 mmol, 83 %). **¹H NMR** (400 MHz, DMSO-*d₆*) δ 8.01 (1H, s), 7.72-7.67 (2H, m), 7.51-7.45 (3H, m), 7.07 (1H, s), 6.47 (2H, bs), 6.07 (1H, d, *J* = 7.0 Hz), 4.73 (1H, dd, *J* = 6.9, 4.5 Hz), 4.13 (1H, dd, *J* = 4.4, 1.6 Hz), 3.94 (1H, td, *J* = 6.6, 1.5 Hz), 3.80 (1H, d, *J* = 13.7 Hz), 3.70 (1H, d, *J* = 13.7 Hz), 2.97 (1H, dd, *J* = 13.8, 7.0 Hz), 2.81 (1H, dd, *J* = 13.9, 6.5 Hz), 2.58 (2H, d, *J* = 7.0 Hz), 2.40 (3H, s), 1.77 (1H, non, *J* = 6.7 Hz), 0.90 (9H, s), 0.87 (3H, d, *J* = 6.6 Hz), 0.85 (3H, d, *J* = 6.6 Hz), 0.68 (9H, s), 0.09, 0.08, -0.12, -0.37 (12H, 4×s). **¹³C NMR** (101 MHz, DMSO-*d₆*) δ 168.1, 161.6, 157.8, 151.41, 151.42, 129.9, 129.1, 129.0, 128.0, 120.2, 114.7, 110.3, 102.7, 86.5, 84.2, 74.8, 73.8, 34.8, 34.3, 29.5, 25.9, 25.7, 24.1, 22.3, 22.1, 17.9, 17.7, 11.0, -4.4, -4.5, -4.6, -5.2. **LRMS** Calculated for C₃₈H₆₀N₅O₄SSi₂, [M+H]⁺ m/z: 738.4, [M+H]⁺, found 738.4.

### Example 152

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-pentyl-7H-pyrrolo[2,3-d]pyrimidin-4-amine (132a):

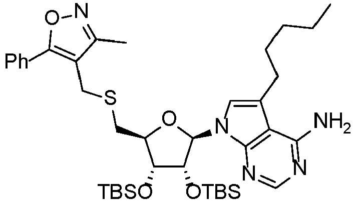

Analogue **132a** was prepared from **122** (0.26 g, 0.32 mmol) and 0.5M solution of pentylzinc bromide in THF (1.92 mL, 0.96 mmol) in analogous fashion to **129a.** FCC (5-60 % acetone in cyclohexane) furnished **132a** (0.19 g, 0.25 mmol, 80 %). **¹H NMR** (400 MHz, DMSO-*d₆*) δ 8.01 (1H, s), 7.72-7.66 (2H, m), 7.50-7.43 (3H, m), 7.06 (1H, s), 6.48 (2H, bs), 6.07 (1H, d, *J* = 7.0 Hz), 4.71 (1H, dd, *J* = 7.0, 4.5 Hz), 4.13 (1H, dd, *J* = 4.4, 1.6 Hz), 3.94 (1H, td, *J* = 6.7, 1.3 Hz), 3.80 (1H, d, *J* = 13.7 Hz), 3.70 (1H, d, *J* = 13.7 Hz), 2.97 (1H, dd, *J* = 13.8, 7.1 Hz), 2.80 (1H, dd, *J* = 13.8, 6.4 Hz), 2.76-2.66 (2H, m), 2.40 (3H, s), 1.51 (2H, p, *J* = 7.4 Hz), 1.36-1.20 (4H, m), 0.90 (9H, s), 0.82 (3H, t, *J* = 7.1 Hz), 0.67 (9H, s), 0.09, 0.08, -0.13, -0.38 (12H, 4×s). **¹³C NMR** (101 MHz, DMSO-*d₆*) δ 168.1, 161.6, 157.8, 151.6, 151.5, 129.8, 129.04, 129.00, 128.0, 119.1, 116.0, 110.3, 102.4, 86.3, 84.2, 74.7, 73.9, 34.3, 30.8, 30.1, 25.9, 25.8, 25.7, 24.1, 22.2, 17.9, 17.7, 14.1, 11.0, -4.46, -4.47, -4.6, -5.3. **LRMS** Calculated for C₃₉H₆₂N₅O₄SSi₂, [M+H]⁺ m/z: 752.4, [M+H]⁺, found 752.4.

### Example 153

### 7-((2R,3R,4R,5S)-3,4-bis((tert-Butyldimethylsilyl)oxy)-5-((((3-methyl-5-phenylisoxazol-4-yl)methyl) thio)methyl)tetrahydrofuran-2-yl)-5-cyclopentyl-7H-pyrrolo[2,3-d]pyrimidin-4-amine (133a):

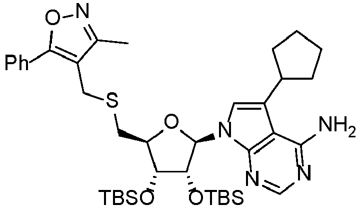

Title compound **133a** was prepared from **122** (0.25 g, 0.31 mmol) and 0.5M solution of cyclopentylzinc bromide in THF (1.86 mL, 0.93 mmol) according to the same procedure as for **129a.** FCC (5-60 % acetone in cyclohexane) provided modified nucleoside **133a** (0.19 g, 0.25 mmol, 80 %). **¹H NMR** (400 MHz, DMSO-*d₆*) δ 8.01 (1H, s), 7.73-7.64 (2H, m), 7.53-7.41 (3H, m), 7.10 (1H, s), 6.48 (2H, bs), 6.08 (1H, d, *J* = 7.3 Hz), 4.74 (1H, dd, *J* = 7.3, 4.5 Hz), 4.11 (1H, dd, *J* = 4.4, 1.2 Hz), 3.94 (1H, td, *J* = 7.2, 1.0 Hz), 3.81 (1H, d, *J=* 13.7 Hz), 3.71 (1H, d, *J* = 13.7 Hz), 3.40-3.29 (1H, m), 3.00 (1H, dd, *J* = 13.9, 7.2 Hz), 2.83 (1H, dd, *J* = 13.8, 6.5 Hz), 2.40 (3H, s), 2.04-1.90 (2H, m), 1.71-1.57 (4H, m), 1.54-1.42 (2H, m), 0.90, 0.65 (18H, 2×s), 0.09, 0.08, -0.14, -0.39 (12H, 4×s). **¹³C NMR** (101 MHz, DMSO-*d₆*) δ 168.1, 161.6, 157.6, 151.6, 151.5, 129.8, 129.02, 128.99, 128.0, 120.3, 117.2, 110.3, 102.3, 86.1, 84.4, 74.7, 73.7, 36.4, 34.4, 33.7, 33.3, 25.9, 25.7, 24.7, 24.2, 17.9, 17.7, 11.0, -4.5, -4.6, -5.3. **LRMS** Calculated for C₃₉H₆₀N₅O₄SSi₂, [M+H]⁺ m/z: 750.4, [M+H]⁺, found 750.4.

### Example 154

### 7-((2R,3R,4R,5S)-3,4-bis((tert-Butyldimethylsilyl)oxy)-5-((((3-methyl-5-phenylisoxazol-4-yl)methyl) thio)methyl)tetrahydrofuran-2-yl)-5-cyclohexyl-7H-pyrrolo[2,3-d]pyrimidin-4-amine (134a):

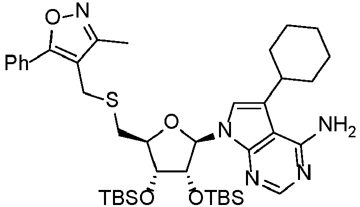

Derivative **134a** was synthesized from **122** (0.2 g, 0.25 mmol) and 0.5M solution of cyclohexylzinc bromide in THF (1.5 mL, 0.75 mmol) following the same methodology as for **129a.** FCC (5-60 % acetone in cyclohexane) afforded **134a** (0.15 g, 0.19 mmol, 77 %). **¹H NMR** (400 MHz, DMSO-*d₆*) δ 8.01 (1H, s), 7.73-7.65 (2H, m), 7.53-7.41 (3H, m), 7.06 (1H, s), 6.43 (2H, bs), 6.09 (1H, d, *J* = 7.4 Hz), 4.73 (1H, dd, *J* = 7.4, 4.5 Hz), 4.11 (1H, dd, *J* = 4.4, 0.7 Hz), 3.94 (1H, br t, *J* = 6.9 Hz), 3.82 (1H, d, *J* = 13.7 Hz), 3.71 (1H, d, *J* = 13.7 Hz), 3.00 (1H, dd, *J* = 13.8, 7.3 Hz), 2.90 (1H, tt, *J* = 11.6, 2.8 Hz), 2.84 (1H, dd, *J i=* 13.9, 6.5 Hz), 2.40 (3H, s), 1.90 (2H, brt, *J* = 15.6 Hz), 1.72-1.65 (3H, m), 1.59-1.40 (2H, m), 1.32-1.03 (3H, m), 0.90, 0.65 (18H, 2×s), 0.09, 0.08, -0.14, -0.40 (12H, 4×s). **¹³C NMR** (101 MHz, DMSO-*d₆*) δ 168.1, 161.6, 157.6, 151.44, 151.40, 129.9, 129.0, 128.0, 122.4, 117.3, 110.3, 101.8, 86.0, 84.5, 74.8, 73.8, 34.5, 34.4, 34.3, 26.1, 25.93, 25.88, 25.7, 24.2, 17.9, 17.7, 11.0, -4.4, -4.47, -4.52, -5.3. **LRMS** Calculated for C₄₀H₆₂N₅O₄SSi₂, [M+H]⁺ m/z: 764.4, [M+H]⁺, found 764.4.

### Example 155

### 7-((2R,3R,4R,5S)-3,4-bis((tert-Butyldimethylsilyl)oxy)-5-((((3-methyl-5-phenylisoxazol-4-yl)methyl) thio)methyl)tetrahydrofuran-2-yl)-5-(cyclohexylmethyl)-7H-pyrrolo[2,3-d]pyrimidin-4-amine (135a):

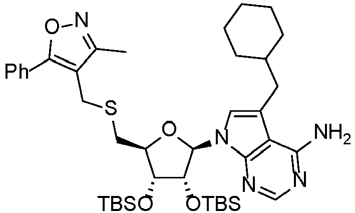

SAH analogue **135a** was prepared from **122** (0.2 g, 0.25 mmol) and 0.5M solution of (cyclohexymethyl)zinc bromide in THF (1.5 mL, 0.75 mmol) in analogous fashion to **129a.** FCC (5-70% acetone in cyclohexane) furnished **135a** (0.16 g, 0.20 mmol, 81 %). **¹H NMR** (400 MHz, DMSO-*d₆*) δ 8.01 (1H, s), 7.74-7.65 (2H, m), 7.51-7.42 (3H, m), 7.06 (1H, s), 6.45 (2H, bs), 6.07 (1H, d, *J* = 7.0 Hz), 4.72 (1H, dd, *J* = 6.9, 4.5 Hz), 4.13 (1H, dd, *J* = 4.4, 1.7 Hz), 3.95 (1H, td, *J* = 6.6, 1.7 Hz), 3.81 (1H, d, *J* = 13.7 Hz), 3.70 (1H, d, *J* = 13.7 Hz), 2.97 (1H, dd, *J* = 13.9, 6.8 Hz), 2.83 (1H, dd, *J* = 13.9, 6.7 Hz), 2.59 (2H, d, *J* = 7.0 Hz), 2.39 (3H, s), 1.75-1.47 (5H, m), 1.47-1.32 (1H, m), 1.16-1.00 (3H, m), 0.96-0.85 (11H, m), 0.68 (9H, s), 0.09, 0.09, -0.12, -0.36 (12H, 4×s). **¹³C NMR** (101 MHz, DMSO-*d₆*) δ 168.3, 161.8, 157.7, 151.5, 151.3, 130.1, 129.22, 129.16, 128.2, 114.4, 110.4, 102.9, 86.5, 84.6, 74.9, 74.1, 34.5, 33.4, 32.8, 32.5, 26.5, 26.1, 25.9, 24.4, 18.1, 17.9, 11.1, -4.3, -4.4, -5.1. **LRMS** Calculated for C₄₁H₆₄N₅O₄SSi₂, [M+H]⁺ m/z: 778.4, [M+H]⁺, found 778.4.

### Example 156

### 5-((1S,4R)-Bicyclo[2.2.1]heptan-2-yl)-7-((2R,3R,4R,5S)-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidin-4-amine (136a):

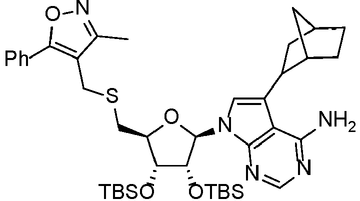

Title compound **136a** was prepared from iodinated analogue **122** (0.26 g, 0.32 mmol) and 0.5M solution of ((1*S*,4*R*)-bicyclo[2.2.1]heptan-2-yl)zinc bromide in THF (1.92 mL, 0.96 mmol) in analogous fashion to **129a.** FCC (5-70 % acetone in cyclohexane) affording derivative **136a** (0.14 g, 0.18 mmol, 58 %). **¹H NMR** (400 MHz, DMSO-*d₆*) δ 8.01 (1H, s), 7.71-7.66 (2H, m), 7.49-7.42 (3H, m), 7.04 (1H, s), 6.34 (2H, bs), 6.08 (1H, d, *J* = 7.5 Hz), 4.78 (1H, dd, *J* = 7.3, 4.8 Hz), 4.11 (1H, dd, *J* = 4.2, 1.4 Hz), 3.96-3.91 (1H, m), 3.82 (1H, d, *J* = 13.7 Hz), 3.70 (1H, d, *J* = 13.7 Hz), 3.06-2.97 (2H, m), 2.84 (1H, dd, *J* = 13.8, 6.7 Hz), 2.40 (3H, s), 2.26-2.16 (2H, m), 1.76-1.61 (1H, m), 1.55-1.46 (4H, m), 1.46-1.39 (1H, m), 1.28-1.20 (1H, m) 1.05-0.99 (1H, m), 0.90, 0.66 (18H, 2×s), 0.09, -0.13, -0.40 (12H, 3×s).. **¹³C NMR** (101 MHz, DMSO-*d₆*) δ 168.1, 161.6, 157.8, 151.8, 151.6, 129.9, 129.0, 128.5, 128.0, 121.5, 117.6, 110.3, 102.1, 86.2, 84.5, 74.8, 73.6, 42.6, 42.4, 38.4, 37.8, 37.6, 36.6, 35.5, 35.4, 34.4, 29.2, 29.0, 28.7, 26.0, 25.7, 24.2, 17.9, 17.7, 11.0, -4.4, -4.5, -5.3. **LRMS** Calculated for C₄₁H₆₄N₅O₄SSi₂, [M+H]⁺ m/z: 776.4, [M+H]⁺, found 776.4.

### Example 157

### 4-(4-Amino-7-((2R,3R,4R,5S)-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidin-5-yl)butanenitrile (137a):

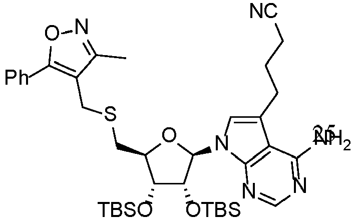

Analogue **137a** was synthesized from derivative **122** (0.35 g, 0.44 mmol) and 0.5M solution of (3-cyanopropyl)zinc bromide in THF (2.64 mL, 1.32 mmol) in analogous fashion to **129a.** FCC (5-100 % acetone in cyclohexane) provided SAH analogue **137a** (0.25 g, 0.34 mmol, 77 %). **¹H NMR** (400 MHz, DMSO-*d₆*) δ 8.03 (1H, s), 7.76-7.71 (2H, m), 7.51-7.46 (3H, m), 7.17 (1H, s), 6.60 (2H, bs), 6.08 (1H, d, *J* = 7.1 Hz), 4.74 (1H, dd, *J* = 7.1, 4.5 Hz), 4.14 (1H, dd, *J* = 4.4, 1.5 Hz), 3.95 (1H, td, *J* = 6.9, 1.5 Hz), 3.92 (1H, *d*, *J* = 13.3 Hz), 3.83 (1H, d, *J* = 13.3 Hz), 3.05 (1H, dd, *J* = 13.8, 7.1 Hz), 2.92 (1H, dd, *J* = 13.8, 6.8 Hz), 2.86-2.74 (2H, m), 2.52-2.48 (2H, m), 2.28 (3H, s), 1.78 (2H, p, *J* = 7.4 Hz), 0.90, 0.67 (18H, 2×s), 0.10, 0.10, -0.12, -0.38 (12H, 4×s). **¹³C NMR** (101 MHz, DMSO-*d₆*) δ 165.0, 160.7, 157.6, 151.8, 151.7, 130.3, 129.3, 127.3, 126.8, 120.7, 119.2, 114.1, 111.1, 102.2, 86.3, 84.5, 74.8, 73.9, 34.6, 26.8, 25.9, 25.7, 25.0, 24.6, 17.9, 17.7, 15.8, -4.5, -4.5 -4.6, -5.3. **LRMS** Calculated for C₃₈H₅₇N₆O₄SSi₂, [M+H]⁺ m/z: 749.4, [M+H]⁺, found 749.4.

### Example 158

### 4-Amino-7-((2R,3R,4R,5S-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidine-5-carbonitrile (138a):

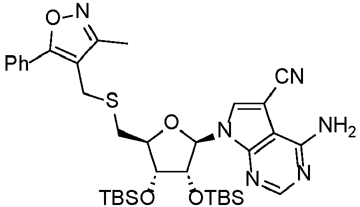

A suspension of **122** (0.3 g, 0.37 mmol), Zn(CN)₂ (0.22 g, 1.86 mmol), Pd₂(dba)₃ (0.02 g, 0.02 mmol), dppf (0.03 g, 0.06 mmol) and zinc (0.04 g, 0.55 mmol) in dry 1,4-dioxane (3 mL) under inert atmosphere and warmed to 100 °C. After stirring for 10 h, H₂O (50 mL) was added, and the reaction mixture was extracted with DCM (2×100 mL). Organic layers were dried over Na₂SO₄, filtrated and concentrated. FCC (10-70 % EtOAc in DCM) gave 138a (0.15 g, 0.22 mmol, 58 %). **¹H NMR** (400 MHz, DMSO-*d₆*) δ 8.44 (1H, s), 8.21 (1H, s), 7.78-7.71 (2H, m), 7.55-7.46 (3H, m), 6.90 (2H, bs), 6.10 (1H, d, *J* = 6.7 Hz), 4.79 (1H, dd, *J* = 6.6, 4.4 Hz), 4.19 (1H, dd, *J* = 4.2, 1.8 Hz), 4.01 (1H, td, *J* = 6.9, 1.7 Hz), 3.91 (1H, d, *J* = 13.2 Hz), 3.83 (1H, d, *J* = 13.2 Hz), 3.09 (1H, dd, *J* = 13.9, 7.0 Hz), 3.00 (1H, dd, *J* = 13.9, 7.1 Hz), 2.29 (3H, s), 0.89, 0.68 (18H, 2×s), 0.10, 0.09, -0.10, -0.39 (12H, 4×s). **¹³C NMR** (101 MHz, DMSO-*d₆*) δ 165.0, 160.7, 157.2, 153.8, 150.7, 132.9, 130.3, 129.3, 127.3, 126.9, 115.3, 111.0, 101.4, 87.6, 85.2, 83.8, 74.6, 74.3, 34.4, 25.9, 25.6, 24.6, 17.9, 17.6, 9.8, -4.5, -4.6, -4.6, -5.5. **LRMS** Calculated for C₃₅H₅₁N₆O₄SSi₂, [M+H]⁺ m/z: 707.3, [M+H]⁺, found 707.3.

### Example 159

### 7-((2R,3R,4r,5S)-3,4-bis((tert-Butyldimethylsilyl)oxy)-5-((((3-methyl-5-phenylisoxazol-4-yl)methyl)thio)methyl)tetrahydrofuran-2-yl)-5-vinyl-7H-pyrrolo[2,3-d]pyrimidin-4-amine (139a):

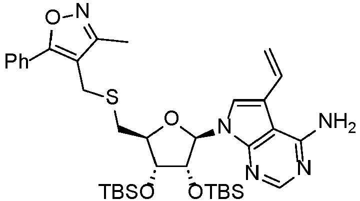

To a solution **of 122** (0.4 g, 0.50 mmol) and Pd(PPh₃)₄ (0.06 g, 0.05 mmol) in dry DMF (5 mL), tributyl(vinyl)tin (0.58 mL, 1.99 mmol) was added under argon atmosphere and the reaction mixture was warmed to 100 °C. After stirring for 10 h, the reaction mixture was cooled to RT and a solvent was removed in vacuo. The residuals were adsorbed to silica and the FCC (10-70 % EtOAc in DCM) gave 139a (0.29 g, 0.40 mmol, 82 %). **¹H NMR** (400 MHz, CDCl₃) δ 8.24 (1H, s), 7.76-7.71 (2H, m), 7.46-7.40 (3H, m), 7.09 (1H, s), 6.89 (1H, ddd, *J* = 17.4, 11.0, 0.8 Hz), 6.00 (1H, d, *J* = 4.7 Hz), 5.48 (1H, dd, *J* = 17.4, 1.4 Hz), 5.37 (2H, bs), 5.30 (1H, dd, *J* = 11.0, 1.5 Hz), 4.79 (1H, t, *J* = 4.5 Hz), ), 4.27-4.19 (2H, m), 3.82 (1H, d, *J* = 13.1 Hz), 3.70 (1H, d, *J* = 13.1 Hz), 3.00 (1H, dd, *J* = 13.9, 5.1 Hz), 2.92 (1H, dd, *J* = 13.9, 5.8 Hz), 2.31 (3H, s), 0.93, 0.81 (18H, 2×s), 0.11, -0.07, -0.21 (12H, 3×s). **¹³C NMR** (101 MHz, DMSO-*d₆*) δ 166.1, 160.8, 157.4, 152.1, 151.2, 130.0, 129.2, 129.0, 127.9, 127.3, 121.6, 116.5, 115.0, 110.5, 102.3, 90.1, 83.8, 74.7, 74.7, 34.9, 26.0, 25.9, 25.9, 18.2, 18.1, 10.3, -4.1, -4.4, -4.6, -4.8. **LRMS** Calculated for C₃₆H₅₄N₅O₄SSi₂, [M+H]⁺ m/z: 708.3, [M+H]⁺, found 708.3.

### Example 160

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-((triisopropylsilyl)ethynyl)-7H-pyrrolo[2,3-d]pyrimidin-4-amine (140a):

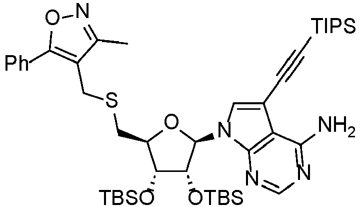

To a suspension of **122** (0.25 g, 0.31 mmol), Pd(PPh₃)₄ (0.04 g, 0.03 mmol) and CuI (0.02 g, 0.1 mmol) in dry THF (2 mL), (triisopropylsilyl)acetylene (0.21 mL, 0.94 mmol) was added followed by TEA (0.13 mL, 0.94 mmol) at RT under argon atmosphere and the reaction mixture was warmed to 70 °C. After stirring for 2 h, H₂O (50 mL) was added and the reaction mixture was extracted with DCM (2×100 mL). Organic part was dried over Na₂SO₄, filtrated and the filtrate was concentrated. FCC (15-60 % EtOAc in DCM) gave **140a** (0.25 g, 0.29 mmol, 93 %). **¹H NMR** (400 MHz, DMSO-*d₆*) δ 8.14 (1H, s), 7.87 (1H, s), 7.76-7.68 (2H, m), 7.53-7.44 (3H, m), 6.09 (1H, d, *J* = 6.9 Hz), 4.79 (1H, dd, *J* = 6.9, 4.5 Hz), 4.16 (1H, dd, *J* = 4.4, 1.5 Hz), 3.96 (1H, td, *J* = 6.9, 1.5 Hz), 3.91 (1H, d, *J* = 13.1 Hz), 3.82 (1H, d, *J* = 13.1 Hz), 3.09 (1H, dd, *J* = 13.9, 6.9 Hz), 2.99 (1H, dd, *J* = 13.9, 7.2 Hz), 2.27 (3H, s), 1.10-1.04 (21H, m), 0.90, 0.68 (18H, 2×s), 0.10, 0.09, -0.10, -0.37 (12H, 4×s). **¹³C NMR** (101 MHz, DMSO-*d₆*) δ 165.0, 160.6, 157.7, 153.3, 150.2, 130.3, 129.3, 127.9, 127.3, 126.8, 111.0, 102.6, 101.0, 95.4, 92.8, 86.7, 84.9, 74.7, 74.0, 34.6, 25.9, 25.6, 24.8, 18.7, 17.9, 17.6, 10.9, 9.8, -4.5, -4.5, -4.6, -5.4. **LRMS** Calculated for C₄₅H₇₂N₅O₄SSi₂, [M+H]⁺ m/z: 862.5, [M+H]⁺, found 862.5.

### Example 161

### 4-(4-Amino-7-((2R,3R,4R,5S)-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidin-5-yl)cyclohex-3-ene-1-carbonitrile (141a):

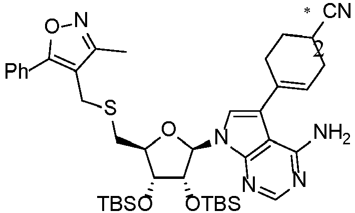

To a solution of intermediate **122** (0.18 g, 0.22 mmol), Pd(dppf)Cl₂·DCM (0.02 g, 0.02 mmol) and Na₂CO₃ (0.12 g, 1.11 mmol) in the mixture of DME/EtOH/H₂O (4:1:1, 6 mL), (4-cyanocyclohex-1-en-1-yl)boronic acid pinacol ester (0.08 g, 0.34 mmol) was added at RT under argon atmosphere and the reaction mixture was warmed to 100 °C. After stirring for 4 h, H₂O (50 mL) was added the reaction mixture was extracted with DCM (2×100 mL). Organic part was dried over Na₂SO₄, filtrated and concentrated. FCC (10-70 % EtOAc in DCM) afforded modified nucleoside **141a** (0.16 g, 0.21 mmol, 93 %) as mixture of diastereomers. **¹H NMR** (400 MHz, DMSO-*d₆)* δ 8.08 (2H, s), 7.75-7.63 (2H, m), 7.73-7.72 (2H, m), 7.51-7.47 (6H, m), 7.39 (1H, s), 7.39(1H, s), 6.38 (4H bs), 6.14 (1H, d, *J* = 7.5 Hz), 6.13 (1H, d, *J* = 7.3 Hz), 5.66-5.59 (2H, m), 4.76 (1H, dd, *J* = 7.5, 3.9 Hz), 4.76 (1H, dd, *J* = 7.8, 4.0 Hz), 4.15 (2H, d, *J=* 4.4 Hz), 3.96 (2H, t, *J* = 6.9 Hz), 3.92 (2H, d, *J=* 13.3 Hz), 3.83 (2H, d, *J* = 13.2 Hz), 3.20-3.12 (2H, m), 3.09 (1H, dd, *J* = 13.8, 7.5 Hz), 3.09 (1H, dd, *J* = 13.8, 7.4 Hz), 2.94 (1H, dd, *J* = 13.8, 6.7 Hz), 2.93 (1H, dd, *J* = 13.9, 6.7 Hz), 2.61-2.50 (2H, m), 2.46-2.32 (6H, m), 2.28 (3H, s), 2.28 (3H, s), 2.11-2.00 (2H, m), 2.11-2.00 (2H, m), 1.99-1.90 (2H, m), 0.91 (18H, s), 0.65, 0.65 (18H, 2×s), 0.11, 0.10 (12H, 2×s), -0.12 (6H, s), -0.40, -0.40 (6H, 2×s). **¹³C NMR** (101 MHz, DMSO-*d₆*) δ 165.0, 160.7, 157.4, 151.9, 151.2, 151.2, 131.4, 131.3, 130.3, 129.3, 127.3, 126.9, 123.24, 123.18, 122.8, 122.8, 120.2, 120.2, 118.0, 111.0, 100.4, 100.3, 86.2, 86.0, 84.7, 84.6, 74.8, 73.9, 73.9, 34.7, 34.6, 28.3, 28.3, 27.8, 27.7, 26.5, 25.9, 25.7, 25.2, 25.1, 24.6, 23.4, 17.9, 17.7, 9.8, -4.5, -4.5, -4.5, -4.5, -5.4, -5.4. **LRMS** Calculated for C₄₁H₅₉N₆O₄SSi₂, [M+H]⁺ m/z: 787.4, [M+H]⁺, found 787.4.

### Example 162

### (2R,3R,4S,5S)-2-(4-Amino-5-isopropyl-7H-pyrrolo[2,3-d]pyrimidin-7-yl)-5-(((((3-methyl-5-phenylisoxazol-4-yl)methyl)thio)methyl)tetrahydrofuran-3,4-diol (129b):

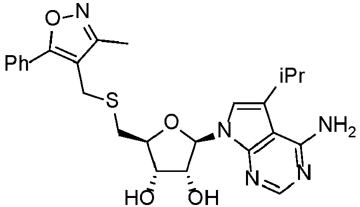

Derivative **129b** was synthesized from **129a** (0.15 g, 0.20 mmol) in analogous fashion to **47c.** Reverse-phase FCC (10-60 % MeCN in H₂O) provided **129b** (0.08 g, 0.16 mmol, 81 %). **¹H NMR** (400 MHz, DMSO-*d₆*) δ 8.05 (1H, s), 7.77-7.64 (2H, m), 7.57-7.39 (3H, m), 7.06 (1H, s), 6.62 (2H, bs), 6.05 (1H, d, *J* = 5.1 Hz), 5.34 (1H, bs), 5.22 (1H, bs), 4.51-4.38 (1H, m), 4.06-3.98 (1H, m), 3.98-3.92 (1H, m), 3.74 (1H, d, *J* = 14.3 Hz), 3.68 (1H, d, *J* = 14.2 Hz), 3.42-3.21 (1H, m), 2.87 (1H, dd, *J* = 13.2, 4.4 Hz), 2.74 (1H, dd, *J* = 13.6, 6.5 Hz), 2.38 (3H, s), 1.20 (1H, d, *J* = 6.2 Hz), 1.16 (1H, d, *J* = 6.1 Hz). **¹³C NMR** (101 MHz, DMSO-*d₆*) δ 168.5, 161.9, 157.5, 151.6, 151.5, 130.2, 129.4, 129.2, 128.3, 123.7, 117.1, 110.6, 102.0, 87.1, 83.3, 73.2, 73.0, 34.6, 25.5, 24.3, 24.1, 11.2. **HRMS** (ESI) Calculated for C₂₅H₃₀N₅O₄S, [M+H]⁺ m/z: 496.2013, [M+H]⁺, found 496.2008.

### Example 163

### (2R,3R,4S,5S)-2-(4-Amino-5-(sec-butyl)-7H-pyrrolo[2,3-d]pyrimidin-7-yl)-5-((((3-methyl-5-phenylisoxazol-4-yl)methyl)thio)methyl)tetrahydrofuran-3,4-diol (130b):

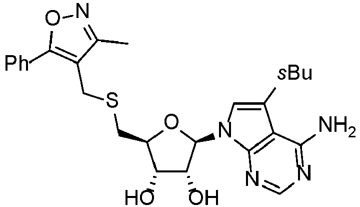

**130b** was prepared from **130a** (0.14 g, 0.19 mmol) following the same methodology as for **47c.** Reverse-phase FCC (10-60 % MeCN in H₂O) furnished **130b** (0.09 g, 0.18 mmol, 93 %) as a mixture of diastereomers. **¹H NMR** (400 MHz, DMSO-*d₆*) δ 8.04 (2H, s), 7.75-7.66 (4H, m), 7.52-7.43 (6H, m), 7.06 (2H, s), 6.57 (4H, bs), 6.06 (1H, d, *J=* 5.9 Hz), 6.06 (1H, d, *J* = 5.9 Hz), 5.33 (2H, d, *J* = 6.0 Hz), 5.21 (2H, d, *J* = 4.8 Hz), 4.49-4.40 (2H, m), 4.06-3.99 (2H, m), 3.99-3.90 (2H, m), 3.74 (1H, d, *J* = 13.7 Hz), 3.73 (1H, d, *J* = 13.7 Hz), 3.69 (1H, d, *J* = 13.6 Hz), 3.69 (1H, d, *J* = 13.6 Hz), 3.09 (2H, h, *J* = 6.6 Hz), 2.87 (2H, dd, *J* = 13.8, 5.0 Hz), 2.74 (1H, dd, *J* = 13.9, 6.8 Hz), 2.74 (1H, dd, *J=* 14.0, 6.9 Hz), 2.37 (6H, s), 1.74-1.55 (2H, m), 1.52-1.36 (2H, m), 1.17 (3H, d, *J* = 6.7 Hz), 1.13 (3H, d, *J=* 6.7 Hz), 0.88 (3H, t, *J* = 7.4 Hz), 0.81 (3H, t, *J* = 7.3 Hz). **¹³C NMR** (101 MHz, DMSO-*d₆*) δ 168.8, 162.2, 162.2, 157.7, 151.6, 151.5, 151.4, 130.5, 129.6, 129.4, 128.4, 122.7, 118.0, 118.0, 110.8, 102.5, 87.4, 87.2, 83.6, 83.5, 73.6, 73.6, 73.1, 34.9, 34.9, 32.2, 30.6, 24.6, 24.6, 21.7, 21.7, 12.0, 11.9, 11.4, 11.4. **HRMS** (ESI) Calculated for C₂₆H₃₂N₅O₄S [M+H]⁺ m/z: 510.2170, [M+H]⁺, found 510.2166.

### Example 164

### (2R,3R,4S,5S)-2-(4-Amino-5-isobutyl-7H-pyrrolo[2,3-d]pyrimidin-7-yl)-5-((((3-methyl-5-phenylisoxazol-4-yl)methyl)thio)methyl)tetrahydrofuran-3,4-diol (131b):

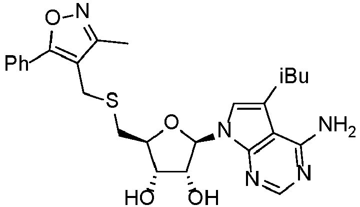

Analogue **131b** was synthesized from modified nucleoside **131a** (0.14 g, 0.19 mmol) using the same conditions as for **47c.** Reverse-phase FCC (10-60 % MeCN in H₂O) afforded title compound **131b** (0.09 g, 0.17 mmol, 90%). **¹H NMR** (400 MHz, DMSO-*d₆*) δ 8.06 (1H, s), 7.75-7.68 (2H, m), 7.53-7.45 (3H, m), 7.05 (1H, s), 6.63 (2H, bs), 6.06 (1H, d, *J* = 5.6 Hz), 5.35 (br d, *J* = 5.6), 5.22 (br d, *J* = 4.5), 4.39 (1H, q, *J* = 5.1 Hz), 4.02 (1H, q, *J* = 4.1 Hz), 3.97 (1H, q, *J* = 5.5 Hz), 3.74 (1H, d, *J* = 13.6 Hz), 3.69 (1H, d, *J* = 13.6 Hz), 2.87 (1H, dd, *J* = 14.0, 5.1 Hz), 2.74 (1H, dd, *J* = 14.0, 6.7 Hz), 2.58 (1H, dd, *J* = 6.3, 5.5 Hz), 2.38 (3H, s), 1.74 (1H, hept, *J* = 6.7 Hz), 0.86 (1H, d, *J* = 6.6 Hz), 0.83 (1H, d, *J* = 6.6 Hz), 0.90 (9H, s), 0.65 (9H, s), 0.09 (3H, s), 0.08 (3H, s), -0.14 (3H, s), -0.40 (3H, s). **¹³C NMR** (101 MHz, DMSO-*d₆*) δ 168.3, 161.7, 157.4, 151.1, 151.0, 130.0, 129.2, 129.1, 128.1, 120.1, 114.9, 110.4, 102.4, 86.9, 83.2, 73.4, 72.9, 34.9, 34.5, 29.3, 24.2, 22.31, 22.25, 11.0. **HRMS** (ESI) Calculated for C₂₆H₃₂N₅O₄S, [M+H]⁺ m/z: 510.2170, [M+H]⁺, found 510.2171.

### Example 165

### (2R,3R,4S,5S)-2-(4-Amino-5-pentyl-7H-pyrrolo[2,3-d]pyrimidin-7-yl)-5-((((3-methyl-5-phenylisoxazol-4-yl)methyl)thio)methyl)tetrahydrofuran-3,4-diol (132b):

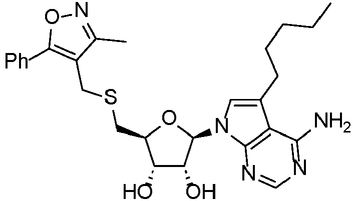

Pentyl analogue **132b** was synthesized from derivative **132a** (0,18 g, 0.23 mmol) according to the same methodology as for **47c.** Reverse-phase FCC (10-80 % MeCN in H₂O) furnished SAH analogue **132b** (0.12 g, 0.23 mmol, 97 %). **¹H NMR** (400 MHz, DMSO-*d₆*) δ 8.03 (1H, s), 7.75-7.68 (2H, m), 7.52-7.44 (3H, m), 7.03 (1H, s), 6.53 (2H, bs), 6.05 (1H, d, *J* = 5.6 Hz), 5.32 (1H, d, *J* = 5.9 Hz), 5.20 (1H, d, *J* = 5.2 Hz), 4.39 (1H, q, *J* = 5.4 Hz), 4.02 (1H, q, *J* = 4.8 Hz), 3.96 (1H, q, *J* = 5.1 Hz), 3.74 (1H, d, *J* = 13.6 Hz), 3.69 (1H, d, *J* = 13.6 Hz), 2.87 (1H, dd, *J* = 14.0, 5.2 Hz), 2.80-2.62 (3H, m), 2.37 (3H, s), 1.51 (2H, p, *J* = 7.3 Hz), 1.38-1.19 (4H, m), 0.82 (3H, t, *J* = 6.8 Hz). **¹³C NMR** (101 MHz, DMSO-*d₆*) δ 168.4, 161.8, 157.9, 151.8, 151.5, 130.1, 129.3, 129.2, 128.2, 118.9, 116.3, 110.5, 102.4, 86.9, 83.1, 73.4, 72.9, 34.6, 31.1, 30.2, 26.0, 24.3, 22.3, 14.3, 11.1. **HRMS** (ESI) Calculated for C₂₇H₃₄N₅O₄S, [M+H]⁺ m/z: 524.2326, [M+H]⁺, found 524.2325.

### Example 166

### (2R,3R,4S,5S)-2-(4-Amino-5-cyclopentyl-7H-pyrrolo[2,3-d]pyrimidin-7-yl)-5-((((3-methyl-5-phenyl isoxazol-4-yl)methyl)thio)methyl)tetrahydrofuran-3,4-diol (133b):

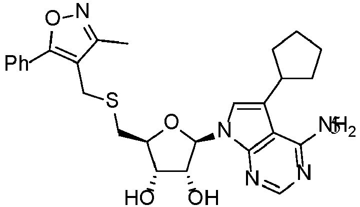

Derivative **133b** was prepared from protected analogue **133a** (0.17 g, 0.23 mmol) following the same procedure as for **47c.** Reverse-phase FCC (10-70 % MeCN in H₂O) provided **133b** (0.11 g, 0.21 mmol, 90 %). **¹H NMR** (400 MHz, DMSO-*d₆*) δ 8.04 (1H, s), 7.74-7.68 (2H, m), 7.51-7.45 (3H, m), 7.08 (1H, s), 6.56 (2H, bs), 6.06 (1H, d, *J* = 5.5 Hz), 5.32 (1H, d, *J* = 5.0 Hz), 5.20 (1H, d, *J* = 3.9 Hz), 4.44 (1H, q, *J* = 4.8 Hz), 4.08-3.99 (1H, m), 3.99-3.90 (1H, m), 3.74 (1H, d, *J* = 13.5 Hz), 3.69 (1H, d, *J* = 13.7 Hz), 3.44-3.26 (1H, m), 2.88 (1H, dd, *J* = 13.7, 4.8 Hz), 2.74 (1H, dd, *J* = 13.7, 6.7 Hz), 2.38 (3H, s), 2.08-1.88 (2H, m), 1.76-1.56 (4H, m), 1.56-1.38 (2H, m). **¹³C NMR** (101 MHz, DMSO-*d₆*) δ 168.3, 161.7, 157.6, 151.6, 151.5, 130.0, 129.2, 129.1, 128.1, 120.4, 117.0, 110.4, 102.3, 86.9, 83.1, 73.1, 72.8, 36.6, 34.5, 33.7, 33.5, 24.8, 24.7, 24.1, 11.0. **HRMS** (ESI) Calculated for C₂₇H₃₂N₅O₄S, [M+H]⁺ m/z: 522.2170, [M+H]⁺, found 522.2165.

### Example 167

### (2R,3R,4S,5S)-2-(4-Amino-5-cyclohexyl-7H-pyrrolo[2,3-d]pyrimidin-7-yl)-5-((((3-methyl-5-phenylisoxazol-4-yl)methyl)thio)methyl)tetrahydrofuran-3,4-diol (134b):

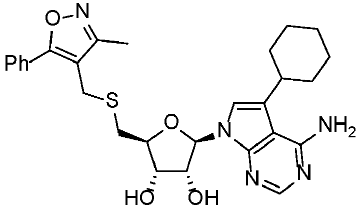

Derivative **134b** was synthesized from **134a** (0.13 g, 0.17 mmol) using the same methodology as for **47c.** Reverse-phase FCC (10-70 % MeCN in H₂O) furnished **134b** (0.08 g, 0.16 mmol, 89 %). **¹H NMR** (400 MHz, DMSO-*d₆*) δ 8.05 (1H, s), 7.76-7.67 (2H, m), 7.52-7.44 (3H, m), 7.04 (1H, s), 6.54 (2H, bs), 6.06 (1H, d, *J* = 5.8 Hz), 5.32 (1H, d, *J* = 6.1 Hz), 5.20 (1H, d, *J* = 4.7 Hz), 4.44 (1H, q, *J* = 5.2 Hz), 4.02 (1H, q, *J* = 4.2 Hz), 3.99-3.92 (1H, m), 3.74 (1H, d, *J* = 13.6 Hz), 3.69 (1H, d, *J* = 13.6 Hz), 2.97-2.83 (2H, m), 2.75 (1H, dd, *J* = 13.9, 6.8 Hz), 2.37 (3H, s), 1.95 (1H, br d, *J* = 12.6 Hz), 1.88 (1H, br d, *J* = 12.0 Hz), 1.77-1.62 (3H, m), 1.57-1.41 (2H, m), 1.30-1.11 (3H, m). **¹³C NMR** (101 MHz, DMSO-*d₆*) δ 168.4, 161.8, 157.5, 151.4, 151.2, 130.1, 129.3, 129.2, 128.2, 122.8, 117.3, 110.5, 101.9, 87.0, 83.2, 73.2, 72.9, 34.7, 34.6, 34.5, 26.2, 26.1, 24.2, 11.1. **HRMS** (ESI) Calculated for C₂₈H₃₄N₅O₄S, [M+H]⁺ m/z: 536.2326, [M+H]⁺, found 536.2324.

### Example 168

### (2R,3R,4S,5S)-2-(4-Amino-5-(cyclohexylmethyl)-7H-pyrrolo[2,3-d]pyrimidin-7-yl)-5-((((3-methyl-5-phenylisoxazol-4-yl)methyl)thio)methyl)tetrahydrofuran-3,4-diol (135b):

Title compound **135b** was prepared **135a** (0.14 g, 0.18 mmol) using the same conditions as for **47c.** Reverse-phase FCC (10-60 % MeCN in H₂O) afforded cyclohexylmethyl derivative **135b** (0.1 g, 0.17 mmol, 95 %). **¹H NMR** (400 MHz, DMSO-*d₆*) δ 8.05 (1H, s), 7.80-7.65 (2H, m), 7.52-7.41 (3H, m), 7.03 (1H, s), 6.60 (2H, bs), 6.06 (1H, d, *J* = 5.5 Hz), 5.35 (1H, bs), 5.23 (1H, bs), 4.46-4.35 (1H, 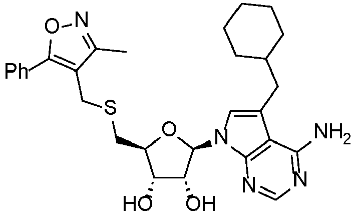 m), 4.09-3.93 (2H, m), 3.74 (1H, d, *J* = 13.4 Hz), 3.68 (1H, d, *J* = 13.6 Hz), 2.88 (1H, dd, *J* = 14.0, 4.9 Hz), 2.75 (1H, dd, *J* = 14.0, 6.6 Hz), 2.68-2.52 (2H, m), 2.36 (3H, s), 1.76-1.45 (5H, m), 1.43-1.29 (1H, m), 1.15-0.97 (3H, m), 0.95-0.71 (2H, m). **¹³C NMR** (101 MHz, DMSO-*d₆*) δ 168.4, 161.7, 157.4, 151.2, 151.1, 130.0, 12923, 129.15, 128.1, 1202, 114.4, 110.5, 102.5, 86.8, 83.4, 73.4, 72.9, 67.4, 38.9, 34.6, 33.5, 32.7, 32.6, 30.8, 26.4, 26.0, 24.4, 11.0. **HRMS** (ESI) Calculated for C₂₉H₃₆N₅O₄S, [M+H]⁺ m/z: 550.2483, [M+H]⁺, found 550.2474.

### Example 169

### (2R,3R,4S,5S)-2-(4-Amino-5-((1S,4R)-bicyclo[2.2.1]heptan-2-yl)-7H-pyrrolo[2,3-d]pyrimidin-7-yl)-5-((((3-methyl-5-phenylisoxazol-4-yl)methyl)thio)methyl)tetrahydrofuran-3,4-diol (136b):

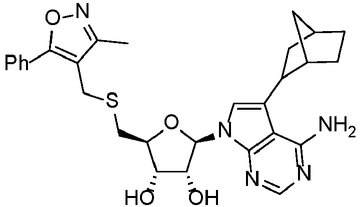

SAH analogue **136b** was synthesized from derivative **136a** (0.13 g, 0.17 mmol) according to the same procedure as for **47c.** Reverse-phase FCC (10-60 % MeCN in H₂O) furnished modified nucleoside **136b** (0.07 g, 0.13 mmol, 79 %). **¹H NMR** (400 MHz, DMSO-*d₆*) δ 8.05 (1H, s), 7.75-7.66 (2H, m), 7.52-7.43 (3H, m), 7.03 (1H, s), 6.47 (2H, bs), 6.05 (1H, d, *J* = 5.8 Hz), 5.33 (1H, br d, *J* = 5.2 Hz), 5.20 (1H, br d, *J* = 4.2 Hz), 4.46 (1H, q, *J* = 5.2 Hz), 4.03 (1H, q, *J* = 4.2 Hz), 3.99-3.90 (1H, m), 3.74 (1H, d, *J i=* 13.6 Hz), 3.68 (1H, d, *J* = 13.7 Hz), 3.08-2.98 (1H, m), 2.89 (1H, dd, *J* = 13.9, 5.4 Hz), 2.74 (1H, dd, *J* = 14.0, 6.9 Hz), 2.37 (3H, s), 2.30-2.20 (2H, m), 1.73-1.62 (1H, m), 1.54-1.43 (4H, m), 1.36 (1H, dd, *J* = 17.3, 9.3 Hz), 1.28-1.18 (1H, m) 1.00 (1H, dd, *J* = 17.3, 9.4 Hz). **¹³C NMR** (101 MHz, DMSO-*d₆*) δ 168.5, 161.9, 157.6, 151.7, 151.4, 130.1, 129.3, 129.2, 128.2, 122.0, 117.5, 110.5, 102.1, 87.0, 83.2, 73.2, 72.9, 42.8, 42.5, 38.8, 37.9, 37.6 36.9, 36.7, 35.7, 35.5, 34.6, 29.2, 29.0, 24.4, 11.1. **HRMS** (ESI) Calculated for C₂₉H₃₄N₅O₄S, [M+H]⁺ m/z: 548.2326, [M+H]⁺, found 548.2322.

### Example 170

### 4-(4-Amino-7-((2R,3R,4S,5S)-3,4-dihydroxy-5-((((3-methyl-5-phenylisoxazol-4-yl)methyl)thio)methyl)tetrahydrofuran-2-yl)-7H-pyrrolo[2,3-d]pyrimidin-5-yl)butanenitrile (137b):

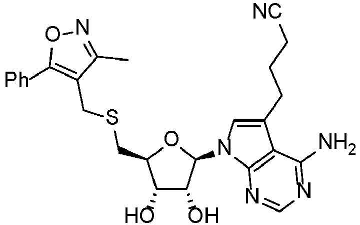

Modified nucleoside **137b** was prepared from protected analogue **137a** (0.25 g, 0.33 mmol) in analogous fashion to **47c.** Reverse-phase FCC (10-60 % MeCN in H₂O) furnished **137b** (0.16 g, 0.31 mmol, 95 %). **¹H NMR** (400 MHz, DMSO-*d₆*) δ 8.05 (1H, s), 7.78-7.72 (2H, m), 7.55-7.45 (3H, m), 7.13 (1H, s), 6.61 (2H, bs), 6.08 (1H, d, *J* = 5.9 Hz), 5.35 (1H, d, *J* = 6.2 Hz), 5.23 (1H, d, *J* = 5.2 Hz), 4.44 (1H, q, *J* = 5.9 Hz), 4.05 (1H, q, *J* = 5.1 Hz), 4.02-3.97 (1H, m), 3.86 (1H, d, *J* = 13.0 Hz), 3.81 (1H, d, *J* = 13.0 Hz), 2.95 (1H, dd, *J* = 13.9, 5.4 Hz), 2.86 (1H, dd, *J* = 13.9, 6.9 Hz), 2.80 (2H, t, *J* = 7.7 Hz), 2.53-2.46 (2H, m), 2.26 (3H, s), 1.79 (2H, p, *J* = 7.3 Hz). **¹³C NMR**(101 MHz, DMSO-*d₆*) δ 165.4, 161.3, 157.9, 152.1, 151.8, 130.7, 129.8, 127.2, 121.2, 119.8, 114.5, 111.5, 102.5, 87.0, 83.5, 73.5, 73.2, 35.2, 26.7, 25.4, 24.9, 16.1, 10.1. **HRMS** (ESI) Calculated for C₂₆H₂₉N₆O₄S, [M+H]⁺ m/z: 521.1966, [M+H]⁺, found 521.1965.

### Example 171

### 4-Amino-7-((2R,3R,4S,5S)-3,4-dihydroxy-5-((((3-methyl-5-phenylisoxazol-4-yl)methyl)thio)methyl)tetrahydrofuran-2-yl)-7H-pyrrolo[2,3-d]pyrimidine-5-carbonitrile (138b):

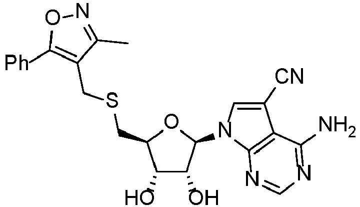

Analogue **138b** was prepared from **138a** (0.05 g, 0.07 mmol) using the same conditions as for **47c.** Reverse-phase FCC (10-60 % MeCN in H₂O) afforded **138b** (0.03 g, 0.07 mmol, 95 %). **¹H NMR** (400 MHz, DMSO-*d₆*) δ 8.41 (1H, s), 8.23 (1H, s), 7.81-7.73 (2H, m), 7.56-7.47 (3H, m), 6.89 (2H, bs), 6.10 (1H, d, *J* = 5.7 Hz), 5.55 (1H, d, *J* = 5.8 Hz), 5.36 (1H, d, *J* = 3.6 Hz), 4.51 (1H, q, *J* = 4.6 Hz), 4.11-4.02 (2H, m), 3.86 (1H, d, *J* = 13.1 Hz), 3.82 (1H, d, *J* = 13.1 Hz), 2.97 (1H, dd, *J* = 14.1, 5.7 Hz), 2.93 (1H, dd, *J* = 13.9, 6.8 Hz), 2.28 (3H, s). **¹³C NMR** (101 MHz, DMSO-*d₆*) δ 164.9, 160.8, 157.2, 153.9, 150.6, 132.7, 130.3, 129.4, 127.3, 126.9, 115.4, 111.1, 101.4, 87.8, 84.0, 83.7, 73.5, 72.8, 34.7, 24.4, 9.8. **HRMS** (ESI) Calculated for C₂₃H₂₃N₆O₄S, [M+H]⁺ m/z: 479.1496, [M+H]⁺, found 479.1493.

### Example 172

### (2R,3R,4S,5S)-2-(4-Amino-5-vinyl-7H-pyrrolo[2,3-d]pyrimidin-7-yl)-5-((((3-methyl-5-phenylisoxazol-4-yl)methyl)thio)methyl)tetrahydrofuran-3,4-diol (139b):

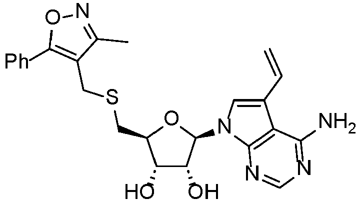

Analogue **139b** was prepared from **139a** (0.27 g, 0.38 mmol) in analogous fashion to **47c.** Reverse-phase FCC (10-60 % MeCN in H₂O) gave **139b** (0.16 g, 0.33 mmol, 87 %). **¹H NMR** (400 MHz, DMSO-*d₆*) δ 8.07 (1H, s), 7.79-7.70 (2H, m), 7.63 (1H, s), 7.57-7.44 (3H, m), 7.10 (1H, dd, *J=* 17.2, 10.9 Hz), 6.72 (2H, bs), 6.11 (1H, d, *J* = 6.0 Hz), 5.56 (1H, dd, *J=* 17.2, 1.4 Hz), 5.41 (1H, d, *J* = 6.2 Hz), 5.27 (1H, d, *J* = 5.1 Hz), 5.12 (1H, dd, *J* = 10.9, 1.5 Hz), 4.50 (1H, q, *J* = 5.9 Hz), 4.10-3.99 (2H, m), 3.87 (1H, d, *J* = 13.0 Hz), 3.82 (1H, d, *J* = 13.0 Hz), 2.97 (1H, dd, *J* = 13.9, 5.7 Hz), 2.89 (1H, dd, *J* = 13.9, 6.9 Hz), 2.27 (3H, s). **¹³C NMR** (101 MHz, DMSO-*d₆*) δ 164.9, 160.8, 157.8, 151.9, 151.4, 130.3, 129.4, 129.1, 127.3, 126.8, 118.9, 114.8, 113.4, 111.1, 100.8, 86.8, 83.3, 73.2, 72.9, 34.8, 24.4, 9.8. **HRMS** (ESI) Calculated for C₂₄H₂₆N₅O₄S, [M+H]⁺ m/z: 480.1700, [M+H]⁺, found 480.1691.

### Example 173

### (2R,3R,4S,5S)-2-(4-Amino-5-ethynyl-7H-pyrrolo[2,3-d]pyrimidin-7-yl)-5-((((3-methyl-5-phenylisoxazol-4-yl)methyl)thio)methyl)tetrahydrofuran-3,4-diol (140b):

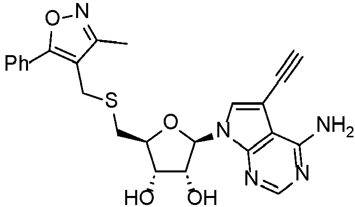

A solution of ethynyl derivative **140a** (0.25 g, 0.29 mmol), CsF (0.18 g, 1.15 mmol) and dry MeOH (0.28 mL) in dry DMSO (2 mL) was stirred at RT under argon atmosphere for 10 h. The volatiles were lyophilized. FCC (10-70 % MeCN in H₂O) gave **140b** (0.08 g, 0.16 mmol, 60 %). **¹H NMR** (400 MHz, DMSO-*d₆*) δ 8.14 (1H, s), 7.79 (1H, s), 7.78-7.74 (2H, m), 7.56-7.47 (3H, m), 6.67 (2H, bs), 6.06 (1H, d, *J* = 6.0 Hz), 5.45 (1H, d, *J* = 6.2 Hz), 5.30 (1H, d, *J* = 5.0 Hz), 4.49 (1H, q, *J* = 6.0 Hz), 4.29 (1H, s), 4.10-3.97 (2H, m), 3.86 (1H, d, *J* = 13.0 Hz), 3.81 (1H, d, *J* = 13.0 Hz), 2.96 (1H, dd, *J* = 13.8, 5.7 Hz), 2.89 (1H, dd, *J* = 13.9, 6.8 Hz), 2.28 (3H, s). **¹³C NMR** (101 MHz, DMSO-*d₆*) δ 164.9, 160.8, 157.7, 153.2, 150.1, 130.3, 129.4, 127.5, 127.3, 126.9, 111.1, 102.5, 94.9, 87.1, 83.3, 77.4, 73.2, 72.8, 34.8, 24.4, 9.8. **HRMS** (ESI) Calculated for C₂₄H₂₄N₅O₄S, [M+H]⁺ m/z: 478.1544, [M+H]⁺, found 478.1535.

### Example 174

### 4-(4-Amino-7-((2R,3R,4S,5S)-3,4-dihydroxy-5-((((3-methyl-5-phenyhsoxazol-4-yl)methyl)thio) methyl)tetrahydrofuran-2-yl)-7H-pyrrolo[2,3-d]pyrimidin-5-yl)cyclohex-3-ene-1-carbonitrile (141b):

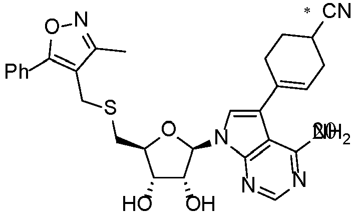

SAH analogue **141b** was prepared from derivative **141a** (0.15 g, 0.19 mmol) using the same conditions as for **47c.** Reverse-phase FCC (10-60 % MeCN in H₂O) afforded title compound **141b** (0.09 g, 0.16 mmol, 84 %) as a mixture of diastereomers. **¹H NMR** (400 MHz, DMSO-*d₆*) δ 8.11 (2H, s), 7.78-7.71 (4H, m), 7.63 (1H, s), 7.55-7.46 (6H, m), 7.41 (1H, s), 7.39 (1H, s), 6.45 (4H, bs), 6.12 (1H, d, *J i=* 6.2 Hz), 6.11 (1H, d, *J=* 6.1 Hz), 5.63 (2H, bs), 5.40 (1H, d, *J* = 5.8 Hz), 5.38 (1H, d, *J* = 5.8 Hz), 5.26 (2H, d, *J* = 4.2 Hz), 4.48 (2H, q, *J* = 4.8 Hz), 4.10-3.95 (4H, m), 3.86 (2H, d, *J* = 13.0 Hz), 3.81 (2H, d, *J* = 13.0 Hz), 3.20-3.09 (2H, m), 2.96 (2H, dd, *J* = 13.9, 5.6 Hz), 2.87 (2H, dd, *J* = 13.9, 6.9 Hz), 2.60-2.48 (4H, m), 2.45-2.34 (4H, m), 2.26, 2.26 (3H, 2×s), 2.10-1.99 (2H, m), 1.90-1.87 (2H, m). **¹³C NMR** (101 MHz, DMSO-*d₆*) δ 165.2, 161.1, 157.1, 151.5, 151.0, 151.0, 131.4, 130.6, 129.6, 127.5, 127.0, 123.5, 123.22, 123.19, 120.4, 118.5, 111.3, 100.3, 100.3, 86.9, 86.9, 83.6, 83.5, 73.3, 73.3, 73.0, 35.0, 28.5, 27.7, 27.6, 35.3, 25.3, 24.7, 23.6, 9.9. **HRMS** (ESI) Calculated for C₂₄H₂₆N₅O₄S, [M+H]⁺ m/z: 480.1700, [M+H]⁺, found 480.1691.

### Example 175

### (2R,3R,4S,5S)-2-(4-Amino-5-ethyl-7H-pyrrolo[2,3-d]pyrimidin-7-yl)-5-((((3-methyl-5-phenylisoxazol-4-yl)methyl)thio)methyl)tetrahydrofuran-3,4-diol (142):

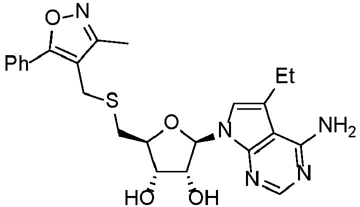

Ethyl derivative **142** was synthesized from vinyl analogue **139b** (0.1 g, 0.21 mmol) following the similar procedure as for **6.** Reverse-phase FCC (10-50 % MeCN in H₂O) furnished modified nucleoside **142** (0.06 g, 0.13 mmol, 60 %). **¹H NMR** (400 MHz, DMSO-*d₆*) δ 8.09 (1H, s), 7.79-7.72 (2H, m), 7.55-7.47 (3H, m), 7.11 (1H, s), 6.83 (2H, bs), 6.07 (1H, *d, J=* 5.7 Hz), 5.36 (1H, bs), 5.25 (1H, bs), 4.45 (1H, t, *J=* 5.3 Hz), 4.08-3.97 (2H, m), 3.86 (1H, d, *J=* 13.0 Hz), 3.81 (1H, d, *J=* 13.0 Hz), 2.95 (1H, dd, *J=* 13.9, 5.5 Hz), 2.86 (1H, dd, *J=* 13.9, 6.9 Hz), 2.75 (2H, q, *J=* 7.3 Hz), 2.26 (3H, s), 1.15 (3H, t, *J* = 7.3 Hz). **¹³C NMR** (101 MHz, DMSO-*d₆*) δ 164.9, 160.8, 156.6, 150.9, 150.1, 130.3, 129.4, 127.3, 126.8, 118.7, 118.2, 111.1, 102.0, 86.7, 83.2, 73.1, 72.8, 34.9, 24.5, 19.3, 15.0, 9.8. **HRMS** (ESI) Calculated for C₂₄H₂₈N₅O₄S, [M+H]⁺ m/z: 482.1857, [M+H]⁺, found 482.1852.

Aromatic substituents were installed into the C-7 position using the Suzuki-Miyaura and the Stille cross-coupling on either **122** or the deprotected **123** to afford compounds **143a-148a** and **149-156.** Subsequent deprotection with HF-TEA furnished derivatives **143b-148b.** Hydrogenation of the nitro group of **144b** to amino group by hydrogenation gave nucleoside **157.** This way we obtained a small library of compounds containing substitution with variously modified phenyls (compounds **143b-147b, 149, 150, 157),** bicyclic (het)aryls (compounds **148b** and **151)** and five- and six-membered hetaryls (compounds **152-156, Scheme 9).**

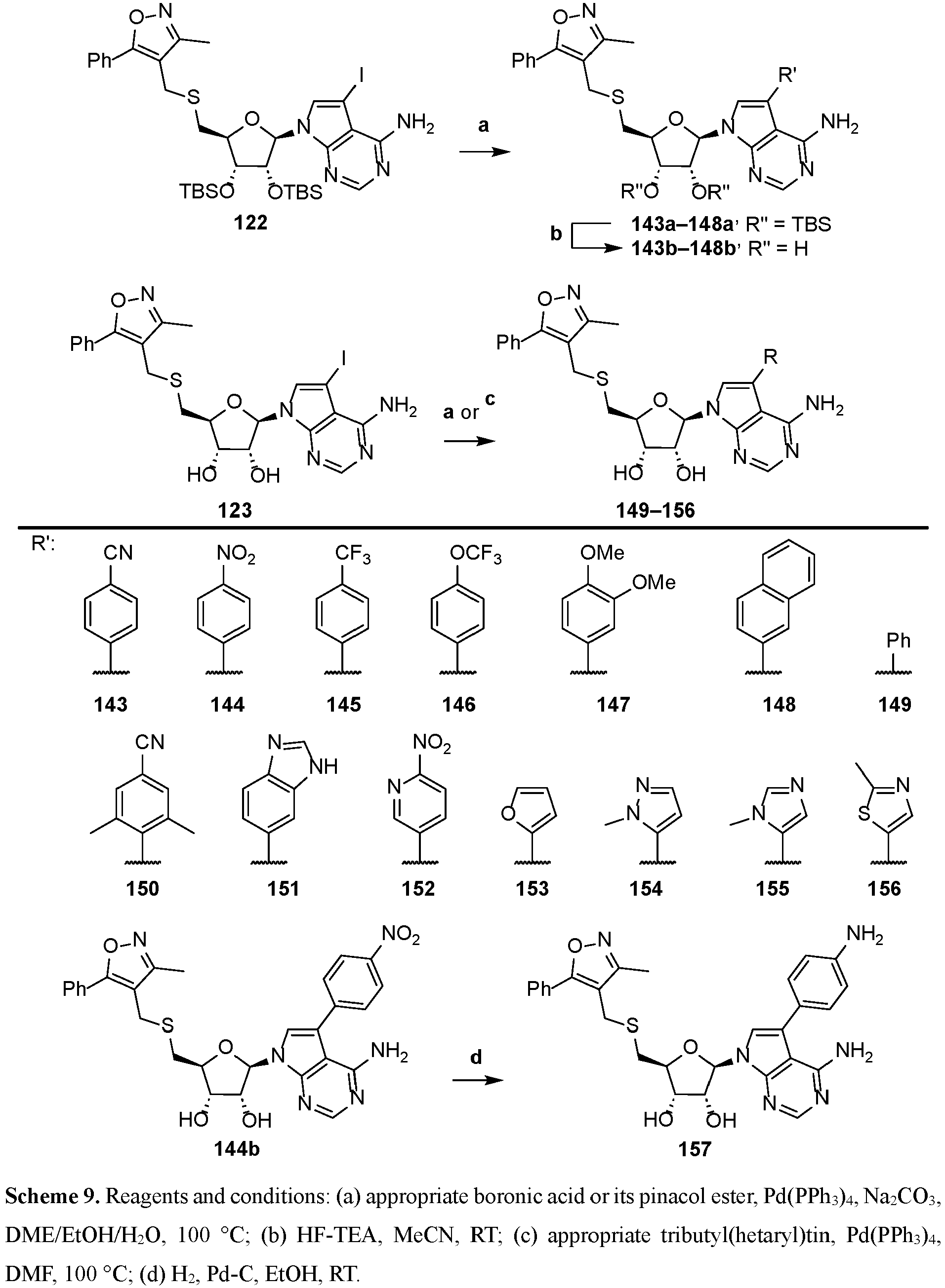

### Example 176

### 4-(4-Amino-7-((2R,3R,4R,5S)-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidin-5-yl)benzonitrile (143a):

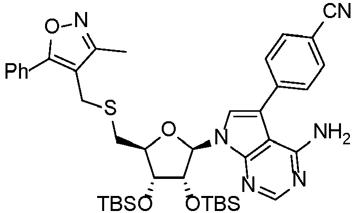

A suspension of **122** (0.2 g, 0.25 mmol), Pd(PPh₃)₄ (0.03 g, 0.03 mmol), Na₂CO₃ (0.13 g, 1.25 mmol) and (4-cyanophenyl) boronic acid (0.07 g, 0.50 mmol) in the mixture of DME/EtOH/H₂O (4:1:1, 6 mL) was placed in an inert atmosphere and heated to the 100 °C. After stirring for 10 h, H₂O (50 mL) was added and the reaction mixture was extracted with DCM (2×100 mL). Organic layers were dried over Na₂SO₄, filtrated and concentrated. FCC (10-60 % acetone in cyclohexane) gave **143a** (0.16 g, 0.20 mmol, 80 %). **¹H NMR** (400 MHz, DMSO-*d₆*) δ 8.17 (1H, s), 7.91 (2H, ddd, *J* = 8.3, 1.9, 1.4 Hz), 7.78-7.69 (3H, m), 7.58 (2H, ddd, *J* = 8.3, 2.0, 1.4 Hz), 7.51-7.43 (3H, m), 6.31 (2H, bs), 6.20 (1H, d, *J=* 7.2 Hz), 4.83 (1H, dd, *J=* 7.2, 4.4 Hz), 4.18 (1H, dd, *J* = 4.3, 1.1 Hz), 3.99 (1H, td, *J* = 7.2, 1.1 Hz), 3.91 (1H, d, *J* = 13.2 Hz), 3.82 (1H, d, *J* = 13.2 Hz), 3.11 (1H, dd, *J=* 13.8, 7.4 Hz), 2.96 (1H, dd, *J=* 14.0, 6.7 Hz), 2.26 (3H, s), 0.92, 0.67 (18H, 2×s), 0.12, 0.11, -0.10, -0.36 (12H, 4×s). **¹³C NMR** (101 MHz, DMSO-*d₆*) δ 165.0, 160.6, 157.5, 152.3, 152.1, 139.4, 132.9, 130.3, 129.3, 129.1, 127.3, 126.8, 122.7, 119.3, 111.0, 109.2, 100.2, 86.5, 84.8, 74.8, 74.1, 34.6, 25.9, 25.6, 24.6, 17.9, 17.7, 9.8, -4.5, -4.5, -4.5, -5.4. **LRMS** Calculated for C₄₁H₅₅N₆O₄SSi₂, [M+H]⁺ m/z: 783.4, [M+H]⁺, found 783.4.

### Example 177

### 7-((2R,3R,4R,5S)-3,4-bis((tert-Butyldlmethylsilyl)oxy)-5-((((3-methyl-5-phenyllsoxazol-4-yl)methyl) thio)methyl)tetrahydrofuran-2-yl)-5-(4-nitrophenyl)-7H-pyrrolo[2,3-d]pyrimidin-4-amine (144a):

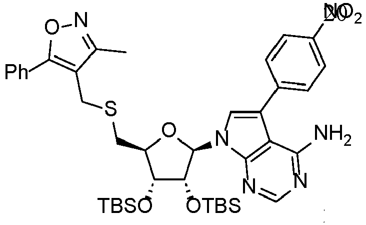

SAH analogue **144a** was prepared from **122** (0.2 g, 0.25 mmol) and (4-nitrophenyl)boronic acid (0.08 g, 0.50 mmol) following the same procedure as for **143a.** FCC (10-60 % acetone in cyclohexane) afforded **144a** (0.15 g, 0.19 mmol, 77 %). **¹H NMR** (400 MHz, DMSO-*d₆*) δ 8.31 (2H, dt, *J=* 8.8, 2.0 Hz), 8.18 (1H, s), 7.79 (1H, s), 7.76-7.69 (2H, m), 7.65 (2H, dt, *J* = 8.8, 2.0 Hz), 7.48-7.44 (3H, m), 6.38 (2H, bs), 6.21 (1H, d, *J=* 7.2 Hz), 4.85 (1H, dd, *J=* 7.2, 4.4 Hz), 4.18 (1H, dd, *J=* 4.4, 1.2 Hz), 4.00 (1H, td, *J=* 7.5, 1.0 Hz), 3.91 (1H, d, *J=* 13.3 Hz), 3.82 (1H, d, *J=* 13.3 Hz), 3.11 (1H, dd, *J=* 13.8, 7.4 Hz), 2.97 (1H, dd, *J* = 13.8, 6.7 Hz), 2.26 (3H, s), 0.92, 0.67 (18H, 2×s), 0.12, 0.11, -0.10, -0.37 (12H, 4×s). **¹³C NMR** (101 MHz, DMSO-*d₆*) δ 165.0, 160.7, 157.6, 152.4, 152.3, 146.1, 141.6, 130.3, 129.4, 129.2, 127.3, 126.9, 124.3, 123.2, 115.6, 111.1, 100.2, 86.6, 84.9, 74.8, 74.2, 34.6, 25.9, 25.7, 24.6, 17.9, 17.7, 9.8, -4.4, -4.5, -4.5, -5.4. **LRMS** Calculated for C₄₀H₅₅N₆O₆SSi₂, [M+H]⁺ m/z: 803.3, [M+H]⁺, found 803.3.

### Example 178

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(4-(trifluoromethyl)phenyl)-7H-pyrrolo[2,3-d]pyrimidin-4-amine (145a):

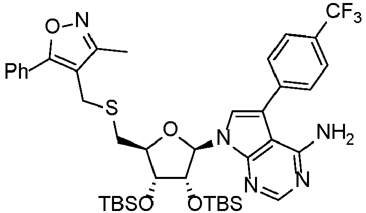

Title compound **145a** was synthesized from **122** (0.2 g, 0.25 mmol) and (4-(trifluoromethyl)phenyl)boronic acid (0.1 g, 0.50 mmol) in analogous fashion to **143a.** FCC (10-50 % acetone in cyclohexane) furnished SAH analogue **145a** (0.16 g, 0.20 mmol, 79 %). **¹H NMR** (400 MHz, DMSO-*d₆*) δ 8.17 (1H, s), 7.81 (2H, d, *J* = 8.1 Hz), 7.75-7.71 (2H, m), 7.68 (1H, s), 7.62 (2H, d, *J* = 8.0 Hz) 7.49-7.44 (3H, m), 6.26 (2H, bs), 6.21 (1H, d, *J* = 7.2 Hz), 4.84 (1H, dd, *J=* 7.2, 4.4 Hz), 4.18 (1H, dd, *J=* 4.4, 1.2 Hz), 4.00 (1H, td, *J=* 7.1, 1.0 Hz), 3.92 (1H, d, *J=* 13.3 Hz), 3.82 (1H, d, *J* = 13.2 Hz), 3.11 (1H, dd, *J* = 13.8, 7.4 Hz), 2.96 (1H, dd, *J* = 13.8, 6.8 Hz), 2.26 (3H, s), 0.92, 0.67 (18H, 2×s), 0.12, 0.11, -0.09, -0.36 (12H, 4×s). **¹³C NMR** (101 MHz, DMSO-*d₆*) *δ* 165.0, 160.7, 157.5, 152.2, 152.0, 138.7, 130.3, 129.3, 129.0, 127.3, 127.2 (q, *J* = 32.3 Hz), 126.8, 125.9 (q, *J* = 3.9 Hz), 124.7 (q, *J* = 272.1 Hz), 122.4, 115.9, 111.0, 100.3, 86.5, 84.8, 74.8, 74.2, 34.6, 25.9, 25.6, 24.6, 17.9, 17.7, 9.8, -4.46, -4.5, -4.5, -5.4. **¹⁹F NMR** (377 MHz, DMSO-*d6*) δ -60.73. **LRMS** Calculated for C₄₁H₅₅F₃N₅O₄SSi₂, [M+H]⁺ m/z: 826.4, [M+H]⁺, found 826.4.

### Example 179

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(4-(trifluoromethoxy)phenyl)-7H-pyrrolo[2,3-d]pyrimidin-4-amine (146a):

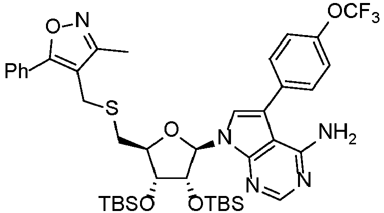

Derivative **146a** was prepared from **122** (0.2 g, 0.25 mmol) and (4-(trifluoromethoxy)phenyl)boronic acid (0.1 g, 0.50 mmol) in analogous fashion to **143a.** FCC (10-50 % acetone in cyclohexane) gave **146a** (0.16 g, 0.19 mmol, 78 %). **¹H NMR** (400 MHz, DMSO-*d₆*) δ 8.15 (1H, s), 7.75-7.71 (2H, m), 7.59 (1H, s), 7.53-7.50 (2H, m), 7.49-7.40 (5H, m), 6.20 (1H, d, *J=* 7.2 Hz), 6.18 (2H, bs), 4.81 (1H, dd, *J=* 7.2, 4.4 Hz), 4.18 (1H, dd, *J=* 4.4, 1.3 Hz), 3.98 (1H, td, *J=* 7.0, 1.3 Hz), 3.92 (1H, d, *J=* 13.2 Hz), 3.82 (1H, d, *J=* 13.3 Hz), 3.10 (1H, dd, *J* = 13.8, 7.4 Hz), 2.95 (1H, dd, *J* = 13.8, 6.7 Hz), 2.26 (3H, s), 0.92, 0.67 (18H, 2×s), 0.12, 0.11, -0.10, -0.35 (12H, 4×s). **¹³C NMR** (101 MHz, DMSO-*d₆*) δ 165.0, 160.7, 157.5, 152.1, 151.7, 147.5 (q, *J* = 1.8 Hz), 133.9, 130.3, 129.3, 127.31, 127.29, 126.8, 121.7, 120.3 (q, *J* = 256.1 Hz), 115.8, 111.0, 100.5, 86.4, 84.7, 74.8, 74.2, 34.7, 25.9, 25.6, 24.6, 17.9, 17.7, 9.8, -4.5, -4.5, -4.6, -5.4. **¹⁹F NMR** (377 MHz, DMSO-*d6*) δ -56.71. **LRMS** Calculated for C₄₁H₅₅F₃N₅O₄SSi₂, [M+H]⁺ m/z: 826.4, [M+H]⁺, found 826.4.

### Example 180

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(3,4-dimethoxyphenyl)-7H-pyrrolo[2,3-D]pyrimidin-4-amine (147a):

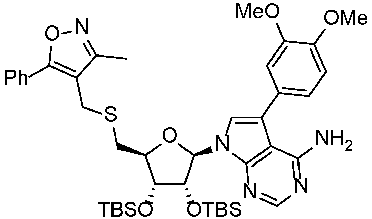

Modified nucleoside **147a** was synthesized from **122** (0.2 g, 0.25 mmol) and (3,4-dimethoxyphenyl)boronic acid (0.09 g, 0.50 mmol) in analogous fashion to **143a.** FCC (10-60 % acetone in cyclohexane) furnished **147a** (0.17 g, 0.20 mmol, 82 %). **¹H NMR** (400 MHz, DMSO-*d₆*) δ 8.14 (1H, s), 7.75-7.69 (2H, m), 7.48-7.42 (4H, m), 7.05 (1H, d, *J=* 8.8 Hz), 6.93-6.90 (2H, m), 6.19 (1H, *d, J=* 7.2 Hz), 6.10 (2H, bs), 4.81 (1H, dd, J = 7.2, 4.4 Hz), 4.16 (1H, dd, *J* = 4.4, 1.1 Hz), 3.99 (1H, td, *J* = 7.1, 1.1 Hz), 3.93 (1H, d, *J* = 13.2 Hz), 3.83 (1H, d, *J* = 13.2 Hz), 3.79 (3H, s), 3.74 (3H, s), 3.08 (1H, dd, *J* = 13.9, 7.2 Hz), 2.95 (1H, dd, *J* = 13.8, 6.9 Hz), 2.26 (3H, s), 0.91, 0.68 (18H, 2×s), 0.11, 0.11, -0.09, -0.33 (12H, 4×s). **¹³C NMR** (101 MHz, DMSO-*d₆*) δ 165., 160.7, 157.5, 152.0, 151.4, 149.1, 148.3, 130.3, 129.3, 127.3, 127.0, 126.9, 120.8, 120.6, 117.1, 112.6, 112.5, 111.1, 101.0, 86.3, 84., 74.8, 74.1, 55.8, 55.5, 34.8, 26.0, 25.7, 24.7, 18.0, 17.7, 9.8, -4.4, -4.5, -4.5, -5.3. **LRMS** Calculated for C₄₂H₆₀N₅O₆SSi₂, [M+H]⁺ m/z: 818.4, [M+H]⁺, found 818.4.

### Example 181

### 7-((2R,3R,4R,5S)-3,4-bis((tert-Butyldimethylsilyl)oxy)-5-((((3-methyl-5-phenylisoxazol-4-yl)methyl) thio)methyl)tetrahydrofuran-2-yl)-5-(naphthalen-2-yl)-7H-pyrrolo[2,3-d]pyrimidin-4-amine (148a):

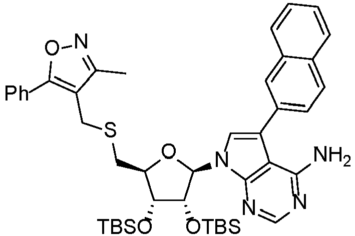

SAH analogue **148a** was prepared from modified nucleoside **122** (0.2 g, 0.25 mmol) and naphthalen-2-ylboronic acid (0.08 g, 0.50 mmol) using the same procedure as for **143a.** FCC (10-50 % acetone in cyclohexane) afforded title compound **148a** (0.16 g, 0.20 mmol, 80 %). **¹H NMR** (400 MHz, DMSO-*d₆*) δ 8.18 (1H, s), 8.02 (1H, d, *J=* 8.6 Hz), 7.99-7.95 (1H, m); 7.93-7.89 (2H, m), 7.74-7.70 (2H, m), 7.65 (1H, s); 7.60-7.51 (3H, m), 7.48-7.38 (3H, m), 6.25 (1H, d, *J=* 7.2 Hz), 6.17 (2H, bs), 4.86 (1H, dd, *J=* 7.2, 4.4 Hz), 4.19 (1H, dd, *J=* 4.4, 1.2 Hz), 4.01 (1H, td, *J=* 6.9, 1.1 Hz), 3.93 (1H, *d, J=* 13.2 Hz), 3.84 (1H, d, *J=* 13.2 Hz), 3.12 (1H, dd, *J* = 13.8, 7.3 Hz), 2.98 (1H, dd, *J* = 13.8, 6.8 Hz), 2.26 (3H, s), 0.93, 0.69 (18H, 2×s), 0.12, 0.12, -0.07, -0.31 (12H, 4×s). **¹³C NMR** (101 MHz, DMSO-*d₆*) δ 165.0, 160.7, 157.5, 152.1, 151.8, 133.3, 132.06, 132.05, 130.3, 129.3, 128.6, 127.92, 127.85, 127.3, 127.1, 126.82, 126.79, 126.76, 126.1, 121.5, 117.1, 111.0, 100.8, 86.4, 84.7, 74.8, 74.2, 34.7, 25.9, 25.7, 24.7, 17.9, 17.7, 9.8, -4.46, -4.48, -4.51, -5.3. **LRMS** Calculated for C₄₄H₅₈N₅O₄SSi₂, [M+H]⁺ m/z: 808.4, [M+H]⁺, found 808.4.

### Example 182

### 4-(4-Amino-7-((2R,3R,4S,5S)-3,4-dihydroxy-5-((((3-methyl-5-phenylisoxazol-4-yl)methyl)thio)methyl)tetrahydrofuran-2-yl)-7H-pyrrolo[2,3-d]pyrimidin-5-yl)benzonitrile (143b):

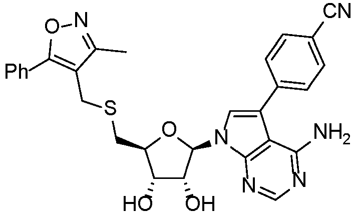

Modified nucleoside **143b** was synthesized from protected analogue **143a** (0.15 g, 0.19 mmol) using the same conditions as for **47c.** Reverse-phase FCC (10-70 % MeCN in H₂O) provided derivative **143b** (0.1 g, 0.17 mmol, 89 %). **¹H NMR** (400 MHz, DMSO-*d₆*) δ 8.19 (1H, s), 7.90 (2H, dt, *J =* 8.5, 1.8 Hz), 7.77-7.72 (2H, m), 7.70 (1H, s), 7.62 (2H, dt, *J=* 8.5, 1.8 Hz), 7.55-7.42 (3H, m), 6.33 (2H, bs), 6.18 (1H, d, *J=* 5.9 Hz), 5.46 (1H, d, *J=* 6.3 Hz), 5.30 (1H, d, *J=* 5.1 Hz), 4.56 (1H, q, *J* = 5.9 Hz), 4.14-4.00 (2H, m), 3.86 (1H, *d, J=* 13.0 Hz), 3.81 (1H, d, *J=* 13.0 Hz), 2.98 (1H, dd, *J=* 13.9, 5.7 Hz), 2.90 (1H, dd, *J=* 13.9, 6.9 Hz), 2.24 (3H, s). **¹³C NMR** (101 MHz, DMSO-*d₆*) δ 164.9, 160.8, 157.6, 152.3, 152.0, 139.4, 132.9, 130.3, 129.4, 129.1, 127.3, 126.8, 122.6, 119.3, 115.8, 111.1, 109.1, 100.1, 87.0, 83.4, 73.2, 72.9, 34.8, 24.4, 9.7. **HRMS** (ESI) Calculated for C₂₉H₂₇N₆O₄S, [M+H]⁺ m/z: 555.1809, [M+H]⁺, found 555.1808.

### Example 183

### (2R,3R,4S,5S)-2-(4-Amino-5-(4-nitropheny1)-7H-pyrrolo[2,3-d]pyrimidin-7-y1)-5-(((3-methyl-5-phenylisoxazol-4-yl)methyl)thio)methyl)tetrahydrofuran-3,4-diol (144b):

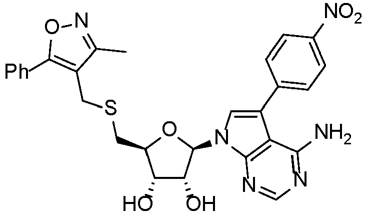

Derivative **144b** was prepared from modified nucleoside **144a** (0.14 g, 0.18 mmol) according to the same methodology as for **47c.** Reverse-phase FCC (10-70 % MeCN in H₂O) furnished derivative **144b** (0.09 g, 0.17 mmol, 92 %). **¹H NMR** (400 MHz, DMSO-*d₆*) δ 8.30 (2H, dt, *J* = 8.8, 1.9 Hz), 8.21 (1H, s), 7.78 (1H, s), 7.74 (2H, dd, *J =* 8.0, 1.6 Hz), 7.69 (2H, dt, *J=* 8.9, 1.9 Hz), 7.54-7.42 (3H, m), 6.41 (2H, bs), 6.20 (1H, d, *J=* 5.9 Hz), 5.47 (1H, d, *J=* 6.3 Hz), 5.31 (1H, d, *J=* 5.1 Hz), 4.58 (1H, q, *J=* 5.9 Hz), 4.15-4.01 (2H, m), 3.86 (1H, d, *J=* 13.0 Hz), 3.81 (1H, d, *J* = 13.0 Hz), 2.99 (1H, dd, *J* = 13.9, 5.7 Hz), 2.91 (1H, dd, *J=* 13.9, 6.9 Hz), 2.25 (3H, s). **¹³C NMR** (101 MHz, DMSO-*d₆*) δ 164.9, 160.8, 157.6, 152.4, 152.1, 145.9, 141.6, 130.3, 129.4, 129.2, 127.3, 126.8, 124.2, 123.0, 115.4, 111.1, 100.0, 87.1, 83.5, 73.3, 72.9, 34.8, 24.4, 9.7. **HRMS** (ESI) Calculated for C₂₈H₂₇N₆O₆S, [M+H]⁺ m/z: 575.1707, [M+H]⁺, found 575.1706.

### Example 184

### (2R,3R,4S,5S)-2-(4-Amino-5-(4-(trifluoromethyl)phenyl)-7H-pyrrolo[2,3-d]pyrimidin-7-yl)-5-(((3-methyl-5-phenylisoxazol-4-yl)methyl)thio)methyl)tetrahydrofuran-3,4-diol (145b):

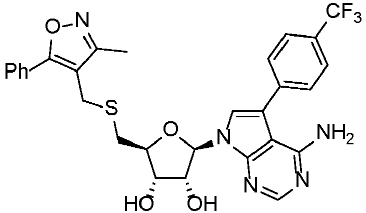

Title compound **145b** was synthesized from modified nucleoside **145a** (0.15 g, 0.19 mmol) following the same procedure as for **47c.** Reverse-phase FCC (10-80 % MeCN in H₂O) gave SAH analogue **145b** (0.09 g, 0.16 mmol, 84 %). **¹H NMR** (400 MHz, DMSO-*d₆*) δ 8.19 (1H, s), 7.80 (2H, d, *J=* 8.1 Hz), 7.75 (2H, dd, *J* = 8.0, 1.6 Hz), 7.66 (1H, s), 7.65 (2H, d, *J* = 7.8 Hz) 7.52-7.42 (3H, m), 6.28 (2H, bs), 6.19 (1H, d, *J* = 5.9 Hz), 5.45 (1H, d, *J* = 6.2 Hz), 5.30 (1H, d, *J=* 5.1 Hz), 4.56 (1H, q, *J=* 5.9 Hz), 4.15-3.98 (2H, m), 3.86 (1H, d, *J=* 13.0 Hz), 3.81 (1H, d, *J=* 13.0 Hz), 2.98 (1H, dd, *J=* 13.9, 5.6 Hz), 2.90 (1H, dd, *J =* 13.9, 6.9 Hz), 2.25 (3H, s). **¹³C NMR** (101 MHz, DMSO-*d₆*) δ 164.9, 160.8, 157.6, 152.3, 151.8, 138.7, 130.3, 129.4, 129.0, 127.3, 127.1 (q, *J* = 31.8 Hz), 126.8, 125.9 (q, *J* = 3.7Hz), 124.7 (q, *J* = 271.9 Hz), 122.2, 115.8, 111.1, 100.2, 87.0, 83.4, 73.3, 72.9, 34.8, 24.4, 9.7. **¹⁹F NMR** (377 MHz, DMSO-*d6*) δ -60.72. **HRMS** (ESI) Calculated for C₂₉H₂₇F₃N₅O₄S, [M+H]⁺ m/z: 598.1730, [M+H]⁺, found 598.1729.

### Example 185

### (2R,3R,4S,5S)-2-(4-Amino-5-(4-(trifluoromethoxy)phenyl)-7H-pyrrolo[2,3-d]pyrimidin-7-yl)-5-((((3-methyl-5-phenylisoxazol-4-yl)methyl)thio)methyl)tetrahydrofuran-3,4-diol (146b):

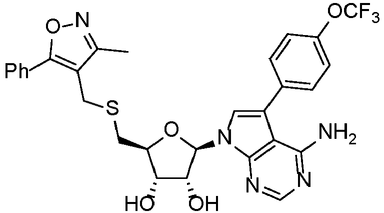

SAH analogue **146b** was prepared from derivative **146a** (0.15 g, 0.18 mmol) using the same conditions as for **47c.** Reverse-phase FCC (10-90 % MeCN in H₂O) provided modified nucleoside **146b** (0.1 g, 0.16 mmol, 86 %). **¹H NMR** (400 MHz, DMSO-*d₆*) δ 8.18 (1H, s), 7.75 (2H, dd, *J=* 8.2, 1.7 Hz), 7.61-7.41 (8H, m), 6.20 (2H, bs), 6.18 (1H, d, *J* = 5.9 Hz), 5.44 (1H, d, *J* = 6.2 Hz), 5.29 (1H, d, *J* = 5.1 Hz), 4.54 (1H, q, J = 5.9 Hz), 4.15-3.99 (2H, m), 3.86 (1H, d, *J* = 13.0 Hz), 3.81 (1H, d, *J* = 13.0 Hz), 2.97 (1H, dd, *J* = 13.9, 5.6 Hz), 2.89 (1H, dd, *J*= 13.9, 6.9 Hz), 2.24 (3H, s). **¹³C NMR** (101 MHz, DMSO-*d₆*) δ 164.9, 160.8, 157.5, 152.2, 151.6, 147.5 (q, *J* = 2.0 Hz), 133.9, 130.3, 129.4, 127.3, 126.8, 121.6, 121.5, 120.3 (q, *J =* 256.4 Hz), 115.7, 111.1, 100.4, 87.0, 83.3, 73.3, 72.9, 34.8, 24.4, 9.7. **¹⁹F NMR** (377 MHz, DMSO-*d6*) δ -56.70. **HRMS** (ESI) Calculated for C₂₉H₂₆F₃N₅O₅NaS, [M+Na]⁺ m/z: 636.1492, [M+Na]⁺, found 636.1499.

### Example 186

### (2R,3R,4S,5S)-2-(4-Amino-5-(3,4-dimethoxyphenyl)-7H-pyrrolo[2,3-d]pyrimidin-7-yl)-5-(((3-methyl-5-phenylisoxazol-4-yl)methyl)thio)methyl)tetrahydrofuran-3,4-diol (147b):

Title compound **147b** was synthesized from **147a** (0.16 g, 0.19 mmol) in analogous fashion to **47c.** 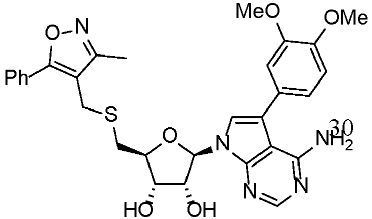 Reverse-phase FCC (10-70 % MeCN in H₂O) afforded **147b** (0.1 g, 0.17 mmol, 91 %). **¹H NMR** (400 MHz, DMSO-*d₆*) δ 8.15 (1H, s), 7.78-7.70 (2H, m), 7.53-7.39 (3H, m), 7.44 (1H, s), 7.04 (1H, d, *J=* 8.3 Hz), 6.97 (1H, d, *J=* 2.0 Hz), 6.94 (1H, dd, *J=* 8.3, 2.0 Hz), 6.18 (1H, d, *J=* 6.0 Hz), 6.14 (2H, bs), 5.42 (1H, d, *J=* 6.3 Hz), 5.28 (1H, d, *J=* 5.1 Hz), 4.55 (1H, q, *J* = 6.0 Hz), 4.11-4.00 (2H, m), 3.88 (1H, d, *J* = 13.0 Hz), 3.82 (1H, *d, J=* 13.0 Hz), 3.79 (3H, s), 3.76 (3H, s), 2.97 (1H, dd, *J* = 13.9, 5.5 Hz), 2.89 (1H, dd, *J* = 13.9, 7.0 Hz), 2.25 (3H, s). **¹³C NMR** (101 MHz, DMSO-*d₆*) δ 164.9, 160.8, 157.5, 152.0, 151.3, 149.1, 148.2, 130.3, 129.4, 127.3, 127.0, 126.8, 120.7, 120.3, 117.0, 112.6, 112.4, 111.1, 100.8, 86.9, 83.3, 73.2, 72.9, 55.8, 55.6, 34.9, 24.5, 9.7. **HRMS** (ESI) Calculated for C₃₀H₃₂N₅O₆S, [M+H]⁺ m/z: 590.2068, [M+H]⁺, found 590.2065.

### Example 187

### (2R,3R,4S,5S)-2-(4-Amino-5-(naphthalen-2-yl)-7H-pyrrolo[2,3-d]pyrimidin-7-yl)-5-((((3-methyl-5-phenylisoxazol-4-yl)methyl)thio)methyl)tetrahydrofuran-3,4-diol (148b):

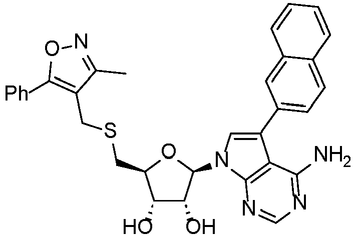

Analogue **148b** was prepared from **148a** (0.15 g, 0.19 mmol) in analogous fashion to **47c.** Reverse-phase FCC (10-80 % MeCN in H₂O) furnished **148b** (0.1 g, 0.18 mmol, 94 %). **¹H NMR** (400 MHz, DMSO-*d₆*) δ 8.20 (1H, s), 8.01 (1H, d, *J* = 8.6 Hz), 7.98-7.89 (3H, m), 7.77-7.72 (2H, m), 7.63 (1H, s), 7.61 (1H, dd, *J* = 8.4, 1.7 Hz), 7.58-7.50 (2H, m), 7.50-7.40 (3H, m), 6.23 (1H, d, *J=* 6.0 Hz), 6.20 (2H, bs), 5.46 (1H, d, *J=* 6.3 Hz), 5.30 (1H, d, *J=* 5.1 Hz), 4.59 (1H, q, *J=* 6.0 Hz), 4.14-4.02 (2H, m), 3.88 (1H, d, *J=* 13.0 Hz), 3.83 (1H, d, *J=* 13.0 Hz), 2.99 (1H, dd, *J=* 13.9, 5.6 Hz), 2.92 (1H, dd, *J=* 13.9, 6.9 Hz), 2.25 (3H, s). **¹³C NMR** (101 MHz, DMSO-*d₆*) δ 164.9, 160.8, 157.6, 152.2, 151.7, 133.4, 132.0, 130.3, 129.4, 128.6, 128.0, 127.9, 127.3, 127.2, 126.9, 126.81, 126.75, 126.1, 121.3, 117.0, 111.1, 100.7, 86.9, 83.4, 73.3, 72.9, 34.9, 24.5, 9.7. **HRMS** (ESI) Calculated for C₃₂H₃₀N₅O₄S, [M+H]⁺ m/z: 580.2013, [M+H]⁺, found 580.2012.

### Example 188

### (2R,3R,4S,5S)-2-(4-Amino-5-phenyl-7H-pyrrolo[2,3-d]pyrimidin-7-yl)-5-((((3-methyl-5-phenylisoxazol-4-yl)methyl)thio)methyl)tetrahydrofuran-3,4-diol (149):

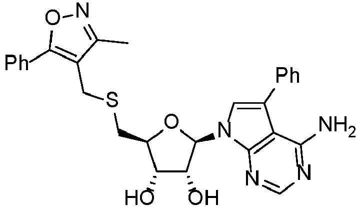

A suspension of **123** (0.07 g, 0.12 mmol), Pd(PPh₃)₄ (0.01 g, 0.01 mmol), Na₂CO₃ (0.06 g, 0.60 mmol) and PhB(OH)₂ (0.03 g, 0.24 mmol) in the mixture of DME/EtOH/H₂O (4:1:1, 6 mL) was placed in an inert atmosphere and heated to 100 °C. After stirring for 10 h, the reaction mixture was cooled to RT, concentrated and purified by FCC (0-50 % EtOAc/acetone/EtOH/H₂O (84/12/2.4/1.6) mixture in EtOAc/acetone/EtOH/H₂O (68/12/12/8) mixture) to gain phenyl derivative **149** (0.04 g, 0.07 mmol, 58 %). **¹H NMR** (400 MHz, DMSO-*d₆*) δ 8.17 (1H, s), 7.77-7.72 (2H, m), 7.51 (1H, s), 7.50-7.42 (7H, m), 7.37 (1H, tt, *J* = 6.4, 1.6 Hz), 6.18 (1H, d, *J=* 6.0 Hz), 6.11 (2H, bs), 5.44 (1H, d, *J=* 6.3 Hz), 5.29 (1H, d, *J =* 5.1 Hz), 4.56 (1H, q, *J =* 6.0 Hz), 4.10-4.06 (1H, m), 4.06-4.02 (1H, m), 3.87 (1H, d, *J=* 13.0 Hz), 3.82 (1H, d, *J=* 13.0 Hz), 2.97 (1H, dd, *J=* 13.8, 5.7 Hz), 2.90 (1H, dd, *J=* 13.9, 7.0 Hz), 2.25 (3H, s). **¹³C NMR** (101 MHz, DMSO-*d₆*) δ 164.9, 160.8, 157.5, 152.1, 151.5, 134.6, 130.3, 129.3, 128.6, 127.3, 127.2, 126.8, 120.9, 117.0, 111.1, 100.6, 86.9, 83.3, 73.2, 72.9, 34.9, 24.5, 9.7. **HRMS** (ESI) Calculated for C₂₈H₂₈N₅O₄S, [M+H]⁺ m/z: 530.1857, [M+H]⁺, found 530.1852.

### Example 189

### 4-(4-Amino-7-((2R,3R,4S,5S)-3,4-dihydroxy-5-((((3-methyl-5-phenylisoxazol-4-yl)methyl)thio) methyl)tetrahydrofuran-2-yl)-7H-pyrrolo[2,3-d]pyrimidin-5-yl)-3,5-dimethylbenzonitrile (150):

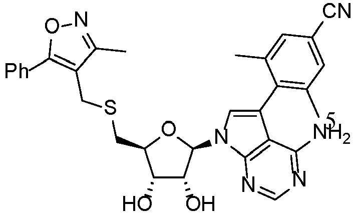

**150** was prepared from **123** (0.14 g, 0.24 mmol) and (4-cyano-2,6-dimethylphenyl)boronic acid pinacol ester (0.12 g, 0.48 mmol) in analogous fashion to **149.** FCC (0-50 % EtOAc/acetone/EtOH/H₂O (84/12/2.4/1.6) mixture in EtOAc/acetone/EtOH/H₂O (68/12/12/8) mixture) afforded **150** (0.05 g, 0.09 mmol, 37 %). **¹H NMR** (400 MHz, DMSO-*d₆*) δ 8.17 (1H, s), 7.74-7.70 (2H, m), 7.65-7.62 (1H, m), 7.60-7.57 (1H, m), 7.46-7.38 (3H, m), 7.32 (1H, s), 6.20 (1H, d, *J=* 6.0 Hz), 5.73 (2H, bs), 5.45 (1H, d, *J=* 6.2 Hz), 5.29 (1H, d, *J=* 4.8 Hz), 4.54 (1H, q, *J* = 6.0 Hz), 4.09-4.03 (2H, m), 3.85 (1H, d, *J* = 12.9 Hz), 3.77 (1H, d, *J* = 12.9 Hz), 2.95 (1H, dd, *J=* 14.0, 4.9 Hz), 2.89 (1H, dd, *J=* 14.0, 7.2 Hz), 2.22 (3H, s), 2.07 (3H, s), 1.88 (3H, s). **¹³C NMR** (101 MHz, DMSO-*d₆*) δ 164.9, 160.7, 157.3, 152.2, 151.2, 139.6, 139.5, 139.0, 130.9, 130.2, 129.3, 127.3, 127.2, 126.7, 120.2, 119.2, 111.8, 111.0, 110.5, 101.5, 87.0, 83.8, 73.3, 72.9, 34.9, 24.7, 20.5, 20.2, 9.7. **HRMS** (ESI) Calculated for C₃₁H₃₁N₆O₄S, [M+H]⁺ m/z: 583.2122, [M+H]⁺, found 583.2121.

### Example 190

### (2R,3R,4S,5S)-2-(4-Amino-5-(1H-benzo[d]imidazol-6-yl)-7H-pyrrolo[2,3-d]pyrimidin-7-yl)-5-((((3-methyl-5-phenylisoxazol-4-yl)methyl)thio)methyl)tetrahydrofuran-3,4-diol (151):

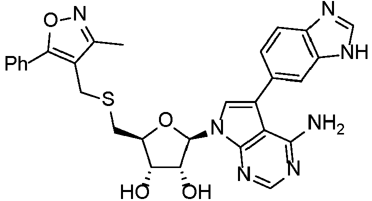

Analogue **151** was synthesized from **123** (0.11 g, 0.18 mmol) and (1-(*tert*-butoxycarbonyl)-1H-benzo[*d*]imidazol-6-yl)boronic acid pinacol ester (0.12 g, 0.36 mmol) following the same procedure as for **149.** FCC (10-100 % EtOAc/acetone/EtOH/H₂O (84/12/2.4/1.6) mixture in EtOAc/acetone/EtOH/H₂O (68/12/12/8) mixture) provided modified nucleoside **151** (0.04 g, 0.06 mmol, 34 %). **¹H NMR** (400 MHz, DMSO-*d₆*) δ 12.55 (1H, bs), 8.26 (1H, s), 8.17 (1H, s), 7.78-7.71 (2H, m), 7.68 (1H, br d, *J =* 8.2 Hz), 7.62 (1H, bs), 7.52-7.41 (4H, m), 7.27 (1H, dd, *J=* 8.3, 1.4 Hz), 6.19 (1H, d, *J=* 6.0 Hz), 6.12 (2H, bs), 5.48 (1H, bs), 5.32 (1H, bs), 4.58 (1H, t, *J=* 5.5 Hz), 4.12-4.00 (2H, m), 3.87 (1H, d, *J=* 13.0 Hz), 3.82 (1H, d, *J=* 13.0 Hz), 2.97 (1H, dd, *J* = 13.8, 5.7 Hz), 2.90 (1H, dd, *J* = 13.8, 7.0 Hz), 2.25 (3H, s). **¹³C NMR** (101 MHz, DMSO-*d₆*) δ 164.9, 160.8, 157.5, 152.0, 151.3, 130.2, 129.4, 127.3, 126.8, 123.0, 120.5, 117.7, 111.1, 100.9, 86.9, 83.3, 73.2, 72.9, 34.9, 24.5, 9.7. **HRMS** (ESI) Calculated for C₂₉H₂₈N₇O₄S, [M+H]⁺ m/z: 570.1918, [M+H]⁺, found 570.1914.

### Example 191

### (2R,3R,4S,5S)-2-(4-Amino-5-(6-nitropyridin-3-yl)-7H-pyrrolo[2,3-d]pyrimidin-7-yl)-5-((((3-methyl-5-phenylisoxazol-4-yl)methyl)thio)methyl)tetrahydrofuran-3,4-diol (152):

Modified nucleoside **152** was prepared from **123** (0.14 g, 0.24 mmol) and 2-nitropyridine-5-boronic acid pinacol ester (0.12 g, 0.48 mmol) in analogous fashion to **149.** FCC (0-90 % EtOAc/acetone/EtOH/H₂O 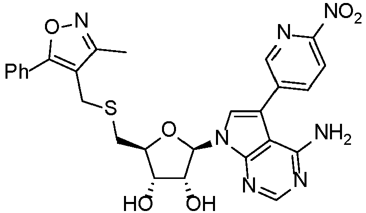 (84/12/2.4/1.6) mixture in EtOAc/acetone/EtOH/H₂O (68/12/12/8) mixture) furnished **152** (0.03 g, 0.06 mmol, 23 %). **¹H NMR** (400 MHz, DMSO-*d₆*) δ 8.70 (1H, dd, *J=* 2.3, 0.5 Hz), 8.39 (1H, dd, *J=* 8.4, 0.5 Hz), 8.22 (1H, s), 8.17 (1H, dd, *J =* 8.4, 2.3 Hz), 7.88 (1H, s), 7.78-7.71 (2H, m), 7.55-7.42 (3H, m), 6.57 (2H, bs), 6.20 (1H, d, *J =* 5.8 Hz), 5.51 (1H, d, *J=* 6.0 Hz), 5.34 (1H, d, *J=* 4.9 Hz), 4.56 (1H, q, *J=* 5.6 Hz), 4.14-4.01 (2H, m), 3.86 (1H, d, *J=* 13.0 Hz), 3.82 (1H, d, *J* = 13.0 Hz), 2.98 (1H, dd, *J* = 13.9, 5.7 Hz), 2.91 (1H, dd, *J=* 13.9, 7.1 Hz), 2.25 (3H, s). **¹³C NMR** (101 MHz, DMSO-*d₆*) δ 164.9, 160.8, 157.8, 152.6, 152.2, 147.7, 139.1, 136.6, 130.3, 129.4, 127.3, 126.8, 123.7, 118.6, 112.0, 111.1, 100.2, 87.2, 83.5, 73.4, 72.9, 34.8, 24.4, 9.7. **HRMS** (ESI) Calculated for C₂₇H₂₆N₇O₆S, [M+H]⁺ m/z: 576.1660, [M+H]⁺, found 576.1658.

### Example 192

### (2R,3R,4S,5S)-2-(4-Amino-5-(furan-2-yl)-7H-pyrrolo[2,3-d]pyrimidin-7-yl)-5-(((3-methyl-5-phenylisoxazol-4-yl)methyl)thio)methyl)tetrahydrofuran-3,4-diol (153):

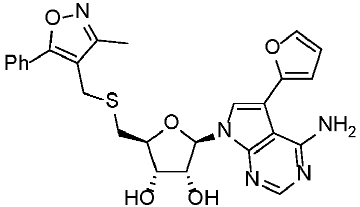

Furyl derivative **153** was synthesized from **123** (0.11 g, 0.18 mmol) and tributyl(furan-2-yl)stannane (0.12 mL, 0.38 mmol) using the same conditions as for **139a.** FCC (0-30 % EtOAc/acetone/EtOH/H₂O (84/12/2.4/1.6) mixture in EtOAc/acetone/EtOH/H₂O (68/12/12/8) mixture) furnished modified nucleoside **153** (0.06 g, 0.12 mmol, 66 %). **¹H NMR** (400 MHz, DMSO-*d₆*) δ 8.15 (1H, s), 7.81 (1H, s), 7.78 (1H, d, *J=* 1.6 Hz), 7.77-7.72 (2H, m), 7.58-7.42 (3H, m), 6.91 (2H, bs), 6.66 (1H, d, *J=* 3.2 Hz), 6.60 (1H, dd, *J=* 3.2, 1.9 Hz), 6.15 (1H, d, *J=* 5.9 Hz), 5.47 (1H, d, *J=* 6.2 Hz), 5.30 (1H, d, *J=* 5.1 Hz), 4.52 (1H, q, *J=* 5.8 Hz), 4.12-4.00 (2H, m), 3.87 (1H, d, *J=* 13.0 Hz), 3.82 (1H, d, *J=* 13.0 Hz), 2.98 (1H, dd, *J=* 13.9, 5.6 Hz), 2.90 (1H, dd, *J=* 13.9, 6.9 Hz), 2.26 (3H, s). **¹³C NMR** (101 MHz, DMSO-*d₆*) δ 164.9, 160.8, 157.4, 152.5, 151.4, 148.7, 142.2, 130.3, 129.4, 127.3, 126.8, 120.3, 112.6, 111.1, 106.8, 105.6, 99.4, 86.9, 83.4, 73.2, 72.9, 34.8, 24.4, 9.7. **HRMS** (ESI) Calculated for C₂₆H₂₆N₅O₅S, [M+H]⁺ m/z: 520.1649, [M+H]⁺, found 520.1648.

### Example 193

### (2R,3R,4S,5S)-2-(4-Amino-5-(1-methyl-1H-pyrazol-5-yl)-7H-pyrrolo[2,3-d]pyrimidin-7-yl)-5-(((3-methyl-5-phenylisoxazol-4-yl)methyl)thio)methyl)tetrahydrofuran-3,4-diol (154):

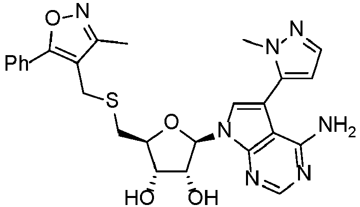

SAH analogue **154** was prepared from **123** (0.07 g, 0.12 mmol) and 1-methyl-5-(tributylstannyl)-1*H*-pyrazole (0.08 mL, 0.25 mmol) following the same procedure as for **139a.** FCC (0-60 % EtOAc/acetone/EtOH/H₂O (84/12/2.4/1.6) mixture in EtOAc/acetone/EtOH/H₂O (60/12/16/12) mixture) furnished **154** (0.03 g, 0.06 mmol, 53 %). **¹H NMR** (400 MHz, DMSO-*d₆*) δ 8.19 (1H, s), 7.78-7.72 (2H, m), 7.66 (1H, s), 7.54 (1H, d, *J=* 1.8 Hz), 7.50-7.42 (3H, m), 6.34 (1H, d, *J=* 1.8 Hz), 6.18 (1H, d, *J=* 6.0 Hz), 6.15 (2H, bs), 5.51 (1H, bs), 5.34 (1H, bs), 4.56 (1H, t, *J=* 5.1 Hz), 4.11-4.01 (2H, m), 3.87 (1H, d, *J=* 13.0 Hz), 3.82 (1H, d, *J=* 13.0 Hz), 3.70 (3H, s), 2.97 (1H, dd, *J=* 13.9, 5.5 Hz), 2.91 (1H, dd, *J=* 13.9, 7.1 Hz), 2.25 (3H, s). **¹³C NMR** (101 MHz, DMSO-*d₆*) δ 164.9, 160.8, 157.4, 152.6, 151.0, 138.5, 135.6, 130.3, 129.4, 127.3, 126.8, 122.9, 111.1, 107.0, 104.1, 101.4, 87.1, 83.6, 73.3, 72.9, 36.9, 34.9, 24.5, 9.7. **HRMS** (ESI) Calculated for C₂₆H₂₈N₇O₄S, [M+H]⁺ m/z: 534.1918, [M+H]⁺, found 534.1914.

### Example 194

### (2R,3R,4S,5S)-2-(4-Amino-5-(1-methyl-1H-imidazol-5-yl)-7H-pyrrolo[2,3-d]pyrimidin-7-yl)-5-((((3-methyl-5-phenylisoxazol-4-yl)methyl)thio)methyl)tetrahydrofuran-3,4-diol (155):

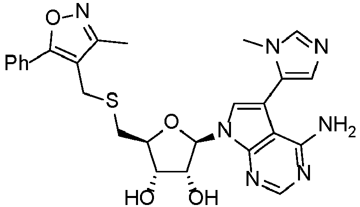

Title compound **155** was synthesized from **123** (0.07 g, 0.12 mmol) and 1-methyl-5-(tributylstannyl)-1*H*-imidazole (0.08 mL, 0.26 mmol) according to the same methodology as for **139a.** FCC (0-100 % EtOAc/acetone/EtOH/H₂O (84/12/2.4/1.6) mixture in EtOAc/acetone/EtOH/H₂O (60/12/16/12) mixture) provided **155** (0.03 g, 0.06 mmol, 47 %). **¹H NMR** (400 MHz, DMSO-*d₆*) δ 8.17 (1H, s), 7.76-7.71 (3H, m), 7.55 (1H, s), 7.50-7.41 (3H, m), 6.94 (1H, d, *J=* 1.0 Hz), 6.17 (1H, d, *J=* 6.0 Hz), 6.12 (2H, bs), 5.51 (1H, bs), 5.35 (1H, bs), 4.55 (1H, t, *J=* 5.4 Hz), 4.12-3.99 (2H, m), 3.87 (1H, *d, J=* 13.0 Hz), 3.81 (1H, *d, J=* 13.0 Hz), 3.45 (3H, s), 2.96 (1H, dd, *J* = 13.9, 5.5 Hz), 2.90 (1H, dd, *J* = 13.9, 7.1 Hz), 2.25 (3H, s). **¹³C NMR** (101 MHz, DMSO-*d₆*) δ 164.9, 160.8, 157.4, 152.4, 150.9, 139.6, 130.2, 129.3, 128.5, 127.3, 126.8, 122.8, 111.1, 103.0, 101.8, 87.1, 83.5, 73.3, 72.9, 34.9, 31.6, 24.5, 9.7. **HRMS** (ESI) Calculated for C₂₆H₂₈N₇O₄S, [M+H]⁺ m/z: 534.1918, [M+H]⁺, found 534.1913.

### Example 195

### (2R,3R,4S,5S)-2-(4-Amino-5-(2-methylthiazol-5-yl)-7H-pyrrolo[2,3-d]pyrimidin-7-yl)-5-((((3-methyl-5-phenylisoxazol-4-yl)methyl)thio)methyl)tetrahydrofuran-3,4-diol (156):

**156** was prepared from **123** (0.07 g, 0.12 mmol) and 2-methyl-5-(tributylstannyl) thiazole (0.09 g, 0.24 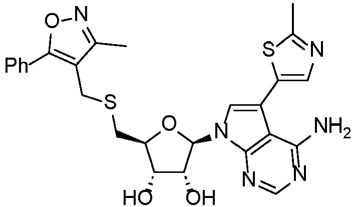 mmol) in analogous fashion to **139a.** FCC (0-60 % EtOAc/acetone/EtOH/H₂O (84/12/2.4/1.6) mixture in EtOAc/acetone/EtOH/H₂O (68/12/12/8) mixture) afforded **156** (0.04 g, 0.07 mmol, 57 %). **¹H NMR** (400 MHz, DMSO-*d₆*) δ 8.17 (1H, s), 7.78-7.69 (2H, m), 7.61 (1H, s), 7.57 (1H,s), 7.53-7.42 (3H, m), 6.37 (2H, bs), 6.14 (1H, d, *J* = 6.1 Hz), 5.45 (1H, d, *J=* 6.2 Hz), 5.30 (1H, d, *J=* 4.9 Hz), 4.54 (1H, q, *J=* 6.0 Hz), 4.10-3.99 (2H, m), 3.86 (1H, d, *J=* 13.0 Hz), 3.81 (1H, d, *J* = 13.0 Hz), 2.97 (1H, dd, *J=* 13.9, 5.6 Hz), 2.89 (1H, dd, *J* = 13.9, 6.8 Hz), 2.67 (3H, s), 2.26 (3H, s). **¹³C NMR** (101 MHz, DMSO-*d₆*) δ 165.3, 164.9, 160.8, 157.4, 152.5, 151.3, 140.3, 130.3, 129.8, 129.4, 127.3, 126.8, 122.8, 111.1, 105.3, 100.9, 87.0, 83.5, 73.2, 72.8, 34.8, 24.5, 19.1, 9.7. **HRMS** (ESI) Calculated for C₂₆H₂₇N₆O₄S₂, [M+H]⁺ m/z: 551.1530, [M+H]⁺, found 551.1529.

### Example 196

### (2R,3R,4S,5S)-2-(4-Amino-5-(4-aminophenyl)-7H-pyrrolo[2,3-d]pyrimidin-7-yl)-5-(((3-methyl-5-phenylisoxazol-4-yl)methyl)thio)methyl)tetrahydrofuran-3,4-diol (157):

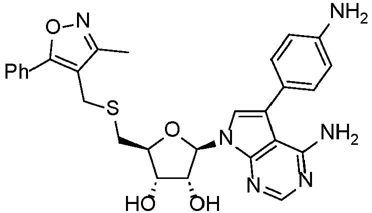

Derivative **157** was synthesized from nitrophenyl analogue **144b** (0.03 g, 0.06 mmol) according to the similar procedure as for **6.** Reverse-phase FCC (0-50 % MeCN in H₂O) furnished **157** (0.02 g, 0.04 mmol, 69 %). **¹H NMR** (400 MHz, DMSO-*d₆*) δ 8.12 (1H, s), 7.76-7.73 (2H, m), 7.53-7.45 (3H, m), 7.28 (1H, s), 7.07 (2H, dt, *J* = 8.4, 1.9 Hz), 6.65 (2H, dt, *J* = 8.4, 1.9 Hz), 6.14 (1H, d, *J=* 6.0 Hz), 6.07 (2H, bs), 5.41 (1H, d, *J=* 6.0 Hz), 5.26 (1H, d, *J=* 4.3 Hz), 5.20 (2H, s), 4.52 (1H, q, *J=* 5.4 Hz), 4.06 (1H, q, *J=* 4.0 Hz), 4.04-4.00 (1H, m), 3.87 (1H, d, *J* = 13.0 Hz), 3.82 (1H, d, *J* = 13.1 Hz), 2.96 (1H, dd, *J* = 13.8, 5.7 Hz), 2.88 (1H, dd, *J* = 13.8, 6.9 Hz), 2.25 (3H, s). **¹³C NMR(**101 MHz, DMSO-*d₆*) δ 164.9, 160.8, 157.5, 151.9, 151.0, 148.2, 130.3, 129.5, 129.4, 127.3, 126.8, 121.4, 119.4, 117.6, 114.4, 111.1, 101.0, 86.8, 83.2, 73.2, 72.9, 34.9, 24.5, 9.7. **HRMS** (ESI) Calculated for C₂₈H₂₉N₆O₄S, [M+H]⁺ m/z: 545.1966, [M+H]⁺, found 545.1962.

To explore more distant parts of the lateral cavity we decided to synthesize a series of compounds with different aromatic substituents attached to the nucleobase through linkers of various lengths and rigidity. The introduction of suitable substituents was inspired by published procedures (Botha, F., et al., Org Biomol Chem 2016/14, 10018-10022, Čapek, P., et al., *Chem Eur J* **2007**/*13,* 6196-6203, Otava, T., et al., ACS Infect Dis 2021/7, 2214-2220, Snášel, J., et al., J Med Chem 2014/57, 8268-8279). Diversely substituted acetylenes **158b-168b** were synthesized from appropriate arylbromides **158a-168a** by Sonogashira cross-coupling reaction with triisopropylacetylene and subsequent removal of the TIPS protecting group by TBAF **(Scheme 10,** Otava, T., et al., ACS Infect Dis 2021/7, 2214-2220).

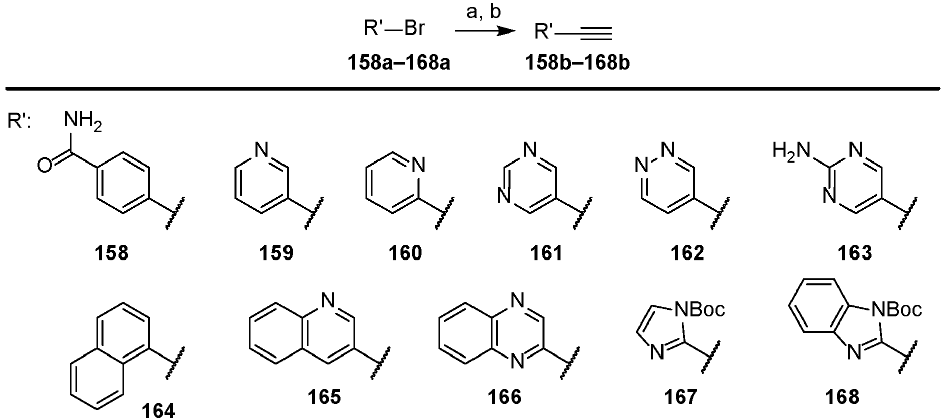

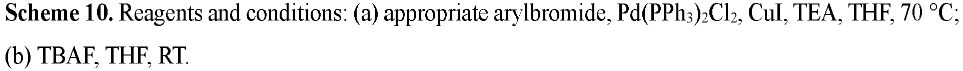

### Example 197

### 4-Ethynylbenzamide (158b):

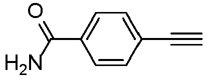

To a suspension of bromo 4-bromobenzamide **158a** (0.4 g, 2.00 mmol), (triisopropylsilyl)acetylene (1.15 mL, 5.14 mmol), CuI (0.06 g, 0.30 mmol) and

Pd(PPh₃)₂Cl₂ (0.07 g, 0.1 mmol) in dry THF (7 mL) was added TEA (0.82 mL, 5.92 mmol) at RT, the mixture was warmed to 60 °C and stirred under argon atmosphere for 1 h. The resulting solution was then filtered through a plug of silica, and concentrated under reduced pressure. The residue was dissolved in THF (8 mL) and cooled down to 0 °C. 1M solution of TBAF in THF (3.1 mL, 3.1 mmol) was added to the solution and the reaction mixture was stirred for 1 h at RT. Volatiles were removed in vacuo and the crude product was subjected to FCC (1-10 % EtOH in DCM) providing the desired acetylene **158b** (0.2 g, 1.36 mmol, 73 %). NMR spectra were consistent with published data (Ye, F., et al., J Am Chem Soc 2017/139, 7184-7187).

### Example 198

### 3-Ethynylpyridine (159b):

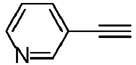

Derivative **159b** was prepared from 3-bromopyridine **159a** (0.25 mL, 2.60 mmol) following the same methodology as for **158b.** FCC (5-25 % EtOAc in DCM) afforded acetylene **159b** (0.23 g, 2.18 mmol, 84 %). NMR spectra were consistent with published data (Gonzalo Rodriguez, J., et al., J Chem Soc, Perkin Trans 1 1997/5, 709-714).

### Example 199

### 2-Ethynylpyridine (160b):

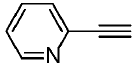

Acetylene **160b** was synthesized from 2-bromopyridine **160a** (0.24 mL, 2.52 mmol) according to the same procedure as for **158b.** FCC (5-20 % EtOAc in DCM) furnished title compound **160b** (0.19 g, 1.86 mmol, 74 %). NMR spectra were consistent with published data (Gonzalo Rodriguez, J., et al., J Chem Soc, Perkin Trans 1 1997/5, 709-714).

### Example 200

### 5-Ethynylpyrimidine (161b):

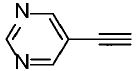

**161b** was prepared from **161a** (0.4 g, 2.52 mmol) in analogous fashion to **158b.** FCC (5-25 % EtOAc in DCM) gave derivative **161b** (0.23 g, 2.25 mmol, 89 %). NMR spectra were consistent with published data (Richardson, C., J Org Chem 2007/72, 4750-4755).

### Example 201

### 4-Ethynylpyridazine (162b):

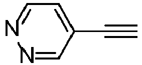

Derivative **162b** was synthesized from **162a** (0.4 g, 2.52 mmol) in analogous fashion to **158b.** FCC (5-30 % EtOAc in DCM) provided acetylene **162b** (0.22 g, 2.08 mmol, 83 %). **¹H NMR** (400 MHz, DMSO-*d₆*) δ 9.29 (1H, dd, J = 2.3, 1.3 Hz), 9.27 (1H, dd, *J* = 5.3, 1.3 Hz), 7.81 (1H, dd, *J=* 5.3, 2.3 Hz), 4.90 (s, 1H). **¹³C NMR** (101 MHz, DMSO-*d₆*) δ 152.5, 151.4, 128.7, 128.6, 122.2, 89.5, 78.5. **LRMS** Calculated for C₆H₅N₂, [M+H]⁺ m/z: 105.1, [M+H]⁺, found 105.0.

### Example 202

### 5-Ethynylpyrimidin-2-amine (163b):

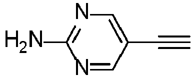

Title compound **163b** was synthesized from 5-bromopyrimidin-2-amine **163a** (0.4 g, 2.30 mmol) using the same methodology as for **158b.** FCC (5-20 % EtOAc in DCM) furnished derivative **163b** (0.15 g, 1.29 mmol, 56 %). NMR spectra were consistent with published data (Cee, V. J., et al., J Med Chem 2007/50, 627-640).

### Example 203

### 1-Ethynylnaphthalene (164b):

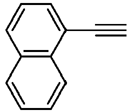

Acetylene **164b** was prepared from 1-bromonaphthalene **164a** (0.27 mL, 1.93 mmol) using the same procedure as for **158b.** FCC (0-5 % EtOAc in cyclohexane) afforded title compound **164b** (0.23 g, 1.48 mmol, 77 %). NMR spectra were consistent with published data (Beshai, M., et al., Tetrahedron Lett 2008/49, 6794-6796).

### Example 204

### 3-Ethynylquinoline (165b):

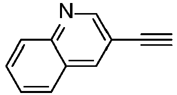

Title compound **165b** was synthesized from 3-bromoquinoline **165a** (0.26 mL, 1.92 mmol) following the same methodology as for **158b.** FCC (5-15 % EtOAc in DCM) furnished derivative **165b** (0.18 g, 1.18 mmol, 62 %). NMR spectra were consistent with published data (Smeyanov, A., Synth Commun 2013/43, 2809-2816).

### Example 205

### 2-Ethynylquinoxaline (166b):

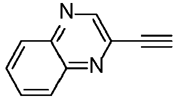

Derivative **166b** was prepared from 2-bromoquinoxaline **166a** (0.51 g, 2.43 mmol) using the same procedure as for **158b.** FCC (5-20 % EtOAc in DCM) provided acetylene **166b** (0.33 g, 2.15 mmol, 89 %). NMR spectra were consistent with published data (Armengol, M., JChem Soc, Perkin Trans 1 2001/2, 154-158).

### Example 206

### tert-Butyl 2-ethynyl-1H-imidazole-1-carboxylate (167b):

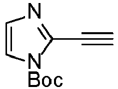

Acetylene **167b** was prepared from **167a** (0.4 g, 1.62 mmol) following the same procedure as for **158b.** FCC (5-50 % EtOAc in cyclohexane) afforded derivative **167b** (0.27 g, 1.39 mmol, 86 %). **¹H NMR** (400 MHz, DMSO-*d₆*) δ 7.62 (1H, s), 7.06 (1H, s), 4.62 (s, 1H), 1.58 (s, 9H). **¹³C NMR** (101 MHz, DMSO-*d₆*) δ 146.3, 129.8, 120.1, 86.5, 84.8, 74.2, 27.5. **LRMS** Calculated for C₁₀H₁₂O₂N₂, [M+H]⁺ m/z: 192.1, [M+H]⁺, found 192.1.

### Example 207

### tert-Butyl 2-ethynyl-1H-benzo[d]imidazole-1-carboxylate (168b):

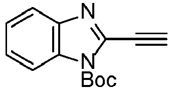

Derivative **168b** was synthesized from **168a** (0.6 g, 2.02 mmol) using the same conditions as for **158b.** FCC (5-80 % EtOAc in cyclohexane) gave acetylene **168b** (0.36 g, 1.49 mmol, 73 %). NMR spectra were consistent with published data (Morinaga, A., et al., Angew Chem Int Ed 2015/54, 15859-15862).

Sonogashira cross-coupling with intermediate **122,** followed by deprotection with either HF-TEA or TFA/H₂O furnished SAH analogues **169b-179b.** Phenyl derivative **180** was obtained by Sonogashira cross-coupling with free iodinated derivative **123.** Suzuki-Miyaura cross-coupling reaction followed by a HF-TEA deprotection was performed to afford compounds **181b, 182b** with a *trans* ethenyl bridge. Modified nucleosides **172b** and **180** were further subjected to catalytic hydrogenation to obtain ethylene-bridged analogues **183** and **184.** Derivatives with benzyl-type substituents were prepared via Negishi cross-coupling. Subsequent removal of the protecting groups by HF-TEA afforded **185b** and **186b.** Representatives **187** and **188** having substituents connected *via* aromatic linker were obtained using Suzuki-Miyaura cross-coupling. Pd-catalyzed cross-coupling reaction with various thiols gave modified nucleosides **189b, 190b** and **191** with substituents connected to the nucleobase *via* sulfur **(Scheme 11).**

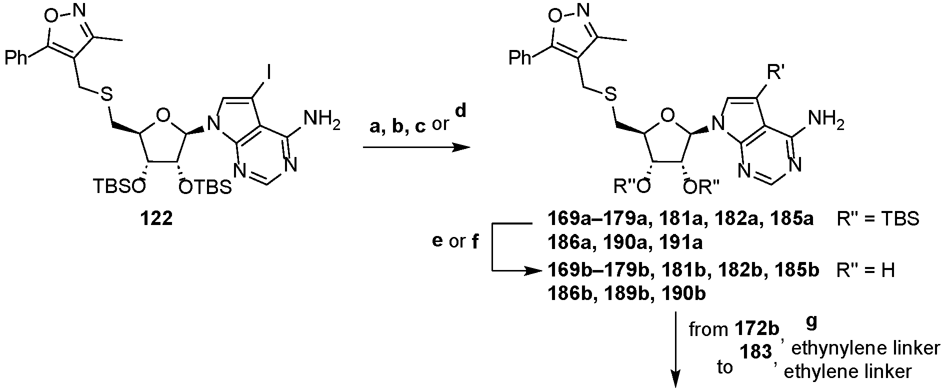

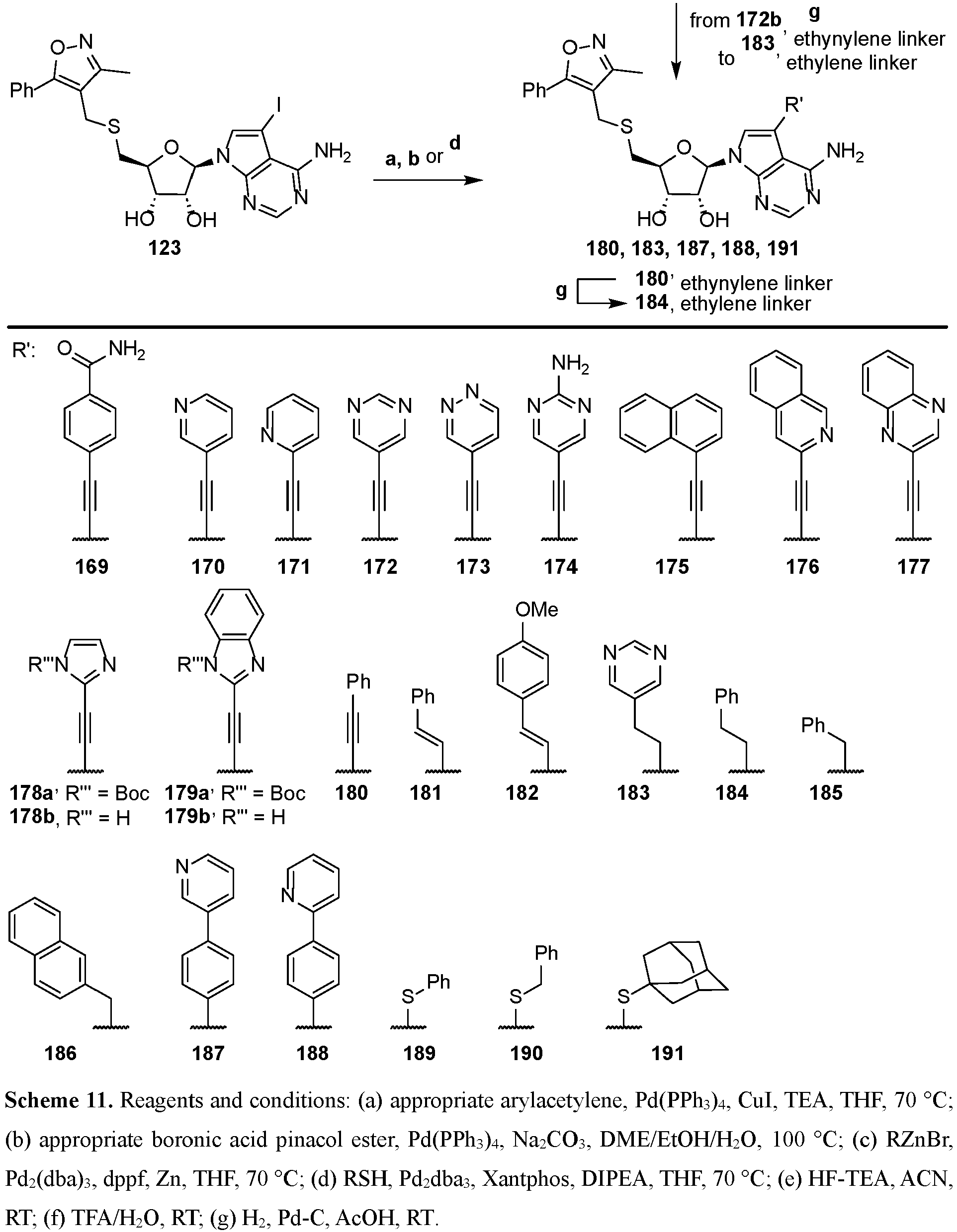

### Example 208

### 4-((4-Amino-7-((2R,3R,4R,5S)-3,4-bis((tert-butyldimethylsilyl)oxy)-5-((((3-methyl-5-phenylisoxazol-4-yl(methyl)thio)methyl)tetrahydrofuran-2-yl)-7H-pyrrolo[2,3-d]pyrimidin-5-yl)ethynyl)benzamide (169a):

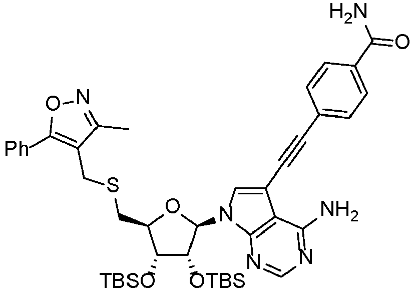

To a suspension of **122** (0.15 g, 0.19 mmol), Pd(PPh₃)₄ (0.02 g, 0.02 mmol), CuI (0.01 g, 0.05 mmol) and acetylene **158b** (0.05 g, 0.36 mmol) in dry THF (2 mL), TEA (0.08 mL, 0.79 mmol) was added. After stirring under argon atmosphere for 10 h at 70 °C, the reaction mixture was cooled to RT, H₂O (50 mL) was added and the resulting mixture was extracted with EtOAc (2×100mL). Collected organic layers were dried over Na₂SO₄, filtrated and concentrated. The reaction mixture was absorbed to silica and the FCC (5-20 % MeOH in EtoAc) afforded title compound **169a** (0.15 g, 0.18 mmol, 97 %). **¹H NMR** (400 MHz, DMSO-*d₆*) δ 8.17 (1H, s), 8.05 (1H, s), 7.94 (1H, s), 7.91 (2H, d, *J=* 8.2 Hz), 7.79-7.72 (2H, m), 7.66 (2H, d, *J=* 8.2 Hz), 7.56-7.44 (4H, m), 6.76 (2H, bs), 6.13 (1H, d, *J=* 6.8 Hz), 4.81 (1H, dd, *J=* 6.3, 4.6 Hz), 4.19 (1H, d, *J=* 3.0 Hz), 4.00 (1H, t, *J=* 7.6 Hz), 3.92 (1H, d, *J=* 13.2 Hz), 3.84 (1H, d, *J=* 13.2 Hz), 3.12 (1H, dd, *J=* 13.7, 7.2 Hz), 2.99 (1H, dd, *J=* 13.7, 6.7 Hz), 2.30 (3H, s), 0.91, 0.69 (18H, 2×s), 0.11, 0.10, -0.09, -0.37 (12H, 4×s). **¹³C NMR** (101 MHz, DMSO-*d₆*) δ 167.3, 165.0, 160.7, 157.8, 153.2, 150.6, 133.9, 131.1, 130.3, 129.4, 127.9, 127.3, 126.9, 125.5, 111.1, 95.3, 91.0, 86.9, 85.1, 84.8, 74.7, 74.3, 34.6, 25.9, 25.7, 24.6, 17.9, 17.6, 9.9, -4.4, -4.5, -4.6, -5.3. **LRMS** Calculated for C₄₃H₅₇N₆O₅SSi₂, [M+H]⁺ m/z: 825.4, [M+H]⁺, found 825.4.

### Example 209

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(pyridin-3-ylethynyl)-7H-pyrrolo[2,3-d]pyrimidin-4-amine (170a):

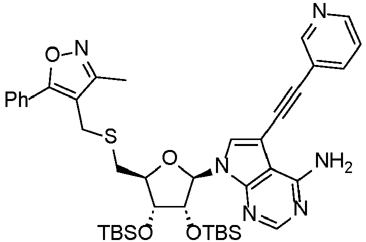

Derivative **170a** was synthesized from **122** (0.2 g, 0.25 mmol) and acetylene **159b** (0.08 g, 0.75 mmol) in analogous fashion to **169a.** FCC (20-100 % EtOAc in cyclohexane) provided **170a** (0.15 g, 0.20 mmol, 79 %). 8.78 (1H, dd, *J*= 2.2, 0.9 Hz), 8.57 (1H, dd, *J=* 4.8, 1.7 Hz), 8.16 (2H, s), 8.00 (1H, ddd, *J=* 7.9, 2.1, 1.7 Hz), 7.95 (1H, s), 7.78-7.73 (2H, m), 7.54-7.48 (3H, m), 7.46 (1H, ddd, *J* = 7.9, 4.9, 0.9 Hz), 6.79 (2H, bs), 6.13 (1H, d, *J* = 6.8 Hz), 4.81 (1H, dd, *J* = 6.8, 4.5 Hz), 4.19 (1H, dd, *J* = 4.4, 1.7 Hz), 3.99 (1H, td, *J=* 7.0, 1.7 Hz), 3.92 (1H, d, *J=* 13.3 Hz), 3.84 (1H, d, *J* = 13.3 Hz), 3.11 (1H, dd, *J* = 13.7, 7.2 Hz), 2.99 (1H, dd, *J* = 13.7, 6.8 Hz), 2.30 (3H, s), 0.91, 0.70 (18H, 2×s), 0.11, 0.10, -0.09, -0.37 (12H, 4×s). **¹³C NMR** (101 MHz, DMSO-*d₆*) δ 165.0, 160.7, 157.7, 153.1, 151.7, 150.6, 148.8, 138.5, 130.3, 129.3, 128.0, 127.3, 126.9, 123.6, 119.9, 111.1, 102.2, 95.1, 88.3, 87.0, 86.1, 84.7, 74.7, 74.3, 34.6, 25.9, 25.6, 24.6, 17.9, 17.6, 9.8, -4.46, -4.53, -4.6, -5.3. **LRMS** Calculated for C₄₁H₅₅N₆O₄SSi₂, [M+H]⁺ m/z: 783.4, [M+H]⁺, found 783.4.

### Example 210

### 7-((2R,3R,4S,5S)-3,4-bis((tert-Butyldimethylsilyl)oxy)-5-((((3-methyl-5-phenylisoxazol-4-yl)methyl)thio)methyl)tetrahydrofuran-2-yl)-5-(pyridin-2-ylethynyl)-7H-pyrrolo[2,3-d]pyrimidin-4-amine (171a):

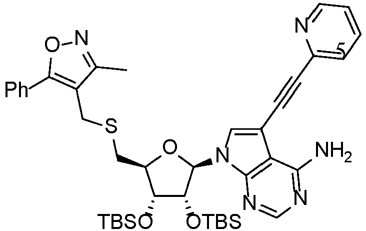

SAH analogue **171a** was prepared from iodinated derivative **122** (0.2 g, 0.25 mmol) and acetylene **160b** (0.08 g, 0.75 mmol) according to the same methodology as for **169a.** FCC (20-100 % EtOAc in cyclohexane) afforded title compound **171a** (0.15 g, 0.20 mmol, 79 %). **¹H NMR** (400 MHz, DMSO-*d₆*) δ 8.60 (1H, ddd, *J=* 4.8, 1.8, 1.0 Hz), 8.17 (1H, s), 8.00 (1H, s), 7.85 (1H, td, *J* = 7.7, 1.8 Hz), 7.77-7.73 (2H, m), 7.67 (1H, dt, *J* = 7.9, 1.1 Hz), 7.56-7.45 (3H, m), 7.40 (1H, ddd, *J=* 7.6, 4.9, 1.2 Hz), 6.80 (2H, bs), 6.12 (1H, d, *J=* 6.9 Hz), 4.82 (1H, dd, *J=* 6.8, 4.4 Hz), 4.19 (1H, dd, *J=* 4.4, 1.7 Hz), 4.00 (1H, td, *J=* 7.0, 1.6 Hz), 3.92 (1H, d, *J* = 13.3 Hz), 3.84 (1H, d,*J*= 13.3 Hz), 3.11 (1H, dd,*J*= 13.7, 7.2 Hz), 3.00 (1H, dd, *J* = 13.7, 6.9 Hz), 2.30 (3H, s), 0.91, 0.69 (18H, 2×s), 0.11, 0.10, -0.09, -0.37 (12H, 4×s). **¹³C NMR** (101 MHz, DMSO-*d₆*) δ 165.0, 160.7, 157.8, 153.3, 150.6, 150.3, 142.6, 136.9, 130.3, 129.4, 128.2, 127.3, 126.9, 126.8, 123.4, 111., 102.5, 94.6, 91.7, 87.0, 84.8, 82.5, 74.7, 74.3, 34.6, 25.9, 25.6, 24.6, 17.9, 17.6, 9.8, -4.5, -4.5, -4.6, -5.3. **LRMS** Calculated for C₄₁H₅₅N₆O₄SSi₂, [M+H]⁺ m/z: 783.4, [M+H]⁺, found 783.4.

### Example 211

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(pyrimidin-5-ylethynyl)-7H-pyrrolo[2,3-d]pyrimidin-4-amine (172a):

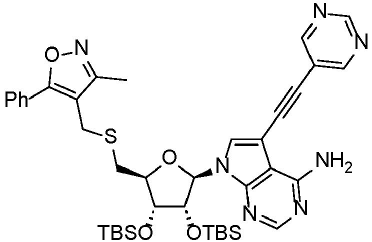

**172a** was prepared from (0.2 g, 0.25 mmol) and **161b** (0.08 g, 0.75 mmol) in analogous fashion to **169a.** FCC (15-70 % EtOAc in cyclohexane) gave **172a** (0.15 g, 0.20 mmol, 79 %). **¹H NMR** (400 MHz, DMSO-*d₆*) δ 9.17 (1H, s), 9.03 (1H, s), 8.16 (1H, s), 7.99 (1H, s), 7.78-7.72 (2H, m), 7.54-7.47 (3H, m), 6.85 (2H, bs), 6.13 (1H, d, *J =* 6.9 Hz), 4.82 (1H, dd, *J=* 6.9, 4.4 Hz), 4.19 (1H, dd, *J=* 4.4, 1.6 Hz), 4.00 (1H, td, *J=* 7.0, 1.5 Hz), 3.92 (1H, d, *J=* 13.3 Hz), 3.84 (1H, *d, J=* 13.3 Hz), 3.12 (1H, dd, *J* = 13.7, 7.3 Hz), 2.98 (1H, dd, *J* = 13.7, 6.8 Hz), 2.30 (3H, s), 0.91, 0.69 (18H, 2×s), 0.11, 0.11, -0.09, -0.38 (12H, 4×s). **¹³C NMR** (101 MHz, DMSO-*d₆*) δ 165.0, 160.7, 158.7, 157.7, 156.6, 153.3, 150.7, 130.3, 129.4, 128.5, 127.3, 126.9, 119.5, 111.1, 102.0, 94.6, 89.6, 87.0, 86.0, 84.8, 74.7, 74.3, 34.5, 25.9, 25.6, 24.6, 17.9, 17.6, 9.8, -4.4, -4.5, -4.6, -5.3. **LRMS** Calculated for C₄₀H₅₄N₇O₄SSi₂, [M+H]⁺ m/z: 784.4, [M+H]⁺, found 784.4.

### Example 212

### 7-((2R,3R,4R,5S)-3,4-bis((tert-Butyldimethylsilyl)oxy)-5-((((3-methyl-5-phenylisoxazol-4-yl)methyl) thio)methyl)tetrahydrofuran-2-yl)-5-(pyridazin-4-ylethynyl)-7H-pyrrolo[2,3-d]pyrimidin-4-amine (173a):

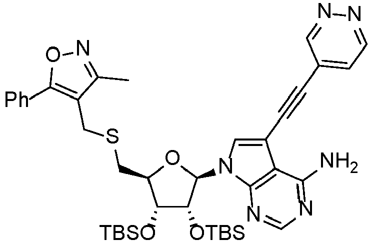

Title compound **173a** was prepared from **122** (0.2 g, 0.25 mmol) and acetylene **162b** (0.08 g, 0.75 mmol) in analogous fashion to **169a.** FCC (5-70 % EtOAc in cyclohexane) afforded **173a** (0.15 g, 0.19 mmol, 78 %). **¹H NMR** (400 MHz, DMSO-*d₆*) δ 9.37 (1H, dd, *J=* 2.1, 1.4 Hz), 9.26 (1H, dd, *J=* 5.4, 1.3 Hz), 8.17 (2H, s), 8.07 (1H, s), 7.86 (1H, dd, *J=* 5.4, 2.3 Hz), 7.77-7.74 (2H, m), 7.55-7.47 (3H, m), 6.90 (2H, bs), 6.13 (1H, d, *J=* 6.9 Hz), 4.82 (1H, dd, J = 6.9, 4.4 Hz), 4.19 (1H, dd, *J=* 4.4, 1.6 Hz), 4.00 (1H, td, *J=* 6.9, 1.5 Hz), 3.92 (1H, d, *J=* 13.3 Hz), 3.84 (1H, d, *J* = 13.3 Hz), 3.12 (1H, dd, *J* = 13.8, 7.3 Hz), 2.99 (1H, dd, *J* = 13.7, 6.8 Hz), 2.30 (3H, s), 0.91, 0.69 (18H, 2×s), 0.11, 0.10, -0.10, -0.39 (12H, 4×s). **¹³C NMR** (101 MHz, DMSO-*d₆*) δ 165.0, 160.7, 157.6, 153.3, 152.0, 151.2, 150.8, 130.3, 129.4, 127.4, 127.3, 126.9, 123.2, 111.1, 102.0, 94.2, 91.4, 87.1, 86.6, 84.9, 74.7, 74.3, 34.5, 25.9, 25.6, 24.5, 17.9, 17.6, 9.8, -4.45, -4.51, -4.6, -5.3. **LRMS** Calculated for C₄₀H₅₄N₇O₄SSi₂, [M+H]⁺ m/z: 784.4, [M+H]⁺, found 784.4.

### Example 213

### 5-((2-Aminopyrimidin-5-yl)ethynyl)-7-((2R,3R,4R,5S)-3,4-bis((tert-butyldimethylsilyl)oxy)-5-(((3-methyl-5-phenylisoxazol-4-yl)methyl)thio)methyl)tetrahydrofuran-2-yl)-7H-pyrrolo[2,3-d]pyrimidin-4-amine (174a):

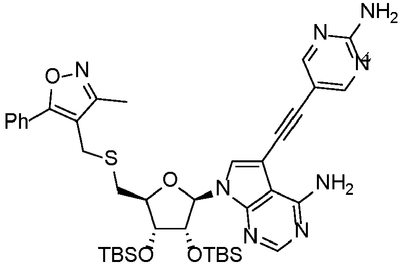

Derivative **174a** was synthesized from intermediate **122** (0.15 g, 0.19 mmol) and acetylene **163b** (0.05 g, 0.38 mmol) according to the same procedure as for **169a.** FCC (5-40 % MeOH in EtOAc) provided SAH analogue **174a** (0.14 g, 0.18 mmol, 96 %). **¹H NMR** (400 MHz, DMSO-*d₆*) δ 8.45 (2H, s), 8.13 (1H, s), 7.82 (1H, s), 7.79-7.68 (2H, m), 7.57-7.43 (3H, m), 7.11 (2H, s), 6.72 (2H, bs), 6.11 (1H, d, *J=* 6.7 Hz), 4.79 (1H, dd, *J=* 6.1, 4.7 Hz), 4.18 (1H, d, *J=* 3.1 Hz), 4.01-3.95 (1H, m), 3.91 (1H, d, *J=* 13.2 Hz), 3.83 (1H, d, *J=* 13.2 Hz), 3.10 (1H, dd, *J=* 13.6, 7.2 Hz), 2.97 (1H, dd, *J=* 13.6, 6.7 Hz), 2.29 (3H, s), 0.90, 0.69 (18H, 2×s), 0.11, 0.10, -0.10, -0.38 (12H, 4×s). **¹³C NMR** (101 MHz, DMSO-*d₆*) δ 165.0, 162.0, 160.7, 160.4, 157.7, 153.1, 150.5, 130.3, 129.4, 127.3, 126.9, 111.1, 106.3, 102.2, 95.8, 86.9, 86.8, 85.0, 84.7, 74.8, 74.3, 34.6, 25.9, 25.7, 24.6, 17.9, 17.7, 9.9, -4.4, -4.5, -4.6, -5.3. **LRMS** Calculated for C₄₀H₅₅N₈O₄SSi₂, [M+H]⁺ m/z: 799.4, [M+H]⁺, found 799.4.

### Example 214

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(naphthalen-1-ylethynyl)-7H-pyrrolo[2,3-d]pyrimidin-4-amine (175a):

Analogue **175a** was prepared from **122** (0.2 g, 0.25 mmol) and acetylene **164b** (0.11 g, 0.75 mmol) in 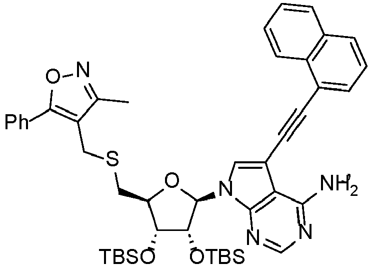 analogous fashion to **169a.** FCC (10-50 % acetone in cyclohexane) gave **175a** (0.17 g, 0.21 mmol, 83 %). **¹H NMR** (400 MHz, DMSO-*d₆*) δ 8.31-8.27 (1H, m), 8.19 (1H, s), 8.05 (1H, s), 8.04-7.98 (2H, m), 7.87 (1H, dd, *J=* 7.2, 1.1 Hz),7.77-7.72 (2H, m), 7.66-7.59 (2H, m), 7.57 (1H, dd, *J=* 8.2, 7.2 Hz), 7.53-7.43 (3H, m), 6.78 (2H, bs), 6.16 (1H, d, *J=* 6.9 Hz), 4.86 (1H, dd, *J=* 6.9, 4.4 Hz), 4.21 (1H, dd, *J* = 4.4, 1.6 Hz), 4.01 (1H,td,*J* = 7.0, 1.6 Hz), 3.94 (1H, d, *J* = 13.2 Hz), 3.86 (1H, d,*J*= 13.2 Hz), 3.13 (1H, dd, *J* = 13.8, 7.2 Hz), 3.02 (1H, dd, *J* = 13.8, 6.9 Hz), 2.30 (3H, s), 0.92, 0.71 (18H, 2×s), 0.12, 0.11, -0.07, -0.33 (12H, 4×s). **¹³C NMR** (101 MHz, DMSO-*d₆*) δ 165.0, 160.7, 157.8, 153.2, 150.6, 133.1, 132.5, 130.5, 130.3, 129.3, 129.1, 128.8, 127.8, 127.4, 127.3, 126.91, 126.87, 125.8, 125.4, 120.1, 111.1, 102.4, 95.5, 89.3, 87.9, 87.0, 84.8, 74.7, 74.2, 34.6, 25.9, 25.7, 24.7, 17.9, 17.7, 9.8, -4.4, -4.5, -4.6, -5.3. **LRMS** Calculated for C₄₆H₅₈N₅O₄SSi₂, [M+H]⁺ m/z: 832.4, [M+H]⁺, found 832.4.

### Example 215

### 7-((2R,3R,4R,5S)-3,4-bis((tert-Butyldimethylsilyl)oxy)-5-((((3-methyl-5-phenylisoxazol-4-yl)methyl) thio)methyl)tetrahydrofuran-2-yl)-5-(quinolin-3-ylethynyl)-7H-pyrrolo[2,3-d]pynmidin-4-amine (176a):

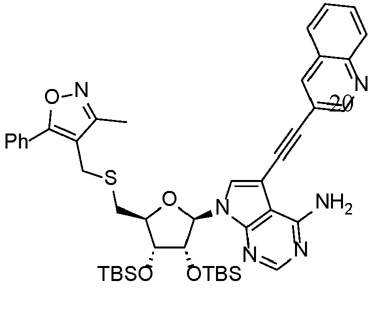

Analogue **176a** was prepared from modified nucleoside **122** (0.2 g, 0.25 mmol) and acetylene **165b** (0.11 g, 0.75 mmol) according to the same procedure as for **169a.** FCC (20-100 % acetone in cyclohexane) afforded **176a** (0.21 g, 0.25 mmol, 99 %). **¹H NMR** (400 MHz, DMSO-*d₆*) δ 9.03 (1H, d, *J* = 2.1 Hz), 8.64 (1H, d, *J* = 1.6 Hz), 8.17 (1H, s), 8.05 (1H, *d, J=* 8.5 Hz), 8.02-7.99 (2H, m), 7.81 (1H, ddd, *J* = 8.2, 6.9, 1.4 Hz), 7.78-7.74 (2H, m), 7.67 (1H, ddd, *J* = 8.0, 7.1, 0.9 Hz), 7.56-7.47 (3H, m), 6.87 (2H, bs), 6.14 (1H, d, *J* = 6.9 Hz), 4.83 (1H, dd, *J* = 6.8, 4.5 Hz), 4.20 (1H, dd, *J=* 4.3, 1.4 Hz), 4.01 (1H, td, *J=* 6.9, 1.1 Hz), 3.93 (1H, d, *J=* 13.2 Hz), 3.85 (1H, d, *J* = 13.2 Hz), 3.13 (1H, dd, *J* = 13.7, 7.2 Hz), 3.00 (1H, dd, *J* = 13.7, 6.8 Hz), 2.30 (3H, s), 0.91, 0.70 (18H, 2×s), 0.12, 0.11, -0.08, -0.36 (12H, 4×s). **¹³C NMR** (101 MHz, DMSO-*d₆*) δ 165.0, 160.7, 157.7, 153.2, 151.8, 150.6, 146.3, 138.2, 130.5, 130.3, 129.4, 129.0, 128.2, 127.7, 127.3, 127.1, 126.9, 117.0, 111.1, 102.2, 95.2, 88.9, 87.0, 86.2, 84.8, 74.8, 74.3, 34.6, 25.9, 25.7, 24.6, 17.9, 17.7, 9.9, -4.4, -4.5, -4.6, -5.3. **LRMS** Calculated for C₄₅H₅₇N₆O₄SSi₂, [M+H]⁺ m/z: 833.4, [M+H]⁺, found 833.4.

### Example 216

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(quinoxalin-2-ylethynyl)-7H-pyrrolo[2,3-d]pyrimidin-4-amine (177a):

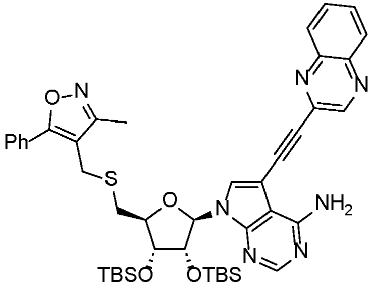

SAH analogue **177a** was synthesized from intermediate **122** (0.2 g, 0.25 mmol) and acetylene **166b** (0.12 g, 0.75 mmol) using the same methodology as for **169a.** FCC (10-60 % acetone in cyclohexane) provided **177a** (0.2 g, 0.24 mmol, 97 %). **¹H NMR** (400 MHz, DMSO-*d₆*) δ 9.17 (1H, s), 8.20 (1H, s), 8.18 (1H, s), 8.13-8.10 (1H, m), 8.08-8.05 (1H, m), 7.93-7.86 (2H, m), 7.78-7.73 (2H, m), 7.55-7.46 (3H, m), 6.96 (2H, bs), 6.15 (1H, d, *J=* 6.9 Hz), 4.85 (1H, dd, *J=* 6.8, 4.4 Hz), 4.21 (1H, dd, *J* = 4.3, 1.6 Hz), 4.01 (1H, td, *J=* 7.0, 1.5 Hz), 3.93 (1H, d, *J=* 13.3 Hz), 3.85 (1H, d, *J* = 13.3 Hz), 3.13 (1H, dd, *J* = 13.8, 7.2 Hz), 3.03 (1H, dd,*J*= 13.7, 6.9 Hz), 2.30 (3H, s), 0.91, 0.70 (18H, 2×s), 0.12, 0.11, -0.08, -0.35 (12H, 4×s). **¹³C NMR** (101 MHz, DMSO-*d₆*) δ 165.0, 160.7, 157.7, 153.4, 150., 147.7, 141.7, 140.3, 139.5, 131.1, 130.8, 130.3, 130.1, 129.4, 129.1, 128.9, 127.3, 126.9, 111.1, 102.2, 94.0, 90.1, 87.4, 87.1, 85.0, 74.8, 74.3, 34.6, 25.9, 25.6, 24.6, 17.9, 17.6, 9.9, -4.4, -4.5, -4.6, -5.3. **LRMS** Calculated for C₄₄H₅₆N₇O₄SSi₂, [M+H]⁺ m/z: 834.4, [M+H]⁺, found 834.4.

### Example 217

### tert-Butyl 2-((4-amino-7-((2R,3R,4R,5S)-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidin-5-yl)ethynyl)-1H-imidazole-1-carboxylate (178a):

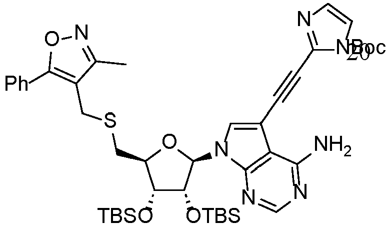

Modified nucleoside **178a** was prepared from iodinated derivative **122** (0.25 g, 0.31 mmol) and acetylene **167b** (0.18 g, 0.94 mmol) in analogous fashion to **169a.** FCC (5-50 % acetone in cyclohexane) furnished **178a** (0.25 g, 0.28 mmol, 91 %). **¹H NMR** (400 MHz, DMSO-*d₆*) δ 8.16 (1H, s), 8.05 (1H, s), 7.78-7.71 (2H, m), 7.63 (1H, d, *J=* 1.6 Hz), 7.55-7.47 (3H, m), 7.12 (1H, d, *J* = 1.5 Hz), 6.12 (1H, d, *J* = 7.0 Hz), 4.82 (1H, dd, *J* = 6.9, 4.5 Hz), 4.18 (1H, dd, *J=* 4.4, 1.1 Hz), 4.00 (1H, td, *J=* 6.9, 1.2 Hz), 3.92 (1H, d, *J=* 13.2 Hz), 3.84 (1H, d, *J* = 13.2 Hz), 3.11 (1H, dd, *J* = 13.7, 7.2 Hz), 3.00 (1H, dd, *J* = 13.6, 7.0 Hz), 2.29 (3H, s), 1.57 (9H, s), 0.91, 0.69 (18H, 2×s), 0.11, 0.10, -0.10, -0.38 (12H, 4×s). **¹³C NMR** (101 MHz, DMSO-*d₆*) δ 165.0, 160.7, 157.6, 153.5, 150.7, 146.9, 130.4, 129.4, 127.3, 126.9, 119.8, 111.1, 102.0, 94.2, 87.0, 86.6, 86.5, 85.0, 82.6, 74.8, 74.2, 34.6, 27.6, 25.9, 25.6, 24.6, 17.9, 17.6, 9.9, -4.5, -4.5, -4.6, -5.3. **LRMS** Calculated for C₄₄H₆₂N₇O₆SSi₂, [M+H]⁺ m/z: 872.4, [M+H]⁺, found 872.4.

### Example 218

### tert-Butyl 2-((4-amino-7-((2R,3R,4R,5S)-3,4-bis((tert-butyldimethylsilyl)oxy)-5-((((3-methyl-5-phenylisoxazol-4-yl)methyl)thio)methyl)tetrahydrofuran-2-yl)-7H-pyrrolo[2,3-d]pyrimidin-5-yl)ethynyl)-1H-benzo[d]imidazole-1-carboxylate (179a):

**179a** was synthesized from **122** (0.2 g, 0.25 mmol) and **169b** (0.18 g, 0.75 mmol) in analogous fashion 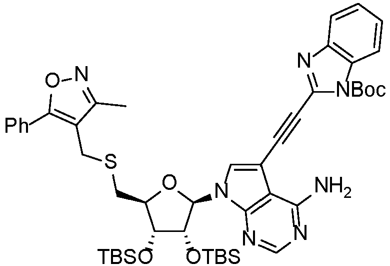 to **169a.** FCC (5-50 % acetone in cyclohexane) afforded **179a** (0.16 g, 0.17 mmol, 69 %). **¹H NMR** (400 MHz, DMSO-*d₆*) δ 8.18 (1H, s), 8.16 (1H, s), 7.89 (1H, ddd, *J=* 8.2, 1.1, 1.0 Hz), 7.77-7.74 (2H, m), 7.72 (1H, ddd, *J* = 8.0, 1.2, 0.6 Hz), 7.55-7.49 (3H, m), 7.46 (1H, dd, *J=* 8.2, 1.4 Hz), 7.42 (1H, dd, *J* = 8.0, 1.3 Hz), 6.13 (1H, d, *J* = 6.9 Hz), 4.86 (1H, dd, *J* = 6.9, 4.4 Hz), 4.20 (1H, dd, J = 4.3, 1.5 Hz), 4.01 (1H, td, *J=* 6.9, 1.5 Hz), 3.93 (1H, d, *J=* 13.3 Hz), 3.85 (1H, d, *J=* 13.3 Hz), 3.13 (1H, dd, *J=* 13.7, 7.2 Hz), 3.03 (1H, dd, *J=* 13.7, 6.9 Hz), 2.30 (3H, s), 1.68 (9H, s), 0.91, 0.69 (18H, 2×s), 0.12, 0.11, -0.09, -0.37 (12H, 4×s). **¹³C NMR** (101 MHz, DMSO-*d₆*) δ 165.0, 160.7, 157.6, 153.6, 150.8, 148.0, 142.8, 136.1, 131.4, 130.4, 129.4, 127.3, 126.9, 125.1, 120.0, 115.1, 111.1, 102.2, 93.8, 89.3, 87.2, 86.8, 85.1, 83.5, 74.8, 74.2, 34.6, 27.8, 25.9, 25.6, 24.6, 17.9, 17.6, 9.8, -4.46, -4.53, -4.6, -5.3. **LRMS** Calculated for C₄₈H₆₄N₇O₆SSi₂, [M+H]⁺ m/z: 922.4, [M+H]⁺, found 922.4.

### Example 219

### 4-((4-Amino-7-((2R,3R,4S,5S)-3,4-dihydroxy-5-((((3-methyl-5-phenylisoxazol-4-yl)methyl)thio) methyl)tetrahydrofuran-2-yl)-7H-pyrrolo[2,3-d]pyrimidin-5-yl)ethynyl)benzamide (169b):

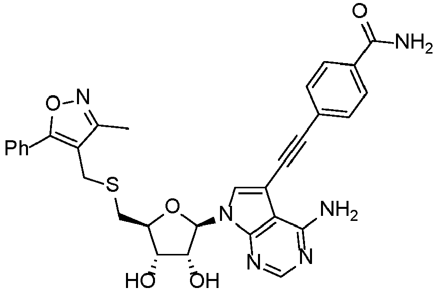

Title compound **169b** was prepared from **169a** (0.14 g, 0.17 mmol) following the same procedure as for **47c.** Reverse-phase FCC (5-70 % MeCN in H₂O) provided **169b** (0.09 g, 0.15 mmol, 90 %). **¹H NMR** (400 MHz, DMSO-*d₆*) δ 8.18 (1H, s), 8.05 (1H, bs), 7.92 (1H, s), 7.91 (2H, d, *J* = 7.1 Hz), 7.79-7.74 (2H, m), 7.65 (2H, d, *J=* 8.5 Hz), 7.56-7.44 (4H, m), 6.75 (2H, bs), 6.12 (1H, d, *J* = 5.9 Hz), 5.48 (1H, d, *J* = 6.2 Hz), 5.32 (1H, d, *J=* 5.0 Hz), 4.52 (1H, q, *J=* 5.2 Hz), 4.10-4.02 (2H, m), 3.87 (1H, d, *J=* 13.0 Hz), 3.83 (1H, d, *J=* 13.0 Hz), 2.99 (1H, dd, *J=* 13.8, 5.5 Hz), 2.92 (1H, dd, *J=* 13.8, 6.8 Hz), 2.29 (3H, s). **¹³C NMR** (101 MHz, DMSO-*d₆*) δ 167.3, 164.9, 160.8, 157.8, 153.2, 150.5, 133.9, 131.1, 130.3, 129.4, 127.9, 127.3, 126.9, 125.5, 111.1, 102.2, 95.1, 91.0. 87.2, 85.2, 83.6, 73.4, 72.9, 34.8, 24.4, 9.8. **HRMS** (ESI) Calculated for C₃₁H₂₉N₆O₅S, [M+H]⁺ m/z: 597.1915, [M+H]⁺, found 597.1913.

### Example 220

### (2R,3R,4S,5S)-2-(4-Amino-5-(pyridin-3-ylethynyl)-7H-pyrrolo[2,3-d]pyrimidin-7-yl)-5-((((3-methyl-5-phenylisoxazol-4-yl)methyl)thio)methyl)tetrahydrofuran-3,4-diol (170b):

Analogue **170b** was synthesized from **170a** (0.14 g, 0.18 mmol) in analogous fashion to **47c.** Reverse-phase FCC (10-70 % MeCN in H₂O) gave **170b** (0.09 g, 0.16 mmol, 88 %). **¹H NMR** (400 MHz, MSO-*d₆*) δ 8.78 (1H, d, *J* = 1.3 Hz), 8.57 (1H, dd, *J* = 4.8, 1.4 Hz), 8.18 (1H, s), 8.00 (1H, dt, *J=* 7.9, 1.8 Hz), 7.93 (1H, s), 7.80-7.73 (2H, m), 7.52-7.43 (3H, m), 7.42 (1H, ddd, J = 7.9, 4.8, 0.8 Hz), 6.79 (2H, bs), 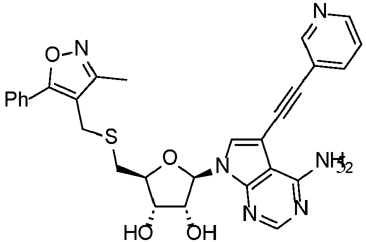 6.12 (1H, d, *J=* 5.9 Hz), 5.48 (1H, d, *J=* 6.2 Hz), 5.32 (1H, d, *J=* 4.9 Hz), 4.52 (1H, q, *J* = 5.7 Hz), 4.13-3.96 (2H, m), 3.87 (1H, *d, J=* 13.0 Hz), 3.83 (1H, *d, J=* 13.1 Hz), 2.98 (1H, dd, *J=* 13.9, 5.6 Hz), 2.92 (1H, dd, *J=* 13.9, 6.8 Hz), 2.28 (3H, s). **¹³C NMR** (101 MHz, DMSO-*d₆*) δ 165.0, 160.8, 157.7, 153.3, 151.7, 150.5, 148.8, 138.6, 130.3, 129.5, 127.9, 127.4, 126.9, 123.7, 120.0, 111.2, 102.1, 95.0, 88.3, 87.2, 86.2, 83.6, 73.4, 72.9, 34.8, 24.4, 9.8. **HRMS** (ESI) Calculated for C₂₉H₂₇N₆O₄S, [M+H]⁺ m/z: 555.1809, [M+H]⁺, found 555.1808.

### Example 221

### (2R,3R,4S,5S)-2-(4-Amino-5-(pyridin-2-ylethynyl)-7H-pyrrolo[2,3-d]pyrimidin-7-yl)-5-((((3-methyl-5-phenylisoxazol-4-yl)methyl)thio)methyl)tetrahydrofuran-3,4-diol (171b):

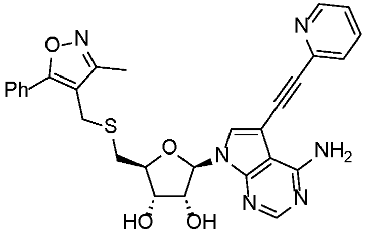

Modified nucleoside **171b** was prepared from protected analogue 171a (0.14 g, 0.18 mmol) according to the same methodology as for **47c.** Reverse-phase FCC (10-70 % MeCN in H₂O) furnished title compound **171b** (0.08 g, 0.15 mmol, 83 %). **¹H NMR** (400 MHz, DMSO-*d₆*) δ 8.60 (1H, ddd, *J=* 4.9, 1.8, 1.0 Hz), 8.19 (1H, s), 7.97 (1H, s), 7.85 (1H, td, *J* = 7.7, 1.8 Hz), 7.79-7.73 (2H, m), 7.66 (1H, dt, *J=* 7.9, 1.1 Hz), 7.56-7.46 (3H, m), 7.40 (1H, ddd, *J=* 7.6, 4.9, 1.2 Hz), 6.80 (2H, bs), 6.11 (1H, d, *J=* 5.9 Hz), 5.50 (1H, d, *J=* 6.2 Hz), 5.33 (1H, d, *J* = 5.0 Hz), 4.53 (1H, q, *J=* 5.8 Hz), 4.11-4.01 (2H, m), 3.87 (1H, d, *J=* 13.0 Hz), 3.83 (1H, d, *J=* 13.1 Hz), 2.98 (1H, dd, *J=* 13.9, 5.7 Hz), 2.93 (1H, dd, *J=* 13.9, 6.9 Hz), 2.28 (3H, s). **¹³C NMR** (101 MHz, DMSO-*d₆*) δ 164.9, 160.8, 157.8, 153.4, 150.5, 150.4, 142.7, 137.0, 130.3, 129.4, 128.1, 127.3, 126.9, 126.8, 123.5, 111.2, 102.4, 94.5, 91.7, 87.3, 83.7, 82.6, 73.4, 72.9, 34.8, 24.4, 9.8. **HRMS** (ESI) Calculated for C₂₉H₂₇N₆O₄S, [M+H]⁺ m/z: 555.1809, [M+H]⁺, found 555.1808.

### Example 222

### (2R,3R,4S,5S)-2-(4-Amino-5-(pyrimidin-5-ylethynyl)-7H-pyrrolo[2,3-d]pyrimidin-7-yl)-5-(((3-methyl-5-phenylisoxazol-4-yl)methyl)thio)methyl)tetrahydrofuran-3,4-diol (172b):

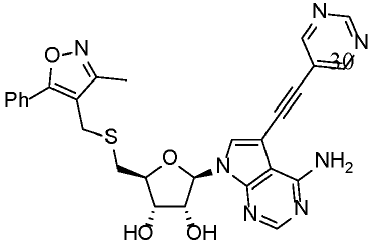

Derivative **172b** was synthesized from **172a** (0.14 g, 0.18 mmol) in analogous fashion to **47c.** Reverse-phase FCC (10-60 % MeCN in H₂O) gave **172b** (0.09 g, 0.17 mmol, 94 %). **¹H NMR** (400 MHz, DMSO-*d₆*) δ 9.17 (1H, s), 9.02 (1H, s), 8.18 (1H, s), 7.97 (1H, s), 7.79-7.73 (2H, m), 7.55-7.46 (3H, m), 6.90 (2H, bs), 6.12 (1H, d, *J =* 5.9 Hz), 5.49 (1H, d, *J=* 6.2 Hz), 5.34 (1H, d, *J=* 5.0 Hz), 4.52 (1H, q, *J=* 5.9 Hz), 4.10-4.02 (2H, m), 3.87 (1H, d, *J=* 13.0 Hz), 3.83 (1H, *d, J=* 13.0 Hz), 2.98 (1H, dd, *J=* 13.9, 5.6 Hz), 2.92 (1H, dd, *J=* 13.9, 6.8 Hz), 2.28 (3H, s). **¹³C NMR** (101 MHz, DMSO-*d₆*) δ 164.9, 160.8, 158.7, 157.7, 156.6, 153.3, 150.6, 130.3, 129.4, 128.4, 127.3, 126.9, 119.6, 111.1, 102.0, 94.5, 89.7, 87.2, 85.0, 83.6, 73.4, 72.9, 34.8, 24.4, 9.8. **HRMS** (ESI) Calculated for C₂₈H₂₆N₇O₄S, [M+H]⁺ m/z: 556.1762, [M+H]⁺, found 556.1761.

### Example 223

### (2R,3R,4S,5S)-2-(4-Amino-5-(pyridazin-4-ylethynyl)-7H-pyrrolo[2,3-d]pyrimidin-7-yl)-5-(((3-methyl-5-phenylisoxazol-4-yl)methyl)thio)methyl)tetrahydrofuran-3,4-diol (173b):

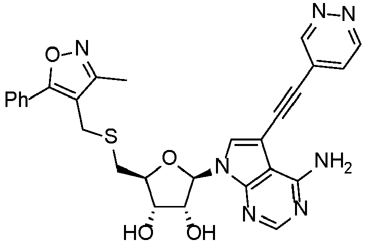

SAH analogue **173b** was prepared from derivative **173a** (0.14 g, 0.18 mmol) following the same methodology as for **47c.** Reverse-phase FCC (10-70 % MeCN in H₂O) provided title compound **173b** (0.1 g, 0.17 mmol, 94 %). **¹H NMR** (400 MHz, DMSO-*d₆*) δ 9.36 (1H, dd, *J=* 2.2, 1.3 Hz), 9.26 (1H, dd, *J=* 5.4, 1.2 Hz), 8.19 (1H, s), 8.05 (1H, s), 7.85 (1H, dd, *J=* 5.4, 2.3 Hz), 7.80-7.72 (2H, m), 7.59-7.44 (3H, m), 6.90 (2H, bs), 6.12 (1H, d, *J=* 5.9 Hz), 5.50 (1H, d, *J=* 5.7 Hz), 5.34 (1H, d, *J=* 3.4 Hz), 4.53 (1H, q, *J=* 4.9 Hz), 4.13-4.01 (2H, m), 3.87 (1H, d, *J=* 13.0 Hz), 3.83 (1H, d, *J=* 13.0 Hz), 2.99 (1H, dd, *J=* 13.9, 5.5 Hz), 2.92 (1H, dd, *J=* 13.9, 6.8 Hz), 2.28 (3H, s). **¹³C NMR** (101 MHz, DMSO-*d₆*) δ 164.9, 160.8, 157.6, 153.4, 152.0, 151.2, 150.7, 130.3, 129.6, 129.4, 127.4, 127.3, 126.8, 123.3, 111.1, 101.9, 94.1, 91.6, 87.3, 86.5, 83.7, 73.4, 72.9, 34.8, 24.4, 9.8. **HRMS** (ESI) Calculated for C₂₈H₂₆N₇O₄S, [M+H]⁺ m/z: 556.1762, [M+H]⁺, found 556.1761.

### Example 224

### (2R,3R,4S,5S)-2-(4-Amino-5-((2-aminopyrimidin-5-yl)ethynyl)-7H-pyrrolo[2,3-d]pyrimidin-7-yl)-5-((((3-methyl-5-phenylisoxazol-4-yl)methyl)thio)methyl)tetrahydrofuran-3,4-diol (174b):

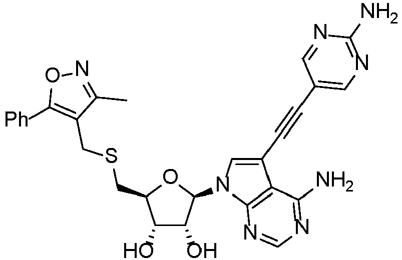

Title compound **174b** was synthesized from **174a** (0.14 g, 0.18 mmol) according to the same procedure as for **47c.** Reverse-phase FCC (5-70 % MeCN in H₂O) furnished SAH analogue **174b** (0.06 g, 0.11 mmol, 60 %). **¹H NMR** (400 MHz, DMSO-*d₆*) δ 8.45 (2H, s), 8.15 (1H, s), 7.80 (1H, s), 7.79-7.74 (2H, m), 7.58-7.44 (3H, m), 7.10 (2H, bs), 6.72 (2H, bs), 6.10 (1H, d, *J=* 5.8 Hz), 5.47 (1H, d, *J* = 6.0 Hz), 5.31 (1H, d, *J =* 4.7 Hz), 4.50 (1H, q, *J=* 5.1 Hz), 4.10-3.99 (2H, m), 3.87 (1H, d, *J=* 13.1 Hz), 3.82 (1H, *d, J=* 13.1 Hz), 2.97 (1H, dd, J= 13.8, 5.4 Hz), 2.91 (1H, dd, J = 13.9, 6.8 Hz), 2.28 (3H, s). **¹³C NMR** (101 MHz, DMSO-*d₆*) δ 164.9, 162.0, 160.8, 160.4, 157.7, 153.1, 150.3, 130.3, 129.4, 127.3, 126.9, 111.1, 106.4, 102.1, 95.7, 87.1, 86.7, 85.1, 83.5, 73.4, 72.9, 34.8, 24.4, 9.8. **HRMS** (ESI) Calculated for C₂₈H₂₆N₇O₄S, [M+H]⁺ m/z: 571.1871, [M+H]⁺, found 858.4686.

### Example 225

### (2R,3R,4S,5S)-2-(4-Amino-5-(naphthalen-1-ylethynyl)-7H-pyrrolo[2,3-d]pyrimidin-7-yl)-5-((((3-methyl-5-phenylisoxazol-4-yl)methyl)thio)methyl)tetrahydrofuran-3,4-diol (175b):

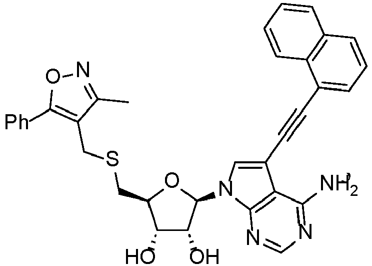

Modified nucleoside **175b** was prepared from SAH analogue **175a** (0.16 g, 0.19 mmol) using the same methodology as for **47c.** FCC (5-80 % MeCN in H₂O) gave title compound **175b** (0.1 g, 0.17 mmol, 91 %). **¹H NMR** (400 MHz, DMSO-*d₆*) δ 8.36-8.28 (1H, m), 8.20 (1H, s), 8.05 (1H, s), 8.04-7.97 (2H, m), 7.87 (1H, dd, *J=* 7.2, 1.1 Hz),7.80-7.73 (2H, m), 7.68-7.59 (2H, m), 7.57 (1H, dd, *J=* 8.2, 7.2 Hz), 7.55-7.44 (3H, m), 6.78 (2H, bs), 6.15 (1H, d, *J* = 5.9 Hz), 5.50 (1H, d, *J* = 6.2 Hz), 5.32 (1H, d, *J* = 5.0 Hz), 4.57 (1H, q, *J=* 5.9 Hz), 4.14-4.02 (2H, m), 3.89 (1H, d, *J=* 13.0 Hz), 3.84 (1H, d, *J=* 13.0 Hz), 3.01 (1H, dd, *J=* 13.9, 5.7 Hz), 2.95 (1H, dd, *J* = 13.9, 6.9 Hz), 2.29 (3H, s). **¹³C NMR** (101 MHz, DMSO-*d₆*) δ 165.0, 160.9, 157.9, 153.3, 150.6, 133.1, 132.6, 130.5, 130.3, 129.5, 129.1, 128.8, 127.6, 127.5, 127.4, 127.0, 126.9, 125.9, 125.6, 120.2, 111.2, 102.3, 95.5, 89.4, 88.0, 87.1, 83.7, 73.4, 72.9, 34.9, 24.5, 9.8. **HRMS** (ESI) Calculated for C₃₄H₃₀N₅O₄S, [M+H]⁺ m/z: 604.2013, [M+H]⁺, found 604.2012.

### Example 226

### (2R,3R,4S,5S)-2-(4-Amino-5-(quinolin-3-ylethynyl)-7H-pyrrolo[2,3-d]pyrimidin-7-yl)-5-((((3-methyl-5-phenylisoxazol-4-yl)methyl)thio)methyl)tetrahydrofuran-3,4-diol (176b):

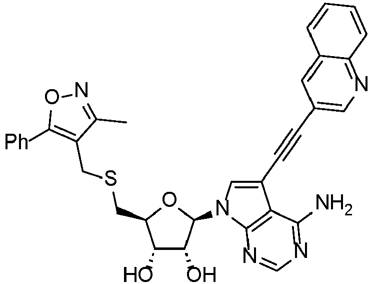

**176b** was prepared from **176a** (0.2 g, 0.23 mmol) in analogous fashion to **47c.** Reverse-phase FCC (5-80 % MeCN in H₂O) gave **176b** (0.13 g, 0.22 mmol, 93%). **¹H NMR** (400 MHz, DMSO-*d₆*) δ 9.03 (1H, d, *J=* 1.8 Hz), 8.63 (1H, *d, J=* 1.8 Hz), 8.19 (1H, s), 8.05 (1H, d, *J=* 8.4 Hz), 8.00 (1H, d, *J=* 8.3 Hz), 7.98 (1H, s), 7.85-7.74 (3H, m), 7.67 (1H, t, *J=* 7.4 Hz), 7.58-7.45 (3H, m), 6.86 (2H, bs), 6.14 (1H, *d, J=* 5.8 Hz), 5.50 (1H, *d, J=* 6.1 Hz), 5.34 (1H, d, *J* = 4.9 Hz), 4.54 (1H, q, *J* = 5.7 Hz), 4.14-3.99 (2H, m), 3.88 (1H, d, *J* = 13.1 Hz), 3.84 (1H, d, *J* = 13.1 Hz), 3.00 (1H, dd, *J* = 13.9, 5.5 Hz), 2.94 (1H, dd, *J* = 13.9, 6.8 Hz), 2.29 (3H, s). **¹³C NMR** (101 MHz, DMSO-*d₆*) δ 165.0, 160.9, 157.8, 153.4, 151.9, 150.6, 146.4, 138.2, 130.7, 130.4, 129.5, 129.1, 128.3, 128.1, 127.8, 127.4, 127.2, 126.9, 117.2, 111.2, 102.2, 95.1, 89.0, 87.3, 86.4, 83.7, 73.5, 72.9, 34.9, 24.5, 9.9. **HRMS** (ESI) Calculated for C₃₃H₂₉N₆O₄S, [M+H]⁺ m/z: 605.1966, [M+H]⁺, found 605.1965.

### Example 227

### (2R,3R,4S,5S)-2-(4-Amino-5-(quinoxalin-2-ylethynyl)-7H-pyrrolo[2,3-d]pyrimidin-7-yl)-5-((((3-methyl-5-phenylisoxazol-4-yl)methyl)thio)methyl)tetrahydrofuran-3,4-diol (177b):

Title compound **177b** was synthesized from SAH analogue **177a** (0.19 g, 0.22 mmol) using the same conditions as for **47c.** Reverse-phase FCC (10-70 % MeCN in H₂O) gave derivative **177b** (0.12 g, 0.20 mmol, 89 %). **¹H NMR** (400 MHz, DMSO-*d₆*) δ 9.16 (1H, s), 8.22 (1H, s), 8.15 (1H, s), 8.14-8.09 (1H, m), 8.09-8.03 (1H, m), 7.94-7.84 (2H, m), 7.79-7.74 (2H, m), 7.57-7.45 (3H, m), 6.95 (2H, bs), 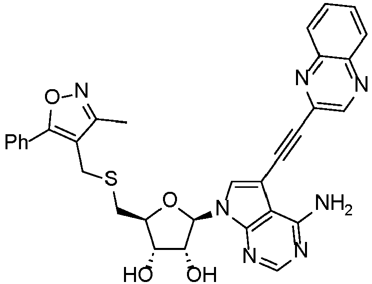 6.14 (1H, d, *J* = 5.8 Hz), 5.51 (1H, d, *J* = 6.1 Hz), 5.34 (1H, d, *J=* 4.8 Hz), 4.56 (1H, q, *J=* 5.7 Hz), 4.14-4.03 (2H, m), 3.88 (1H, d, *J=* 13.1 Hz), 3.84 (1H, d, *J=* 13.0 Hz), 3.00 (1H, dd, *J=* 14.1, 6.0 Hz), 2.98 (1H, dd, *J=* 14.1, 7.1 Hz), 2.29 (3H, s). **¹³C NMR** (101 MHz, DMSO-*d₆*) δ 165.0, 160.9, 157.8, 153.5, 150.7, 147.8, 141.8, 140.4, 139.6, 131.3, 130.9, 130.4, 129.9, 129.5, 129.2, 128.9, 127.4, 126.9, 111.2, 102.2, 94.0, 90.2, 87.6, 87.4, 83.8, 73.5, 72.9, 34.8, 24.5, 9.8. **HRMS** (ESI) Calculated for C₃₂H₂₈N₇O₄S, [M+H]⁺ m/z: 606.1918, [M+H]⁺, found 606.1917.

### Example 228

### (2R,3R,4S,5S)-2-(5-((1H-Imidazol-2-yl)ethynyl)-4-amino-7H-pyrrolo[2,3-d]pyrimidin-7-yl)-5-((((3-methyl-5-phenylisoxazol-4-yl)methyl)thio)methyl)tetrahydrofuran-3,4-diol (178b):

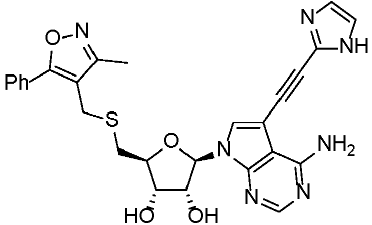

Protected derivative **178a** (0.23 g, 0.26 mmol) was dissolved in TFA/H₂O (9:1, 5 mL) at RT. After stirring for 3 h at RT, the reaction mixture was concentrated and the residue was co-evaporated with MeOH. The residual acid was neutralized with NH₄OH, the volatiles were removed under reduced pressure and the crude product was subjected to reverse-phase FCC (10-60 % MeCN in H₂O) affording desired product **178b** (0.13 g, 0.23 mmol, 87 %). **¹H NMR** (400 MHz, DMSO-*d₆*) δ 12.84 (1H, s), 8.18 (1H, s), 7.92 (1H, s), 7.80-7.73 (2H, m), 7.58-7.45 (3H, m), 7.27 (1H, bs), 7.04 (1H, s), 6.80 (2H, bs), 6.10 (1H, d, *J=* 5.9 Hz), 5.48 (1H, d, *J=* 6.0 Hz), 5.31 (1H, d, *J=* 4.8 Hz), 4.53 (1H, q, *J=* 5.7 Hz), 4.11-4.00 (2H, m), 3.87 (1H, d, *J=* 13.1 Hz), 3.83 (1H, d, *J=* 13.0 Hz), 2.97 (1H, dd, *J=* 14.0, 5.8 Hz), 2.92 (1H, dd, *J=* 13.8, 7.0 Hz), 2.28 (3H, s). **¹³C NMR** (101 MHz, DMSO-*d₆*) δ 164.9, 160.8, 157.7, 153.3, 150.4, 130.3, 129.4, 129.2, 127.7, 127.3, 126.8, 111.1, 102.1, 94.4, 87.3, 83.6, 83.2, 82.1, 73.3, 72.9, 34.8, 24.4, 9.8. **HRMS** (ESI) Calculated for C₂₇H₂₆N₇O₄S, [M+H]⁺ m/z: 544.1762, [M+H]⁺, found 544.1761.

### Example 229

### (2R,3R,4S,5S)-2-(5-((1H-Benzo[d]imidazol-2-yl)ethynyl)-4-amino-7H-pyrrolo[2,3-d]pyrimidin-7-yl)-5-((((3-methyl-5-phenylisoxazol-4-yl)methyl)thio)methyl)tetrahydrofiiran-3,4-diol (179b):

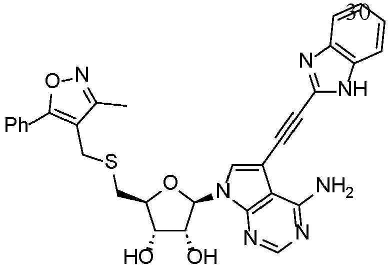

Modified nucleoside **179b** was prepared from **179a** (0.14 g, 0.15 mmol) according to the same procedure as for **178b.** Reverse-phase FCC (5-90 % MeCN in H₂O) furnished **179b** (0.08 g, 0.13 mmol, 83 %). **¹H NMR** (400 MHz, DMSO-*d₆*) δ 8.21 (1H, bs), 8.07 (1H, s), 7.80-7.74 (2H, m), 7.59 (2H, bs), 7.56-7.51 (2H, m), 7.51-7.47 (1H, m), 7.26 (2H, dd, *J=* 6.0, 3.0 Hz), 6.92 (2H, bs), 6.12 (1H, d, *J=* 5.9 Hz), 5.50 (1H, d, *J* = 5.9 Hz), 5.33 (1H, d, *J* = 4.2 Hz), 4.56 (1H, q, *J* = 5.4 Hz), 4.11-4.03 (2H, m), 3.87 (1H, d, *J=* 13.1 Hz), 3.84 (1H, d, *J* = 13.1 Hz), 2.99 (1H, dd, *J=* 13.9, 5.8 Hz), 2.93 (1H, dd, *J=* 13.9, 7.0 Hz), 2.29 (3H, s). **¹³C NMR** (101 MHz, DMSO-*d₆*) δ 164.9, 160.8, 157.7, 153.4, 150.6, 135.4, 130.3, 129.4, 129.1, 127.3, 126.9, 111.1, 102.1, 93.6, 87.4, 85.0, 83.7, 83.2, 73.3, 72.9, 34.8, 24.4, 9.8. **HRMS** (ESI) Calculated for C₃₁H₂₈N₇O₄S, [M+H]⁺ m/z: 594.1918, [M+H]⁺, found 594.1917.

### Example 230

### (2R,3R,4S,5S)-2-(4-Amino-5-(phenylethynyl)-7H-pyrrolo[2,3-d]pyrimidin-7-yl)-5-((((3-methyl-5-phenylisoxazol-4-yl)methyl)thio)methyl)tetrahydrofuran-3,4-diol (180):

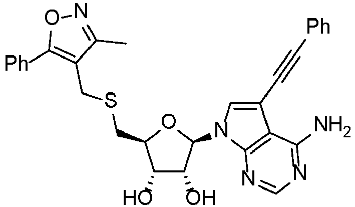

To a suspension of **123** (0.07 g, 0.12 mmol), Pd(PPh₃)₄ (0.01 g, 0.01 mmol), CuI (0.01 g, 0.03 mmol) and phenylacetylene (0.04 mL, 0.36 mmol) in dry THF (3 mL), TEA (0.04 mL, 0.40 mmol) was added. After stirring under argon atmosphere for 10 h at 70 °C, the reaction mixture was cooled to RT, the volatiles were evaporated in vacuo. FCC (0-25 % EtOAc/acetone/EtOH/H₂O (84/12/2.4/1.6) mixture in EtOAc/acetone/EtOH/H₂O (68/12/12/8) mixture) gave **180** (0.05 g, 0.10 mmol, 79 %). **¹H NMR** (400 MHz, DMSO-*d₆*) δ 8.18 (1H, bs), 7.88 (1H, s), 7.79-7.74 (2H, m), 7.59-7.56 (2H, m), 7.56-7.51 (2H, m), 7.51-7.46 (1H, m), 7.45-7.40 (3H, m), 6.71 (2H, bs), 6.11 (1H, d, *J=* 5.9 Hz), 5.48 (1H, d, *J=* 6.2 Hz), 5.32 (1H, d, *J* = 5.0 Hz), 4.52 (1H, q, *J* = 6.0 Hz), 4.11-4.00 (2H, m), 3.87 (1H, d, *J* = 13.0 Hz), 3.83 (1H, d, *J=* 13.0 Hz), 2.98 (1H, dd, *J=* 13.8, 5.7 Hz), 2.92 (1H, dd, *J=* 13.8, 6.9 Hz), 2.29 (3H, s). **¹³C NMR** (101 MHz, DMSO-*d₆*) δ 164.9, 160.8, 157.8, 153.2, 150.4, 131.3, 130.3, 129.4, 128.9, 128.7, 127.3, 127.2, 126.9, 122.7, 111.1, 95.4, 91.4, 87.1, 83.6, 83.1, 73.3, 72.9, 34.8, 24.4, 9.8. **HRMS** (ESI) Calculated for C₃₀H₂₈N₅O₄S, [M+H]⁺ m/z: 554.1857, [M+H]⁺, found 554.1856.

### Example 231

### 7-((2R,3R,4R,5S)-3,4-bis((tert-Butyldimethylsilyl)oxy)-5-(((3-methyl-5-phenylisoxazol-4-yl)methyl) thio)methyl)tetrahydrofuran-2-yl)-5-((E)-styryl)-7H-pyrrolo[2,3-d]pyrimidin-4-amine (181a):

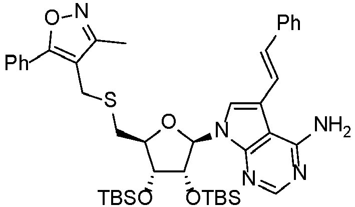

Analogue **181a** was prepared from **122** (0.09 g, 0.11 mmol) and *trans*-2-phenylvinylboronic acid (0.03 g, 0.21 mmol) using the same procedure as for **143a.** FCC (10-65 % EtOAc in DCM) afforded title compound **181a** (0.07 g, 0.09 mmol, 86 %). **¹H NMR** (400 MHz, DMSO-*d₆*) δ 8.07 (1H, s), 7.83 (1H, s), 7.77-7.72 (2H, m), 7.61 (2H, d, *J=* 7.4 Hz), 7.56-7.44 (4H, m), 7.35 (2H, t, *J=* 7.7 Hz), 7.22 (1H, t, *J=* 7.3 Hz), 6.96 (1H, d, *J=* 16.0 Hz), 6.90 (2H, bs), 6.16 (1H, d, *J=* 7.2 Hz), 4.84 (1H, dd, *J=* 7.2, 4.4 Hz), 4.19 (1H, dd, *J* = 4.3, 1.0 Hz), 4.00 (1H, td, *J=* 7.4, 1.0 Hz), 3.95 (1H, d, *J=* 13.2 Hz), 3.86 (1H, d, *J=* 13.2 Hz), 3.13 (1H, dd, *J* = 13.9, 7.3 Hz), 3.00 (1H, dd, *J=* 13.8, 6.8 Hz), 2.28 (3H, s), 0.92, 0.68 (18H, 2×s), 0.12, 0.11, -0.10, -0.37 (12H, 4×s). **¹³C NMR** (101 MHz, DMSO-*d₆*) δ 165.0, 160.7, 157.9, 151.9, 151.7, 137.5, 130.3, 129.4, 128.7, 127.9, 127.3, 127.2, 126.9, 126.6, 120.6, 119.2, 114.7, 111.5, 111.1, 108.7, 101.2, 86.4, 84.8, 74.8, 74.0, 34.7, 25.9, 25.7, 24.7, 17.9, 17.7, 9.8, -4.4, -4.5, -4.5, -5.3. **LRMS** Calculated for C₄₂H₅₈N₅O₄SSi₂, [M+H]⁺ m/z: 784.4, [M+H]⁺, found 784.4.

### Example 232

### 7-((2R,3R,4R,5S)-3,4-bis((-----tert-Butyldimethylsilyl)oxy)-5-(((3-methyl-5-phenylisoxazol-4-yl)methyl)thio)methyl)tetrahydrofuran-2-yl)-5-((E)-4-methoxystyryl)-7H-pyrrolo[2,3-d]pyrimidin-4-amine (182a):

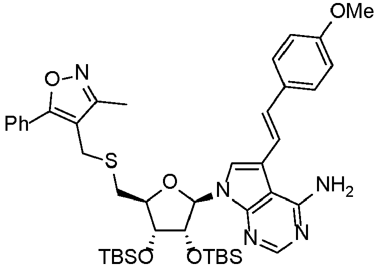

Modified nucleoside **182a** was prepared from **122** (0.2 g, 0.25 mmol) and t*rans*-2-(4-methoxyphenyl)vinylboronic acid (0.09 g, 0.49 mmol) following the same conditions as for **143a.** FCC (15-75 % EtOAc in DCM) gave SAH analogue **182a** (0.12 g, 0.14 mmol, 57%). **¹H NMR** (400 MHz, DMSO-*d₆*) δ 8.06 (1H, s), 7.78-7.71 (3H, m), 7.55 (2H, d, *J* = 8.7 Hz), 7.52-7.45 (3H, m), 7.36 (1H, d, *J* = 16.0 Hz), 6.95-6.83 (5H, m), 6.15 (1H, d, *J=* 7.2 Hz), 4.82 (1H, dd, *J=* 7.1, 4.4 Hz), 4.19 (1H, dd, *J=* 4.3, 0.9 Hz), 3.99 (1H, t, *J=* 7.4 Hz), 3.95 (1H, d, *J=* 13.2 Hz), 3.86 (1H, d, *J=* 13.2 Hz), 3.77 (3H, s), 3.12 (1H, dd, *J* = 13.8, 7.3 Hz), 2.99 (1H, dd, *J* = 13.8, 6.8 Hz), 2.28 (3H, s), 0.92, 0.68 (18H, 2×s), 0.12, 0.11, -0.10, -0.37 (12H, 4×s). **¹³C NMR** (101 MHz, DMSO-*d₆*) δ 165.0, 160.7, 158.7, 157.9, 151.9, 151.7, 130.4, 130.3, 129.4, 127.9, 127.7, 127.3, 126.9, 118.5, 118.3, 115.1, 114.1, 111.1, 101.2, 86.4, 84.7, 74.8, 74.0, 55.3, 34.7, 25.9, 25.7, 24.7, 17.9, 17.7, 9.8, -4.4, -4.45, -4.52, -5.3. **LRMS** Calculated for C₄₃H₆₀N₅O₄SSi₂, [M+H]⁺ m/z: 814.4, [M+H]⁺, found 814.4.

### Example 233

### (2R,3R,4S,5S)-2-(4-Amino-5-((E)-styryl)-7H-pyrrolo[2,3-d]pyrimidin-7-yl)-5-((((3-methyl-5-phenylisoxazol-4-yl)methyl)thio)methyl)tetrahydrofuran-3,4-diol (181b):

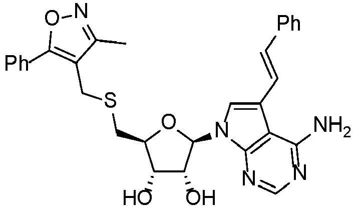

Title compound **181b** was prepared from protected analogue **181a** (0.06 g, 0.08 mmol) using the same conditions as for **47c.** Reverse-phase FCC (5-80 % MeCN in H₂O) afforded styryl derivative **181b** (0.03 g, 0.05, 69 %). **¹H NMR** (400 MHz, DMSO-*d₆*) δ 8.10 (1H, s), 7.83 (1H, s), 7.76 (2H, d, *J* = 6.8 Hz), 7.61 (2H, d, *J=* 7.0 Hz), 7.57-7.43 (4H, m), 7.35 (2H, t, *J=* 6.9 Hz), 7.22 (1H, t, *J=* 7.1 Hz), 7.01 (1H, *d, J=* 16.2 Hz), 6.96 (2H, bs), 6.15 (1H, d, *J=* 5.2 Hz), 5.45 (1H, d, *J* = 4.8 Hz), 5.29 (1H, d, *J*=4.8 Hz), 4.53 (1H, q, *J=* 4.7 Hz), 4.15-3.99 (2H, m), 3.88 (1H, d, *J=* 13.2 Hz), 3.84 (1H, d, *J=* 13.1 Hz), 3.00 (1H, dd, *J=* 14.1, 5.8 Hz), 2.92 (1H, dd, *J=* 14.0, 7.2 Hz), 2.27 (3H, s). **¹³C NMR** (101 MHz, DMSO-*d₆*) δ 164.9, 160.8, 157.8, 151.8, 151.5, 137.6, 130.3, 129.4, 128.7, 128.0, 127.3, 127.2, 126.8, 126.6, 120.4, 118.8, 114.7, 111.1, 101.0, 86.8, 83.3, 73.3, 72.9, 34.9, 24.5, 9.8. **HRMS** (ESI) Calculated for C₃₀H₃₀N₅O₄S, [M+H]⁺ m/z: 556.2013, [M+H]⁺, found 556.2008.

### Example 234

### (2R,3R,4S,5S)-2-(4-Amino-5-((E)-4-methoxystyryl)-7H-pyrrolo[2,3-d]pyrimidin-7-yl)-5-((((3-methyl-5-phenylisoxazol-4-yl)methyl)thio)methyl)tetrahydrofuran-3,4-diol (182b):

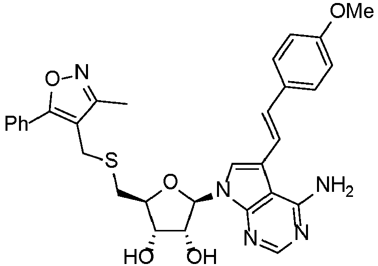

Derivative **182b** was synthesized from modified nucleoside **182a** (0.11 g, 0.13 mmol) following the same methodology as for **47c.** FCC (10-90 % MeCN in H₂O) provided SAH analogue **182b** (0.05 g, 0.10 mmol, 77 %). **¹H NMR** (400 MHz, DMSO-*d₆*) δ 8.08 (1H, s), 7.80-7.71 (3H, m), 7.57 (2H, d, *J* = 8.7 Hz), 7.55-7.44 (3H, m), 7.36 (1H, d, *J* = 16.0 Hz), 6.94 (1H, d, *J=* 15.9 Hz), 6.92 (1H, d, *J=* 8.8 Hz), 6.89 (2H, bs), 6.15 (1H, d, *J=* 5.9 Hz), 5.44 (1H, d, *J=* 6.2 Hz), 5.28 (1H, d, *J=* 5.2 Hz), 4.52 (1H, q, *J=* 5.8 Hz), 4.10 (1H, q, *J=* 5.0 Hz), 4.04 (1H, q, *J=* 6.1 Hz), 3.88 (1H, d, *J=* 13.0 Hz), 3.83 (1H, d, *J* = 13.0 Hz), 3.77 (3H, s), 2.99 (1H, dd, *J=* 13.9, 5.7 Hz), 2.92 (1H, dd, *J=* 13.9, 7.0 Hz), 2.27 (3H, s). **¹³C NMR** (101 MHz, DMSO-*d₆*) δ 164.9, 160.8, 158.7, 157.9, 151.9, 151.5, 130.4, 130.3, 129.4, 127.9, 127.7, 127.3, 126.8, 118.2, 118.1, 115.0, 114.1, 111.2, 101.0, 86.8, 83.3, 73.3, 72.9, 55.3, 34.9, 24.5, 9.8. **HRMS** (ESI) Calculated for C₃₁H₃₂N₅O₅S, [M+H]⁺ m/z: 586.2119, [M+H]⁺, found 586.2116.

### Example 235

### (2R,3R,4S,5S)-2-(4-Amino-5-(2-(pyrimidin-5-yl)ethyl)-7H-pyrrolo[2,3-d]pyrimidin-7-yl)-5-((((3-methyl-5-phenylisoxazol-4-yl)methyl)thio)methyl)tetrahydrofuran-3,4-diol (183):

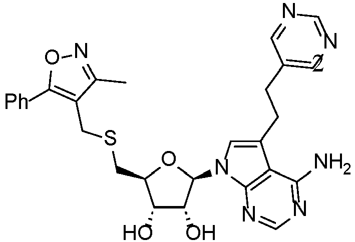

Saturated derivative **183** was synthesized from unsaturated analogue **172b** (0.07 g, 0.13 mmol) using the similar conditions as for **6.** Reverse-phase FCC (10-60 % MeCN in H₂O) afforded title compound **183** (0.05 g, 0.10 mmol, 75 %). **¹H NMR** (400 MHz, DMSO-*d₆*) δ 8.98 (1H, s), 8.65 (2H, s), 8.05 (1H, s), 7.79-7.71 (2H, m), 7.56-7.43 (3H, m), 7.08 (1H, s), 6.66 (2H, bs), 6.07 (1H, d, *J=* 5.5 Hz), 5.37 (1H, d, *J=* 6.1 Hz), 5.24 (1H, d, *J=* 5.2 Hz), 4.38 (1H, q, *J=* 5.6 Hz), 4.07-3.95 (2H, m), 3.85 (1H, d, *J=* 13.0 Hz), 3.80 (1H, d, *J=* 13.0 Hz), 3.09 (1H, dd, *J=* 8.2, 7.2 Hz), 2.93 (1H, dd, *J=* 13.9, 5.2 Hz), 2.87 (1H, dd, *J* = 8.2, 7.6 Hz), 2.82 (1H, dd, *J=* 13.8, 6.6 Hz), 2.26 (3H, s). **¹³C NMR** (101 MHz, DMSO-*d₆*) δ 164.9, 160.8, 157.9, 157.0, 156.5, 151.9, 151.4, 134.8, 130.3, 129.4, 127.3, 126.8, 119.1, 114.6, 111.2, 102.1, 86.8, 83.0, 73.3, 72.9, 34.8, 30.5, 26.7, 24.5, 9.8. **HRMS** (ESI) Calculated for C₂₈H₃₀N₇O₄S, [M+H]⁺ m/z: 560.2074, [M+H]⁺, found 560.2073.

### Examole 236

### (2R,3R,4S,5S)-2-(4-Amino-5-phenethyl-7H-pyrrolo[2,3-d]pyrimidin-7-yl)-5-((((3-methyl-5-phenylisoxazol-4-yl)methyl)thio)methyl)tetrahydrofuran-3,4-diol (184):

**184** was prepared from **180** (0.07 g, 0.13 mmol) in analogous fashion to **6.** Reverse-phase FCC (10-90 % MeCN in H₂O) provided **184** (0.06 g, 0.11 mmol, 84 %). **¹H NMR** (400 MHz, DMSO-*d₆*) δ 8.05 (1H, s), 7.79-7.72 (2H, m), 7.55-7.44 (3H, m), 7.27-7.20 (4H, m), 7.17-7.11 (1H, m), 7.07 (1H, s), 6.57 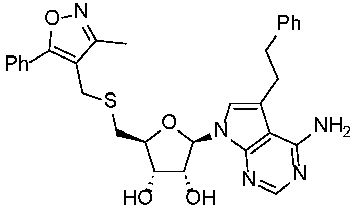 (2H, bs), 6.08 (1H, d, *J* = 5.7 Hz), 5.36 (1H, d, *J* = 6.2 Hz), 5.24 (1H, d, *J=* 5.1 Hz), 4.39 (1H, q, *J =* 5.7 Hz), 4.05-3.96 (2H, m), 3.85 (1H, d, *J=* 13.0 Hz), 3.79 (1H, d, *J=* 13.0 Hz), 3.05 (1H, dd, *J=* 8.6, 7.2 Hz), 2.94 (1H, dd, *J=* 13.9, 5.2 Hz), 2.89-2.79 (3H, m), 2.26 (3H, s). **¹³C NMR** (101 MHz, DMSO-*d₆*) δ 164.9, 160.8, 158.0, 151.8, 151.4, 141.7, 130.3, 129.4, 128.6, 128.3, 127.3, 126.8, 125.9, 118.9, 115.4, 111.2, 102.4, 86.8, 83.1, 73.2, 72.9, 36.0, 34.9, 27.7, 24.6, 9.8. **HRMS** (ESI) Calculated for C₃₀H₃₂N₅O₄S, [M+H]⁺ m/z: 558.2170, [M+H]⁺, found 558.2166.

### Example 237

### 5-Benzyl-7-((2R,3R,4R,5S)-3,4-bis((tert-Butyldimethylsilyl)oxy)-5-(((3-methyl-5-phenylisoxazol-4-yl)methyl)thio)methyl)tetrahydrofuran-2-yl)-7H-pyrrolo[2,3-d]pyrimidin-4-amine (185a):

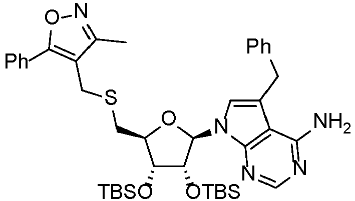

Title compound **185a** was prepared from **122** (0.2 g, 0.25 mmol) and 0.5M solution of benzylzinc bromide in THF (1.5 mL, 0.75 mmol) following the same procedure as for **129a.** FCC (10-80 % EtOAc in DCM) gave (0.16 g, 0.21 mmol, 84 %). **¹H NMR** (400 MHz, CDCl₃) δ 8.19 (1H, s), 7.77-7.71 (2H, m), 7.45-7.38 (3H, m), 7.30-7.22 (3H, m), 7.19-7.15 (2H, m), 6.83 (1H, s), 6.00 (1H, d, *J =* 5.1 Hz), 4.85 (2H, bs), 4.83 (1H, dd, *J =* 4.9, 4.3 Hz), 4.24-4.17 (2H, m), 4.08 (2H, s), 3.81 (1H, d, *J=* 13.1 Hz), 3.64 (1H, d, *J=* 13.1 Hz), 2.99 (1H, dd, *J=* 14.0, 5.2 Hz), 2.89 (1H, dd, *J=* 13.9, 5.8 Hz), 2.31 (3H, s), 0.93, 0.82 (18H, 2×s), 0.11, 0.11, -0.06, -0.20 (12H, 4×s). **¹³C NMR** (101 MHz, DMSO-*d₆*) δ 166.1, 160.8, 157.2, 151.8, 151.7, 139.6, 130.0, 129.1, 129.0, 128.5, 127.9, 127.3, 127.1, 122.5, 113.7, 110.5, 104.0, 89.8, 84.0, 74.7, 74.5, 73.5, 34.9, 32.9, 26.0, 25.92, 25.88, 18.2, 18.1, 10.3, -4.2, -4.4, -4.5, -4.8. **LRMS** Calculated for C₄₁H₅₈N₅O₄SSi₂, [M+H]⁺ m/z: 772.4, [M+H]⁺, found 772.5.

### Example 238

### 7-((2R,3R,4R,5S)-3,4-bis((tert-Butyldimethylsilyl)oxy)-5-((((3-methyl-5-phenylisoxazol-4-yl)methyl)thio)methyl)tetrahydrofuran-2-yl)-5-(naphthalen-2-ylmethyl)-7H-pyrrolo[2,3-d]pyrimidin-4-amine (186a):

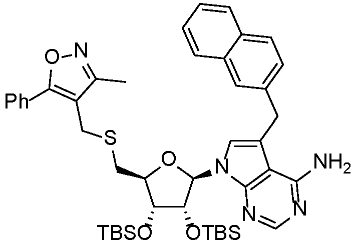

**186a** was synthesized from (0.2 g, 0.25 mmol) and 0.5M solution of (naphthalen-2-ylmethyl)zinc bromide in THF (1.5 mL, 0.75 mmol) in analogous fashion to **129a.** FCC (5-70 % acetone in cyclohexane) provided **186a** (0.18 g, 0.22 mmol, 88 %). **¹H NMR** (400 MHz, DMSO-*d₆*) δ 8.04 (1H, s), 7.85-7.81 (1H, m), 7.77 (1H, d, *J=* 8.6 Hz), 7.72-7.66 (4H, m), 7.48-7.42 (5H, m), 7.37 (1H, dd, J = 8.5, 1.7 Hz), 7.08 (1H, s), 6.40 (2H, bs), 6.09 (1H, d, *J=* 6.9 Hz), 4.68 (1H, dd, *J=* 6.9, 1.8 Hz), 4.32 (1H, d, *J=* 16.4 Hz), 4.27 (1H, d, *J=* 16.4 Hz), 4.09 (1H, dd, *J=* 4.5, 1.8 Hz), 3.94 (1H, td, *J=* 6.6, 1.8 Hz), 3.74 (1H, d, *J=* 13.8 Hz), 3.59 (1H, d, *J* = 13.8 Hz), 2.91 (1H, dd, *J* = 13.9, 6.9 Hz), 2.75 (1H, dd, *J* = 13.9, 6.3 Hz), 2.35 (3H, s), 0.88, 0.68 (18H, 2×s), 0.06, 0.06, -0.13, -0.36 (12H, 4×s). **¹³C NMR** (101 MHz, DMSO-*d₆*) δ 165.0, 160.8, 157.7, 151.9, 151.8, 138.6, 133.3, 131.9, 129.8, 129.1, 129.0, 128.0, 127.9, 127.7, 127.5, 127.4, 126.4, 126.2, 125.6, 120.8, 114.5, 110.2, 102.5, 86.4, 84.2, 74.6, 74.0, 34.2, 31.9, 25.9, 25.7, 24.1, 17.9, 17.7, 11.0, -4.5, -4.5, -5.2. **LRMS** Calculated for C₄₅H₆₀N₅O₄SSi₂, [M+H]⁺ m/z: 822.4, [M+H]⁺, found 822.4.

### Example 239

### (2R,3R,4S,5S)-2-(4-Amino-5-benzyl-7H-pyrrolo[2,3-d]pyrimidin-7-yl)-5-((((3-methyl-5-phenylisoxazol-4-yl)methyl)thio)methyl)tetrahydrofuran-3,4-diol (185b):

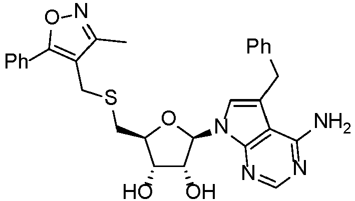

Analogue **185b** was prepared from **185a** (0.15 g, 0.19 mmol) using the same procedure as for **47c.** Reverse-phase FCC (5-70 % MeCN in H₂O) gave **185b** (0.07 g, 0.14 mmol, 73 %). **¹H NMR** (400 MHz, DMSO-*d₆*) δ 8.07 (1H, s), 7.77-7.69 (2H, m), 7.53-7.44 (3H, m), 7.21-7.15 (4H, m), 7.15-7.09 (1H, m), 7.08 (1H, s), 6.51 (2H, bs), 6.08 (1H, d, *J=* 5.9 Hz), 5.39 (1H, br d, *J* = 5.9 Hz), 5.26 (1H, bs), 4.40 (1H, q, *J* = 4.5 Hz), 4.13 (1H, d, *J=* 17.2 Hz), 4.09 (1H, d, *J=* 17.1 Hz), 4.04-3.97 (2H, m), 3.83 (1H, d, *J=* 13.0 Hz), 3.74 (1H, d, *J* = 13.0 Hz), 2.91 (1H, dd, *J* = 13.8, 4.6 Hz), 2.82 (1H, dd, *J* = 13.9, 6.6 Hz), 2.23 (3H, s). **¹³C NMR** (101 MHz, DMSO-*d₆*) δ 164.9, 160.8, 157.4, 151.5, 151.4, 140.8, 130.3, 129.4, 128.6, 128.5, 127.3, 126.8, 126.2, 120.4, 114.7, 111.2, 102.2, 86.7, 83.4, 73.3, 72.8, 34.8, 31.7, 24.7, 9.8. **HRMS** (ESI) Calculated for C₂₉H₃₀N₅O₄S, [M+H]⁺ m/z: 544.2013, [M+H]⁺, found 544.2009.

### Example 240

### (2R,3R,4S,5S)-2-(4-Amino-5-(naphthalen-2-ylmethyl)-7H-pyrrolo[2,3-d]pyrimidin-7-yl)-5-((((3-methyl-5-phenylisoxazol-4-yl)methyl)thio)methyl)tetrahydrofuran-3,4-diol (186b):

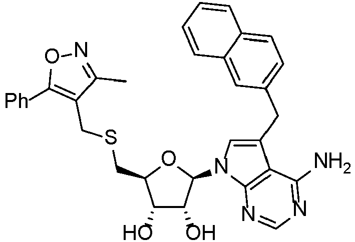

Derivative **186b** was synthesized from protected analogue **186a** (0.17 g, 0.20 mmol) following the same methodology as for **47c.** Reverse-phase FCC (10-70 % MeCN in H₂O) afforded **186b** (0.12 g, 0.20 mmol, 97 %). **¹H NMR** (400 MHz, DMSO-*d₆*) δ 8.05 (1H, s), 7.82 (1H, dd, *J=* 5.8, 3.2 Hz), 7.79-7.64 (5H, m), 7.51-7.40 (5H, m), 7.36 (1H, d, *J* = 8.4 Hz), 7.06 (1H, s), 6.43 (2H, bs), 6.08 (1H, d, *J* = 5.4 Hz), 5.37 (1H, d, *J* = 5.9 Hz), 5.21 (1H, d, J = 4.3 Hz), 4.35 (1H, q, *J* = 5.3 Hz), 4.29 (2H, s), 4.02-3.92 (2H, m), 3.68 (1H, d, *J* = 13.5 Hz), 3.59 (1H, d, *J* = 13.6 Hz), 2.82 (1H, dd, *J=* 13.9, 4.1 Hz), 2.69 (1H, dd, *J=* 14.0, 5.9 Hz), 2.33 (3H, s). **¹³C NMR** (101 MHz, DMSO-*d₆*) δ 165.0, 160.8, 157.8, 152.1, 151.7, 138.7, 133.4, 132.0, 130.0, 129.23, 129.17, 128.2, 127.8, 127.6, 126.6, 126.4, 125.8, 120.6, 114.7, 110.4, 102.5, 87.0, 83.2, 73.4, 72.8, 34.4, 32.1, 24.2, 11.0. **HRMS** (ESI) Calculated for C₃₃H₃₂N₅O₄S, [M+H]⁺ m/z: 594.2170, [M+H]⁺, found 594.2167.

### Example 241

### (2R,3R,4S,5S)-2-(4-Amino-5-(4-(pyridin-3-yl)phenyl)-7H-pyrrolo[2,3-d]pyrimidin-7-yl)-5-(((3-methyl-5-phenylisoxazol-4-yl)methyl)thio)methyl)tetrahydrofuran-3,4-diol (187):

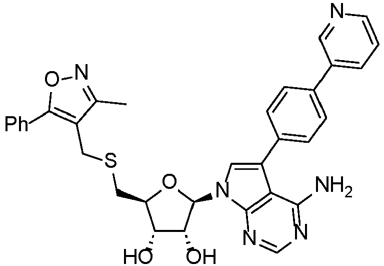

**187** was prepared from **123** (0.07 g, 0.12 mmol) and (4-(pyridin-3-yl)phenyl)boronic acid pinacol ester (0.07 g, 0.25 mmol) in analogous fashion to **149.** FCC (5-80 % EtOAc/acetone/EtOH/H₂O (84/12/2.4/1.6) mixture in EtOAc/acetone/EtOH/H₂O (68/12/12/8) mixture) furnished **187** (0.02 g, 0.04 mmol, 31 %). **¹H NMR** (400 MHz, DMSO-*d₆*) δ 8.96 (1H, dd, *J=* 1.8, 0.6 Hz), 8.59 (1H, dd,

*J=* 4.7, 1.5 Hz), 8.19 (1H, s), 8.14 (1H, ddd, J = 7.9, 2.3, 1.7 Hz), 7.85 (1H, *d, J=* 8.3 Hz), 7.78-7.73 (2H, m), 7.60 (1H, s), 7.58 (1H, d, *J=* 8.3 Hz), 7.55-7.44 (4H, m), 6.24 (2H, bs), 6.20 (1H, d, *J* = 5.9 Hz), 5.52 (1H, bs), 5.37 (1H, bs), 4.55 (1H, t, *J=* 5.1 Hz), 4.12-4.08 (1H, m), 4.08-4.03 (1H, m). 3.87 (1H, d, *J=* 13.0 Hz), 3.83 (1H, d, *J=* 13.0 Hz), 2.99 (1H, dd, *J=* 13.8, 5.7 Hz), 2.91 (1H, dd, *J=* 13.9, 7.0 Hz), 2.25 (3H, s). **¹³C NMR** (101 MHz, DMSO-*d₆*) δ 164.9, 160.8, 157.6, 152.2, 151.6, 148.7, 147.8, 135.5, 135.3, 134.4, 134.1, 130.3, 129.4, 129.2, 127.6, 127.3, 126.8, 124.1, 121.3, 116.5, 111.1, 100.5, 87.0, 83.4, 73.3, 72.9, 34.9, 24.5, **9.7.HRMS** (ESI) Calculated for C₃₃H₃₁N₆O₄S, [M+H]⁺ m/z: 607.2122, [M+H]⁺, found 607.2117.

### Example 242

### (2R,3R,4S,5S)-2-(4-Amino-5-(4-(pyridin-2-yl)phenyl)-7H-pyrrolo[2,3-d]pyrimidin-7-yl)-5-(((3-methyl-5-phenylisoxazol-4-yl)methyl)thio)methyl)tetrahydrofuran-3,4-diol (188):

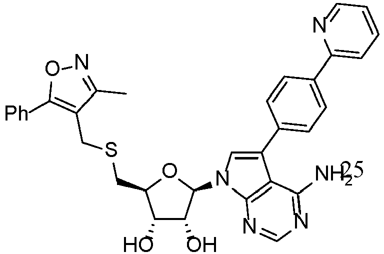

Modified nucleoside **188** was synthesized from **123** (0.07 g, 0.12 mmol) and (4-(pyridin-2-yl)phenyl)boronic acid pinacol ester (0.07 g, 0.25 mmol) using the same conditions as for **149.** FCC (5-70 % EtOAc/acetone/EtOH/H₂O (84/12/2.4/1.6) mixture in EtOAc/acetone/EtOH/H₂O (68/12/12/8) mixture) gave 188 (0.04 g, 0.06 mmol, 49 %). **¹H NMR** (400 MHz, DMSO-*d₆*) δ 8.69 (1H, ddd, *J=* 4.8, 1.8, 0.9 Hz), 8.22-8.18 (3H, m), 8.03 (1H, dt, *J* = 8.0, 0.9 Hz), 7.90 (1H, td, *J* = 7.7, 1.9 Hz), 7.77-7.73 (2H, m), 7.62 (1H, s), 7.57 (1H, dt, *J=* 8.4, 1.9 Hz), 7.52-7.43 (3H, m), 7.36 (1H, ddd, *J=* 7.5, 4.8, 1.0 Hz), 6.23 (2H, bs), 6.21 (1H, d, *J=* 6.0 Hz), 5.46 (1H, d, *J=* 6.3 Hz), 5.31 (1H, d, *J=* 5.1 Hz), 4.58 (1H, q, *J =* 6.0 Hz), 4.11-4.08 (1H, m), 4.07-4.03 (1H, m). 3.88 (1H, d, *J=* 13.0 Hz), 3.83 (1H, d, *J=* 13.0 Hz), 2.99 (1H, dd, *J=* 13.9, 5.7 Hz), 2.91 (1H, dd, *J=* 13.8, 7.0 Hz), 2.26 (3H, s). **¹³C NMR** (101 MHz, DMSO-*d₆*) δ 164.9, 160.8, 157.6, 155.8, 152.2, 151.7, 149.8, 137.5, 137.2, 135.3, 130.3, 129.4, 128.9, 127.3, 127.2, 126.8, 122.8, 121.3, 120.3, 116.6, 111.1, 100.5, 86.9, 83.4, 73.2, 72.9, 34.9, 24.5, 9.8. **HRMS** (ESI) Calculated for C₃₃H₃₁N₆O₄S, [M+H]⁺ m/z: 607.2122, [M+H]⁺, found 607.2117.

### Example 243

### 7-((2R,3R,4R,5S)-3,4-bis((tert-Butyldimethylsilyl)oxy)-5-(((3-methyl-5-phenylisoxazol-4-yl)methyl) thio)methyl)tetrahydrofuran-2-yl)-5-(phenylthio)-7H-pyrrolo[2,3-d]pyrimidin-4-amine (189a):

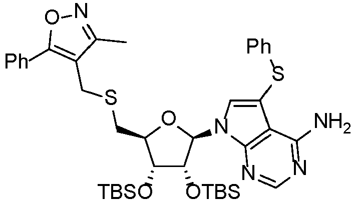

To a solution of **122** (0.2 g, 0.25 mmol), Pd₂dba₃ (0.01 g, 0.01 mmol), Xantphos (0.02 g, 0.03 mmol) and thiophenol (0.06 mL, 0.59 mmol) in 1,4-dioxane (3 mL), DIPEA (0.11 mL, 0.63 mmol) was added and the reaction mixture was stirred under argon atmosphere at 80°C for 10 h. The reaction mixture was concentrated and the crude product purified by FCC (5-50 % acetone in cyclohexane) to obtain **189a** (0.15 g, 0.18 mmol, 74%). **¹H NMR** (400 MHz, DMSO-*d₆*) δ 8.14 (1H, s), 7.91 (1H, s), 7.76-7.70 (2H, m), 7.52-7.44 (3H, m), 7.24-7.17 (2H, m), 7.16-7.11 (1H, m), 7.11-7.06 (2H, m), 6.16 (1H, d, *J=* 7.2 Hz), 4.90 (1H, dd, *J=* 7.2, 4.5 Hz), 4.18 (1H, dd, *J=* 4.5, 1.0 Hz), 4.01 (1H, td, *J=* 7.2, 0.9 Hz), 3.91 (1H, d, *J* = 13.1 Hz), 3.81 (1H, d, *J* = 13.1 Hz), 3.10 (1H, dd, J = 13.8, 7.0 Hz), 3.02 (1H, dd, *J=* 13.8, 7.2 Hz), 2.26 (3H, s), 0.91, 0.69 (18H, 2×s), 0.11, 0.11, -0.08, -0.35 (12H, 4×s). **¹³C NMR** (101 MHz, DMSO-*d₆*) δ 165.0, 160.7, 157.6, 153.0, 151.8, 138.1, 130.3, 129.31, 129.28, 127.3, 126.8, 126.1, 126.0, 111.0, 103.4, 100.3, 86.9, 85.2, 74.8, 74.0, 34.6, 25.9, 25.7, 24.7, 17.9, 17.7, 9.8, -4.5, -5.2. **LRMS** Calculated for C₄₀H₅₆N₅O₄S₂Si₂, [M+H]⁺ m/z: 790.3, [M+H]⁺, found 790.3.

### Example 244

### 5-(Benzylthio)-7-((2R,3R,4R,5S)-3,4-bis((tert-Butyldimethylsilyl)oxy)-5-(((3-methyl-5-phenyl isoxazol-4-yl)methyl)thio)methyl)tetrahydrofuran-2-yl)-7H-pyrrolo[2,3-d]pyrimidin-4-amine (190a):

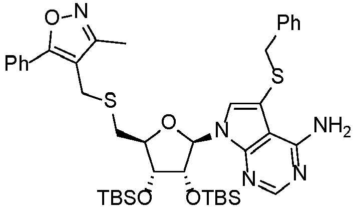

SAH analogue **190a** was prepared from iodinated derivative **122** (0.2 g, 0.25 mmol) and phenylmethanethiol (0.11 mL, 0.51 mmol) following the same procedure as for **189a.** FCC (5-50 % acetone in cyclohexane) afforded derivative **190a** (0.17 g, 0.21 mmol, 84 %). **¹H NMR** (400 MHz, DMSO-*d₆*) δ 8.09 (1H, s), 7.77-7.71 (2H, m), 7.54-7.47 (3H, m), 7.45 (1H, s), 7.23-7.14 (3H, m), 7.13-7.08 (2H, m), 6.73 (2H, bs), 6.07 (1H, d, *J* = 6.8 Hz), 4.73 (1H, dd, *J* = 6.7, 4.5 Hz), 4.14 (1H, dd, *J* = 4.4, 1.8 Hz), 3.96 (1H, td, *J=* 6.9, 1.8 Hz), 3.91 (1H, d, *J=* 12.6 Hz), 3.90 (1H, d, *J=* 13.2 Hz), 3.86 (1H, d, *J=* 12.6 Hz), 3.81 (1H, d, *J=* 13.2 Hz), 3.03 (1H, dd, *J=* 13.8, 7.1 Hz), 2.89 (1H, dd, *J* = 13.8, 6.8 Hz), 2.28 (3H, s), 0.90, 0.68 (18H, 2×s), 0.10, 0.09, -0.11, -0.38 (12H, 4×s). **¹³C NMR** (101 MHz, DMSO-*d₆*) δ 165.0, 160.7, 157.8, 152.6, 151.1, 137.7, 130.3, 129.3, 128.9, 128.5, 128.4, 127.3, 127.2, 126.9, 111.0, 103.7, 103.3, 86.7, 84.6, 74.7, 74.1, 42.7, 34.4, 25.9, 25.7, 24.6, 17.9, 17.7, 9.8, -4.4, -4.5, -4.6, -5.3. **LRMS** Calculated for C₄₁H₅₈N₅O₄S₂Si₂, [M+H]⁺ m/z: 804.4, [M+H]⁺, found 804.4.

### Example 245

### (2R,3R,4S,5S)-2-(4-Amino-5-(phenylthio)-7H-pyrrolo[2,3-d]pyrimidin-7-yl)-5-((((3-methyl-5-phenylisoxazol-4-yl)methyl)thio)methyl)tetrahydrofuran-3,4-diol (189b):

Modified nucleoside **189b** was synthesized from protected analogue **189a** (0.13 g, 0.17 mmol) according 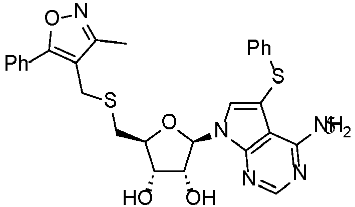 to the same methodology as for **47c.** Reverse-phase FCC (10-70 % MeCN in H₂O) provided title compound **189b** (0.08 g, 0.15 mmol, 89 %). **¹H NMR** (400 MHz, DMSO-*d₆*) δ 8.16 (1H, s), 7.88 (1H, s), 7.77-7.72 (2H, m), 7.53-7.42 (3H, m), 7.22-7.15 (2H, m), 7.14-7.10 (1H, m), 7.10-7.05 (2H, m), 6.64 (2H, bs), 6.15 (1H, d, *J* = 6.2 Hz), 5.48 (1H, d, *J* = 6.2 Hz), 5.31 (1H, d, *J* = 4.7 Hz), 4.59 (1H, q, *J* = 6.1 Hz), 4.10-4.02 (2H, m), 3.86 (1H, d, *J* = 12.9 Hz), 3.79 (1H, d, *J=* 12.9 Hz), 3.01-2.89 (2H, m), 2.24 (3H, s). **¹³C NMR** (101 MHz, DMSO-*d₆*) δ 164.9, 160.7, 157.6, 153.0, 151.7, 138.1, 130.3, 130.0, 129.4, 127.3, 126.8, 126.0, 125.9, 111.1, 103.2, 100.1, 87.1, 83.9, 73.2, 72.9, 34.8, 24.5, 9.7. **HRMS** (ESI) Calculated for C₂₈H₂₈N₅O₄S₂, [M+H]⁺ m/z: 562.1577, [M+H]⁺, found 562.1572.

### Example 246

### (2R,3R,4S,5S)-2-(4-Amino-5-(benzylthio)-7H-pyrrolo[2,3-d]pyrimidin-7-yl)-5-((((3-methyl-5-phenylisoxazol-4-yl)methyl)thio)methyl)tetrahydrofuran-3,4-diol (190b):

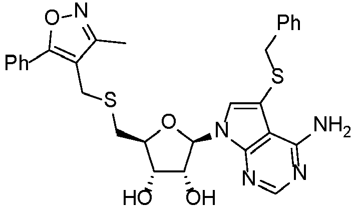

Title compound **190b** was prepared from derivative **190a** (0.16 g, 0.19 mmol) using the same conditions as for **47c.** Reverse-phase FCC (10-70 % MeCN in H₂O) furnished **190b** (0.1 g, 0.17 mmol, 87 %). **¹H NMR** (400 MHz, DMSO-*d₆*) δ 8.11 (1H, s), 7.78-7.73 (2H, m), 7.55-7.47 (3H, m), 7.29 (1H, s), 7.22-7.12 (3H, m), 7.10-7.04 (2H, m), 6.77 (2H, bs), 6.05 (1H, d, *J =* 5.5 Hz), 5.42 (1H, d, *J =* 6.0 Hz), 5.28 (1H, d, *J=* 5.0 Hz), 4.36 (1H, q, *J=* 5.5 Hz), 4.05-3.95 (2H, m), 3.88 (2H, s), 3.85 (1H, d, J = 13.1 Hz), 3.79 (1H, d, *J* = 13.0 Hz), 2.92 (1H, dd, *J =* 13.9, 5.3 Hz), 2.83 (1H, dd, *J* = 13.9, 6.8 Hz), 2.27 (3H, s). **¹³C NMR** (101 MHz, DMSO-*d₆*) δ 164.9, 160.8, 157.8, 152.6, 150.8, 137.7, 130.3, 129.4, 129.0, 128.4, 128.0, 127.3, 127.2, 126.8, 111.1, 103.24, 103.21, 87.1, 83.4, 73.5, 72.8, 42.4, 34.7, 24.5, 9.8. **HRMS** (ESI) Calculated for C₂₉H₃₀N₅O₄S₂, [M+H]⁺ m/z: 576.1734, [M+H]⁺, found 576.1732.

### Example 247

### (2R,3R,4S,5S)-2-(5-((Adamantan-1-yl)thio)-4-amino-7H-pyrrolo[2,3-d]pyrimidin-7-yl)-5-((((3-methyl-5-phenylisoxazol-4-yl)methyl)thio)methyl)tetrahydrofuran-3,4-diol (191):

Analogue **191** was prepared from **123** (0.08 g, 0.14 mmol) and 1-adamantanethiol (0.05 g, 0.30 mmol) according to the same methodology as for **189a.** FCC (0-20 % EtOAc/acetone/EtOH/H₂O (84/12/2.4/1.6) mixture in EtOAc/acetone/EtOH/H₂O (68/12/12/8) mixture) gave **191** (0.05 g, 0.07 mmol, 52 %). **¹H NMR** (400 MHz, DMSO-*d₆*) δ 8.13 (1H, s), 7.78-7.69 (2H, m), 7.53 (1H, s), 7.51-7.42 (3H, m), 6.14 (1H, d, *J* = 6.0 Hz), 5.43 (1H, d, *J* = 6.1 Hz), 5.29 (1H, d, *J=* 4.3 Hz), 4.56 (1H, q, *J* = 5.4 Hz), 4.15-4.01 (2H, m), 3.89 (1H, d, *J=* 12.8 Hz), 3.79 (1H, d, *J=* 12.8 Hz), 3.04-2.88 (2H, m), 2.23 (3H, s), 1.82 (3H, bs), 1.67 (6H, t, *J=* 14.4 Hz), 1.50 (3H, d, *J=* 11.6 Hz), 1.40 (3H, d, *J=* 11.6 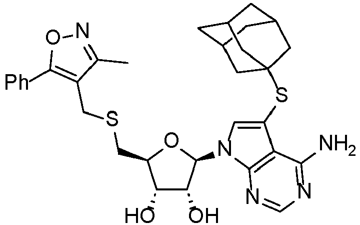 Hz), 1.40 (3H, d, *J=* 11.1 Hz). **¹³C NMR** (101 MHz, DMSO-*d₆*) δ 164.9, 160.7, 158.0, 152.4, 150.8, 130.2, 130.0, 129.3, 127.3, 126.7, 111.0, 104.5, 99.0, 86.9, 84.0, 73.3, 72.8, 47.9, 42.7, 35.6, 34.8, 24.7, 9.7. **HRMS** (ESI) Calculated for C₃₂H₃₈N₅O₄S₂, [M+H]⁺ m/z: 620.2360, [M+H]⁺, found 620.2357.

Next, we decided to evaluate the effect of substituents in other positions of the nucleobase. We chose fluorine and chlorine atoms and a methyl group, as suitable substitutions in the position 2. Starting material for the synthesis of 2-fluoro derivative, nucleoside **192,** was prepared according to a published procedure (Hulpia, F., et al., Eur J Med Chem 2020/188, 112018). The C-2 aminogroup was transformed to a diazonium salt and the fluorine atom was thus introduced using the Baltz-Schiemann reaction with an Olah reagent as a fluorine source. Since the quantitative removal of all protecting groups occured, the isopropyliden protection was installed to recieve aprropriate intermediate **194** that was subjected to the Mitsunobu reaction to introduce an S-acetyl group to the C-5' position (see **Scheme 1).** Installation of the appropriate 5'-substituent was performed under the same conditions as mentioned above **(Scheme 3).** Final deprotection under acidic conditions furnished desired SAH analogue **198.** The synthesis of 2-chloro derivative started from nucleoside **199** that was obtained according to the published procedure (Beigelman, L., et al. WO2021119325, 2021-06-17). Sandmayer reaction with SbCl₃ provided intermediate **200.** Subsequent reduction of the azide afforded derivative **201,** that was subjected to the same reaction pathway, as for **198,** to obtain desired SAH analogue **204.** A C-2 methyl analogue **208** was prepared from protected nucleoside **205** (Wu, T., et al. US2023071711, 2023-03-09). The C-5' substituent was introduced following a similar procedure as above. **(Scheme 3)** Final deprotection under acidic conditions furnished SAH analogue **208 (Scheme 12).**

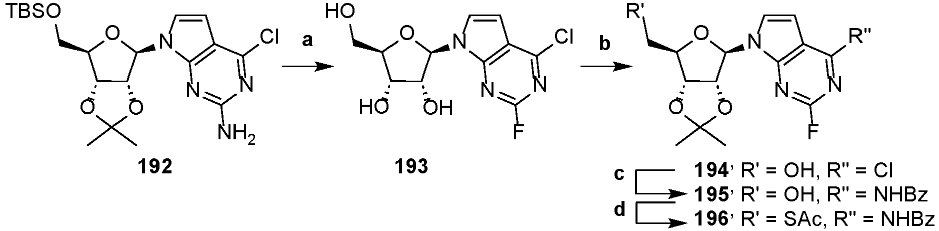

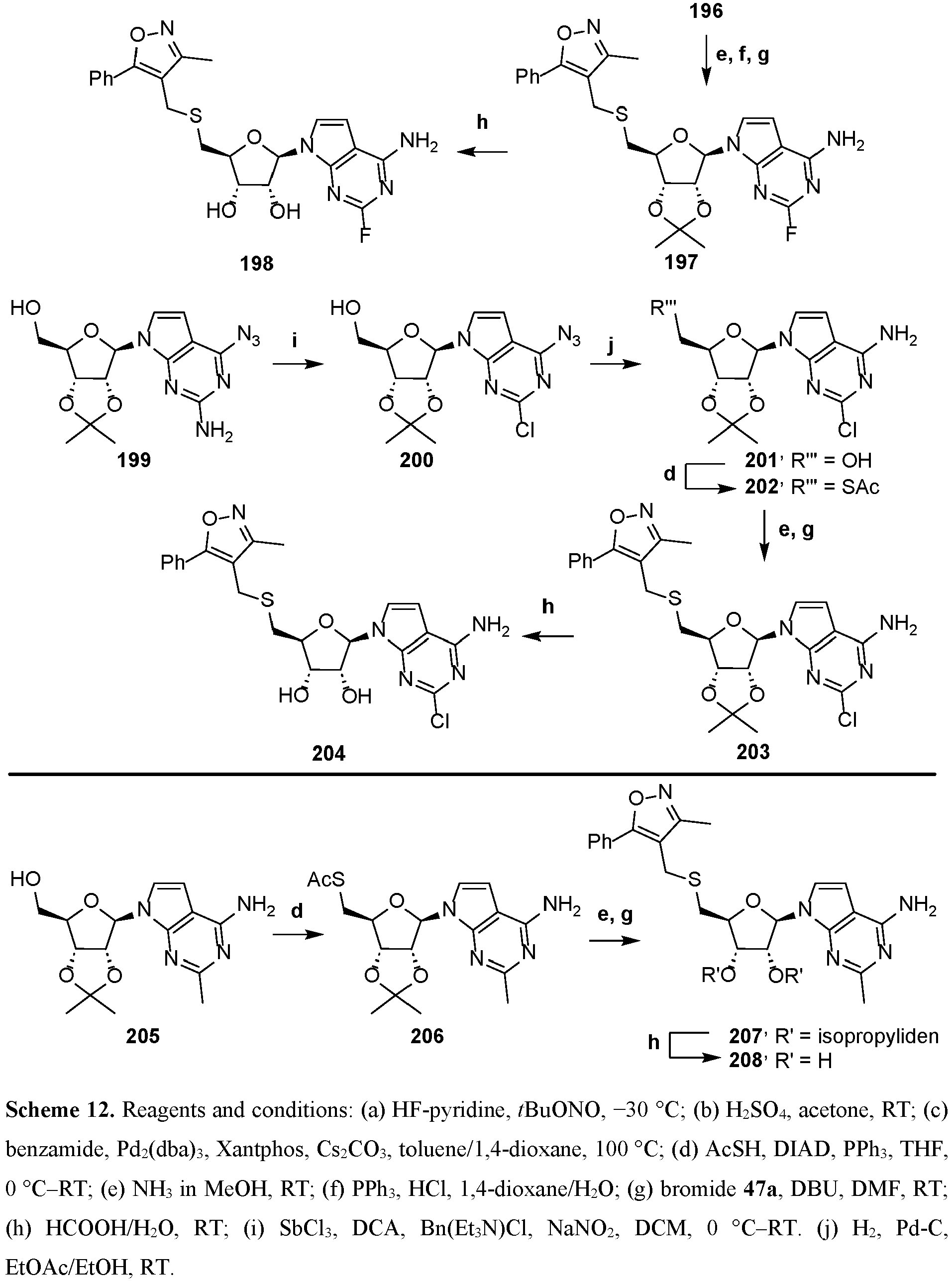

### Example 248

### (2R,3R,4S,5R)-2-(4-Chloro-2-fluoro-7H-pyrrolo[2,3-d]pyrimidin-7-yl)-5-(hydroxymethyl) tetrahydrofuran-3,4-diol (193):

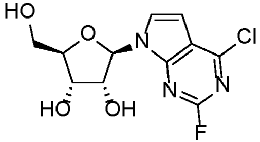

Protected nucleoside **192** (2.4 g, 5.27 mmol) was dissolved in pre-cooled HF-py (9.5 mL) at -30 °C. tBuONO (1.3 mL, 10.55 mmol) was added dropwise and the reaction mixture was stirred at -30--20 °C After stirring for 30 min, the reaction mixture was diluted with EtOAc (300 mL) and washed with saturated solution of NaHCO₃. Aqueous layer was once again extracted with EtOAc (300 mL), combined organic layers were dried over Na₂SO₄ and evaporated. FCC (5-15 % EtOH in DCM) afforded desired 2-fluoroderivative **193** (1.05 g, 3.46 mmol, 66%). NMR spectra were consistent with published data (Malnuit, V., et al., ChemMedChem 2015/10, 1079-1093).

### Example 249

### ((3aR,4R,6R,6aR)-6-(4-Chloro-2-fluoro-7H-pyrrolo[2,3-d]pyrimidin-7-yl)-2,2-dimethyltetrahydrofuro[3,4-d][1,3]dioxol-4-yl)methanol (194):

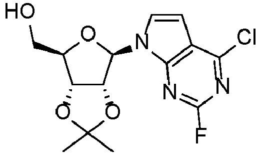

To the suspension of **193** (0.1 g, 0.33 mmol) in acetone (7 mL), H₂SO₄ (0.06 mL, 0.99 mmol) was carefully added and the reaction mixture was stirred at RT for 2 h. Then it was poured in a saturated solution of NaHCO₃ (20 mL) and extracted with EtOAc (2×50 mL). Pooled organic phases were dried over Na₂SO₄, filtered and evaporated affording **194** (0.11 g, 0.32 mmol, 97 %). **¹H NMR** (400 MHz, CDCl₃) δ 7.31 (1H, d, *J=* 3.8 Hz), 6.64 (1H, d, *J=* 3.8 Hz), 5.91 (1H, d, *J=* 4.5 Hz), 5.17 (1H, dd, *J=* 6.2, 4.5 Hz), 5.09 (1H, dd, *J=* 6.3, 2.3 Hz), 4.43 (1H, q, *J=* 2.2 Hz), 3.96 (1H, dd, *J=* 12.5, 2.2 Hz), 3.82 (1H, dd, *J* = 12.5, 2.6 Hz), 1.63 (3H, s), 1.37 (3H, s). **¹³C NMR** (101 MHz, CDCl₃) δ 157.2 (d, *J* = 219.1 Hz), 154.6 (d, *J=* 16.9 Hz), 151.6 (d, *J=* 15.4 Hz), 129.6 (d, *J* = 3.9 Hz), 118.0 (d, *J* = 4.4 Hz), 114.7, 101.0, 94.4, 85.7, 83.3, 81.1, 63.2, 27.6, 25.4. **¹⁹F NMR** (377 MHz, CDCl₃) δ -51.42. **LRMS** Calculated for C₁₄H₁₆ClFN₃O₄, [M+H]⁺ m/z: 344.1, [M+H]⁺, found 344.1

### Example 250

### N-(2-Fluoro-7-((3aR,4R,6R,6aR)-6-(hydroxymethyl)-2,2-dimethyltetrahydrofuro[3,4-d][1,3]dioxol-4-yl)-7H-pyrrolo[2,3-d]pyrimidin-4-yl)benzamide (195):

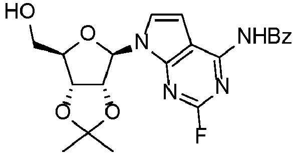

A mixture of Pd₂(dba)₃ (0.15 g, 0.15 mmol) and XantPhos (0.17 g, 0.29 mmol) in dry toluene (30 mL) under atmosphere of argon was heated to 45 °C for 15 min. To the formed solution of catalyst was added Cs₂CO₃ (1.04 g, 3.20 mmol) and a solution of benzamide (0.42 g, 3.49 mmol) and protected nucleoside **194** (1 g, 2.91 mmol) in dry 1,4-dioxane (30 mL). Reaction mixture was heated in a sealed vessel at 100 °C for 4 h, diluted with EtOAc (400 mL) and washed with H₂O and brine. Organic layer was dried over Na₂SO₄ and evaporated. FCC (20-80 % EtOAc in cyclohexane) afforded intermediate **195** (0.48 g, 1.11 mmol, 38 %). **¹H NMR** (400 MHz, CDCl₃) δ 8.73 (1H, s), 8.00-7.94 (2H, m), 7.67-7.60 (1H, m), 7.57-7.51 (2H, m), 7.16 (1H, d, *J*= 3.8 Hz), 7.14 (1H, d, *J* = 3.8 Hz), 5.89 (1H, d, *J* = 4.6 Hz), 5.23 (1H, dd, *J* = 6.2, 4.6 Hz), 5.11 (1H, dd, *J* = 6.3, 2.3 Hz), 4.43 (1H, q, *J=* 2.2 Hz), 3.98 (1H, dd, *J* = 12.5, 2.0 Hz), 3.87-3.78 (1H, m), 1.64 (3H, s), 1.37 (3H, s). **¹³C NMR** (101 MHz, CDCl₃) δ 164.9, 157.7 (d, *J* = 212.9 Hz), 153.8 (d, *J* = 15.0 Hz), 152.6 (d, *J* = 16.7 Hz), 133.4, 133.0, 129.2, 127.9, 126.8 (d, *J* = 3.6 Hz), 114.5, 108.0 (d, *J* = 4.0 Hz), 105.4, 94.6, 85.6, 83.1, 81.2, 63.4, 27.7, 25.5.**¹⁹F NMR** (377 MHz, CDCl₃) δ-54.20. **LRMS** Calculated for C₂₁H₂₂FN₄O₅, [M+H]⁺ m/z: 429.2, [M+H]⁺, found 429.1

### Example 251

### S-(((3aS,4S,6R,6aR)-6-(4-Benzamido-2-fluoro-7H-pyrrolo[2,3-d]pyrimidin-7-yl)-2,2-dimethyltetrahydrofuro[3,4-d][1,3]dioxol-4-yl)methyl) thioacetate (196):

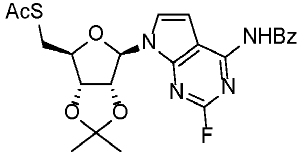

Thioacetate **196** was prepared from **195** (0.46 g, 1.07 mmol) using the same methodology as for **5.** FCC (20-80 % EtOAc in cyclohexane) afforded derivative **196** (0.46 g, 0.94 mmol, 88 %). **¹H NMR** (400 MHz, CDCl₃) δ 8.73 (1H, bs), 8.00-7.95 (2H, m), 7.66-7.61 (1H, m), 7.57-7.51 (2H, m), 7.21 (1H, d, *J=* 3.8 Hz), 7.19 (1H, d, *J=* 3.8 Hz), 6.19 (1H, d, *J=* 2.7 Hz), 5.22 (1H, dd, *J* = 6.6, 2.8 Hz), 4.85 (1H, dd, *J=* 6.5, 3.8 Hz), 4.30 (1H, td, *J* = 6.3, 3.8 Hz), 3.30 (1H, dd, *J* = 13.9, 6.7 Hz), 3.23 (1H, dd, *J* = 13.9, 6.0 Hz), 2.37 (3H, s), 1.61 (3H, s), 1.37 (3H, s). **¹³C NMR** (101 MHz, CDCl₃) δ 194.9, 165.0, 158.1 (d, *J* = 211.3 Hz), 154.8 (d, *J=* 16.1 Hz), 152.2 (d, *J* = 16.6 Hz), 133.3, 133.1, 129.2, 127.9, 125.1 (d, *J* = 3.6 Hz), 115.0, 107.1 (d, *J* = 4.1 Hz), 106.1,90.5, 84.7, 84.6, 83.2, 31.4, 30.7, 27.3, 25.6. **¹⁹F NMR** (377 MHz, CDCl₃) δ -53.91. **LRMS** Calculated for C₂₃H₂₄FN₄O₅S, [M+H]⁺ m/z: 487.2, [M+H]⁺, found 487.1.

### Example 252

### 7-((3aR,4R,6S,6aS)-2,2-Dimethyl-6-((((3-methyl-5-phenylisoxazol-4-yl)methyl)thio)methyl) tetrahydrofuro[3,4-d][1,3]dioxol-4-yl)-2-fluoro-7H-pyrrolo[2,3-d]pyrimidin-4-amine (197):

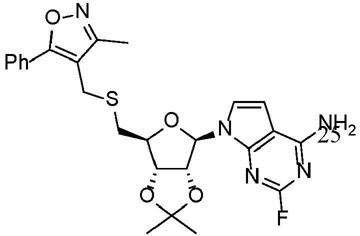

Thioacetate **196** (0.15 g, 0.31 mmol) was treated with 10M methanolic ammonia (5 mL) at RT. After 18 h of stirring, the reaction mixture was concentrated and co-evaporated with 1,4-dioxane. To a solution of the obtained disulfide in 1,4-dioxane/H₂O mixture (4:1, 2.5 mL) was added PPh₃ (0.08 g, 0.31 mmol) and one drop of HCl and the reaction mixture was stirred at RT for 2 h. Volatiles were evaporated and the crude mixture was azeotroped with DMF and used as is. A solution of the bromide **47a** (obtained from 7 (0.07 g, 0.37 mmol)) in dry DMF (1 mL) was added to a solution of the provided thiol in dry DMF (1 mL) at 0 °C. A solution of DBU (0.09 mL, 0.62 mmol) in dry DMF (1 mL) was added dropwise. After 1 h of stirring, volatiles were evaporated and the residue was purified on FCC (20-90 % EtOAc in cyclohexane) to furnish SAH analogue 197 (0.11 g, 0.21 mmol, 70 %). **¹H NMR** (400 MHz, CDCl₃) δ 7.75-7.71 (2H, m), 7.48-7.42 (3H, m), 6.96 (1H, d, *J* = 3.7 Hz), 6.36 (1H, d, *J* = 3.7 Hz), 6.04 (1H, d, *J* = 2.6 Hz), 5.57 (2H, br, s), 5.27 (1H, dd, *J* = 6.6, 2.6 Hz), 4.98 (1H, dd, J = 6.6, 3.9 Hz), 4.29 (1H, td, J = 6.5, 3.9 Hz), 3.74 (1H, d, *J* = 12.9 Hz), 3.64 (1H, d, *J* = 12.8 Hz), 2.89 (1H, dd, *J* = 13.9, 6.7 Hz), 2.84 (1H, dd, *J* = 13.9, 6.4 Hz), 2.33 (3H, s), 1.60 (3H, s), 1.38 (3H, s). **¹³C NMR** (101 MHz, CDCl₃) δ 166.3, 160.8, 159.3 (d, *J=* 208.2 Hz), 158.7 (d, *J* = 19.0 Hz), 151.4 (d, *J* = 17.8 Hz), 130.1, 129.1, 127.9, 127.3, 123.9 (d, *J* = 3.5 Hz), 114.9, 110.3, 101.9 (d, *J=* 3.8 Hz), 99.6, 90.9, 85.9, 84.4, 83.7, 34.9, 27.3, 25.6, 25.4, 10.2. **¹⁹F NMR** (377 MHz, CDCl₃) δ -52.87. **LRMS** Calculated for C₂₃H₂₄FN₄O₅S, [M+H]⁺ m/z: 511.2, [M+H]⁺, found 511.1.

### Example 253

### (2R,3R,4S,5S)-2-(4-Amino-2-fluoro-7H-pyrrolo[2,3-d]pyrimidin-7-yl)-5-((((3-methyl-5-phenylisoxazol-4-yl)methyl)thio)methyl)tetrahydrofuran-3,4-diol (198):

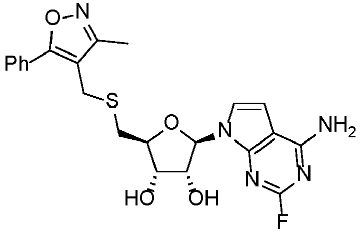

Title compound **198** was prepared from **197** (0.1 g, 0.20 mmol) in analogous fashion to **128.** Reverse-phase FCC (0-100 % MeCN in H₂O) furnished derivative **198** (0.07 g, 0.14 mmol, 71 %). **¹H NMR** (400 MHz, DMSO-*d₆*) δ 7.77-7.73 (2H, m), 7.56-7.47 (3H, m), 7.26 (1H, d, *J* = 3.7 Hz), 6.61 (1H, d, *J=* 3.7 Hz), 5.91 (1H, d, *J=* 5.9 Hz), 5.42 (1H, d, *J=* 6.2 Hz), 5.30 (1H, d, *J* = 4.7 Hz), 4.42 (1H, q, *J=* 5.7 Hz), 4.05-3.99 (2H, m), 3.86 (1H, d, *J=* 13.0 Hz), 3.81 (1H, d, *J=* 13.1 Hz), 2.94 (1H, dd, *J=* 13.9, 5.2 Hz), 2.86 (1H, dd, *J=* 13.9, 6.7 Hz), 2.27 (3H, s). **¹³C NMR** (101 MHz, DMSO-*d₆*) δ 164.7, 160.6, 159.4 (d, *J =* 20.3 Hz), 159.3 (d, *J* = 200.8 Hz), 151.4 (d, *J* = 18.8 Hz), 130.1, 129.3, 127.2, 126.7, 121.7 (d, *J =* 3.2 Hz), 111.0, 100.8, 100.7 (d, *J =* 3.7 Hz), 86.9, 83.2, 73.1, 72.7, 34.7, 24.3, 9.6. **¹⁹F NMR** (377 MHz, DMSO-*d₆*) δ -53.61. **HRMS** (ESI) Calculated for C₂₂H₂₂FN₅O₄SNa, [M+Na]⁺ m/z: 494.1269, [M+Na]⁺, found 494.1268.

### Example 254

### ((3aR,4R,6R,6aR)-6-(4-Azido-2-chloro-7H-pyrrolo[2,3-d]pyrimidin-7-yl)-2,2-dimethyltetrahydrofuro[3,4-d][1,3]dioxol-4-yl)methanol (200):

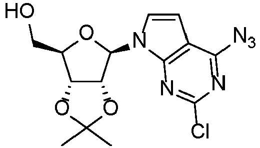

To a protected nucleoside **199** (0.2 g, 0.58 mmol), Bn(Et₃N)Cl (0.13 g, 0.58 mmol) and NaNO₂ (0.8 g, 11.5 mmol) in DCM (20 mL) was added SbCl₃ (0.26 g, 1.15 mmol) in DCM (3 mL) followed by DCA (0.19 mL, 2.30 mmol). The reaction mixture was sitrred at RT for 10 h, diluted with EtOAc (100 mL) and washed with NaHCO₃ and brine. Organic layer was dried over Na₂SO₄ and evaporated. FCC (20-80 % acetone in cyclohexane) furnished derivative **200** (0.19 g, 0.52 mmol, 90 %). **¹H NMR** (400 MHz, CDCl₃) δ 7.14 (1H, d, *J* = 3.7 Hz), 6.47 (1H, d, *J* = 3.7 Hz), 5.80 (1H, d, *J=* 4.9 Hz), 5.20 (1H, dd, *J=* 6.1, 5.0 Hz), 5.12 (1H, dd, *J=* 6.2, 1.8 Hz), 4.46 (1H, q, *J=* 1.8 Hz), 3.99 (1H, dd, *J=* 12.6, 1.9 Hz), 3.82 (1H, dd, *J =* 12.6, 2.2 Hz), 1.63 (3H, s), 1.37 (3H, s). **¹³C NMR** (101 MHz, CDCl₃) δ 156.3, 152.6, 151.7, 128.6, 114.4, 109.3, 99.6, 95.4, 85.6, 83.0, 81.4, 63.5, 27.7, 25.4. **LRMS** Calculated for C₁₄H₁₆ClN₆O₄, [M+H]⁺ m/z: 367.1, [M+H]⁺, found 367.0

### Example 255

### ((3aR,4R,6R,6aR)-6-(4-Amino-2-chloro-7H-pyrrolo[2,3-d]pyrimidin-7-yl)-2,2-dimethyltetrahydrofuro[3,4-d][1,3]dioxol-4-yl)methanol (201):

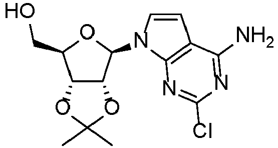

Analogue **201** was synthesized from **200** (0.19 g, 0.52 mmol) in analogous fashion to 6. FCC (50-100 % EtOAc in cyclohexane) gave **201** (0.16 g, 0.48 mmol, 92 %). **¹H NMR** (400 MHz, CDCl₃) δ 6.99 (1H, d, *J=* 3.8 Hz), 6.35 (1H, d, *J=* 3.7 Hz), 5.78 (2H, bs), 5.73 (1H, d, *J=* 5.0 Hz), 5.23 (1H, dd, *J* = 6.0, 5.0 Hz), 5.12 (1H, ddd, *J* = 6.1, 1.7, 0.4 Hz), 4.46 (1H, q, *J* = 1.7 Hz), 3.99 (1H, dd, *J* = 12.6, 1.7 Hz), 3.81 (1H, dd, *J* = 12.6, 2.1 Hz), 1.63 (3H, s), 1.37 (3H, *d, J=* 0.5 Hz). **¹³C NMR** (101 MHz, DMSO-*d₆*) δ 157.8, 153.0, 149.8, 125.9, 114.2, 103.7, 98.6, 95.7, 85.5, 83.0, 81.5, 63.6, 27.8, 25.4. **LRMS** Calculated for C₁₄H₁₈N₄O₄, [M+H]⁺ m/z: 341.1, [M+H]⁺, found 341.1.

### Example 256

### S-(((3aS,4S,6R,6aR)-6-(4-Amino-2-chloro-7H-pyrrolo[2,3-d]pyrimidin-7-yl)-2,2-dimethyltetrahydrofuro[3,4-d][1,3]dioxol-4-yl)methyl)thioacetate (202):

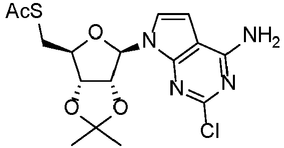

Thioacetate **202** was synthesized from **201** (0.08 g, 0.24 mmol) in analogous fashion to **5.** FCC (3-10 % EtOH in DCM) gave **202** (0.08 g, 0.19 mmol, 80 %). **¹H NMR** (400 MHz, CDCl₃) δ 7.03 (1H, d, *J =* 3.7 Hz), 6.38 (1H, d, *J =* 3.7 Hz), 6.08 (1H, d, *J=* 2.4 Hz), 5.62 (2H, bs), 5.25 (1H, dd, *J=* 6.5, 2.5 Hz), 4.90 (1H, dd, *J=* 6.5, 3.7 Hz), 4.27 (1H, td, *J=* 6.6, 3.7 Hz), 3.30 (1H, dd, *J=* 13.8, 6.8 Hz), 3.24 (1H, dd, *J=* 13.8, 6.3 Hz), 2.36 (3H, s), 1.59 (3H, s), 1.37 (3H, s). **¹³C NMR** (101 MHz, CDCl₃) δ 195.0, 157.7, 153.4, 151.0, 124.2, 114.8, 102.6, 99.4, 91.0, 85.2, 84.8, 83.6, 31.5, 30.7, 27.3, 25.6. **LRMS** Calculated for C₁₆H₂₀ClN₄O₄S, [M+H]⁺ m/z: 399.1, [M+H]⁺, found 399.0.

### Example 257

### 2-Chloro-7-((3aR,4R,6S,6aS)-2,2-dimethyl-6-(((3-methyl-5-phenylisoxazol-4-yl)methyl) thio)methyl)tetrahydrofuro[3,4-d][1,3]dioxol-4-yl)-7H-pyrrolo[2,3-d]pyrimidin-4-amine (203):

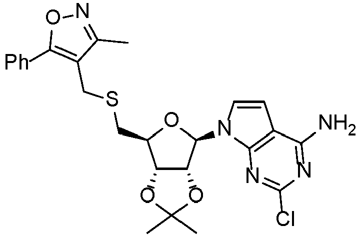

Title compound **203** was synthesized from bromide **47a** (obtained from **7** (0.04 g, 0.21 mmol)) and thiol (provided from **202** (0.07 g, 0.17 mmol)) following the same procedure as for **47b.** FCC (10-80 % EtOAc in cyclohexane) afforded title compound **203** (0.08 g, 0.14 mmol, 85 %). **¹H NMR** (400 MHz, CDCl₃) δ 7.77-7.70 (2H, m), 7.52-7.39 (3H, m), 6.99 (1H, d, *J=* 3.7 Hz), 6.36 (1H, d, *J=* 3.7 Hz), 6.06 (1H, d, *J=* 2.4 Hz), 5.62 (2H, bs), 5.25 (1H, dd, *J* = 6.6, 2.4 Hz), 5.03 (1H, dd, *J* = 6.6, 3.9 Hz), 4.30 (1H, td, *J* = 6.6, 3.9 Hz), 3.75 (1H, d, *J* = 12.8 Hz), 3.65 (1H, d, *J=* 12.8 Hz), 2.93 (1H, dd, *J* = 13.8, 6.9 Hz), 2.87 (1H, dd, *J=* 13.8, 6.3 Hz), 2.33 (3H, s), 2.30 (3H, s), 1.60 (3H, s), 1.39 (3H, s). **¹³C NMR** (101 MHz, CDCl₃) δ 166.3, 160.8, 157.5, 152.9, 150.7, 130.1, 129.1, 127.9, 127.3, 124.4, 114.8, 110.3, 102.6, 99.5, 91.1, 86.4, 84.8, 83.9, 34.9, 27.4, 25.6, 25.4, 10.3. **LRMS** Calculated for C₂₅H₂₇ClN₅O₄S, [M+H]⁺ m/z: 528.2, [M+H]⁺, found 528.1.

### Example 258

### (2R,3R,4S,5S)-2-(4-Amino-2-chloro-7H-pyrrolo[2,3-d]pyrimidin-7-yl)-5-((((3-methyl-5-phenylisoxazol-4-yl)methyl)thio)methyl)tetrahydrofuran-3,4-diol (204):

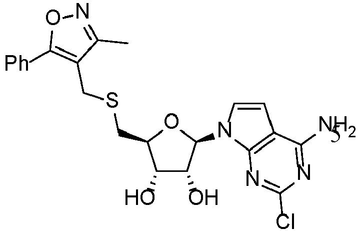

Title compound **204** was prepared from protected analogue **203** (0.07 g, 0.13 mmol) according to the same procedure as for **128.** Reverse-phase FCC (0-100 % MeCN in H₂O) furnished derivative **204** (0.05 g, 0.10 mmol, 82 %). **¹H NMR** (400 MHz, DMSO-*d₆*) δ 7.81-7.72 (2H, m), 7.60-7.45 (5H, m), 7.32 (1H, d, *J=* 3.7 Hz), 6.61 (1H, d, *J* = 3.7 Hz), 5.97 (1H, d, *J* = 6.0 Hz), 5.44 (1H, *d, J=* 6.1 Hz), 5.33 (1H, d, J = 4.6 Hz), 4.44 (1H, q, J = 5.4 Hz), 4.07-3.98 (2H, m), 3.85 (1H, d, *J* = 13.0 Hz), 3.80 (1H, d, *J* = 13.0 Hz), 2.95 (1H, dd, J = 13.9, 5.1 Hz), 2.87 (1H, dd, *J* = 13.9, 6.7 Hz), 2.27 (3H, s). **¹³C NMR** (101 MHz, DMSO-*d₆*) δ 164.9, 160.8, 158.6, 153.2, 151.3, 130.3, 129.4, 127.3, 126.8, 122.3, 111.1, 101.7, 100.8, 87.0, 83.5, 73.2, 72.9, 34.9, 24.5, 9.8. **HRMS** (ESI) Calculated for C₂₂H₂₂ClN₅O₄SNa, [M+Na]⁺ m/z: 550.1286, [M+Na]⁺, found 550.1282.

### Example 259

### S-(((3aS,4S,6R,6aR)-6-(4-Amino-2-methyl-7H-pyrrolo[2,3-d]pyrimidin-7-yl)-2,2-dimethyltetrahydrofuro[3,4-d][1,3]dioxol-4-yl)methyl) thioacetate (206):

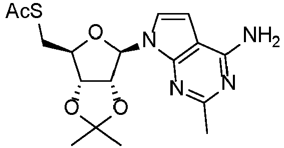

**206** was prepared from **205** (0.5 g, 1.77 mmol) in analogous fashion to **5.** FCC (3-10 % EtOH in DCM) provided **206** (0.46 g, 1.22 mmol, 78 %). **¹H NMR** (400 MHz, CDCl₃) δ 6.98 (1H, d, *J =* 3.7 Hz), 6.34 (1H, d, *J=* 3.7 Hz), 6.11 (1H, d, *J=* 2.2 Hz), 5.35 (1H, dd, *J=* 6.4, 2.2 Hz), 5.28 (2H, bs), 4.96 (1H, dd, *J=* 6.4, 3.5 Hz), 4.31-4.23 (1H, m), 3.32 (1H, dd, *J* = 13.7, 7.3 Hz), 3.19 (1H, dd, *J* = 13.7, 6.2 Hz), 2.56 (3H, s), 2.35 (3H, s), 1.59 (3H, s), 1.37 (3H, s). **¹³C NMR** (101 MHz, CDCl₃) δ 195.0, 161.0, 156.5, 151.1, 123.5, 114.4, 101.7, 98.9, 91.2, 85.5, 84.9, 83.9, 31.5, 30.7, 27.3, 25.7, 25.6. **LRMS** Calculated for C₁₇H₂₃N₄O₄S, [M+H]⁺ m/z: 379.1, [M+H]⁺, found 379.1.

### Example 260

### 7-((3aR,4R,6S,6aS)-2,2-Dimethyl-6-((((3-methyl-5-phenylisoxazol-4-yl)methyl)thio)methyl) tetrahydrofuro[3,4-d][1,3]dioxol-4-yl)-2-methyl-7H-pyrrolo[2,3-d]pyrimidin-4-amine (207):

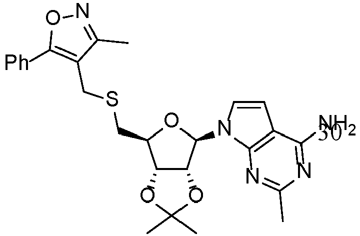

SAH analogue **207** was prepared from bromide **47a** (obtained from 7 (0.06 g, 0.32 mmol)) and thiol (provided from **206** (0.14 g, 0.38 mmol)) using the same procedure as for **47b.** FCC (10-70 % acetone in cyclohexane) furnished modified nucleoside **207** (0.14 g, 0.27 mmol, 70 %). **¹H NMR** (400 MHz, CDCl₃) δ 7.76-7.68 (2H, m), 7.49-7.39 (3H, m), 6.92 (1H, d, *J* = 3.7 Hz), 6.32 (1H, d, *J* = 3.7 Hz), 6.10 (1H, d, *J* = 2.2 Hz), 5.35 (1H, dd, *J* = 6.5, 2.2 Hz), 5.33 (2H, bs), 5.06 (1H, dd, *J=* 6.5, 3.8 Hz), 4.31 (1H, td, *J=* 6.6, 3.8 Hz), 3.71 (1H, d, *J=* 12.6 Hz), 3.56 (1H, d, *J* = 12.8 Hz), 2.87 (1H, dd, *J* = 13.8, 6.9 Hz), 2.82 (1H, dd, J = 13.8, 6.4 Hz), 2.53 (3H, s), 2.30 (3H, s), 1.60 (3H, s), 1.38 (3H, s). **¹³C NMR** (101 MHz, CDCl₃) δ 166.2, 160.8, 156.5, 150.9, 130.0, 129.1, 127.9, 127.3, 123.4, 114.4, 110.3, 101.7, 99.1, 91.0, 86.4, 84.8, 84.1, 77.5, 77.2, 76.8, 34.9, 27.3, 25.7, 25.6, 25.2, 10.2. **LRMS** Calculated for C₂₆H₃₀N₅O₄S, [M+H]⁺ m/z: 508.2, [M+H]⁺, found 508.2.

### Example 261

### (2R,3R,4S,5S)-2-(4-Amino-2-methyl-7H-pyrrolo[2,3-d]pyrimidin-7-yl)-5-((((3-methyl-5-phenylisoxazol-4-yl)methyl)thio)methyl)tetrahydrofuran-3,4-diol (208):

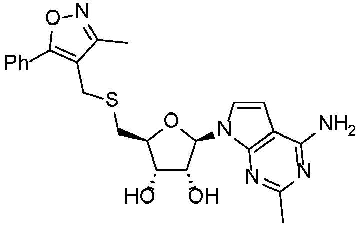

Modified nucleoside **208** was synthesized from (0.2 g, 0.39 mmol) according to the same procedure as for **128.** Reverse-phase FCC (25-100 % MeCN in H₂O) furnished **208** (0.11 g, 0.23 mmol, 57 %). **¹H NMR** (400 MHz, DMSO-*d₆*) δ 7.78-7.73 (2H, m), 7.56-7.46 (3H, m), 7.20 (1H, d, *J* = 3.7 Hz), 6.91 (2H, bs), 6.53 (1H, d, *J* = 3.6 Hz), 6.07 (1H, d, *J* = 5.9 Hz), 5.36 (1H, d, *J=* 6.2 Hz), 5.25 (1H, d, *J=* 5.1 Hz), 4.44 (1H, q, *J=* 5.8 Hz), 4.08-3.97 (2H, m), 3.85 (1H, *d, J=* 13.0 Hz), 3.80 (1H, d, *J=* 13.0 Hz), 2.94 (1H, dd, *J=* 13.9, 5.4 Hz), 2.86 (1H, dd, *J=* 13.9, 6.8 Hz), 2.36 (3H, s), 2.26 (3H, s). **¹³C NMR** (101 MHz, DMSO-*d₆*) δ 164.9, 160.8, 160.4, 157.5, 151.8, 130.3, 129.4, 127.3, 126.9, 120.9, 111.2, 100.7, 100.3, 86.7, 83.1, 73.2, 73.0, 34.9, 25.7, 24.5, 9.8. **HRMS** (ESI) Calculated for C₂₃H₂₆N₅O₄S, [M+H]⁺ m/z: 468.1700, [M+H]⁺, found 468.1697.

### Third generation

8-aza-7-deazaadenosine nucleobase and its N-6 and C-7 derivatives were employed in order to study modification of nucleobase core. Starting material for the synthesis of the unsubstituted derivative was the protected nucleoside **209,** prepared according to the published methodology (Chen, J. M., et al. US2010104532, 2010-04-29). The nucleobase amino group was protected with Boc and the Mitsunobu reaction carried out under the same conditions as above **(Scheme 1)** provided 5'-thioacetyl nucleoside **211.** Nucleophilic substitution of bromine of the freshly prepared bromide **47a** with an *in situ* generated thiolate followed by deprotection under acidic conditions afforded the SAH analogue **213.** Analogously, the C-7 iododerivative **217** was synthesized from a known nucleoside **214** (Huang, S.-C., et al., Bioorg Med Chem 2020/28, 115681). The Sonogashira cross-coupling was used to form derivative **218a** bearing the C-7 substituent connected via triple bond. The Suzuki-Miyaura reaction provided representatives **219a-222a** with a directly attached aromatic substituent. The Negishi cross-coupling reaction was used to synthesize analogue **223a** bearing an alkyl substituent. Final removal of the protection under acidic conditions furnished modified nucleosides **218b-223b (Scheme 13).**

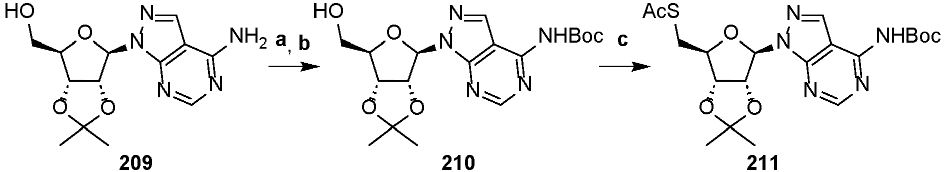

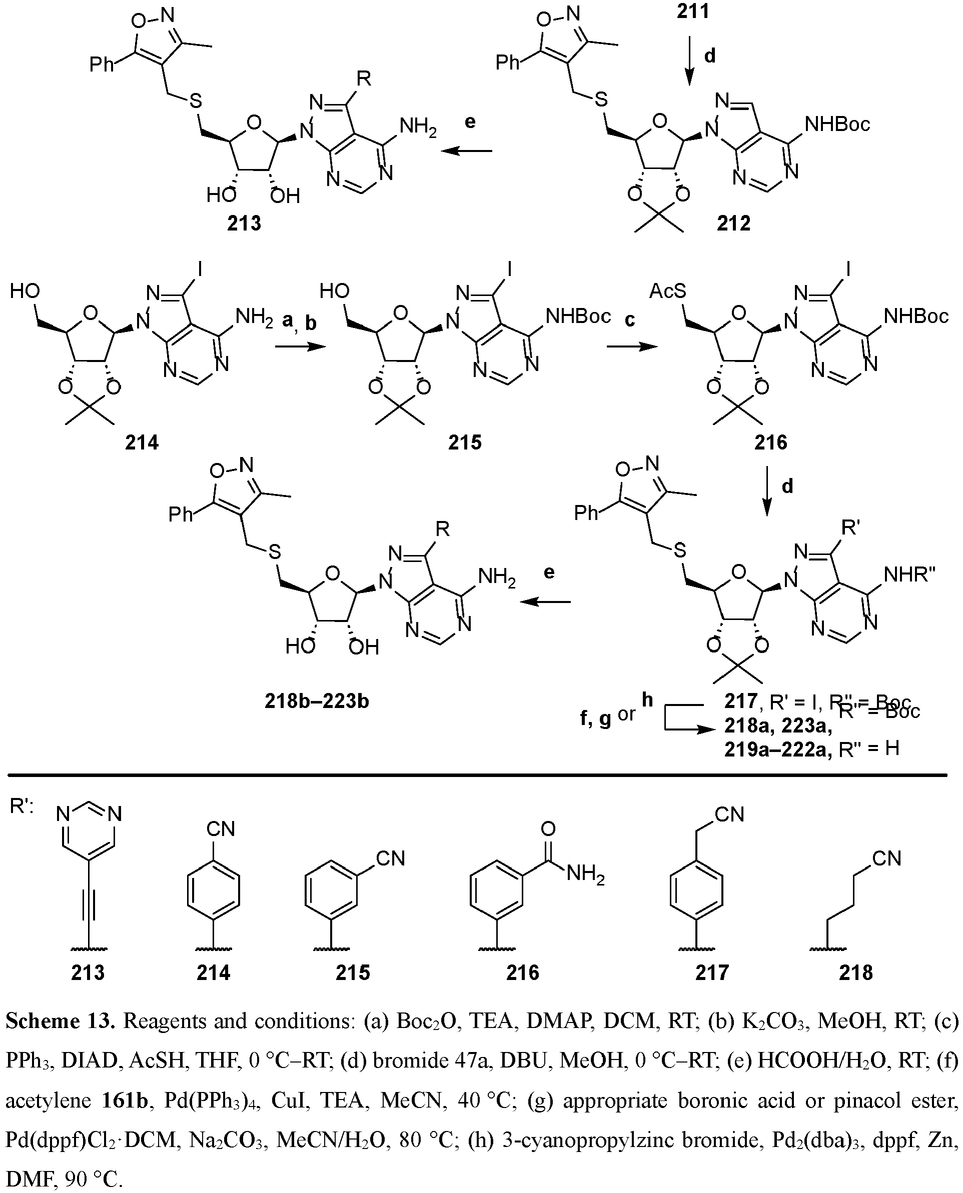

### Example 262

### tert-Butyl (1-((3aR,4R,6R,6aR)-6-(hydroxymethyl)-2,2-dimethyltetrahydrofuro[3,4-d][1,3]dioxol-4-yl)-1H-pyrazolo[3,4-d]pyrimidin-4-yl)carbamate (210):

To a suspension of nucleoside **209** (0.9 g, 2.93 mmol) in DCM (40 mL) was added Boc₂O (1.92 g, 8.79 mmol), TEA (0.4 mL, 2.88 mmol) and DMAP (0.02 g, 0.12 mmol). The reaction mixture was stirred at RT for 5 h, then it was diluted with EtOAc (120 mL), washed with H₂O and brine, dried over Na₂SO₄, 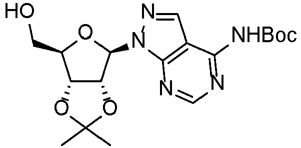 and evaporated. The residue was dissolved in MeOH (35 mL), then K₂CO₃ (0.44 g, 3.18 mmol) was added and the mixture was stirred at RT for 10 h. The solvent was evaporated, the residue was dissolved in EtOAc (120 mL), washed with sat. NH₄Cl and brine, dried over Na₂SO₄, and concentrated. FCC (15-100 % EtOAc in cyclohexane) afforded title compound **210** (0.73 g, 1.79 mmol, 61 %). **¹H NMR** (400 MHz, CDCl₃) δ 9.23 (1H, s), 8.80 (1H, d, *J=* 0.6 Hz), 8.62 (1H, s), 6.66 (1H, d, *J=* 3.0 Hz), 5.24 (1H, dd, *J=* 5.9, 3.0 Hz), 5.18 (1H, bs), 5.12 (1H, dd, *J=* 5.9, 1.3 Hz), 4.56 (1H, dt, *J* = 3.0, 1.6 Hz), 3.92 (1H, dd, *J* = 12.8, 1.9 Hz), 3.77 (1H, dd, *J* = 12.8, 3.0 Hz), 1.66 (3H, s), 1.59 (9H. s), 1.39 (3H, s). **¹³C NMR** (101 MHz, CDCl₃) δ 155.1, 155.0, 153.5, 151.3, 138.6, 113.6, 103.4, 92.7, 88.1, 85.1, 83.3, 82.3, 64.3, 28.3, 27.4, 25.4. **LRMS** Calculated for C₁₈H₂₆N₅O₆, [M+H]⁺ m/z: 408.2, [M+H]⁺, found 408.2.

### Example 263

### S-(((3aS,4S,6R,6aR)-6-(4-((tert-Butoxycarbonyl)amino)-1H-pyrazolo[3,4-d]pyrimidin-1-yl)-2,2-dimethyltetrahydrofuro[3,4-d][1,3]dioxol-4-yl)methyl) thioacetate (211):

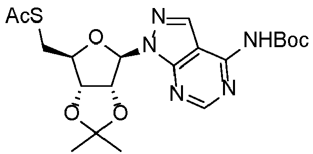

Title compound **211** was prepared from **210** (0.72 g, 1.77 mmol) according to the same procedure as for **5.** FCC (15-50 % EtOAc/EtOH (3/1) mixture in cylcohexane) provided derivative **211** (0.56 g, 1.20 mmol, 68 %). **¹H NMR** (400 MHz, CDCl₃) δ 10.35 (1H, s), 8.76 (1H, d, *J=* 0.6 Hz), 8.70 (1H, s), 6.72 (1H, s), 5.45 (1H, dd, *J* = 6.0, 1.0 Hz), 4.94 (1H, dd, *J=* 6.0, 2.0 Hz), 4.33 (1H, ddd, *J=* 8.5, 6.7, 2.0 Hz), 3.24 (1H, dd, *J=* 13.7, 8.5 Hz), 3.05 (1H, dd, *J* = 13.7, 6.7 Hz), 2.31 (3H, s), 1.60 (9H, s), 1.59 (3H, s), 1.39 (3H, s). **¹³C NMR** (101 MHz, CDCl₃) δ 194.8, 155.6, 155.0, 153.5, 151.6, 138.4, 113.7, 103.4, 89.9, 86.9, 84.8, 84.5, 83.0, 31.8, 30.7, 28.4, 27.0, 25.5. **LRMS** Calculated for C₂₀H₂₈N₅O₆S, [M+H]⁺ m/z: 466.2, [M+H]⁺, found 466.2.

### Example 264

### tert-Butyl (1-((3aR,4R,6S,6aS)-2,2-dimethyl-6-(((3-methyl-5-phenylisoxazol-4-yl)methyl)thio)methyl)tetrahydrofuro[3,4-d][1,3]dioxol-4-yl)-1H-pyrazolo[3,4-d]pyrimidin-4-yl)carbamate (212):

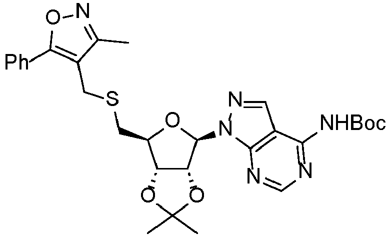

To a solution of derivative **7** (0.1 g, 0.53 mmol) in dry DCM (3 mL) was added PBr₃ (0.06 mL, 0.58 mmol) under argon atmosphere at 0 °C. The reaction mixture was stirred at RT for 1 h, then diluted with DCM (40 mL), washed with sat. NaHCO₃ and brine, dried over Na₂SO₄ and concentrated. Resulting bromide **47a** and thioacetate **211** (0.18 g, 0.39 mmol) were dissolved in dry MeOH (3 mL) under argon atmosphere and the resulting suspension was cooled in the ice bath. DBU (0.09 mL, 0.58 mmol) was added, and the reaction mixture was stirred at 0 °C for 1 h, then allowed to warm to RT and stirred for 4 h. The reaction was quenched with saturated solution of NH₄Cl (20 ml) and extracted with EtOAc (2×50 mL). Pooled organic layers were dried over Na₂SO₄ and concentrated. FCC (15-80 % EtOAc in cyclohexane) gave title compound **212** (0.21 g, 0.35 mmol, 91 %). **¹H NMR** (400 MHz, CDCl₃) δ 9.48 (1H, s), 8.70 (1H, d, *J* = 0.7 Hz), 8.62 (1H, s), 7.77-7.69 (2H, m), 7.52-7.38 (3H, m), 6.74-6.69 (1H, s), 5.42 (1H, dd, *J* = 6.1, 1.2 Hz), 5.03 (1H, dd, *J* = 6.1, 2.3 Hz), 4.41 (1H, ddd, *J* = 8.5, 6.5, 2.3 Hz), 3.68 (1H, d, *J* = 12.7 Hz), 3.57 (1H, d, *J* = 12.7 Hz), 2.82 (1H, dd, *J* = 13.8, 8.5 Hz), 2.74 (1H, dd, *J* = 13.8, 6.5 Hz), 2.32 (3H, s), 1.62 (3H, s), 1.59 (9H, s), 1.42 (3H, s). **¹³C NMR** (101 MHz, CDCl₃) δ 166.2, 160.8, 155.5, 154.7, 153.0, 151.3, 138.2, 130.0, 129.1, 127.9, 127.3, 113.8, 110.2, 103.3, 89.8, 87.4, 84.6, 83.2, 35.1, 28.3, 27.1, 25.6, 24.8, 10.2. **LRMS** Calculated for C₂₉H₃₅N₆O₆S, [M+H]⁺ m/z: 595.2, [M+H]⁺, found 595.2.

### Example 265

### (2R,3R,4S,5S)-2-(4-Amino-1H-pyrazolo[3,4-d]pyrimidin-1-yl)-5-((((3-methyl-5-phenylisoxazol-4-yl)methyl)thio)methyl)tetrahydrofuran-3,4-diol (213):

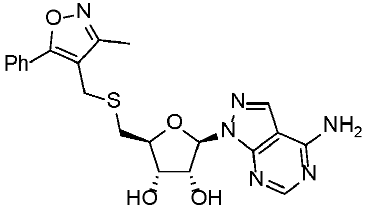

Modified nucleoside **213** was prepared from **212** (0.2 g, 0.34 mmol) using the same conditions as for **128**. Reverse-phase FCC (20-50 MeCN in H₂O) gave **213** (0.11 g, 0.23 mmol, 68 %). **¹H NMR** (400 MHz, CDCl₃) δ 8.20 (1H, s), 8.15 (1H, d, *J* = 0.5 Hz), 7.83 (1H, bs), 7.79-7.69 (2H, m), 7.68 (1H, bs), 7.57-7.45 (3H, m), 6.14 (1H, d, *J* = 4.1 Hz), 5.45 (1H, d, *J* = 5.7 Hz), 5.26 (1H, d, *J* = 5.8 Hz), 4.62 (1H, q, *J* = 5.0 Hz), 4.25 (1H, q, *J* = 5.2 Hz), 4.06 (1H, dt, *J* = 7.4, 4.9 Hz), 3.78 (2H, s), 2.90 (1H, dd, *J* = 13.9, 4.9 Hz), 2.77 (1H, dd, *J* = 13.9, 7.4 Hz), 2.24 (3H, s). **¹³C NMR** (101 MHz, CDCl₃) δ 164.7, 160.6, 158.0, 156.3, 154.3, 133.6, 130.1, 129.2, 127.1, 126.6, 111.0, 100.3, 88.2, 83.5, 73.2, 73.2, 35.0, 24.3, 9.5. **HRMS** (ESI) Calculated for C₂₁H₂₃N₆O₄S, [M+H]⁺ m/z: 455.1496, [M+H]⁺, found 455.1495.

### Example 266

### tert-Butyl(1-((3aR,4R,6R,6aR)-6-(hydroxymethyl)-2,2-dimethyltetrahydrofuro[3,4-d][1,3]dioxol-4-yl)-3-iodo-1H-pyrazolo[3,4-d]pyrimidin-4-yl)carbamate (215):

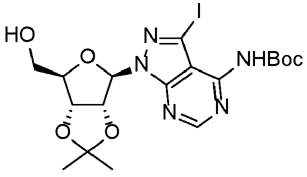

Nucleoside **215** was prepared from **214** (1.9 g, 4.39 mmol) in analogous fashion to **210**. FCC (20-100 % EtOAc in cyclohexane) gave **215** (1.33 g, 2.49 mmol, 57 %). **¹H NMR** (400 MHz, CDCl₃) δ 8.78 (1H, s), 8.16 (1H, bs), 6.49 (1H, d, *J* = 3.4 Hz), 5.20 (1H, dd, *J* = 5.9, 3.4 Hz), 5.07 (1H, dd, *J* = 5.9, 1.4 Hz), 4.57-4.51 (1H, m), 3.92 (1H, dd, *J* = 12.7, 2.1 Hz), 3.76 (1H, dd, *J* = 12.7, 3.0 Hz), 1.64 (3H, s), 1.59 (9H, s), 1.38 (3H, s). **¹³C NMR** (101 MHz, CDCl₃) δ 156.8, 154.1, 153.2, 114.0, 106.0, 93.8, 87.6, 87.2, 84.6, 83.5, 82.0, 63.9, 28.2, 27.4, 25.4. **LRMS** Calculated for C₁₈H₂₅IN₅O₆, [M+H]⁺ m/z: 534.1, [M+H]⁺, found 534.1.

### Example 267

### S-(((3aS,4S,6R,6aR)-6-(4-((tert-Butoxycarbonyl)amino)-3-iodo-1H-pyrazolo[3,4-d]pyrimidin-1-yl)-2,2-dimethyltetrahydrofuro[3,4-d][1,3]dioxol-4-yl)methyl) thioacetate (216):

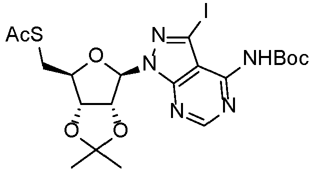

Derivative **216** was synthesized from protected nucleoside **215** (0.97 g, 1.82 mmol) according to the same procedure as for **5**. FCC (2-15 % acetone in DCM) provided title compound **216** (0.38 g, 0.64 mmol, 35 %). **¹H NMR** (400 MHz, CDCl₃) δ 8.79 (1H, s), 8.08 (1H, bs), 6.57 (1H, d, *J* = 1.4 Hz), 5.40 (1H, dd, *J* = 6.1, 1.4 Hz), 4.90 (1H, dd, *J* = 6.1, 2.3 Hz), 4.33 (1H, ddd, *J* = 8.1, 6.9, 2.3 Hz), 3.22 (1H, dd, *J* = 13.7, 8.1 Hz), 3.05 (1H, dd, *J* = 13.7, 6.9 Hz), 2.32 (3H, s), 1.59 (9H, s), 1.58 (3H, s), 1.38 (3H, s). **¹³C NMR** (101 MHz, CDCl₃) δ 194.6, 156.9, 154.8, 152.8, 114.0, 105.7, 90.4, 87.4, 86.9, 84.5, 84.2, 83.3, 31.7, 30.7, 28.2, 27.1, 25.5. **LRMS** Calculated for C₂₀H₂₇IN₅O₆S, [M+H]⁺ m/z: 592.1.2, [M+H]⁺, found 592.1.

### Example 268

### tert-Butyl (1-((3aR,4R,6S,6aS)-2,2-dimethyl-6-((((3-methyl-5-phenylisoxazol-4-yl)methyl)thio)methyl)tetrahydrofuro[3,4-d][1,3]dioxol-4-yl)-3-iodo-1H-pyrazolo[3,4-d]pyrimidin-4-yl)carbamate (217):

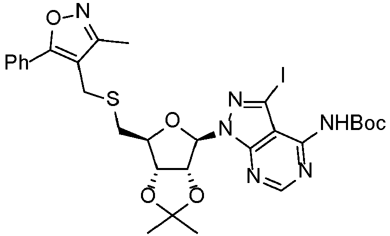

Title compound **217** was prepared from derivative **216** (0.44 g, 0.74 mmol) using the same conditions as for **212**. FCC (15-100 % EtOAc in cyclohexane) afforded modified nucleoside **217** (0.38 g, 0.53 mmol, 71 %). **¹H NMR** (400 MHz, CDCl₃) δ 8.76 (1H, s), 8.05 (1H, bs), 7.78-7.69 (2H, m), 7.52-7.39 (3H, m), 6.57 (1H, d, *J* = 1.6 Hz), 5.37 (1H, dd, *J* = 6.2, 1.6 Hz), 4.98 (1H, dd, *J* = 6.2, 3.4 Hz), 4.40 (1H, td, *J* = 7.3, 2.5 Hz), 3.69 (1H, d, *J* = 12.8 Hz), 3.62 (1H, d, *J* = 12.8 Hz), 2.79-2.75 (2H, m), 2.34 (3H, s), 1.60 (3H, s), 1.58 (9H, s), 1.40 (3H, s). **¹³C NMR** (101 MHz, CDCl₃) δ 166.3, 160.7, 156.9, 154.8, 152.8, 130.1, 129.1, 127.9, 127.3,114.2, 110.1, 105.6, 90.2, 87.5, 87.2, 84.3, 84.2, 83.4, 35.0, 28.2, 27.1, 25.5, 24.9, 10.3. **LRMS** Calculated for C₂₉H₃₄IN₆O₆S, [M+H]⁺ m/z: 721.1, [M+H]⁺, found 721.1.

### Example 269

### tert-Butyl (1-((3aR,4R,6S,6aS)-2,2-dimethyl-6-(((3-methyl-5-phenylisoxazol-4-yl)methyl)thio)methyl)tetrahydrofuro[3,4-d] [1,3]dioxol-4-yl)-3-(pyrimidin-5-ylethynyl)-1H-pyrazolo[3,4-d]pyrimidin-4-yl)carbamate (218a):

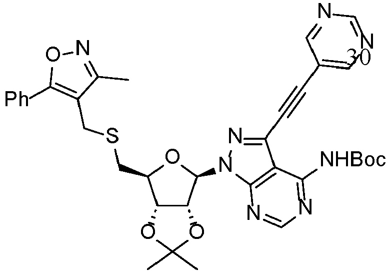

SAH analogue **218a** was prepared from **217** (0.18 g, 0.25 mmol) and acetylene **161b** (0.06 g, 0.58 mmol) following the same procedure as for **169a**. FCC (20-100 % EtOAc in cyclohexane) provided **218a** (0.1 g, 0.14 mmol, 57 %). **¹H NMR** (400 MHz, CDCl₃) δ 9.27 (1H, s), 9.01 (2H, s), 8.81 (1H, s), 7.73-7.69 (2H, m), 7.49-7.39 (3H, m), 6.66 (1H, s), 5.39 (1H, dd, *J* = 6.1, 1.0 Hz), 5.00 (1H, dd, *J* = 6.1, 2.4 Hz), 4.42 (1H, td, *J* = 8.4, 2.3 Hz), 3.70 (1H, d, *J* = 12.9 Hz), 3.62 (1H, d, *J* = 12.9 Hz), 2.80 (1H, dd, *J* = 13.7, 8.4 Hz), 2.73 (1H, dd, *J* = 13.7, 6.5 Hz), 2.32 (3H, s), 1.61 (3H, s), 1.59 (9H, s), 1.40 (3H, s). **¹³C NMR** (101 MHz, CDCl₃) δ 166.3, 160.7, 158.9, 158.0, 157.5, 154.6, 153.1, 148.7, 130.1, 129.1, 127.8, 127.3, 126.0, 114.2, 110., 104., 90.4, 89.6, 87.3, 87.0, 84.4, 84.3, 83.7, 34.7, 28.2, 27.1, 25.5, 24.7, 10.3. **LRMS** Calculated for C₃₅H₃₇N₈O₆S, [M+H]⁺ m/z: 697.3, [M+H]⁺, found 697.3.

### Example 270

### 4-(4-Amino-1-((3aR,4R,6S,6aS)-2,2-dimethyl-6-((((3-methyl-5-phenylisoxazol-4-yl)methyl) thio)methyl)tetrahydrofuro[3,4-d][1,3]dioxol-4-yl)-1H-pyrazolo[3,4-d]pyrimidin-3-yl)benzonitrile (219a):

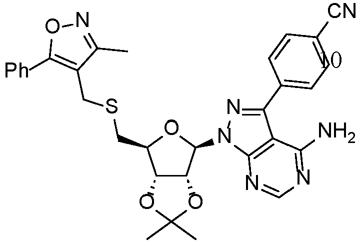

A mixture of modified nucleoside **217** (0.21 g, 0.29 mmol), (4-cyanophenyl)boronic acid (0.08 g, 0.52 mmol), Pd(dppf)Cl₂·DCM (0.01 g, 0.02 mmol) and Na₂CO₃ (0.09 g, 0.87 mmol) in MeCN/H₂O (3:1, 4 mL) was stirred under argon atmosphere at 80 °C for 18 h. The reaction mixture was diluted with EtOAc (100 mL), washed with H₂O and brine, dried over Na₂SO₄ and concentrated. FCC (15-60 % EtOAc/EtOH (3/1) mixture in cyclohexane) afforded SAH analogue **219a** (0.1 g, 0.17 mmol, 59 %).**¹H NMR** (400 MHz, CDCl₃) δ 8.41 (1H, s), 7.81 (2H, d, *J* = 8.2 Hz), 7.75 (2H, d, *J* = 8.2 Hz), 7.72-7.68 (2H, m), 7.48-7.39 (3H, m), 6.65 (1H, d, *J* = 1.6 Hz), 5.90 (2H, s), 5.44 (1H, dd, *J* = 6.2, 1.6 Hz), 4.99 (1H, dd, *J* = 6.2, 2.5 Hz), 4.42 (1H, td, *J* = 7.4, 2.5 Hz), 3.70 (1H, d, *J* = 12.8 Hz), 3.59 (1H, d, *J* = 12.8 Hz), 2.82-2.78 (2H, m), 2.29 (3H, s), 1.61 (3H, s), 1.40 (3H, s). **¹³C NMR** (101 MHz, CDCl₃) δ 166.2, 160.7, 157., 155.8, 155.6, 144.1, 137.0, 133.3, 130.1, 129.1, 127.8, 127.3, 118.2, 114.2, 113.5, 110.1, 98.8, 90.4, 86.9, 84.3, 84., 34.9, 27.2, 25.6, 24.7, 10.2. **LRMS** Calculated for C₃₁H₃₀N₇O₄S, [M+H]⁺ m/z: 596.2, [M+H]⁺, found 596.2.

### Example 271

### 3-(4-Amino-1-((3aR,4R,6S,6aS)-2,2-dimethyl-6-((((3-methyl-5-phenylisoxazol-4-yl)methyl)thio) methl)tetrahdrofuro[3,4-d][1,3]dioxol-4-yl)-1H-pyrazolo[3,4-d]pyrimidin-3-yl)benzonitrile (220a):

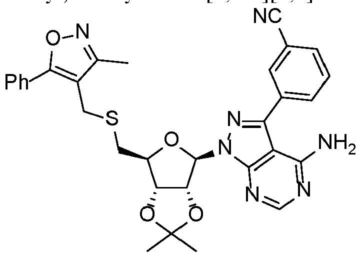

**220a** was synthesized from (0.18 g, 0.25 mmol) and (3-cyanophenyl)boronic acid (0.08 g, 0.52 mmol) using the same methodology as for **219a**. FCC (15-60 % EtOAc/EtOH (3/1) mixture in cyclohexane) furnished **220a** (0.12 g, 0.20 mmol, 81 %). **¹H NMR** (400 MHz, CDCl₃) δ 8.38 (1H, s), 7.94 (1H, t, *J* = 1.5 Hz), 7.85 (1H, d, *J* = 7.8 Hz), 7.80 (1H, d, *J* = 7.8 Hz), 7.73-7.69 (2H, m), 7.67 (1H, t, *J* = 7.8 Hz), 7.50-7.39 (3H, m), 6.65 (1H, d, *J* = 1.6 Hz), 6.06 (2H, s), 5.46 (1H, dd, *J* = 6.2, 1.6 Hz), 5.00 (1H, dd, *J* = 6.2, 2.5 Hz), 4.42 (1H, td, *J* = 7.3, 2.5 Hz), 3.70 (1H, d, *J* = 12.8 Hz), 3.59 (1H, d, *J* = 12.8 Hz), 2.80 (2H, d, *J* = 7.3 Hz), 2.29 (3H, s), 1.62 (3H, s), 1.41 (3H, s). **¹³C NMR** (101 MHz, CDCl₃) δ 166.2, 160.7, 157.3, 155.5, 143.8, 133.9, 133.1, 132.5, 131.9, 130.5, 130.1, 129.1, 127.8, 127.3, 118.0, 114.2, 114.1, 110.0, 98.7, 90.3, 86.9, 84.3, 84.2, 34.9, 27.2, 25.6, 24.7, 10.2. **LRMS** Calculated for C₃₁H₃₀N₇O₄S, [M+H]⁺ m/z: 596.2, [M+H]⁺, found 596.2.

### Example 272

### 3-(4-Amino-1-((3aR,4R,6S,6aS)-2,2-dimethyl-6-((((3-methyl-5-phenylisoxazol-4-yl)methyl)thio) methyl)tetrahydrofuro[3,4-d][1,3]dioxol-4-yl)-1H-pyrazolo[3,4-d]pyrimidin-3-yl)benzamide (221a):

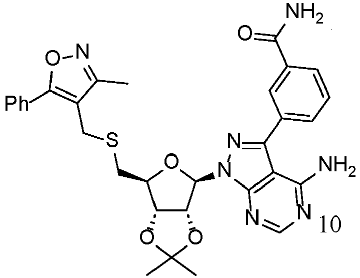

**221a** was prepared from intermediate **217** (0.18 g, 0.25 mmol) and (3-carbamoylphenyl)boronic acid (0.05 g, 0.30 mmol) according to the same procedure as for **219a**. FCC (15-60 % EtOAc/EtOH (3/1) mixture in cyclohexane) gave **221a** (0.11 g, 0.18 mmol, 72 %). **¹H NMR** (400 MHz, CDCl₃) δ 8.37 (1H,s), 8.11 (1H, s), 7.92 (1H, *d, J* = 7.5 Hz), 7.77 (1H, d, *J* = 7.5 Hz), 7.71-7.67 (2H, m), 7.61 (1H, t, *J* = 7.5 Hz), 7.48-7.37 (3H, m), 6.64 (1H, d, *J* = 1.2 Hz), 6.43 (1H, bs), 6.07 (1H, bs), 5.92 (2H, bs), 5.47 (1H, dd, *J* = 6.1, 1.2 Hz), 4.99 (1H, dd, *J* = 6.1, 2.3 Hz), 4.42 (1H, td, *J* = 7.4, 2.3 Hz), 3.70 (1H, d, *J* = 12.9 Hz), 3.60 (1H, d, *J* = 12.9 Hz), 2.80 (2H, d, *J* = 7.4 Hz), 2.26 (3H, s), 1.61 (3H, s), 1.40 (3H, s). **¹³C NMR** (101 MHz, CDCl₃) δ 168.9, 166.2, 160.8, 157.8, 156.2, 155.6, 145.0, 134.8, 133.2, 131.7, 130.1, 129.9, 129.1, 128.4, 127.7, 127.6, 127.2, 114.1, 110.2, 98.9, 90.2, 86.9, 84.4, 84.2, 35.0, 27.2, 25.6, 24.7, 10.1. **LRMS** Calculated for C₃₁H₃₂N₇O₅S, [M+H]⁺ m/z: 614.2, [M+H]⁺, found 614.2.

### Example 273

### 2-(4-(4-Amino-1-((3aR,4R,6S,6aS)-2,2-dimethyl-6-((((3-methyl-5-phenylisoxazol-4-yl)methyl)thio) methyl)tetrahydrofuro[3,4-d][1,3]dioxol-4-yl)-1H-pyrazolo[3,4-d]pyrimidin-3-yl)phenyl)acetonitrile (222a):

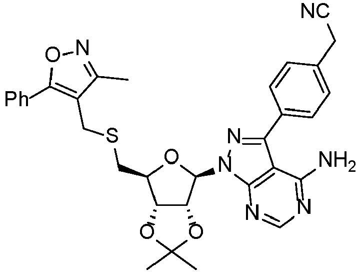

Derivative **222a** was synthesized from **217** (0.22 g, 0.31 mmol) and 2-((4-cyanomethyl)phenyl)boronic acid pinacol ester (0.11 g, 0.31 mmol) following the same conditions as for **219a**. FCC (15-60 % EtOAc/EtOH (3/1) mixture in cyclohexane) afforded **222a** (0.08 g, 0.13 mmol, 42%). **¹H NMR** (400 MHz, CDCl₃) δ 8.41 (1H, s), 7.79-7.70 (2H, m), 7.67 (2H, d, *J* = 8.0 Hz), 7.53 (2H, d, *J* = 8.0 Hz), 7.51-7.42 (3H, m), 6.69 (1H, *d, J* = 1.6 Hz), 5.74 (2H, s), 5.48 (1H, dd, *J* = 6.2, 1.6 Hz), 5.04 (1H, dd, *J* = 6.2, 2.5 Hz), 4.45 (1H, td, *J* = 7.3, 2.5 Hz), 3.87 (2H, s), 3.72 (1H, d, *J* = 12.7 Hz), 3.61 (1H, d, *J* = 12.7 Hz), 2.88 (1H, dd, *J* = 14.0, 7.7 Hz), 2.83 (1H, dd, *J* = 14.0, 6.9 Hz), 2.31 (3H, s), 1.64 (3H, s), 1.42 (3H, s). **¹³C NMR** (101 MHz, CDCl₃) δ 166.2, 160.8, 157.6, 156.2, 155.6, 145.0, 132.7, 131.6, 130.1, 129.3, 129.3, 129.1, 127.8, 127.3, 117.4, 114.0, 110.1, 98.9, 90.2, 87.0, 84.4, 84.3, 35.0, 27.2, 25.6, 24.8, 23.7, 10.2. **LRMS** Calculated for C₃₂H₃₂N₇O₄S, [M+H]⁺ m/z: 610.2, [M+H]⁺, found 610.2.

### Example 274

### tert-Butyl (3-(3-cyanopropyl)-1-((3aR,4R,6S,6aS)-2,2-dimethyl-6-((3-methyl-5-phenylisoxazol-4-yl)methyl)thio)methyl)tetrahydrofuro[3,4-d][1,3]dioxol-4-yl)-1H-pyrazolo[3,4-d]pyrimidin-4-yl)carbamate (223a):

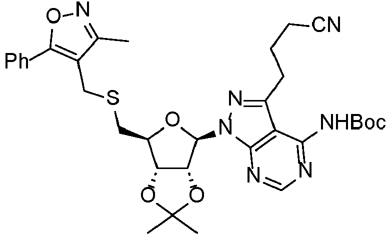

**223a** was prepared from **217** (0.18 g, 0.25 mmol) and 0.5M solution of (3-cyanopropyl)zinc bromide in THF (1.5 mL, 0.75 mmol) in analogous fashion to **129a**. FCC (20-100 % EtOAc in cyclohexane) provided **223a** (0.06 g, 0.09 mmol, 37 %). **¹H NMR** (400 MHz, CDCl₃) δ 8.51 (1H, s), 7.79-7.64 (2H, m), 7.54-7.44 (4H, m), 6.59 (1H, d, *J* = 1.4 Hz), 5.35 (1H, dd, *J* = 6.1, 1.4 Hz), 4.98 (1H, dd, *J* = 6.1, 2.5 Hz), 4.39 (1H, td, *J* = 7.4, 2.5 Hz), 3.71 (1H, d, *J* = 12.8 Hz), 3.61 (1H, d, *J* = 12.8 Hz), 3.16-3.09 (2H, m), 2.79 (1H, dd, *J* = 13.8, 8.0 Hz), 2.74 (1H, dd, *J* = 13.8, 6.8 Hz), 2.45 (2H, t, *J* = 7.1 Hz), 2.33 (3H, s), 2.22-2.05 (2H, m), 1.61 (3H, s), 1.56 (9H, s), 1.41 (3H, s). **LRMS** Calculated for C₃₃H₄₀N₇O₆S, [M+H]⁺ m/z: 662.2, [M+H]⁺, found 610.2.

### Example 275

### (2R,3R,4S,5S-2-(4-Amino-3-(pyrimidin-5-ylethynyl)-1H-pyrazolo[3,4-d]pyrimidin-1-yl)-5-((((3-methyl-5-phenylisoxazol-4-yl)methyl)thio)methyl)tetrahydrofuran-3,4-diol (218b):

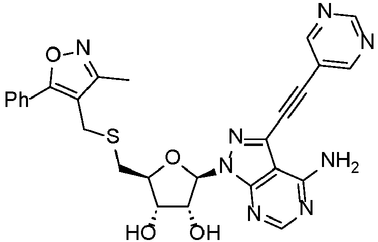

**218b** was synthesized from protected analogue **218a** (0.09 g, 0.13 mmol) using the same conditions as for **128**. Reverse-phase FCC (20-50 % MeCN in H₂O) furnished **218b** (0.05 g, 0.09 mmol, 68 %). **¹H NMR** (400 MHz, DMSO-*d₆*) δ 9.24 (1H, s), 9.13 (2H, s), 8.30 (1H, s), 8.10 (1H, bs), 7.78-7.71 (2H, m), 7.56-7.47 (3H, m), 7.06 (1H, bs), 6.19 (1H, d, *J* = 4.4 Hz), 5.55 (1H, d, *J* = 5.7 Hz), 5.34 (1H, d, *J* = 5.6 Hz), 4.69-4.61 (1H, m), 4.24 (1H, q, *J* = 5.1 Hz), 4.11 (1H, dt, *J* = 7.2, 5.0 Hz), 3.81 (2H, s), 2.92 (1H, dd, *J* = 13.9, 4.9 Hz), 2.80 (1H, dd, *J* = 13.9, 7.2 Hz), 2.26 (3H, s). **¹³C NMR** (101 MHz, DMSO-*d₆*) δ 164.7, 160.6, 159.4, 157.6, 157.3, 157.0, 154.5, 130.1, 129.2, 127.1, 126.6, 126.3, 118.0, 111.0, 100.7, 88.3, 87.3, 86.5, 83.8, 73.2, 73.1, 34.9, 24.3, 9.6. **HRMS** (ESI) Calculated for C₂₇H₂₅N₈O₄S, [M+H]⁺ m/z: 557.1714, [M+H]⁺, found 557.1722.

### Example 276

### 4-(4-Amino-1-((2R,3R,4S,5S)-3,4-dihydroxy-5-((((3-methyl-5-phenylisoxazol-4-yl)methyl)thio)methyl)tetrahydrofuran-2-yl)-1H-pyrazolo[3,4-d]pyrimidin-3-yl)benzonitrile (219b):

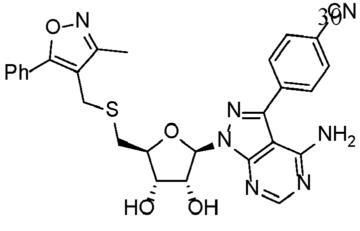

Title compound **219b** was prepared from modified nucleoside **219a** (0.09 g, 0.15 mmol) according to the same procedure as for **128**. Reverse-phase FCC (20-50 % MeCN in H₂O) provided SAH analogue **219b** (0.05 g, 0.09 mmol, 56 %). **¹H NMR** (400 MHz, DMSO-*d₆*) δ 8.30 (1H, s), 7.96 (2H, d, *J* = 8.3 Hz), 7.79 (2H, d, *J* = 8.3 Hz), 7.76-7.67 (2H, m), 7.54-7.42 (3H, m), 7.14 (2H, bs), 6.24 (1H, d, *J* = 4.1 Hz), 5.53 (1H, d, *J* = 5.6 Hz), 5.31 (1H, d, *J* = 5.7 Hz), 4.71-4.63 (1H, m), 4.29 (1H, q, *J* = 5.1 Hz), 4.11 (1H, dt, *J* = 7.4, 4.9 Hz), 3.81 (1H, d, *J* = 13.1 Hz), 3.76 (1H, d, *J* = 13.1 Hz, 1H), 2.92 (1H, dd, *J* = 13.9, 4.8 Hz), 2.81 (1H, dd, *J* = 13.9, 7.4 Hz), 2.19 (3H, s). **¹³C NMR** (101 MHz, DMSO-*d₆*) δ 164.6, 160.6, 158.1, 156.3, 155.7, 143.6, 137.0, 133.0, 130.0, 129.2, 129.1, 127.1, 126.6, 118.8, 111.2, 111.0, 97.8, 88.2, 83.7, 73.3, 73.2, 34.9, 24.3, 9.5. **HRMS** (ESI) Calculated for C₂₈H₂₆N₇O₄S, [M+H]⁺ m/z: 556.1762, [M+H]⁺, found 556.1764.

### Example 277

### 3-(4-Amino-1-((2R,3R,4S,5S)-3,4-dihydroxy-5-((((3-methyl-5-phenylisoxazol-4-yl)methyl)thio)methyl)tetrahydrofuran-2-yl)-1H-pyrazolo[3,4-d]pyrimidin-3-yl)benzonitrile (220b):

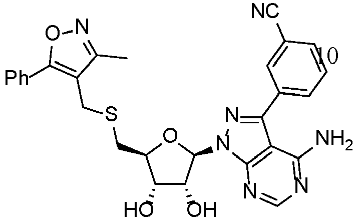

Derivative **220b** was synthesized from protected analogue **220a** (0.11 g, 0.19 mmol) following the same methodology as for **128**. Reverse-phase FCC (20-45 % MeCN in H₂O) afforded **220b** (0.06 g, 0.10 mmol, 52 %). **¹H NMR** (400 MHz, DMSO-*d₆*) δ 8.30 (1H, s), 7.99 (1H, t, *J* = 1.4 Hz), 7.95-7.90 (2H, m), 7.74-7.70 (2H, m), 7.70 (1H, t, *J* = 7.8 Hz), 7.51-7.43 (3H, m), 7.13 (2H, bs), 6.24 (1H, d, *J* = 4.2 Hz), 5.52 (1H, d, *J* = 5.7 Hz), 5.29 (1H, d, *J* = 5.8 Hz), 4.67 (1H, q, *J* = 5.0 Hz), 4.28 (1H, q, *J* = 5.1 Hz), 4.10 (1H, dt, *J* = 7.6, 4.7 Hz), 3.82 (1H, d, *J* = 13.0 Hz), 3.76 (1H, d, *J* = 13.0 Hz), 2.92 (1H, dd, *J* = 13.9, 4.7 Hz), 2.81 (1H, dd, *J* = 13.9, 7.5 Hz), 2.19 (3H, s). **¹³C NMR** (101 MHz, DMSO-*d₆*) δ 164.6, 160.5, 158.1, 156.2, 155.7, 143.3, 133.6, 132.8, 132.4, 131.8, 130.2, 130.0, 129.1, 127.1, 126.6, 118.6, 112.3, 110.9, 97.8, 88.2, 83.7, 73.2, 35.0, 24.3, 9.5. **HRMS** (ESI) Calculated for C₂₈H₂₆N₇O₄S, [M+H]⁺ m/z: 556.1762, [M+H]⁺, found 556.1759.

### Example 278

### 3-(4-Amino-1-((2R,3R,4S,5S)-3,4-dihydroxy-5-((((3-methyl-5-phenylisoxazol-4-yl)methyl)thio)methyl)tetrahydrofuran-2-yl)-1H-pyrazolo[3,4-d]pyrimidin-3-yl)benzamide (221b):

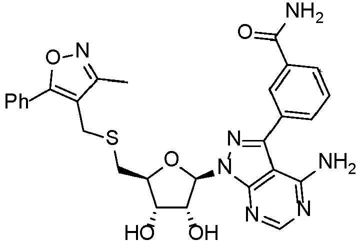

**221b** was prepared from **221a** (0.1 g, 0.16 mmol) in analogous fashion to **128**. Reverse-phase FCC (20-50 % MeCN in H₂O) gave **221b** (0.04 g, 0.07 mmol, 44 %). **¹H NMR** (400 MHz, DMSO-*d₆*) δ 8.29 (1H, s), 8.16 (1H, t, *J* = 1.6 Hz), 8.08 (2H, s), 7.98 (1H, ddd, *J* = 7.6, 1.6, 1.1 Hz), 7.74 (1H, ddd, *J* = 7.8, 1.6, 1.1 Hz, 7.73-7.70 (2H, m), 7.59 (1H, t, *J* = 7.7 Hz), 7.53-7.41 (5H, m), 6.24 (1H, d, *J* = 4.3 Hz), 5.51 (1H, d, *J* = 5.8 Hz), 5.30 (1H, d, *J* = 5.7 Hz), 4.72-4.67 (1H, m), 4.28 (1H, q, *J* = 5.0 Hz), 4.09 (1H, dt, *J* = 7.6, 4.9 Hz), 3.83 (1H, d, *J* = 13.0 Hz), 3.77 (1H, *d, J=* 13.0 Hz), 2.91 (1H, dd, *J* = 13.9, 4.8 Hz), 2.81 (1H, dd, *J* = 13.9, 7.6 Hz), 2.19 (3H, s). **¹³C NMR** (101 MHz, DMSO-*d₆*) δ 167.5, 164.6, 160.6, 158.2, 156.2, 155.6, 144.7, 135.0, 132.6, 130.9, 130.0, 129.2, 129.1, 127.8, 127.5, 127.1, 126.6, 111.0, 97.8, 88.2, 83.7, 73.3, 73.2, 35.0, 24.3, 9.5. **HRMS** (ESI) Calculated for C₂₈H₂₇N₇O₅NaS, [M+Na]⁺ m/z: 596.1687, [M+Na]⁺, found 596.1688.

### Example 279

### 2-(4-(4-Amino-1-((2R,3R,4S,5S)-3,4-dlhydroxy-5-((((3-methyl-5-phenylisoxazol-4-yl)methyl) thio)methyl)tetrahydrofuran-2-yl)-1H-pyrazolo[3,4-d]pyrimidin-3-yl)phenyl)acetonitrile (222b):

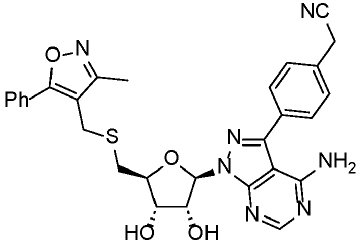

**222b** was synthesized from modified nucleoside **222a** (0.11 g, 0.16 mmol) according to the same methodology as for **128**. Reverse-phase FCC (20-50 % MeCN in H₂O) provided SAH analogue **222b** (0.05 g, 0.09 mmol, 55 %). **¹H NMR** (400 MHz, DMSO-*d₆*) δ 8.28 (1H, s), 7.77-7.69 (2H, m), 7.60-7.62 (2H, m), 7.55-7.42 (5H, m), 6.88 (2H, bs), 6.23 (1H, d, *J* = 4.2 Hz), 5.51 (1H, d, *J* = 5.7 Hz), 5.28 (1H, d, *J* = 5.8 Hz), 4.71-4.63 (1H, m), 4.28 (1H, q, *J* = 5.2 Hz), 4.14 (2H, s), 4.10 (1H, dt, *J* = 7.6, 4.8 Hz), 3.82 (1H, d, *J* = 13.1 Hz), 3.77 (1H, d, *J* = 13.1 Hz), 2.91 (1H, dd, *J* = 13.9, 4.8 Hz), 2.81 (1H, dd, *J* = 13.9, 7.6 Hz), 2.19 (3H, s).**¹³C** NMR (101 MHz, DMSO-*d₆*) δ 164.7, 160.6, 158.2, 156.2, 155.6, 144.6, 131.9, 131.9, 130.1, 129.2, 128.9, 128.8, 127.1, 126.6, 119.1, 111.0, 97.8, 88.2, 83.7, 73.3, 73.2, 35.0, 24.3, 22.3, 9.5. **HRMS** (ESI) Calculated for C₂₉H₂₈N₇O₄S, [M+H]⁺ m/z: 570.1918, [M+H]⁺, found 570.1919.

### Example 280

### 4-(4-Amino-1-((2R,3R,4S,5S)-3,4-dihydroxy-5-((((3-methyl-5-phenylisoxazol-4-yl)methyl)thio)methyl)tetrahydrofuran-2-yl)-1H-pyrazolo[3,4-d]pyrimidin-3-yl)butanenitrile (223b):

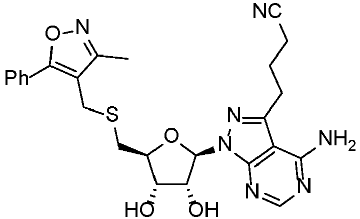

Modified nucleoside **223b** was prepared from protected analogue **223a** (0.05 g, 0.06 mmol) following same procedure as for **128**. Reverse-phase FCC (20-45 % MeCN in H₂O) furnished derivative **223b** (0.02 g, 0.03 mmol, 54 %). **¹H NMR** (400 MHz, DMSO-*d₆*) δ 8.17 (1H, s), 7.78-7.70 (2H, m), 7.57-7.46 (3H, m), 7.37 (2H, bs), 6.10 (1H, d, *J* = 4.1 Hz), 5.41 (1H, d, *J* = 5.6 Hz), 5.22 (1H, d, *J* = 5.8 Hz), 4.57 (1H, td, *J* = 5.4, 4.1 Hz), 4.25 (1H, q, *J* = 5.2 Hz), 4.05 (1H, dt, *J* = 7.7, 4.9 Hz), 3.81 (1H, d, *J* = 13.0 Hz, 1H), 3.76 (1H, d, *J* = 13.0 Hz), 3.00 (2H, t, *J* = 7.5 Hz), 2.89 (1H, dd, *J* = 13.9, 4.6 Hz), 2.77 (1H, dd, *J* = 13.9, 7.7 Hz), 2.56-2.52 (2H, m), 2.23 (3H, s), 1.86 (2H, p, *J* = 7.5 Hz).**¹³C NMR** (101 MHz, DMSO-*d₆*) δ 164.7, 160.6, 158.1, 156.1, 155.4, 144.6, 130.1, 129.2, 127.1, 126.6, 120.4, 111.0, 98.6, 87.9, 83.4, 73.3, 73.1, 35.0, 26.8, 24.3, 24.2, 15.7, 9.5. **HRMS** (ESI) Calculated for C₂₅H₂₈N₇O₄S, [M+H]⁺ m/z: 522.1918, [M+H]⁺, found 522.1917.

### Fourth generation

Based to the results of the first, second and third generation we designed and prepared hybrid molecules based on 7-deazaadenosine or 7-deaza-8-azaadenosine scaffold, which would, ideally, combine the most beneficial features regarding the biological activity, microsomal stability and cell permeability of the inhibitors. As the best performing substituents in the C-7 position of the nucleobase we selected iodo (compound **123**, **Scheme 7**), 4-cyanophenyl (compound **143**, **Scheme 9**), 5-pyrimidinylethynyl (compound **172**, **Scheme 11**) and 4-(cyanomethyl)phenyl (compound **222**, **Scheme 13**). The most suitable reagent for alkylation to the 5' position appeared to be 5-(benzo[1,3]dioxol-5-yl)-4-(bromomethyl)-3-methylisoxazole (reagent **96a**, **Scheme 5**). A sequence of alkylation of **96a** to the 5' free thiol derived from **216**, Suzuki-Miyaura cross coupling of (4-(cyanomethyl)phenyl)boronic acid pinacol ester to the C-7 position and acidic cleavage of the protecting groups gave hybrid **226**. In the series based on 7-deazaadenosine, two different synthetic strategies were employed. In the case of compounds with the 4-cyanophenyl modification we first introduced the 4-cyanophenyl to a protected nucleoside **4** by Suzuki-Miyaura cross-coupling to prepare **227**. The 5'-hydroxyl was converted to a thioacetate *via* the Mitsunobu reaction with thioacetic acid. The intermediate **228** was then alkylated with selected hetaryls (reagents **49a**, **54a**, **56a**, **93a**, **95a** and **96a**) following the same methodology as for SAH analogues in the first generation (**Scheme 3** and **Scheme 5**) affording compounds **230a-232a**, **233**, **235a** and **236a**. The analogue **233** bearing a nitrophenyl group on the isoxazole part was reduced by the treatment with SnCl₂ to amino derivative **234a**. Final deprotection of these molecules with HF-TEA led to the final hybrids **230b-232b** and **234b-236b**. The reversed order of reactions had to be used to synthesize compounds with a 5-pyrimidinylethynyl substitution in the C-7 position. 5'-moieties were first attached to the iodinated thiol **121** by alkylation of the freshly prepared halomethyl hetarenes (reagents **49a**, **54a**, **56a**, **93a**, **95a** and **96a**). Nitro group modification of the derivative **240** was transformed to amino group by SnCl₂ (compound **241a**). The following Sonogashira cross-coupling reactions with 5-ethynylpyrimidine then afforded modified nucleosides **237b-239b** and **241b-243b**. The removal of the protecting groups with HF-TEA afforded final hybrids **237c-239c** and **241c-243c**. The iodo derivatives **244-246** were obtained from protected analogues by the same type of deprotection. (**Scheme 14**).

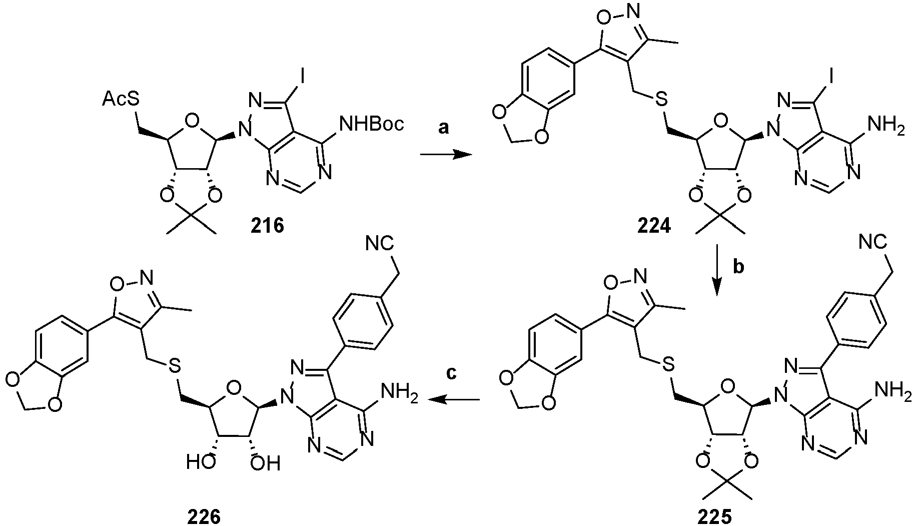

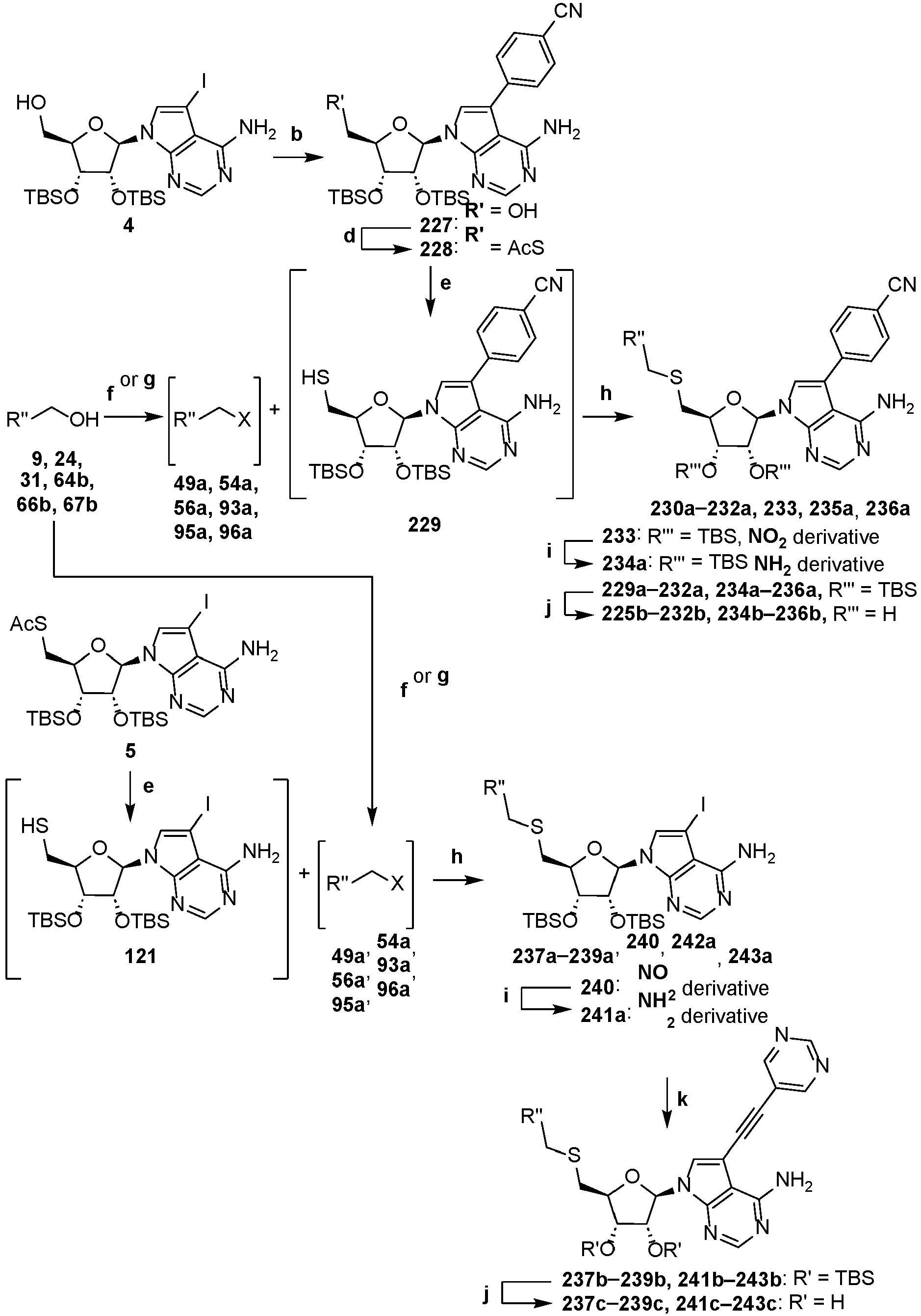

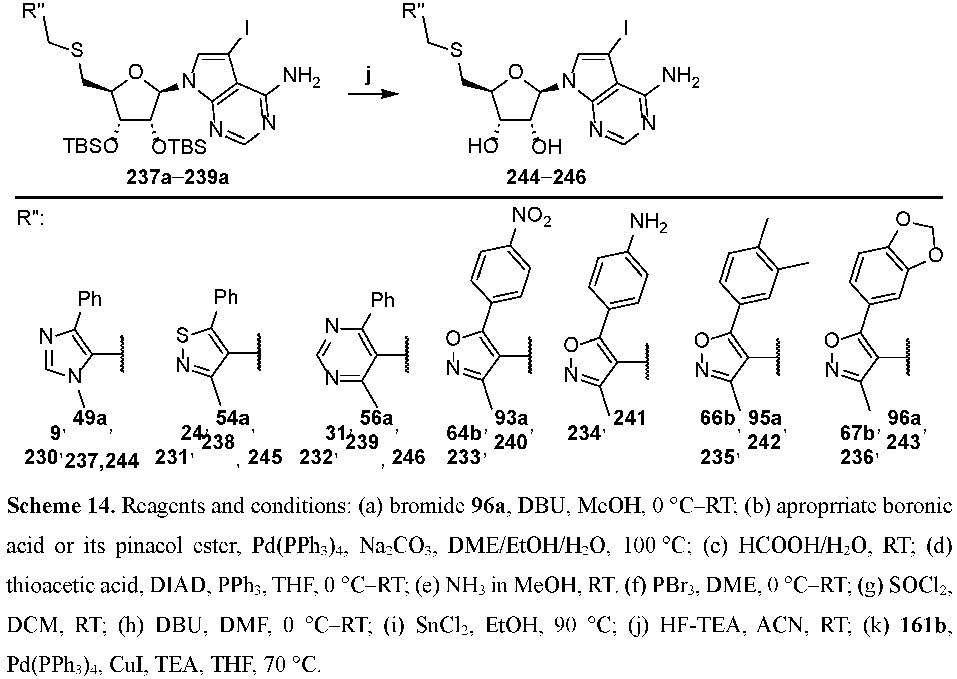

### Example 281

### 1-((3aR,4R,6S,6aS)-6-((((5-(Benzo[d][1,3]dioxol-5-yl)-3-methylisoxazol-4-yl)methyl)thio)methyl)-2,2-dimethyltetrahydrofuro[3,4-d][1,3]dioxol-4-yl)-3-iodo-1H-pyrazolo[3,4-d]pyrimidin-4-amine (224):

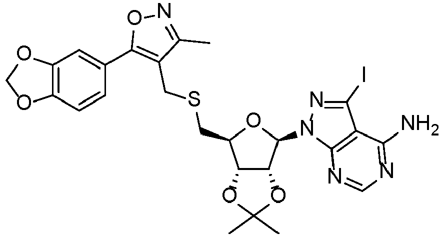

Intermediate **224** was synthesized from modified nucleoside **216** (0.2 g, 0.74 mmol) using the same conditions as for **212**. FCC (10-75 % EtOAc in DCM) afforded iodinated derivative **224** (0.03 g, 0.05 mmol, 14 %). **¹H NMR** (400 MHz, CDCl₃) δ 8.33 (1H, s), 7.26 (1H, dd, *J* = 8.1, 1.7 Hz), 7.22 (1H, d, *J* = 1.6 Hz), 6.90 (1H, d, *J* = 8.1 Hz), 6.52 (1H, d, *J* = 1.5 Hz), 6.03 (4H, bs), 5.38 (1H, dd, *J* = 6.2, 1.5 Hz), 4.98 (1H, dd, *J* = 6.2, 2.5 Hz), 4.39 (1H, td, *J* = 7.3, 2.4 Hz), 3.65 (1H, d, *J* = 12.7 Hz), 3.56 (1H, d, *J* = 12.7 Hz), 2.78 (2H, d, *J* = 7.3 Hz), 2.31 (3H, s), 1.60 (3H, s), 1.40 (3H, s). **¹³C NMR** (101 MHz, CDCl₃) δ 166.0, 160.8, 157.5, 156.8, 154.8, 149.2, 148.3, 122.0, 121.8, 114.1, 109.3, 109.0, 107.6, 104.3, 101.7, 90.1, 88.9, 87.3, 84.3, 84.3, 35.1, 29.8, 27.1, 25.6, 25.0, 10.3. **LRMS** Calculated for C₂₅H₂₆IN₆O₆S, [M+H]⁺ m/z: 665.1, [M+H]⁺, found 665.0.

### Example 282

### 2-(4-(4-Amino-1-((3aR,4R,6S,6aS)-6-((((5-(benzo[d][1,3]dioxol-5-yl)-3-methylisoxazol-4-yl)methyl)thio)methyl)-2,2-dimethyltetrahydrofuro[3,4-d][1,3]dioxol-4-yl)-1H-pyrazolo[3,4-d]pyrimidin-3-yl)phenyl)acetonitrile (225):

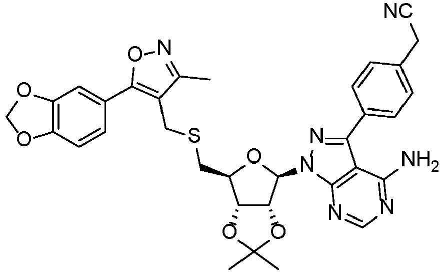

SAH analogue **225** was prepared from **224** (0.03 g, 0.05 mmol) and (4-cyanophenyl)boronic acid (0.02 g, 0.10 mmol) using the same methodology as for **143a**. FCC (15-100 % EtOAc in cyclohexane) furnished derivative **225** (0.03 g, 0.04 mmol, 79 %).**¹H NMR** (400 MHz, CDCl₃) δ 8.39 (1H, s), 7.66-7.62 (2H, m), 7.53-7.48 (2H, m), 7.24 (1H, dd, *J* = 8.1, 1.8 Hz), 7.19 (1H, dd, *J* = 1.7, 0.3 Hz), 6.87 (1H, dd, *J* = 8.1, 0.3 Hz), 6.67 (1H, d, *J* = 1.7 Hz), 6.02 (1H, d, *J* = 1.4 Hz), 6.01 (1H, d, *J* = 1.4 Hz), 5.58 (2H, bs), 5.46 (1H, dd, *J* = 6.2, 1.7 Hz), 5.01 (1H, dd, *J* = 6.2, 2.5 Hz), 4.42 (1H, td, *J* = 7.4, 2.6 Hz), 3.85 (2H, s), 3.65 (1H, d, *J* = 12.7 Hz), 3.54 (1H, d, *J* = 12.7 Hz), 2.85-2.81 (2H, m), 2.27 (3H, s), 1.62 (3H, s), 1.41 (3H, s). **¹³C NMR** (101 MHz, CDCl₃) δ 166.0, 160.7, 157.8, 156.7, 155.7, 149.2, 148.3, 144.9, 132.8, 131.6, 129.3, 129.2, 121.9, 121.7, 114.1, 109.3, 109.0, 107.6, 101.7, 99.0, 90.1, 87.0, 84.4, 84.3, 84.3, 35.0, 27.2, 25.6, 24.8, 23.7, 10.2. **LRMS** Calculated for C₂₅H₂₆IN₆O₆S, [M+H]⁺ m/z: 653.2, [M+H]⁺, found 653.2.

### Example 283

### 2-(4-(4-Amino-1-((2R,3R,4S,5S)-5-((((5-(benzo[d][1,3]dioxol-5-yl)-3-methylisoxazol-4-yl)methyl) thio)methyl)-3,4-dihydroxytetrahydrofuran-2-yl)-1H-pyrazolo[3,4-d]pyrimidin-3-yl)phenyl)acetonitrile (226):

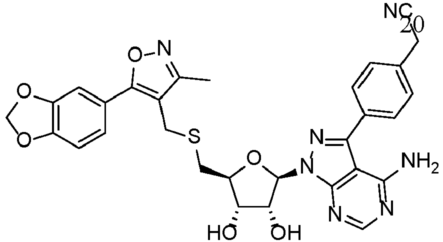

Title compound **226** was synthesized from **225** (0.02 g, 0.03 mmol) in analogous fashion to **128**. Reverse-phase FCC (5-75 % MeCN in H₂O) provided **226** (0.01 g, 0.02 mmol, 53 %). **¹H NMR** (400 MHz, CDCl₃) δ 8.29 (1H, s), 7.64 (2H, d, *J* = 8.1), 7.50 (2H, d, *J* = 8.2), 7.26-7.22 (2H, m), 7.00 (1H, d, *J* = 8.0 Hz), 6.23 (1H, d, *J* = 4.2 Hz), 6.09 (2H, s), 5.53 (1H, bs), 5.30 (1H, bs), 4.66 (1H, t, *J* = 4.7 Hz), 4.27 (1H, t, *J* = 4.8 Hz), 4.13 (2H, s), 4.09 (1H, dt, *J* = 7.5, 4.7 Hz), 3.77 (1H, d, *J* = 13.1 Hz), 3.73 (1H, d, *J* = 13.1 Hz), 2.91 (1H, dd, *J* = 13.9, 4.6 Hz), 2.80 (1H, dd, *J* = 13.9, 7.7 Hz), 2.16 (3H, s). **¹³C NMR** (101 MHz, DMSO-*d₆*) δ 164.8, 160.7, 158.3, 156.3, 155.7, 148.9, 148.1, 144.8, 132.1, 132.1, 129.1, 129.0, 121.6, 121.1, 119.3, 110.1, 109.1, 106.8, 101.9, 97.9, 88.4, 83.9, 73.5, 73.4, 35.1, 24.6, 22.5, 9.7. **HRMS** (ESI) Calculated for C₃₀H₂₈N₇O₆S, [M+H]⁺ m/z: 614.1816, [M+H]⁺, found 614.1815.

### Example 284

### 4-(4-Amino-7-((2R,3R,4R,5R)-3,4-bis((tert-butyldimethylsilyl)oxy)-5-(hydroxymethyl)tetrahydrofuran-2-yl)-7H-pyrrolo[2,3-d]pyrimidin-5-yl)benzonitrile (227):

Substituted nucleoside **227** was prepared from intermediate **4** (3 g, 4.83 mmol) and (4-cyanophenyl)boronic acid (1.3 g, 8.85 mmol) using the same methodology as for **143a**. FCC (10-90 % 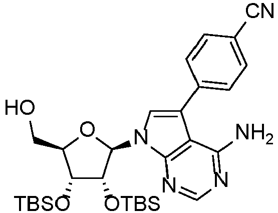 EtOAc in DCM) furnished derivative **227** (2.12 g, 3.56 mmol, 74 %). **¹H NMR** (400 MHz, DMSO-*d₆*) δ 8.17 (1H, s), 7.95-7.90 (2H, m), 7.75 (1H, s), 7.62-7.58 (2H, m),6.35 (2H, bs), 6.15 (1H, d, *J* = 7.2 Hz), 5.44 (1H, dd, *J* = 6.4, 5.1 Hz), 4.73 (1H, dd, *J* = 7.2, 4.5 Hz), 4.26 (1H, dd, *J* = 4.5, 0.9 Hz), 3.95 (1H, t, *J* = 3.3 Hz), 3.69 (1H, dt, *J* = 11.9, 4.7 Hz), 3.57 (1H, ddd, *J* = 11.9, 6.5, 3.3 Hz), 0.93, 0.67 (18H, 2×s), 0.13, 0.11, -0.13, -0.39 (12H, 4×s). **¹³C NMR** (101 MHz, DMSO-*d₆*) δ 157.6, 152.1, 151.7, 139.5, 133.0, 131.7, 131.6, 129.1, 122.9, 119.3, 115.6, 109.2, 100.3, 86.8, 86.7, 75.1, 73.3, 61.6, 26.0, 25.7, 18.0, 17.7, -4.47, -4.52, -4.6, -5.5. **LRMS** Calculated for C₃₀H4₆N₅O₄Si₂, [M+H]⁺ m/z: 596.3, [M+H]⁺, found 596.3.

### Example 285

### S-(((2S,3R,4R,5R)-5-(4-Amino-5-(4-cyanophenyl)-7H-pyrrolo[2,3-d]pyrimidin-7-yl)-3,4-bis((tertbutyldimethylsilyl)oxy)tetrahydrofuran-2-yl)methyl) thioacetate (228):

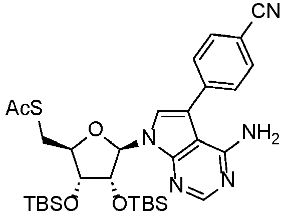

Analogue **228** was prepared from **227** (2.01 g, 3.37 mmol) according to the same procedure as for 5. FCC (10-80 % EtOAc in cyclohexane) afforded **228** (1.73 g, 2.64 mmol, 78 %). **¹H NMR** (400 MHz, DMSO-*d₆*) δ 8.18 (1H, s), 7.96-7.91 (2H, m), 7.77 (1H, s), 7.67-7.62 (2H, m), 6.33 (2H, bs), 6.19 (1H, d, *J* = 7.5 Hz), 4.88 (1H, dd, *J* = 7.5, 4.4 Hz), 4.16 (1H, d, *J* = 4.6 Hz), 3.90 (1H, t, *J* = 7.3 Hz), 3.44 (1H, dd, *J* = 13.9, 7.5 Hz), 3.20 (1H, dd, *J* = 13.9, 7.1 Hz), 2.34 (3H, s), 0.92, 0.66 (18H, 2×s), 0.13, 0.12, -0.09, -0.37 (12H, 4×s). **¹³C NMR** (101 MHz, DMSO-*d₆*) δ 195.2, 157.5, 152.3, 152.1, 139.4, 133.0, 129.1, 122.8, 119.3, 115.9, 109.2, 100.1, 86.3, 84.2, 74.6, 74.1, 31.3, 30.6, 25.9, 25.6, 18.0, 17.7, -4.5, -4.6, -4.6, -5.5. **LRMS** Calculated for C₃₂H₄₈ N₅O₄SSi₂, [M+H]⁺ m/z: 654.3, [M+H]⁺, found 654.3.

### Example 286

### 4-(4-Amino-7-((2R,3R,4R,5S)-3,4-bis((tert-butyldimethylsilyl)oxy)-5-(mercaptomethyl)tetrahydrofuran-2-yl)-7H-pyrrolo[2,3-d]pyrimidin-5-yl)benzonitrile (229):

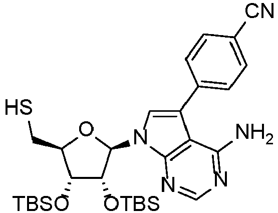

Thiol **229** was prepared from protected analogue **228** (0.2 g, 0.31 mmol) following the same conditions as for **46**. The crude thiol **229** was, after co-evaporation, used without further purification in the next reaction. LRMS Calculated for C₃₀H₄₆N₅O₃SSi₂, [M+H]⁺ m/z: 612.3, [M+H]⁺, found 612.2.

### Example 287

### 4-(4-Amino-7-((2R,3R,4R,5S)-3,4-bis((tert-butyldimethylsilyl)oxy)-5-((((1-methyl-4-phenyl-1H-imidazol-5-yl)methyl)thio)methyl)tetrahydrofuran-2-yl)-7H-pyrrolo[2,3-d]pyrimidin-5-yl)benzonitrile (230a):

**230a** was synthesized from chloride **49a** (obtained from **9** (0.06 g, 0.31 mmol)) and thiol **229** (provided 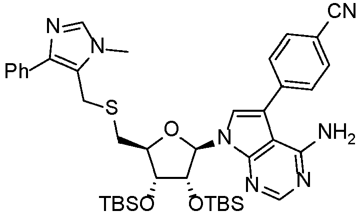 from **228** (0.2 g, 0.31 mmol)) using the same procedure as for **49b**. FCC (2-15 % EtOH in DCM) provided **230a** (0.11 g, 0.13 mmol, 43 %). **¹H NMR** (400 MHz, CDCl₃) δ 8.29 (1H, s), 7.75-7.71 (2H, m), 7.68-7.64 (2H, m), 7.55-7.51 (2H, m), 7.48 (1H, s), 7.37-7.32 (2H, m), 7.26-7.21 (1H, m), 7.14 (1H, s), 6.04 (1H, d, *J* = 5.2 Hz), 5.14 (2H, bs), 4.84 (1H, dd, *J* = 5.3, 4.2 Hz), 4.24-4.17 (2H, m), 4.05 (1H, d, *J* = 13.6 Hz), 3.94 (1H, d, *J* = 13.6 Hz), 3.66 (3H, s), 2.97-2.93 (2H, m), 0.93, 0.79 (18H, 2×s), 0.11, 0.10, -0.06, -0.24 (12H, 4×s). **¹³C NMR** (101 MHz, CDCl₃) δ 156.7, 152.1, 151.4, 140.5, 139.4, 137.9, 134.5, 133.0, 129.3, 128.7, 127.4, 127.1, 123.13, 123.10, 118.7, 115.7, 111.1, 101.9, 90.2, 84.4, 74.8, 74.7, 35.0, 31.8, 26.7, 26.0, 25.9, 18.2, 18.1, -4.2, -4.4, -4.5, -4.9. **LRMS** Calculated for C₄₁H₅₆N₇O₃SSi₂, [M+H]⁺ m/z: 782.4, [M+H]⁺, found 782.4.

### Example 288

### 4-(4-Amino-7-((2R,3R,4R,5S)-3,4-bis((tert-butyldimethylsilyl)oxy)-5-((((3-methyl-5-phenylisothiazol-4-yl)methyl)thio)methyl)tetrahydrofuran-2-yl)-7H-pyrrolo[2,3-d]pyrimidin-5-yl)benzonitrile (231a):

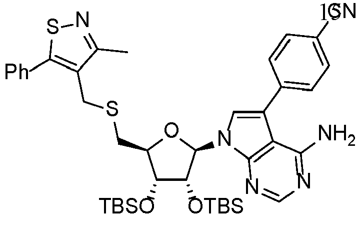

**231a** was prepared from bromide **54a** (obtained from **24** (0.05 g, 0.23 mmol)) and **229** (provided from **228** (0.15 g, 0.23 mmol)) in analogous fashion to **47b**. FCC (10-50 % acetone in cyclohexane) afforded **231a** (0.12 g, 0.15 mmol, 65 %). **¹H NMR** (400 MHz, CDCl₃) δ 8.25 (1H, s), 7.77-7.72 (2H, m), 7.56-7.51 (2H, m), 7.50-7.46 (2H, m), 7.44-7.38 (3H, m), 7.19 (1H, s), 6.04 (1H, d, *J* = 5.4 Hz), 5.64 (2H, bs), 4.78 (1H, dd, *J* = 5.4, 4.2 Hz), 4.20-4.13 (2H, m), 3.85 (1H, d, *J* = 12.5 Hz), 3.75 (1H, d, *J* = 12.5 Hz), 2.95 (1H, dd, *J* = 13.6, 5.6 Hz), 2.84 (1H, dd, *J* = 13.6, 5.6 Hz), 2.53 (3H, s), 0.92, 0.73 (18H, 2×s), 0.11, 0.09, -0.07, -0.25 (12H, 4×s). **¹³C NMR** (101 MHz, CDCl₃) δ 167.4, 164.0, 156.4, 151.1, 150.7, 139.1, 133.1, 131.1, 129.3, 129.2, 128.9, 128.8, 123.3, 118.7, 116.0, 111.4, 101.6, 90.0, 84.2, 74.9, 74.6, 35.3, 28.8, 26.0, 25.8, 18.4, 18.2, 18.1, -4.2, -4.4, -4.5, -4.9. **LRMS** Calculated for C₄₁H₅₅N₆O₃S₂Si₂, [M+H]⁺ m/z: 799.3, [M+H]⁺, found 799.3.

### Example 289

### 4-(4-Amino-7-((2R,3R,4R,5S)-3,4-bis((tert-butyldimethylsilyl)oxy)-5-((((4-methyl-6-phenylpyrimidin-5-yl)methyl)thio)methyl)tetrahydrofuran-2-yl)-7H-pyrrolo[2,3-d]pyrimidin-5-yl)benzonitrile (232a):

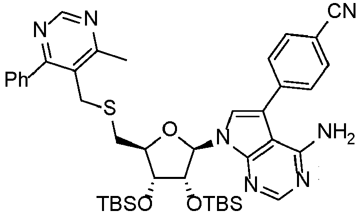

Modified nucleoside **232a** was synthesized from bromide **56a** (obtained from **31** (0.05 g, 0.23 mmol)) and thiol **229** (provided from **228** (0.15 g, 0.23 mmol)) using the same conditions as for **47b**. FCC (10-65 % acetone in cyclohexane) furnished modified derivative **232a** (0.13 g, 0.16 mmol, 71 %). **¹H NMR** (400 MHz, CDCl₃) δ 8.98 (1H, s), 8.22 (1H, s), 7.76-7.73 (2H, m), 7.68-7.64 (2H, m), 7.56-7.52 (2H, m), 7.46-7.41 (3H, m), 7.13 (1H, s), 5.98 (1H, d, *J* = 5.2 Hz), 5.61 (2H, bs), 4.84 (1H, dd, *J* = 5.2, 4.5 Hz), 4.19 (1H, t, *J* = 4.1 Hz), 4.17-4.13 (1H, m), 3.87 (1H, d, *J* = 12.2 Hz), 3.77 (1H, d, *J* = 12.2 Hz), 2.94 (1H, dd, *J* = 13.7, 5.4 Hz), 2.88 (1H, dd, *J* = 13.6, 6.4 Hz), 2.66 (3H, s), 0.92, 0.79 (18H, 2×s), 0.11, 0.11, -0.07, -0.25 (12H, 4×s). **¹³C NMR** (101 MHz, CDCl₃) δ 167.7, 165.8, 156.4, 151.1, 151.0, 139.1, 137.9, 133.1, 129.5, 129.3, 128.9, 128.6, 127.1, 123.4, 118.6, 115.9, 111.4, 101.8, 90.4, 84.2, 74.7, 74.6, 35.9, 32.0, 26.0, 25.8, 22.5, 18.2, 18.1, -4.2, -4.4, -4.9. **LRMS** Calculated for C₄₂H₅₆N₇O₃SSi₂, [M+H]⁺ m/z: 794.4, [M+H]⁺, found 794.4.

### Example 290

### 4-(4-Amino-7-((2R,3R,4R,5S)-3,4-bis((tert-butyldimethylsilyl)oxy)-5-((((3-methyl-5-(4-nitrophenyl)isoxazol-4-yl)methyl)thio)methyl)tetrahydrofuran-2-yl)-7H-pyrrolo[2,3-d]pyrimidin-5-yl)benzonitrile (233):

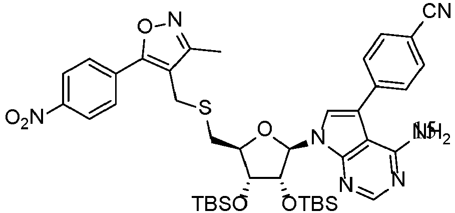

Derivative **233** was prepared from bromide **93a** (obtained from **64b** (0.08 g, 0.35 mmol)) and thiol **229** (provided from **228** (0.2 g, 0.31 mmol)) following the same procedure as for **47b**. FCC (10-70 % EtOAc in DCM) afforded title compound **233** (0.19 g, 0.23 mmol, 77 %). **¹H NMR** (400 MHz, DMSO-*d₆*) δ 8.26 (1H, d, *J* = 8.9 H), 8.16 (1H, s), 8.00 (1H, d, *J* = 8.9 Hz), 7.90 (1H, d, *J* = 8.3 H), 7.72 (1H, s), 7.57 (1H, d, *J* = 8.3 Hz), 6.31 (2H, bs), 6.20 (1H, d, *J* = 7.0 Hz), 4.83 (1H, dd, *J* = 7.0, 4.5 Hz), 4.17 (1H, dd, *J* = 4.3, 1.2 Hz), 4.02-3.97 (1H, m), 3.97 (1H, d, *J* = 13.3 Hz), 3.88 (1H, d, *J* = 13.4 Hz), 3.10 (1H, dd, *J* = 13.9, 7.0 Hz), 2.96 (1H, dd, *J* = 13.9, 7.0 Hz), 2.29 (3H, s), 0.90, 0.67 (18H, 2×s), 0.10, -0.10, -0.36 (12H, 3×s). **¹³C NMR** (101 MHz, DMSO-*d₆*) δ 162.7, 161.2, 157.5, 152.3, 152.1, 147.9, 139.4, 132.9, 132.8, 129.0, 128.1, 124.4, 122.8, 119.3, 115.9, 113.8, 109.2, 100.1, 86.6, 84.7, 74.8, 74.1, 34.5, 25.9, 25.6, 24.2, 17.9, 17.7, 9.8, -4.45, -4.53, -5.4. **LRMS** Calculated for C₄₁H₅₄N₇O₆SSi₂, [M+H]⁺ m/z: 828.3, [M+H]⁺, found 828.3.

### Example 291

### 4-(4-Amino-7-((2R,3R,4R,5S-5-((((5-(4-aminophenyl)-3-methylisoxazol-4-yl)methyl)thio)methyl)-3,4-bis((tert-butyldimethylsilyl)oxy)tetrahydrofuran-2-yl)-7H-pyrrolo[2,3-d]pyrimidin-5-yl)benzonitrile (234a):

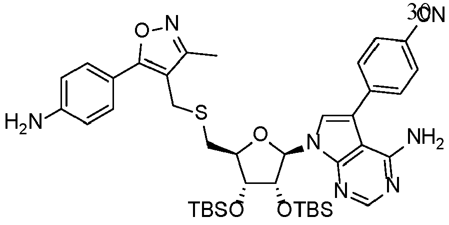

The solution of nitro derivative **233** (0.19 g, 0.23 mmol) and SnCl₂ (0.17 g, 0.91 mmol) in EtOH (2 mL) was refluxed for 1 h. The solvent was removed in vacuo and the residue dissolved in EtOAc (100 mL), washed with H₂O and dried over Na₂SO₄. FCC (10-80 % EtOAc in DCM) furnished desired product **234a** (0.15 g, 0.18 mmol, 79 %). **¹H NMR** (400 MHz, DMSO-*d₆*) δ 8.17 (1H, s), 7.92 (1H, d, *J* = 8.3 H), 7.73 (1H, s), 7.60 (1H, d, *J* = 8.3 Hz), 7.41 (1H, d, *J* = 8.6 H), 6.63 (1H, d, *J* = 8.6 Hz), 6.32 (2H, bs), 6.21 (1H, d, *J* = 7.3 Hz), 5.67 (2H, bs), 4.84 (1H, dd, *J* = 7.2, 4.4 Hz), 4.19 (1H, d, *J* = 4.3 Hz), 4.02 (1H, t, *J* = 7.0 Hz), 3.84 (1H, d, *J* = 13.1 Hz), 3.75 (1H, d, *J* = 13.2 Hz), 3.12 (1H, dd, *J* = 13.8, 7.8 Hz), 2.95 (1H, dd, *J* = 13.8, 6.4 Hz), 2.19 (3H, s), 0.93, 0.66 (18H, 2×s), 0.14, 0.12, -0.10, -0.37 (12H, 3×s). **¹³C NMR** (101 MHz, DMSO-*d₆*) δ 166.2, 160.2, 157.5, 152.3, 152.2, 150.9, 139.4, 133.0, 129.1, 128.0, 122.7, 119.3, 115.9, 114.4, 113.8, 109.2, 107.5, 100.1, 86.4, 85.0, 74.8, 74.2, 34.7, 26.0, 25.6, 25.2, 18.0, 17.7, 9.8, -4.4, -4.4, -4.5, -5.4. LRMS Calculated for C₄₁H₅₆N₇O₄SSi₂, [M+H]⁺ m/z: 798.4, [M+H]⁺, found 798.4.

### Example 292

### 4-(4-Amino-7-((2R,3R,4R,5S)-3,4-bis((tert-butyldimethylsilyl)oxy)-5-((((5-(3,4-dimethylphenyl)-3-methylisoxazol-4-yl)methyl)thio)methyl)tetrahydrofuran-2-yl)-7H-pyrrolo[2,3-d]pyrimidin-5-yl)benzonitrile (235a):

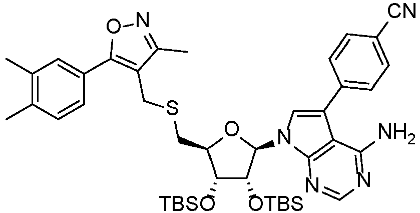

SAH analogue **235a** was prepared from bromide **95a** (obtained from **66b** (0.08 g, 0.35 mmol)) and thiol **229** (provided from **228** (0.2 g, 0.31 mmol)) using the same conditions as for **47b**. FCC (10-70 % EtOAc in DCM) afforded derivative **235a** (0.22 g, 0.27 mmol, 87 %). **¹H NMR** (400 MHz, DMSO-*d₆*) δ 8.17 (1H, s), 7.90 (1H, d, *J* = 8.4 Hz), 7.73 (1H, s), 7.57 (1H, d, *J* = 8.3 Hz), 7.49 (1H, d, *J* = 1.3 Hz), 7.43 (1H, dd, *J* = 7.7, 1.6 Hz), 7.18 (1H, d, *J* = 7.9 Hz), 6.31 (2H, bs), 6.21 (1H, d, *J* = 7.2 Hz), 4.82 (1H, dd, *J* = 7.1, 4.4 Hz), 4.17 (1H, dd, *J* = 4.2, 1.0 Hz), 4.00 (1H, td, *J* = 6.9, 1.3 Hz), 3.89 (1H, d, *J* = 13.2 Hz), 3.79 (1H, d, *J* = 13.2 Hz), 3.09 (1H, dd, *J* = 13.9, 7.2 Hz), 2.95 (1H, dd, *J* = 13.9, 6.9 Hz), 2.25 (3H, s), 2.25 (6H, s), 0.91, 0.67 (18H, 2×s), 0.11, -0.10, -0.36 (12H, 3×s). **¹³C NMR** (101 MHz, DMSO-*d₆*) δ 165.4, 160.5, 157.5, 152.3, 152.1, 139.4, 139.0, 137.4, 132.9, 130.3, 129.1, 127.7, 124.9, 124.3, 122.7, 119.3, 115.9, 110.3, 109.2, 100.1, 86.4, 84.9, 74.8, 74.2, 34.5, 25.9, 25.6, 24.6, 19.5, 17.9, 17.7, 9.8, -4.5, -4.5, -5.4. **LRMS** Calculated for C₄₃H₅₉N₆O₄SSi₂, [M+H]⁺ m/z: 811.4, [M+H]⁺, found 811.4.

### Example 293

### 4-(4-Amino-7-((2R,3R,4R,5S)-5-((((5-(benzo[d][1,3]dioxol-5-yl)-3-methylisoxazol-4-yl)methyl)thio)methyl)-3,4-bis((tert-butyldimethylsilyl)oxy)tetrahydrofuran-2-yl)-7H-pyrrolo[2,3-d]pyrimidin-5-yl)benzonitrile (236a):

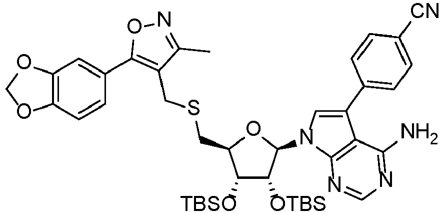

Modified nucleoside **236a** was synthesized from bromide **96a** (obtained from **67b** (0.08 g, 0.35 mmol)) and thiol **229** (provided from intermediate **228** (0.2 g, 0.31 mmol)) in analogous fashion to **47b**. FCC (10-70 % EtOAc in DCM) gave **236a** (0.18 g, 0.21 mmol, 69 %). **¹H NMR** (400 MHz, DMSO-*d₆*) δ 8.17 (1H, s), 7.91 (1H, d, *J* = 8.1 Hz), 7.73 (1H, s), 7.59 (1H, d, *J* = 8.1 Hz), 7.28-7.19 (2H, m), 6.98 (1H, d, *J* = 8.5 Hz), 6.32 (2H, bs), 6.21 (1H, d, *J* = 7.1 H), 6.08 (2H, s), 4.83 (1H, dd, *J* = 6.8, 4.4 Hz), 4.18 (1H, d, *J* = 3.7 Hz), 3.99 (1H, t, *J* = 6.8 Hz), 3.87 (1H, d, *J* = 13.2 Hz), 3.77 (1H, d, *J* = 13.2 Hz), 3.10 (1H, dd, *J* = 13.8, 7.4 Hz), 2.94 (1H, dd, *J* = 13.8, 6.6 Hz), 2.23 (3H, s), 0.92, 0.67 (18H, 2×s), 0.12, 0.11, -0.10, -0.37 (12H, 4×s). **¹³C NMR** (101 MHz, DMSO-*d₆*) δ 164.9, 160.6, 157.5, 152.3, 152.1, 149.0, 148.2, 139.4, 132.9, 129.1, 122.8, 121.6, 121.1, 119.3, 115.9, 110.0, 109.2, 109.1, 106.9, 101.9, 100.1, 86.5, 84.8, 74.8, 74.2, 34.6, 25.9, 25.6, 24.6, 17.9, 17.7, 9.8, -4.45, -4.49, -4.52, -5.4. **LRMS** Calculated for C₄₂H₅₅N₆O₆SSi₂, [M+H]⁺ m/z: 827.3, [M+H]⁺, found 827.3.

### Example 294

### 4-(4-Amino-7-((2R,3R,4S,5S)-3,4-dihydroxy-5-((((1-methyl-4-phenyl-1H-imidazol-5-yl)methyl)thio)methyl)tetrahydrofuran-2-yl)-7H-pyrrolo[2,3-d]pyrimidin-5-yl)benzonitrile (230b):

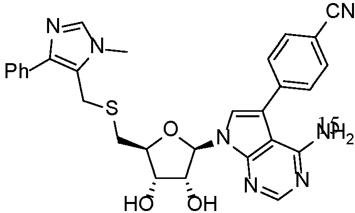

Derivative **230b** was prepared from **230a** (0.11 g, 0.13 mmol) in analogous fashion to **47c**. Reverse-phase FCC (0-70 % MeCN in H₂O) furnished **230b** (0.07 g, 0.13 mmol, 94 %). **¹H NMR** (400 MHz, DMSO-*d₆*) δ 8.19 (1H, s), 7.91-7.87 (2H, m), 7.71 (1H s), 7.68-7.64 (3H, m), 7.64-7.59 (2H, m), 7.38-7.31 (2H, m), 7.23-7.17 (1H, m), 6.34 (2H, bs), 6.19 (1H, d, *J* = 6.0 Hz), 5.46 (1H, d, *J* = 6.3 Hz), 5.31 (1H, d, *J* = 5.0 Hz), 4.57 (1H, q, *J* = 5.8 Hz), 4.11-4.04 (3H, m), 4.02 (1H, d, *J* = 13.3 Hz), 3.63 (3H, s), 3.00 (1H, dd, *J* = 13.9, 5.8 Hz), 2.93 (1H, dd, *J* = 13.9, 6.9 Hz). **¹³C NMR** (101 MHz, DMSO-*d₆*) δ 157.6, 152.3, 152.0, 139.4, 138.3, 138.3, 134.9, 132.9, 129.1, 128.6, 126.6, 126.5, 123.6, 122.6, 119.3, 115.8, 109.0, 100.1, 87.0, 83.5, 73.3, 72.9, 34.9, 31.2, 25.8. **HRMS** (ESI) Calculated for C₂₉H₂₈N₇O₃S, [M+H]⁺ m/z: 554.1969, [M+H]⁺, found 554.1968.

### Example 295

### 4-(4-Amino-7-((2R,3R,4S,5S)-3,4-dihydroxy-5-((((3-methyl-5-phenylisothiazol-4-yl)methyl)thio) methyl)tetrahydrofuran-2-yl)-7H-pyrrolo[2,3-d]pyrimidin-5-yl)benzonitrile (231b):

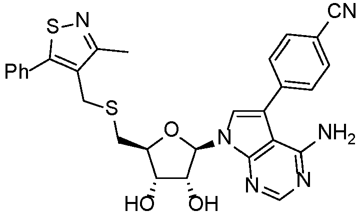

Title compound **231b** was synthesized from modified nucleoside **231a** (0.11 g, 0.14 mmol) using the same methodology as for **47c**. Reverse phase FCC (0-60 % MeCN in H₂O) afforded **231b** (0.06 g, 0.11 mmol, 78 %). **¹H NMR** (400 MHz, DMSO-*d₆*) δ 8.19 (1H, s), 7.93-7.87 (2H, m), 7.68 (1H s), 7.65-7.59 (3H, m), 7.56-7.52 (2H, m), 7.51-7.41 (3H, m), 6.33 (2H, bs), 6.16 (1H, d, *J* = 6.0 Hz), 5.43 (1H, d, *J* = 6.3 Hz), 5.27 (1H, d, *J* = 5.1 Hz), 4.55 (1H, q, *J* = 5.9 Hz), 4.06 (1H, td, *J* = 5.2, 3.6 Hz), 3.98 (1H, td, *J* = 6.1, 3.8 Hz), 3.87 (1H, d, *J* = 12.3 Hz), 3.81 (1H, d, *J* = 12.3 Hz), 2.94 (1H, dd, *J* = 13.9, 5.8 Hz), 2.87 (1H, dd, *J* = 13.9, 7.1 Hz), 2.46 (3H, s). **¹³C NMR** (101 MHz, DMSO-*d₆*) δ 167.8, 162.7, 157.6, 152.3, 152.0, 139.4, 132.9, 130.5, 129.6, 129.5, 129.3, 129.1, 128.4, 122.6, 119.3, 115.8, 109.0, 100.1, 86.9, 83.4, 73.2, 72.9, 35.2, 27.8, 18.0. **HRMS** (ESI) Calculated for C₂₉H₂₇N₆O₃S₂, [M+H]⁺ m/z: 571.1581, [M+H]⁺, found 571.1576.

### Example 296

### 4-(4-Amino-7-((2R,3R,4S,5S)-3,4-dihydroxy-5-((((4-methyl-6-phenylpyrimidin-5-yl)methyl)thio)methyl)tetrahydrofuran-2-yl)-7H-pyrrolo[2,3-d]pyrimidin-5-yl)benzonitrile (232b):

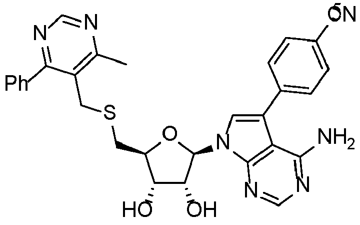

SAH analogue **232b** was prepared from derivative **232a** (0.12 g, 0.15 mmol) following the same conditions as for **47c**. Reverse-phase FCC (0-60 % MeCN in H₂O) provided **232b** (0.05 g, 0.09 mmol, 60 %). **¹H NMR** (400 MHz, DMSO-*d₆*) δ 8.94 (1H, s), 8.18 (1H, s), 7.93-7.89 (2H, m), 7.69-7.64 (3H, m), 7.64-7.60 (3H, m), 7.48-7.43 (3H, m), 6.34 (2H, bs), 6.15 (1H, d, *J* = 5.9 Hz), 5.44 (1H, d, *J* = 6.2 Hz), 5.27 (1H, d, *J* = 5.2 Hz), 4.53 (1H, q, *J* = 5.8 Hz), 4.04 (1H, td, *J* = 5.2, 3.7 Hz), 3.94 (1H, ddd, *J* = 7.2, 5.6, 3.7 Hz), 3.86 (1H, d, *J* = 12.1 Hz), 3.80 (1H, d, *J* = 12.1 Hz), 2.93 (1H, dd, *J* = 13.9, 5.6 Hz), 2.85 (1H, dd, *J* = 13.9, 7.3 Hz), 2.61 (3H, s). **¹³C NMR** (101 MHz, DMSO-*d₆*) δ 167.4, 164.4, 157.4, 156.1, 152.1, 151.8, 139.2, 137.5, 132.7, 129.3, 128.9, 128.6, 128.3, 126.8, 122.4, 119.2, 115.6, 108.9, 99.9, 86.8, 83.0, 73.0, 72.7, 35.5, 30.8, 21.9. **HRMS** (ESI) Calculated for C₃₀H₂₈N₇O₃S, [M+H]⁺ m/z: 566.1969, [M+H]⁺, found 566.1966.

### Example 297

### 4-(4-Amino-7-((2R,3R,4S,5S)-5-((((5-(4-aminophenyl)-3-methylisoxazol-4-yl)methyl)thio)methyl)-3,4-dihydroxytetrahydrofuran-2-yl)-7H-pyrrolo[2,3-d]pyrimidin-5-yl)benzonitrile (234b):

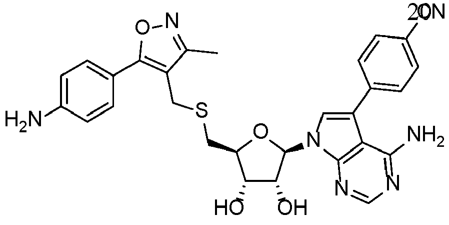

Analogue **234b** was prepared from **234a** (0.14 g, 0.17 mmol) using the same procedure as for **47c**. Reverse- phase FCC (5-70 % MeCN in H₂O) gave **234b** (0.09 g, 0.15 mmol, 90 %). **¹H NMR** (400 MHz, DMSO-*d₆*) δ 8.19 (1H, s), 7.90 (2H, d, *J* = 8.4 Hz), 7.71 (1H, s), 7.63 (2H, d, *J* = 8.4 Hz), 7.44 (2H, d, *J* = 8.7 Hz), 6.45 (2H, d, *J* = 8.7 Hz), 6.34 (2H, bs), 6.19 (1H, d, *J* = 5.9 Hz), 5.67 (2H, bs), 5.45 (1H, d, *J* = 6.2 Hz), 5.31 (1H, d, *J* = 5.1 Hz), 4.56 (1H, q, *J* = 5.9 Hz), 4.13-4.03 (2H, m), 3.79 (1H, d, *J* = 12.9 Hz), 3.74 (1H, d, *J* = 12.9 Hz), 2.97 (1H, dd, *J* = 13.9, 5.8 Hz), 2.89 (1H, dd, *J* = 13.9, 6.7 Hz), 2.17 (3H, s). **¹³C NMR** (101 MHz, DMSO-*d₆*) δ 166.1, 160.3, 157.6, 152.4, 152.0, 150.8, 139.4, 132.9, 129.1, 128.0, 122.6, 119.4, 115.8, 114.5, 113.9, 109.0, 107.6, 100.1, 87.0, 83.6, 73.3, 72.9, 34.8, 25.0, 9.7. **HRMS** (ESI) Calculated for C₂₉H₂₈N₇O₄S, [M+H]⁺ m/z: 570.1918, [M+H]⁺, found 570.1914.

### Example 298

### 4-(4-Amino-7-((2R,3R,4S,5S)-5-((((5-(3,4-dimethylphenyl)-3-methylisoxazol-4-yl)methyl)thio)methyl)-3,4-dihydroxytetrahydrofuran-2-yl)-7H-pyrrolo[2,3-d]pyrimidin-5-yl)benzonitrile (235b):

**235b** was prepared from **235a** (0.18 g, 0.22 mmol) in analogous fashion to **47c**. Reverse-phase FCC 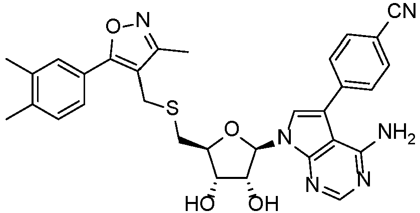 (5-70 % MeCN in H₂O) afforded **235b** (0.1 g, 0.17 mmol, 76 %). **¹H NMR** (400 MHz, DMSO-*d₆*) δ 8.19 (1H, s), 7.89 (1H, d, *J* = 8.2 Hz), 7.70 (1H, s), 7.61 (1H, d, *J* = 8.2 Hz), 7.52 (1H, s), 7.46 (1H, d, *J* = 7.7 Hz), 7.21 (1H, d, *J* = 7.9 Hz), 6.33 (2H, bs), 6.20 (1H, d, *J* = 5.8 Hz), 5.46 (1H, d, *J* = 6.2 Hz), 5.30 (1H, d, *J* = 4.9 Hz), 4.55 (1H, q, *J* = 5.4 Hz), 4.13-4.01 (2H, m), 3.85 (1H, d, *J* = 13.0 Hz), 3.79 (1H, d, *J* = 13.0 Hz), 2.98 (1H, dd, *J* = 13.9, 5.3 Hz), 2.90 (1H, dd, *J* = 13.9, 6.9 Hz), 2.24 (3H, s), 2.23 (6H, s). **¹³C NMR** (101 MHz, DMSO-*d₆*) δ 165.2, 160.6, 157.6, 152.3, 152.0, 139.4, 139.0, 137.5, 132.9, 130.3, 129.1, 127.6, 124.9, 124.2, 122.5, 119.3, 115.8, 110.4, 109.0, 100.1, 87.0, 83.5, 73.3, 72.9, 34.8, 24.6, 19.5, 19.5, 9.7. **HRMS** (ESI) Calculated for C₃₁H₃₁N₆O₄S, [M+H]⁺ m/z: 583.2122, [M+H]⁺, found 583.2117.

### Example 299

4-(4-Amino-7-((2*R*,3*R*,4*S*,5*S*-5-((((5-(benzo[*d*][1,3]dioxol-5-yl)-3-methylisoxazol-4-yl)methyl)thio) methyl)-3,4-dihydroxytetrahydrofuran-2-yl)-7*H*-pyrrolo[2,3-*d*]pyrimidin-5-yl)benzonitrile (**236b**): **236b** was synthesized from **236a** (0.17 g, 0.20 mmol) following the same procedure as for **47c**. Reverse- 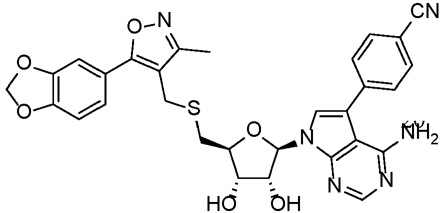 phase FCC (5-60 % MeCN in H₂O) furnished **236b** (0.1 g, 0.16 mmol, 82 %). **¹H NMR** (400 MHz, DMSO-*d₆*) δ 8.19 (1H, s), 7.90 (1H, d, *J* = 8.4 Hz), 7.70 (1H, s), 7.62 (1H, d, *J* = 8.4 Hz), 7.30-7.24 (2H, m), 7.01 (1H, dd, *J* = 7.8, 0.6 Hz), 6.33 (2H, bs), 6.18 (1H, d, *J* = 5.9 Hz), 6.09 (2H, s), 5.46 (1H, d, *J* = 6.2 Hz), 5.29 (1H, d, *J* = 5.1 Hz), 4.55 (1H, q, *J* = 5.3 Hz), 4.11-4.00 (2H, m), 3.81 (1H, d, *J* = 13.0 Hz), 3.77 (1H, d, *J* = 13.1 Hz), 2.97 (1H, dd, *J* = 13.9, 5.6 Hz), 2.89 (1H, dd, *J* = 13.9, 7.0 Hz), 2.21 (3H, s). **¹³C NMR** (101 MHz, DMSO-*d₆*) δ 164.8, 160.7, 157.6, 152.3, 152.0, 149.0, 148.2, 139.4, 132.9, 129.1, 122.6, 121.6, 121.1, 119.3, 115.8, 110.1, 109.1, 109.0, 106.8, 101.9, 100.1, 87.1, 83.4, 73.3, 72.9, 34.8, 24.5, 9.7. **HRMS** (ESI) Calculated for C₃₀H₂₇N₆O₆S, [M+H]⁺ m/z: 599.1707, [M+H]⁺, found 599.1702.

### Example 300

### 7-((2R,3R,4R,5S)-3,4-bis((tert-Butyldimethylsilyl)oxy)-5-((((1-methyl-4-phenyl-1H-imidazol-5-yl)methyl)thio)methyl)tetrahydrofuran-2-yl)-5-iodo-7H-pyrrolo[2,3-d]pyrimidin-4-amine (237a):

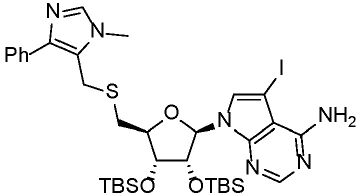

Intermediate **237a** was prepared from chloride **49a** (obtained from 9 (0.1 g, 0.53 mmol)) and thiol **121** (provided from **5** (0.36 g, 0.53 mmol)) using the same conditions as for **49b**. FCC (2-15 % EtOH in DCM) provided iodo derivative **237a** (0.19 g, 0.24 mmol, 46 %). **¹H NMR** (400 MHz, DMSO-*d₆*) δ 8.10 (1H, s), 7.72-7.63 (4H, m), 7.39-7.32 (2H, m), 7.26-7.20 (1H, m), 6.66 (2H, bs), 6.09 (1H, d, *J* = 7.2 Hz), 4.74 (1H, dd, *J* = 7.2, 4.4 Hz), 4.15 (1H, dd, *J* = 4.4, 1.5 Hz), 4.12 (1H, d, *J* = 13.5 Hz), 4.03 (1H, d, *J* = 13.4 Hz), 3.96 (1H, td, *J* = 7.1, 1.5 Hz), 3.66 (3H, s), 3.09 (1H, dd, *J* = 13.7, 7.3 Hz), 2.96 (1H, dd, *J* = 13.7, 6.9 Hz), 0.91, 0.67 (18H, 2×s), 0.12, 0.11, -0.10, -0.39 (12H, 4×s). **¹³C NMR** (101 MHz, DMSO-*d₆*) δ 157.4, 152.3, 150.8, 138.7, 138.5, 135.0, 128.6, 127.4, 126.7, 126.5, 123.4, 103.5, 86.32, 84.9, 74.7, 74.1, 52.7, 34.6, 31.3, 25.9, 25.6, 17.9, 17.7, -4.45, -4.53, -5.4. **LRMS** Calculated for C₃₄H₅₂IN₆O₃SSi₂, [M+H]⁺ m/z: 807.2, [M+H]⁺, found 807.3.

### Example 301

### 7-((2R,3R,4R,5S)-3,4-bis((tert-Butyldimethylsilyl)oxy)-5-((((3-methyl-5-phenylisothiazol-4-yl)methyl)thio)methyl)tetrahydrofuran-2-yl)-5-iodo-7H-pyrrolo[2,3-d]pyrimidin-4-amine (238a):

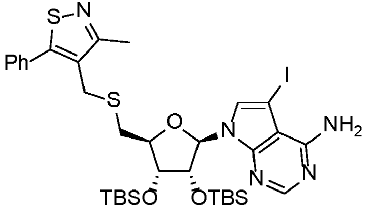

**238a** was synthesized from bromide **54a** (obtained from **24** (0.09 g, 0.44 mmol)) and thiol **121** (provided from **5** (0.3 g, 0.44 mmol)) in analogous fashion to **47b**. FCC (10-50 % EtOAc in cyclohexane) afforded **238a** (0.26 g, 0.31 mmol, 71 %). **¹H NMR** (400 MHz, CDCl₃) δ 8.20 (1H, s), 7.51-7.40 (5H, m), 7.17 (1H, s), 5.94 (1H, d, *J* = 5.0 Hz), 5.82 (2H, bs), 4.68 (1H, dd, *J* = 5.0, 4.4 Hz), 4.14 (1H, t, *J* = 4.2 Hz), 4.13-4.05 (1H, m), 3.83 (1H, d, *J* = 12.6 Hz), 3.74 (1H, d, *J* = 12.6 Hz), 2.89 (1H, dd, *J* = 13.7, 5.6 Hz), 2.80 (1H, dd, *J* = 13.7, 5.8 Hz), 2.56 (3H, s), 0.91, 0.79 (18H, 2×s), 0.10, 0.09, -0.08, -0.24 (12H, 4×s). **¹³C NMR** (101 MHz, CDCl₃) δ 167.5, 164.0, 156.7, 151.6, 150.3, 131.1, 129.3, 129.2, 128.94, 128.91, 128.1, 104.9, 89.9, 83.9, 74.9, 74.5, 50.6, 35.2, 28.9, 26.0, 25.9, 18.5, 18.2, 18.1, -4.2, -4.4, -4.5, -4.9. **LRMS** Calculated for C₃₄H₅₁IN₅O₃S₂Si₂, [M+H]⁺ m/z: 824.2, [M+H]⁺, found 824.2.

### Example 302

### 7-((2R,3R,4R,5S)-3,4-bis((tert-Butyldimethylsilyl)oxy)-5-((((4-methyl-6-phenylpyrimidin-5-yl)methyl)thio)methyl)tetrahydrofuran-2-yl)-5-iodo-7H-pyrrolo[2,3-d]pyrimidin-4-amine (239a):

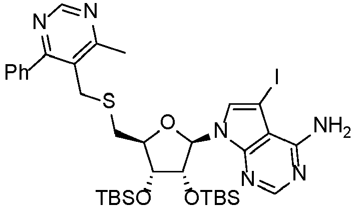

Modified nucleoside **239a** was prepared from bromide **56a** (obtained from **31** (0.12 g, 0.59 mmol)) and **121** (provided from **5** (0.4 g, 0.59 mmol)) following the same conditions as for **47b**. FCC (10-100 % EtOAc in cyclohexane) provided title compound **239a** (0.28 g, 0.34 mmol, 58 %). **¹H NMR** (400 MHz, CDCl₃) δ 9.00 (1H, s), 8.17 (1H, s), 7.68-7.64 (2H, m), 7.48-7.44 (3H, m), 7.13 (1H, s), 5.90 (1H, d, *J* = 4.7 Hz), 5.87 (2H, bs), 4.70 (1H, t, *J* = 4.6 Hz), 4.16 (1H, t, *J* = 4.4 Hz), 4.15-4.09 (1H, m), 3.85 (1H, d, *J* = 12.2 Hz), 3.76 (1H, d, *J* = 12.2 Hz), 2.88 (1H, dd, *J* = 13.9, 5.1 Hz), 2.82 (1H, dd, *J* = 13.9, 6.3 Hz), 2.69 (3H, s), 0.92, 0.81 (18H, 2×s), 0.10, 0.09, -0.07, -0.22 (12H, 4×s). **¹³C NMR** (101 MHz, CDCl₃) δ 167.7, 165.8, 156.7, 156.6, 151.5, 150.2, 138.0, 129.5, 128.9, 128.6, 127.2, 104.9, 90.2, 83.9, 74.8, 74.5, 50.6, 35.8, 32.1, 26.0, 25.9, 22.6, 18.2, 18.1, -4.1, -4.4, -4.5, -4.8. **LRMS** Calculated for C₃₅H₅₂IN₆O₃SSi₂, [M+H]⁺ m/z: 819.2, [M+H]⁺, found 819.3.

### Example 303

### 7-((2R,3R,4R,5S-3,4-bis((tert-Butyldimethylsilyl)oxy)-5-((((3-methyl-5-(4-nitrophenyl)isoxazol-4-yl)methyl)thio)methyl)tetrahydrofuran-2-yl)-5-iodo-7H-pyrrolo[2,3-d]pyrimidin-4-amine (240):

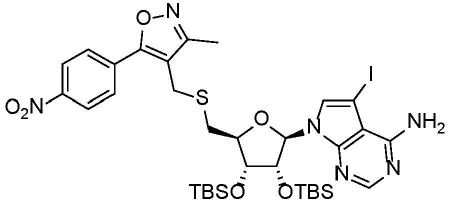

Intermediate **240** was synthesized from bromide **93a** (obtained from **64b** (0.1 g, 0.41 mmol)) and thiol **121** (provided from **5** (0.2 g, 0.30 mmol)) using the same procedure as for **47b**. FCC (15-70 % EtOAc in cyclohexane) to obtain modified nucleoside **240** (0.22 g, 0.26 mmol, 89 %). **¹H NMR** (400 MHz, DMSO-*d₆*) δ 8.29 (1H, d, *J* = 8.9 Hz), 8.09 (1H, s), 8.02 (1H, d, *J* = 8.9 Hz), 7.66 (1H, s), 6.68 (2H, bs), 6.08 (1H, d, *J* = 6.9 Hz), 4.73 (1H, dd, *J* = 6.8, 4.5 Hz), 4.14 (1H, dd, *J* = 4.3, 1.5 Hz), 4.00-3.93 (2H, m), 3.89 (1H, d, *J* = 13.4 Hz), 3.07 (1H, dd, *J* = 13.9, 6.8 Hz), 2.95 (1H, dd, *J* = 13.9, 7.1 Hz), 2.32 (3H, s), 0.89, 0.67 (18H, 2×s), 0.08, -0.11, -0.39 (12H, 3×s). **¹³C NMR** (101 MHz, DMSO-*d₆*) δ 162.7, 161.2, 157.0, 151.8, 150.6, 148.0, 132.9, 128.1, 127.6, 124.4, 113.8, 103.4, 86.5, 84.6, 74.7, 74.1, 52.9, 34.4, 25.9, 25.6, 24.2, 17.9, 17.6, 9.9, -4.5, -4.6, -5.4. **LRMS** Calculated for C₃₄H₅₀IN₆O₆SSi₂, [M+H]⁺ m/z: 853.2, [M+H]⁺, found 853.2.

### Example 304

### 7-((2R,3R,4R,5S)-5-((((5-(4-Aminophenyl)-3-methylisoxazol-4-yl)methyl)thio)methyl)-3,4-bis((tertbutyldimethylsilyl)oxy)tetrahydrofuran-2-yl)-5-iodo-7H-pyrrolo[2,3-d]pyrimidin-4-amine (241a):

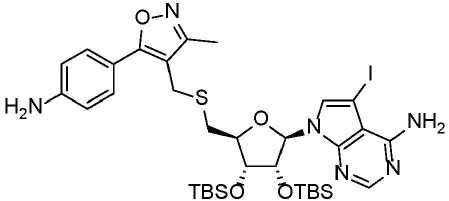

Iodo derivative **241a** was prepared from nitro analogue **240** (0.21 g, 0.25 mmol) according to the same methodology as for **234a**. FCC (10-100 % EtOAc in cyclohexane) afforded **241a** (0.16 g, 0.19 mmol, 78 %). **¹H NMR** (400 MHz, DMSO-*d₆*) δ 8.10 (1H, s), 7.68 (1H, s), 7.43 (1H, d, *J* = 8.6 Hz), 6.79-6.53 (3H, m), 6.09 (1H, d, *J* = 7.2 Hz), 5.68 (2H, bs), 4.75 (1H, dd, *J* = 7.1, 4.4 Hz), 4.15 (1H, dd, *J* = 4.1, 1.2 Hz), 3.98 (1H, t, *J* = 6.7 Hz), 3.84 (1H, d, *J* = 13.1 Hz), 3.76 (1H, d, *J* = 13.2 Hz), 3.09 (1H, dd, *J* = 13.7, 7.6 Hz), 2.93 (1H, dd, *J* = 13.7, 6.5 Hz), 2.22 (3H, s), 0.91, 0.66 (18H, 2×s), 0.12, 0.10, -0.11, -0.39 (12H, 4×s). **¹³C NMR** (101 MHz, DMSO-*d₆*) δ 166.2, 160.2, 157.3, 152.3, 150.8, 128.0, 127.4, 114.4, 113.8, 107.5, 103.4, 86.3, 84.8, 74.7, 74.1, 52.7, 34.6, 25.9, 25.6, 25.1, 17.9, 17.6, 9.8, -4.5, -4.6, -5.4. **LRMS** Calculated for C₃₄H₅₂IN₆O₄SSi₂, [M+H]⁺ m/z: 823.2, [M+H]⁺, found 823.3.

### Example 305

### 7-((2R,3R,4R,5S-3,4-bis((tert-Butyldimethylsilyl)oxy)-5-((((5-(3,4-dimethylphenyl)-3-methylisoxazol-4-yl)methyl)thio)methyl)tetrahydrofuran-2-yl)-5-iodo-7H-pyrrolo[2,3-d]pyrimidin-4-amine (242a):

**242a** was synthesized from bromide **95a** (obtained from **66b** (0.09 g, 0.41 mmol)) and thiol **121** (provided from **5** (0.2 g, 0.30 mmol)) following the same conditions as for **47b**. FCC (15-70 % EtOAc in cyclohexane) gave derivative **242a** (0.23 g, 0.28 mmol, 95 %). **¹H NMR** (400 MHz, DMSO-*d₆*) δ 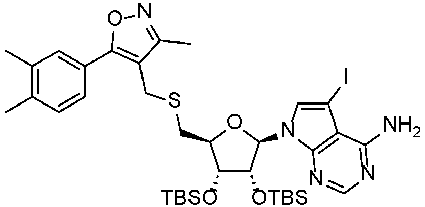 8.09 (1H, s), 7.66 (1H, s), 7.51 (1H, d, *J* = 1.5 Hz), 7.44 (1H, dd, *J* = 7.8, 1.6 Hz), 7.22 (1H, d, *J* = 7.9 Hz), 6.66 (2H, bs), 6.09 (1H, d, *J* = 7.0 Hz), 4.72 (1H, dd, *J* = 7.0, 4.5 Hz), 4.13 (1H, dd, *J* = 4.4, 1.3 Hz), 3.95 (1H, td, *J* = 6.8, 1.4 Hz), 3.89 (1H, d, *J* = 13.2 Hz), 3.80 (1H, d, *J* = 13.2 Hz), 3.05 (1H, dd, *J* = 13.8, 7.0 Hz), 2.92 (1H, dd, *J* = 13.8, 7.0 Hz), 2.27 (3H, s), 2.25 (6H, s), 0.89, 0.66 (18H, 2×s), 0.09, -0.11, -0.39 (12H, 3×s). **¹³C NMR** (101 MHz, DMSO-*d₆*) δ 165.4, 160.6, 157.4, 152.3, 150.9, 139.1, 137.5, 130.4, 127.8, 127.4, 124.9, 124.4, 110.4, 103.5, 86.4, 84.8, 74.7, 74.2, 52.7, 34.5, 25.9, 25.7, 24.7, 19.6, 19.6, 17.9, 17.7, 9.9, -4.4, -4.5, -5.4. **LRMS** Calculated for C₃₆H₅₅IN₅O₄SSi₂, [M+H]⁺ m/z: 836.2, [M+H]⁺, found 836.3.

### Example 306

### 7-((2R,3R,4R,5S-5-((((5-(Benzo[d][1,3]dioxol-5-yl)-3-methylisoxazol-4-yl)methyl)thio)methyl)-3,4-bis((tert-butyldimethylsilyl)oxy)tetrahydrofuran-2-yl)-5-iodo-7H-pyrrolo[2,3-d]pyrimidin-4-amine (243a):

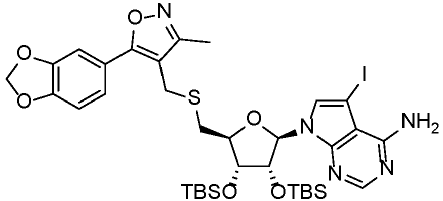

Title compound **243a** was prepared from bromide **96a** (obtained from **67b** (0.1 g, 0.41 mmol)) and thiol **121** (provided from **5** (0.2 g, 0.30 mmol)) following the same conditions as for **47b**. FCC (15-70 % EtOAc in cyclohexane) furnished **243a** (0.19 g, 0.22 mmol, 74 %). **¹H NMR** (400 MHz, DMSO-*d₆*) δ 8.10 (1H, s), 7.67 (1H, s), 7.29-7.21 (2H, m), 7.01 (1H, d, *J* = 8.5 Hz), 6.67 (2H, bs), 6.14-6.04 (3H, m), 4.74 (1H, dd, *J* = 6.6, 4.4 Hz), 4.14 (1H, d, *J* = 3.6 Hz), 3.96 (1H, t, *J* = 6.5 Hz), 3.86 (1H, d, *J* = 13.2 Hz), 3.78 (1H, d, *J* = 13.2 Hz), 3.07 (1H, dd, *J* = 13.8, 7.2 Hz), 2.93 (1H, dd, *J* = 13.7, 6.7 Hz), 2.26 (3H, s), 0.90, 0.67 (18H, 2×s), 0.10, 0.09, -0.11, -0.39 (12H, 4×s). **¹³C NMR** (101 MHz, DMSO-*d₆*) δ 164.9, 160.6, 157.2, 152.2, 150.8, 149.0, 148.2, 127.5, 121.6, 121.1, 110.0, 109.1, 106.9, 103.4, 101.9, 86.4, 84.7, 74.7, 74.1, 68.0, 52.7, 34.5, 25.9, 25.6, 24.6, 22.1, 17.9, 17.6, 9.9, -4.46, -4.52, -4.6, -5.4. **LRMS** Calculated for C₃₅H₅₁IN₅O₆SSi₂, [M+H]⁺ m/z: 852.2, [M+H]⁺, found 852.2.

### Example 307

### 7-((2R,3R,4R,5S)-3,4-bis((tert-Butyldimethylsilyl)oxy)-5-((((1-methyl-4-phenyl-1H-imidazol-5-yl)methyl)thio)methyl)tetrahydrofuran-2-yl)-5-(pyrimidin-5-ylethynyl)-7H-pyrrolo[2,3-d]pyrimidin-4-amine (237b):

**237b** was synthesized from intermediate **237a** (0.12 g, 0.15 mmol) and acetylene **161b** (0.02 g, 0.18 mmol) according to the same methodology as for **169a**. FCC (2-25 % EtOH in DCM) **237b** (0.12 g, 0.15 mmol, 99 %). **¹H NMR** (400 MHz, DMSO-*d₆*) δ 9.17 (1H, s), 9.03 (2H, s), 8.17 (1H, s), 7.99 (1H, s), 7.73 (1H, bs), 7.68-7.62 (2H, m), 7.38-7.32 (2H, m), 7.25-7.19 (1H, m), 6.86 (2H, bs), 6.14(1H, d, *J* = 7.0 Hz), 4.82 (1H, dd, *J* = 7.0, 4.4 Hz), 4.19 (1H, dd, *J* = 4.4, 1.6 Hz), 4.12 (1H, d, *J* = 13.5 Hz), 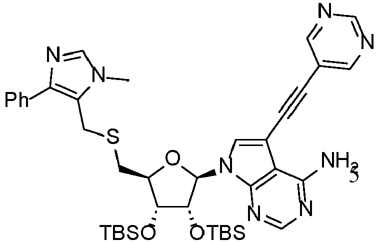 4.04 (1H, d, *J* = 13.5 Hz), 4.00 (1H, td, *J* = 7.1, 1.6 Hz), 3.68 (3H, s), 3.13 (1H, dd, *J* = 13.7, 7.3 Hz), 3.01 (1H, dd, *J* = 13.7, 7.0 Hz), 0.92, 0.69 (18H, 2×s), 0.13, 0.12, -0.09, -0.38 (12H, 4×s). **¹³C NMR** (101 MHz, DMSO-*d₆*) δ 158.5, 157.5, 156.4, 153.1, 150.5, 138.30, 138.26, 134.5, 128.40, 128.36, 128.3, 126.6, 126.5, 119.3, 101.8, 94.5, 89.4, 86.7, 84.9, 84.8, 74.6, 74.1, 34.4, 31.2, 25.8, 25.6, 25.5, 17.8, 17.5, -4.6, -4.65, -4.74, -5.5. **LRMS** Calculated for C₄₀H₅₅N₈O₃SSi₂, [M+H]⁺ m/z: 783.4, [M+H]⁺, found 783.4.

### Example 308

### 7-((2R,3R,4R,5S-3,4-bis((tert-Butyldimethylsilyl)oxy)-5-((((3-methyl-5-phenylisothiazol-4-yl)methyl)thio)methyl)tetrahydrofuran-2-yl)-5-(pyrimidin-5-ylethynyl)-7H-pyrrolo[2,3-d]pyrimidin-4-amine (238b):

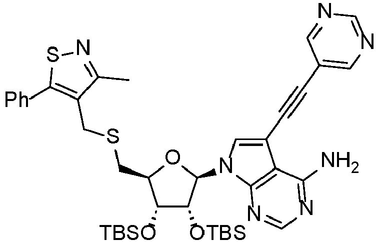

Derivative **238b** was prepared from iodo analogue **238a** (0.07 g, 0.09 mmol) and acetylene **161b** (0.01 g, 0.10 mmol) using the same conditions as for **169a**. FCC (10-60 % acetone in cyclohexane) furnished **238b** (0.06 g, 0.07 mmol, 84 %). **¹H** NMR (400 MHz, CDCl₃) δ 9.17 (1H, s), 8.85 (2H, s), 8.26 (1H, s), 7.52-7.48 (2H, m), 7.46 (1H, s), 7.47-7.40 (3H, m), 5.97 (1H, d, *J* = 5.0 Hz), 5.95 (2H, bs), 4.76-4.70 (1H, m), 4.17-4.12 (2H, m), 3.85 (1H, d, *J* = 12.5 Hz), 3.78 (1H, d, *J* = 12.5 Hz), 2.93 (1H, dd, *J* = 13.6, 5.3 Hz), 2.83 (1H, dd, *J* = 13.7, 5.6 Hz), 2.56 (3H, s), 0.92, 0.81 (18H, 2×s), 0.10, 0.09, -0.06, -0.23 (12H, 4×s). **¹³C NMR** (101 MHz, CDCl₃) δ 167.4, 164.0, 158.5, 157.1, 156.7, 151.8, 149.8, 131.1, 129.4, 129.2, 128.9, 128.84, 128.83, 119.6, 103.7, 95.1, 90.3, 89.5, 85.3, 84.1, 75.0, 74.6, 35.2, 28.8, 26.0, 25.9, 18.5, 18.2, 18.1, -4.2, -4.4, -4.5, -4.8. **LRMS** Calculated for C₄₀H₅₄N₇O₃S₂Si₂, [M+H]⁺ m/z: 800.3, [M+H]⁺, found 800.3.

### Example 309

### 7-((2R,3R,4R,5S-3,4-bis((tert-Butyldimethylsilyl)oxy)-5-((((4-methyl-6-phenylpyrimidin-5-yl) methyl)thio)methyl)tetrahydrofuran-2-yl)-5-(pyrimidin-5-ylethynyl)-7H-pyrrolo[2,3-d]pyrimidin-4-amine (239b):

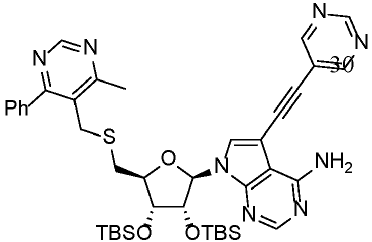

Modified nucleoside **239b** was synthesized from **239a** (0.09 g, 0.11 mmol) and acetylene **161b** (0.01 g, 0.13 mmol) following the same methodology as for **169a**. FCC (2-25 % EtOH in DCM) afforded **239b** (0.06 g, 0.07 mmol, 63 %).**¹H NMR** (400 MHz, CDCl₃) δ 9.17 (1H, s), 8.99 (1H, s), 8.85 (2H, s), 8.25 (1H, s), 7.69-7.66 (2H, m), 7.49-7.44 (3H, m), 7.40 (1H, s), 5.92 (1H, d, *J* = 4.7 Hz), 5.85 (2H, bs), 4.77 (1H, t, *J* = 4.4 Hz), 4.19-4.11 (2H, m), 3.87 (1H, d, *J* = 12.2 Hz), 3.78 (1H, d, *J* = 12.5 Hz), 2.92 (1H, dd, *J* = 13.8, 5.1 Hz), 2.86 (1H, dd, *J* = 13.8, 6.2 Hz), 2.70 (3H, s), 0.92, 0.82 (18H, 2×s), 0.10, 0.09, -0.05, -0.21 (12H, 4×s). **¹³C NMR** (101 MHz, CDCl₃) δ 167.7, 165.8, 158.5, 157.1, 156.8, 156.6, 152.0, 149.7, 137.9, 129.5, 129.0, 128.9, 128.6, 127.1, 119.6, 103.8, 95.0, 90.7, 89.4, 85.4, 84.1, 74.8, 74.6, 35.8, 32.1, 26.0, 25.9, 22.5, 18.2, 18.1, -4.1, -4.4, -4.5, -4.8. **LRMS** Calculated for C₄₁H₅₅N₈O₃SSi₂, [M+H]⁺ m/z: 795.4, [M+H]⁺, found 795.3.

### Example 310

### 7-((2R,3R,4R,5S)-5-((((5-(4-Aminophenyl)-3-methylisoxazol-4-yl)methyl)thio)methyl)-3,4-bis((tertbutyldimethylsilyl)oxy)tetrahydrofuran-2-yl)-5-(pyrimidin-5-ylethynyl)-7H-pyrrolo[2,3-d]pyrimidin-4-amine (241b):

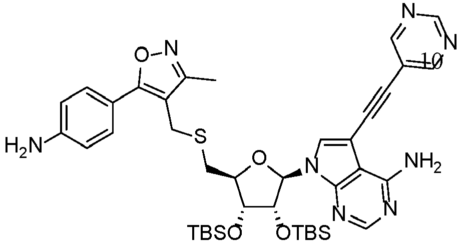

Title compound **241b** was prepared from **241a** (0.07 g, 0.09 mmol) and acetylene **161b** (0.02 g, 0.17 mmol) according to the same procedure as for **169a**. FCC (2-30 % EtOH in EtOAc) provided **241b** (0.06 g, 0.08 mmol, 93 %). **¹H NMR** (400 MHz, DMSO-*d₆*) δ 9.16 (1H, s), 9.03 (2H, s), 8.16 (1H, s), 8.00 (1H, s), 7.43 (2H, d, *J* = 8.6 Hz), 6.87 (2H, bs), 6.64 (2H, d, *J* = 8.7 Hz), 6.14 (1H, d, *J* = 7.0 Hz), 5.67 (2H, bs), 4.83 (1H, dd, *J* = 6.9, 4.4 Hz), 4.20 (1H, dd, *J* = 4.3, 1.2 Hz), 4.02 (1H, t, *J* = 6.5 Hz), 3.85 (1H, d, *J* = 13.1 Hz), 3.76 (1H, d, *J* = 13.1 Hz), 3.13 (1H, dd, *J* = 13.8, 7.6 Hz), 2.97 (1H, dd, *J* = 13.8, 6.5 Hz), 2.23 (3H, s), 0.92, 0.69 (18H, 2×s), 0.14, 0.12, -0.09, -0.38 (12H, 4×s). **¹³C NMR** (101 MHz, DMSO-*d₆*) δ 166.2, 160.3, 158.7, 157.7, 156.6, 153.3, 150.9, 150.7, 128.0, 119.5, 114.4, 113.9, 107.5, 102.1, 94.7, 89.6, 87.0, 85.1, 85.0, 74.8, 74.3, 34.6, 26.0, 25.7, 25.1, 17.9, 17.7, 9.8, -4.4, -4.5, -4.6, -5.3. LRMS Calculated for C₄₀H₅₅N₈O₄SSi₂, [M+H]⁺ m/z: 799.4, [M+H]⁺, found 799.4.

### Example 311

### 7-((2R,3R,4R,5S-3,4-bis((tert-Butyldimethylsilyl)oxy)-5-((((5-(3,4-dimethylphenyl)-3-methylisoxazol-4-yl)methyl)thio)methyl)tetrahydrofuran-2-yl)-5-(pyrimidin-5-ylethynyl)-7H-pyrrolo[2,3-d]pyrimidin-4-amine (242b):

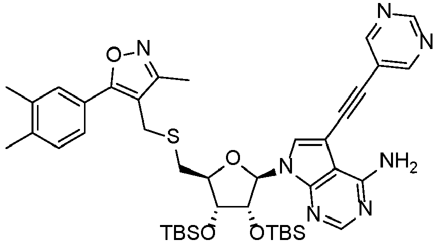

SAH analogue **242b** was synthesized from **242a** (0.19 g, 0.22 mmol) and acetylene **161b** (0.07 g, 0.67 mmol) in analogous fashion to **169a**. FCC (20-100 % EtOAc in cyclohexane) afforded **242b** (0.1 g, 0.12 mmol, 55 %). **¹H NMR** (400 MHz, DMSO-*d₆*) δ 9.17 (1H, s), 9.02 (2H, s), 8.16 (1H, s), 7.99 (1H, s), 7.52 (1H, d, *J* = 1.7 Hz), 7.46 (1H, dd, *J* = 7.8, 1.6 Hz), 7.23 (1H, d, *J* = 7.9 Hz), 6.86 (2H, bs), 6.14 (1H, d, *J* = 6.9 Hz), 4.80 (1H, dd, *J* = 6.8, 4.5 Hz), 4.17 (1H, dd, *J* = 4.3, 1.5 Hz), 3.99 (1H, td, *J=* 6.7, 1.6 Hz), 3.89 (1H, d, *J* = 13.2 Hz), 3.81 (1H, d, *J* = 13.2 Hz), 3.09 (1H, dd, *J* = 13.8, 7.0 Hz), 2.96 (1H, dd, *J* = 13.8, 7.0 Hz), 2.28 (3H, s), 2.25 (3H, s), 2.24 (3H, s), 0.90, 0.69 (18H, 2×s), 0.10, -0.09, -0.38 (12H, 3×s). **¹³C NMR** (101 MHz, DMSO-*d₆*) δ 165.4, 160.5, 158.7, 157.7, 156.6, 153.3, 150.7, 139.0, 137.4, 130.3, 128.5, 127.8, 124.9, 124.3, 119.5, 110.3, 102.0, 94.6, 89.6, 87.0, 85.0, 84.9, 74.8, 74.3, 34.4, 25.9, 25.7, 24.5, 19.5, 19.5, 17.9, 17.6, 9.9, -4.5, -4.55, -4.59, -5.3. **LRMS** Calculated for C₄₂H₅₈N₇O₄SSi₂, [M+H]⁺ m/z: 812.4, [M+H]⁺, found 812.3.

### Example 312

### 7-((2R,3R,4R,5S)-5-((((5-(Benzo[d][1,3]dioxol-5-yl)-3-methylisoxazol-4-yl)methyl)thio)methyl)-3,4-bis((tert-butyldimethylsilyl)oxy)tetrahydrofuran-2-yl)-5-(pyrimidin-5-ylethynyl)-7H-pyrrolo[2,3-d]pyrimidin-4-amine (243b):

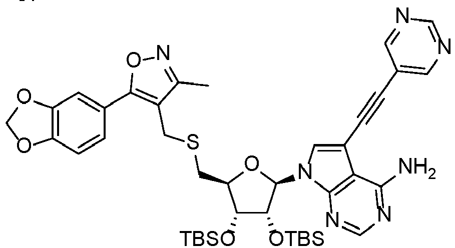

Derivative **243b** was prepared from **243a** (0.15 g, 0.18 mmol) and acetylene **161b** (0.06 g, 0.54 mmol) in analogous fashion to **169a**. FCC (2-10 % MeOH in EtOAc) furnished **243b** (0.07 g, 0.09 mmol, 49 %). **¹H NMR** (400 MHz, DMSO-*d₆*) δ 9.17 (1H, s), 9.02 (2H, s), 8.16 (1H, s), 7.99 (1H, s), 7.29-7.25 (2H, m), 7.02 (1H, d, *J* = 8.5 Hz), 6.85 (2H, bs), 6.13 (1H, d, *J* = 6.8 Hz), 6.09 (2H, s), 4.81 (1H, dd, *J* = 6.8, 4.5 Hz), 4.18 (1H, dd, *J* = 4.3, 1.5 Hz), 4.01 (1H, td, *J* = 6.8, 1.4 Hz), 3.87 (1H, d, *J* = 13.2 Hz), 3.79 (1H, d, *J* = 13.2 Hz), 3.11 (1H, dd, *J* = 13.8, 7.2 Hz), 2.97 (1H, dd, *J* = 13.8, 6.8 Hz), 2.26 (3H, s), 0.91, 0.69 (18H, 2×s), 0.12, 0.10, -0.09, -0.38 (12H, 4×s). **¹³C NMR** (101 MHz, DMSO-*d₆*) δ 164.9, 160.6, 158.7, 157.7, 156.6, 153.2, 150.7, 149.0, 148.2, 128.5, 121.6, 121.1, 119.5, 110.0, 109.1, 107.0, 102.0, 101.9, 94.6, 89.6, 87.0, 85.0, 84.8, 74.7, 74.3, 34.5, 25.9, 25.7, 24.6, 17.9, 17.6, 9.8, -4.4, -4.5, -4.6, -5.3. **LRMS** Calculated for C₄₁H₅₄N₇O₆SSi₂, [M+H]⁺ m/z: 828.3, [M+H]⁺, found 828.3.

### Example 313

### (2R,3R,4S,5S)-2-(4-Amino-5-(pyrimidin-5-ylethynyl)-7H-pyrrolo[2,3-d]pyrimidin-7-yl)-5-((((1-methyl-4-phenyl-1H-imidazol-5-yl)methyl)thio)methyl)tetrahydrofuran-3,4-diol (237c):

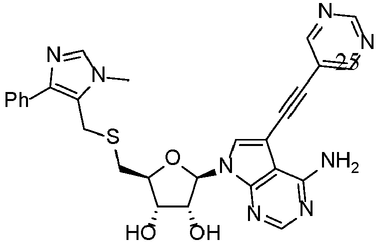

**237c** was synthesized from derivative **237b** (0.12 g, 0.15 mmol) using the same conditions as for **47c**. Reverse-phase FCC (0-60 % MeCN in H₂O) afforded title compound **237c** (0.07 g, 0.13 mmol, 88 %). **¹H NMR** (400 MHz, DMSO-*d₆*) δ 9.17 (1H, s), 9.02 (2H, s), 8.18 (1H, s), 7.97 (1H, s), 7.72-7.65 (3H, m), 7.40-7.34 (2H, m), 7.25-7.19 (1H, m), 6.86 (2H, bs), 6.12 (1H, d, *J* = 6.0 Hz), 5.49 (1H, d, *J* = 6.2 Hz), 5.33 (1H, d, *J*= 4.7 Hz), 4.53 (1H, q, *J* = 5.7 Hz), 4.10-4.00 (4H, m), 3.66 (3H, s), 3.01 (1H, dd, *J* = 14.0, 5.6 Hz), 2.95 (1H, dd, *J* = 13.9, 6.7 Hz). **¹³C NMR** (101 MHz, DMSO-*d₆*) δ 158.5, 157.5, 156.4, 153.1, 150.4, 138.2, 138.1, 134.6, 128.5, 128.2, 126.43, 126.37, 123.4, 119.4, 101.8, 94.3, 89.5, 87.0, 84.8, 83.6, 73.2, 72.7, 34.7, 31.1, 25.6. **HRMS** (ESI) Calculated for C₂₈H₂₇N₈O₃S, [M+H]⁺ m/z: 555.1921, [M+H]⁺, found 555.1915.

### Example 314

### (2R,3R,4S,5S)-2-(4-Amino-5-(pyrimidin-5-ylethynyl)-7H-pyrrolo[2,3-d]pyrimidin-7-yl)-5-((((3-methyl-5-phenylisothiazol-4-yl)methyl)thio)methyl)tetrahydrofuran-3,4-diol (238c):

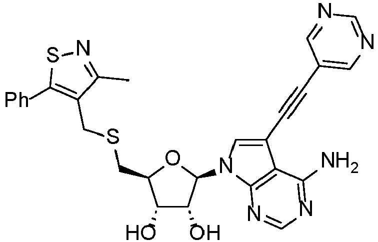

Title compound **238c** was prepared from protected analogue **238b** (0.05 g, 0.07 mmol) following the same procedure as for **47c**. Reverse-phase FCC (0-70 % MeCN in H₂O) provided derivative **238c** (0.03 g, 0.13 mmol, 86%). **¹H NMR** (400 MHz, DMSO-*d₆*) δ 9.17 (1H, s), 9.03 (2H, s), 8.17 (1H, s), 7.95 (1H, s), 7.65-7.43 (5H, m), 6.86 (2H, bs), 6.10 (1H, d, *J* = 6.0 Hz), 5.47 (1H, d, *J* = 6.2 Hz), 5.31 (1H, d, *J* = 5.0 Hz), 4.51 (1H, q, *J* = 5.8 Hz), 4.04 (1H, td, *J* = 5.1, 3.6 Hz), 3.98 (1H, td, *J* = 6.4, 3.5 Hz), 3.88 (1H, d, *J* = 12.3 Hz), 3.83 (1H, d, *J* = 12.3 Hz), 2.95 (1H, dd, *J* = 13.9, 5.8 Hz), 2.89 (1H, dd, *J* = 13.9, 7.1 Hz), 2.49 (3H, s). **¹³C NMR** (101 MHz, DMSO-*d₆*) δ 167.7, 162.5, 158.5, 157.5, 156.4, 153.1, 150.4, 130.3, 129.42, 129.37, 129.2, 128.2, 119.4, 101.8, 94.3, 89.5, 86.9, 84.8, 83.4, 73.2, 72.7, 35.0, 27.6, 17.9. **HRMS** (ESI) Calculated for C₂₈H₂₅NaN₇O₃S₂, [M+Na]⁺ m/z: 594.1353, [M+H]⁺, found 594.1348.

### Example 315

### (2R,3R,4S,5S)-2-(4-Amino-5-(pyrimidin-5-ylethynyl)-7H-pyrrolo[2,3-d]pyrimidin-7-yl)-5-((((4-methyl-6-phenylpyrimidin-5 -yl)methyl)thio)methyl)tetrahydrofuran-3,4-diol (239c):

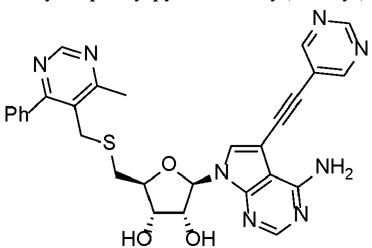

SAH analogue **239c** was synthesized from modified nucleoside **239b** (0.05 g, 0.06 mmol) according to the same methodology as for **47c**. FCC (0-60 % MeCN in H₂O) furnished title compound **239c** (0.03 g, 0.06 mmol, 89 %). **¹H NMR** (400 MHz, DMSO-*d₆*) δ 9.17 (1H, s), 9.03 (2H, s), 8.95 (1H, s), 8.18 (1H, s), 7.96 (1H, s), 7.66-7.46 (5H, m), 6.90 (2H, bs), 6.10 (1H, d, *J* = 6.0 Hz), 5.47 (1H, bs), 5.32 (1H, bs), 4.51 (1H, t, *J* = 4.9 Hz), 4.04 (1H, t, *J* = 4.3 Hz), 3.99 (1H, td, *J* = 6.6, 4.6 Hz), 3.88 (1H, d, *J* = 12.3 Hz), 3.83 (1H, d, *J* = 12.3 Hz), 2.95 (1H, dd, *J* = 14.0, 5.8 Hz), 2.89 (1H, dd, *J* = 13.9, 7.1 Hz), 2.49 (3H, s). **¹³C NMR** (101 MHz, DMSO-*d*) δ 167.6, 164.6, 158.7, 157.6, 156.2, 150.5, 137.7, 129.4, 128.8, 128.5, 128.4, 127.0, 119.5, 102.0, 94.4, 89.7, 87.2, 85.0, 83.4, 73.3, 72.8, 35.6, 30.9, 22.1. **HRMS** (ESI) Calculated for C₂₉H₂₆NaN₈O₃S, [M+Na]⁺ m/z: 589.1741, [M+H]⁺, found 589.1737.

### Example 316

### (2R,3R,4S,5S)-2-(4-Amino-5-(pyrimidin-5-ylethynyl)-7H-pyrrolo[2,3-d]pyrimidin-7-yl)-5-((((5-(4-aminophenyl)-3-methylisoxazol-4-yl)methyl)thio)methyl)tetrahydrofuran-3,4-diol (241c):

Title compound **241c** was prepared from **241b** (0.05 g, 0.07 mmol) using the same procedure as for **47c**. Reverse-phase FCC (5-60 % MeCN in H₂O) furnished **241c** (0.03 g, 0.06 mmol, 83 %). **¹H NMR** (400 MHz, DMSO-*d₆*) δ 9.16 (1H, s), 9.03 (2H, s), 8.18 (1H, s), 7.98 (1H, s), 7.45 (2H, d, *J* = 8.5 Hz), 6.86 (2H, bs), 6.65 (2H, d, *J* = 8.5 Hz), 6.12 (1H, d, *J* = 5.8 Hz), 5.66 (2H, bs), 5.49 (1H, d, *J* = 5.0 Hz), 5.39 (1H, d, *J* = 3.4 Hz), 4.53 (1H, t, *J* = 4.5 Hz), 4.14-4.00 (2H, m), 3.80 (1H, d, *J* = 12.9 Hz), 3.76 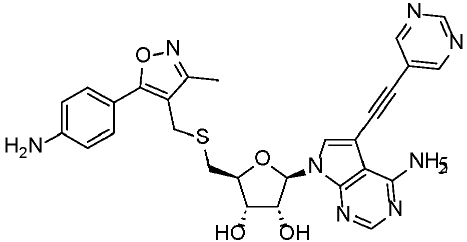 (1H, d, *J* = 13.1 Hz), 2.98 (1H, dd, *J* = 13.8, 5.5 Hz), 2.91 (1H, dd, *J* = 13.8, 6. Hz), 2.21 (3H, s). **¹³C NMR** (101 MHz, DMSO-*d₆*) δ 166.1, 160.4, 158.7, 157.7, 156.6, 153.3, 150.8, 150.6, 128.4, 128.0, 119.6, 114.5, 113.9, 107.6, 102.0, 94.5, 89.7, 87.2, 85.0, 83.7, 73.4, 72.9, 34.8, 24.9, 9.8. **HRMS** (ESI) Calculated for C₂₈H₂₇N₈O₄S, [M+H]⁺ m/z: 571.1871, [M+H]⁺, found 571.1868.

### Example 317

### (2R,3R,4S,5S)-2-(4-Amino-5-(pyrimidin-5-ylethynyl)-7H-pyrrolo[2,3-d]pyrimidin-7-yl)-5-((((5-(3,4-dimethylphenyl)-3-methylisoxazol-4-yl)methyl)thio)methyl)tetrahydrofuran-3,4-diol (242c):

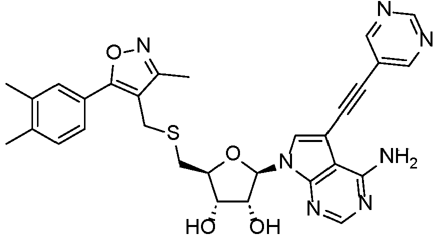

**242c** was synthesized from **242b** (0.1 g, 0.12 mmol) in analogous fashion to **47c**. Reverse-phase FCC (0-70 % MeCN in H₂O) afforded **242c** (0.07 g, 0.11 mmol, 95 %). **¹H NMR** (400 MHz, DMSO-*d₆*) δ 9.17 (1H, s), 9.02 (2H, s), 8.18 (1H, s), 7.97 (1H, s), 7.54 (1H, d, *J* = 1.2 Hz), 7.48 (1H, dd, *J* = 7.8, 1.5 Hz), 7.24 (1H, *d, J* = 7.9 Hz), 6.89 (2H, bs), 6.13 (1H, *d, J* = 5.9 Hz), 5.48 (1H, bs), 5.33 (1H, bs), 4.51 (1H, t, *J* = 4.7 Hz), 4.11-4.02 (2H, m), 3.86 (1H, d, *J* = 13.0 Hz), 3.81 (1H, d, *J* = 13.0 Hz), 2.98 (1H, dd, *J* = 13.9, 5.3 Hz), 2.91 (1H, dd, *J* = 13.8, 6.9 Hz), 2.27 (3H, s), 2.26 (3H, s), 2.24 (3H, s). **¹³C NMR** (101 MHz, DMSO-*d₆*) δ 165.2, 160.7, 158.7, 157.5, 156.6, 153.1, 150.5, 139.0, 137.5, 130.4, 128.4, 127.6, 124.9, 124.3, 119.5, 110.4, 101.9, 94.5, 89.6, 87.2, 85.0, 83.7, 73.4, 72.9, 34.8, 24.5, 19.5, 19.5, 9.8. **HRMS** (ESI) Calculated for C₃₀H₃₀N₇O₄S, [M+H]⁺ m/z: 584.2075, [M+H]⁺, found 584.2071.

### Example 318

### (2R,3R,4S,5S)-2-(4-Amino-5-(pyrimidin-5-ylethynyl)-7H-pyrrolo[2,3-d]pyrimidin-7-yl)-5-((((5-(benzo[d][1,3]dioxol-5-yl)-3-methylisoxazol-4-yl)methyl)thio)methyl)tetrahydrofuran-3,4-diol (243c):

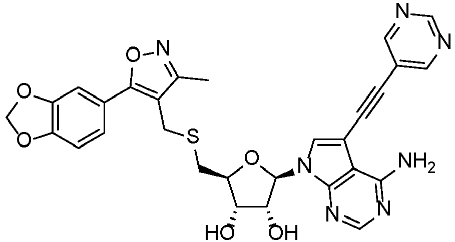

**243c** was prepared from **247b** (0.07 g, 0.09 mmol) according to the same methodology as for **47c**. Reverse-phase FCC (0-60 % MeCN in H₂O) furnished derivative **243c** (0.05 g, 0.09 mmol, 99 %). **¹H NMR** (400 MHz, DMSO-*d₆*) δ 9.16 (1H, s), 9.02 (2H, s), 8.17 (1H, s), 7.97 (1H, s), 7.31-7.26 (2H, m), 7.05 (1H, d, *J* = 8.0 Hz), 6.85 (2H, bs), 6.12 (1H, d, *J* = 5.9 Hz), 6.10 (2H, s), 5.48 (1H, d, *J* = 6.1 Hz), 5.32 (1H, d, *J* = 4.9 Hz), 4.51 (1H, q, *J* = 5.0 Hz), 4.09-4.01 (2H, m), 3.82 (1H, d, *J* = 13.2 Hz), 3.78 (1H, d, *J* = 13.2 Hz), 2.98 (1H, dd, *J* = 13.9, 5.3 Hz), 2.91 (1H, dd, *J* = 13.8, 6.8 Hz), 2.25 (3H, s). **¹³C NMR** (101 MHz, DMSO-*d₆*) δ 164.8, 160.7, 158.7, 157.7, 156.6, 153.3, 150.5, 149.0, 148.2, 128.4, 121.6, 121.1, 119.6, 110.1, 109.2, 106.9, 101.9, 94.5, 89.6, 87.2, 85.0, 83.6, 73.4, 72.9, 34.7, 24.4, 9.8. **HRMS** (ESI) Calculated for C₂₉H₂₆N₇O₆S, [M+H]⁺ m/z: 600.1660, [M+H]⁺, found 600.1656.

### Example 319

### (2R,3R,4S,5S)-2-(4-Amino-5-iodo-7H-pyrrolo[2,3-d]pyrimidin-7-yl)-5-((((1-methyl-4-phenyl-1H-imidazol-5-yl)methyl)thio)methyl)tetrahydrofuran-3,4-diol (244):

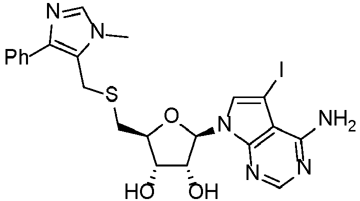

SAH analogue **244** was synthesized from protected derivative **237a** (0.19 g, 0.24 mmol) following the same conditions as for **47c.** Reverse-phase FCC (0-60 % MeCN in H₂O) afforded modified nucleoside **244** (0.12 g, 0.21 mmol, 87 %). **¹H NMR** (400 MHz, DMSO-*d₆*) δ 8.12 (1H, s), 7.99 (1H, bs), 7.67-7.63 (2H, m), 7.43-7.38 (2H, m), 7.31-7.26 (2H, m), 6.70 (2H, bs), 6.06 (1H, d, *J* = 6.2 Hz), 5.43 (1H, bs), 5.28 (1H, bs), 4.51-4.43 (1H, m), 4.07 (1H, d, *J* = 13.5 Hz), 4.05-3.96 (3H, m), 3.70 (3H, s), 2.96 (1H, dd, *J* = 13.9, 5.9 Hz), 2.90 (1H, dd, *J* = 13.9, 6.9 Hz). **¹³C NMR** (101 MHz, DMSO-*d₆*) δ 157.1, 152.0, 150.5, 137.8, 136.7, 133.1, 128.6, 127.0, 126.7, 124.0, 103.2, 86.6, 83.3, 72.9, 72.6, 34.6, 31.6, 25.2. **HRMS** (ESI) Calculated for C₂₂H₂₄IN₆O₃S, [M+H]⁺ m/z: 579.0670, [M+H]⁺, found 579.0664.

### Example 320

### (2R,3R,4S,5S)-2-(4-Amino-5-iodo-7H-pyrrolo[2,3-d]pyrimidin-7-yl)-5-((((3-methyl-5-phenylisothiazol-4-yl)methyl)thio)methyl)tetrahydrofuran-3,4-diol (245):

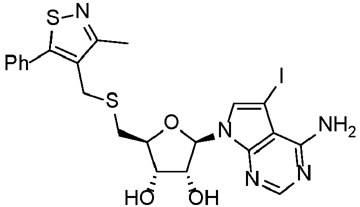

Derivative **245** was prepared from **238a** (0.14 g, 0.17 mmol) in analogous fashion to **47c.** Reverse-phase FCC (0-80 % MeCN in H₂O) gave **245** (0.09 g, 0.15 mmol, 91 %). **¹H NMR** (400 MHz, DMSO-*d₆*) δ 8.14 (1H, s), 7.66 (1H, s), 7.58-7.54 (2H, m), 7.53-7.44 (3H, m), 6.81 (2H, bs), 6.05 (1H, d, *J* = 6.3 Hz), 5.41 (1H, bs), 5.28 (1H, bs), 4.46 (1H, t, *J* = 5.7 Hz), 4.00 (1H, dd, *J* = 5.1, 3.4 Hz), 3.97-3.91 (1H, m), 3.86 (1H, d, *J* = 12.3 Hz), 3.82 (1H, d, *J* = 12.3 Hz), 2.92 (1H, dd, *J* = 13.9, 5.9 Hz), 2.84 (1H, dd, *J* = 13.9, 7.1 Hz), 2.49 (3H, s). **¹³C NMR** (101 MHz, DMSO-*d₆*) δ 167.9, 162.7, 156.9, 151.6, 150.5, 130.5, 129.6, 129.6, 129.4, 128.4, 127.4, 103.3, 86.7, 83.4, 73.1, 72.8, 52.9, 35.2, 27.8, 18.1. **HRMS** (ESI) Calculated for C₂₂H₂₃IN₅O₃S₂, [M+H]⁺ m/z: 596.0282, [M+H]⁺, found 596.0278.

### Example 321

### (2R,3R,4S,5S)-2-(4-Amino-5-iodo-7H-pyrrolo[2,3-d]pyrimidin-7-yl)-5-((((4-methyl-6-phenylpyrimidin-5-yl)methyl)thio)methyl)tetrahydrofuran-3,4-diol (246):

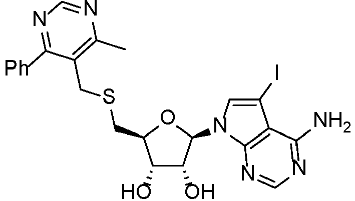

Title compound **246** was prepared from **239a** (0.14 g, 0.17 mmol) in analogous fashion to **47c**. Reverse-phase FCC (0-60 % MeCN in H₂O) furnished **246** (0.09 g, 0.16 mmol, 93 %). **¹H NMR** (400 MHz, DMSO-*d₆*) δ 8.95 (1H, s), 8.11 (1H, s), 7.70-7.66 (2H, m), 7.62 (1H, s), 7.52-7.47 (3H, m), 6.71 (2H, bs), 6.03 (1H, d, *J* = 6.1 Hz), 5.40 (1H, bs), 5.25 (1H, bs), 4.48-4.40 (1H, m), 4.01-3.95 (1H, m), 3.93-3.88 (1H, m), 3.86 (1H, d, *J* = 12.2 Hz), 3.81 (1H, d, *J* = 12.1 Hz), 2.91 (1H, dd, *J* = 13.9, 5.7 Hz), 2.83 (1H, dd, *J* = 13.9, 7.2 Hz), 2.65 (3H, s). **¹³C NMR** (101 MHz, DMSO-*d₆*) δ 167.6, 164.6, 157.2, 156.3, 152.1, 150.6, 137.7, 129.5, 128.8, 128.5, 127.2, 127.0, 103.4, 86.8, 83.2, 73.1, 72.8, 52.8, 35.6, 31.0, 22.2. **HRMS** (ESI) Calculated for C₂₃H₂₄IN₆O₃S, [M+H]⁺ m/z: 591.0670, [M+H]⁺, found 591.0669.

### Biological activity evaluation

### Nsp14 MTase activity inhibition in vitro

To evaluate inhibitory activity of the compounds towards nsp14 methyltransferase from SARS-CoV-2, recombinant nsp14 protein was expressed in E. coli, purified by IMAC chromatography and gel chromatography on HiLoad 16/600 Superdex 200 gel filtration column (GE Healthcare) in 10 mM HEPES, pH 7.5, 150 mM NaCl. The protein was concentrated to 5.5 mg/ml and stored in aliquots at-80°C until needed.

All newly synthesized compounds were dissolved in DMSO to yield 10 mM stock solutions. The compounds were screened first at 12.5 µM concentration and in case that at least 50% inhibition was observed, dose-response curve was generated to calculate IC₅₀ value (half-maximal inhibitory concentration). Sinefungin (Sigma-Aldrich) was used as a reference compound. Synthesized compounds were tested according to a previously published EchoMS assay (Štefek, M., et al., *ACS Omega* **2023**/*8*, 27410-27418, Supporting information, Method 3.1., S30).

Compounds of the invention showed IC₅₀ values in the range between 10 - 5000 nM. The results of the testing are summarized in **Table 1**.

**Table 1: Evaluation of in vitro efficacy of prepared inhibitors. IC₅₀ values are listed in groups labeled: +++++ for IC₅₀ = 10-30 nM, ++++ for IC₅₀ = 30-100 nM, +++ for IC₅₀ = 100-500 nM, ++ for IC₅₀ = 500-2000 nM and+for IC₅₀ = 2000-5000 nM.**

| **Formula** | **Compound** # | **Example** # | **IC₅₀** (+ > +++++) |
|---|---|---|---|
| 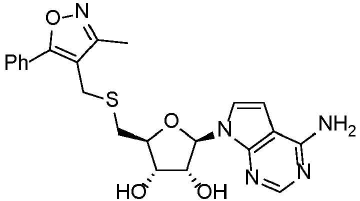 | **47c** | **46** | ++++ |
| 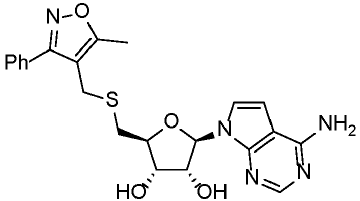 | **48c** | **47** | +++ |
| 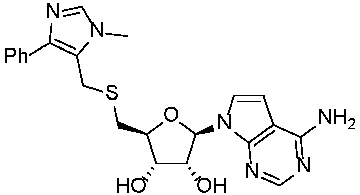 | **49c** | **48** | +++ |
| 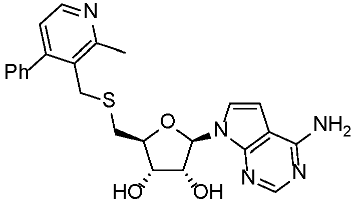 | **50c** | **49** | +++ |
| 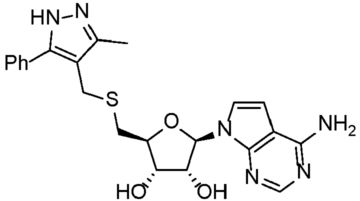 | **51c** | **50** | +++ |
| 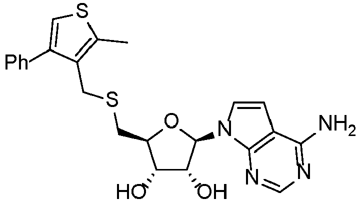 | **52c** | **51** | ++ |
| 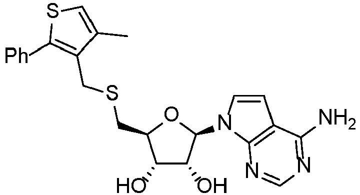 | **53c** | **52** | +++ |
| 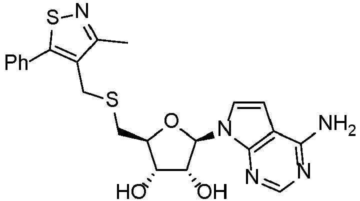 | **54c** | **53** | **++++** |
| 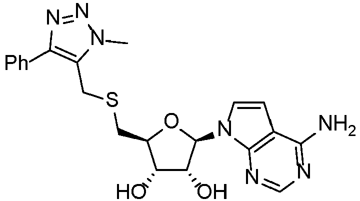 | **55c** | **54** | **++++** |
| 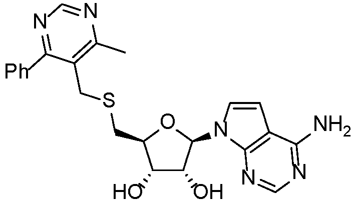 | **56c** | **55** | **+++** |
| 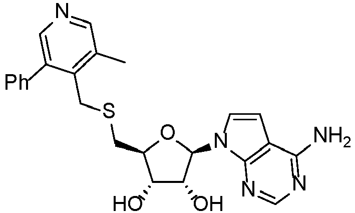 | **57c** | **56** | **++++** |
| 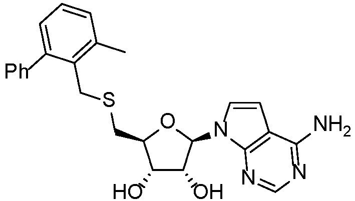 | **58c** | **57** | **++** |
| 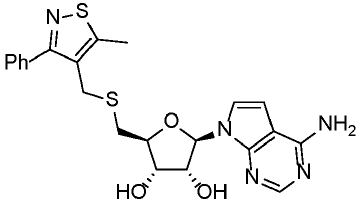 | **59c** | **58** | **++++** |
| 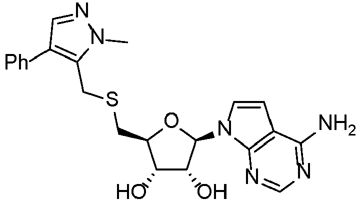 | **60c** | **59** | **++++** |
| 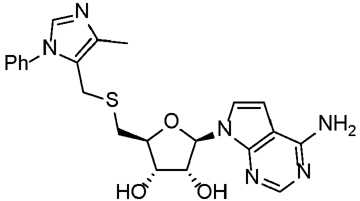 | **61c** | **60** | **+++** |
| 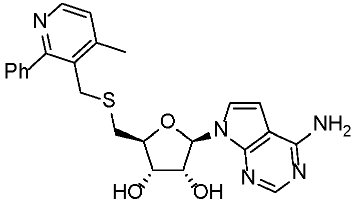 | **62c** | **61** | **++** |
| 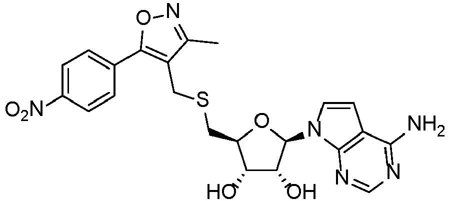 | **93c** | **114** | **+++** |
| 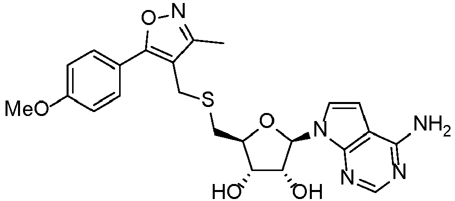 | **94c** | **115** | **+++++** |
| 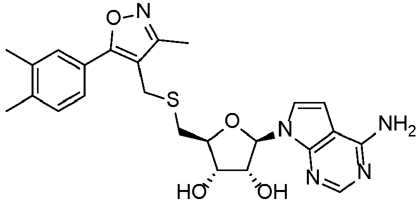 | **95c** | **116** | **+++++** |
| 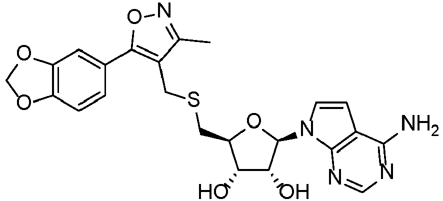 | **96c** | **117** | **+++++** |
| 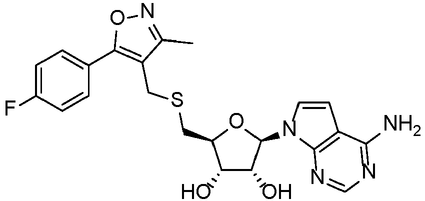 | **97c** | **118** | **+++** |
| 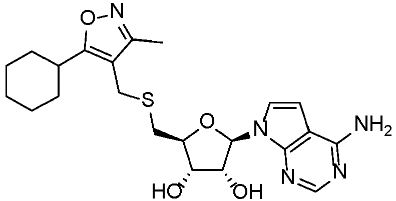 | **98c** | **119** | **++** |
| 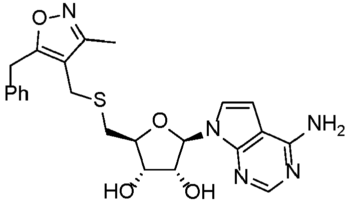 | **99c** | **120** | **+** |
| 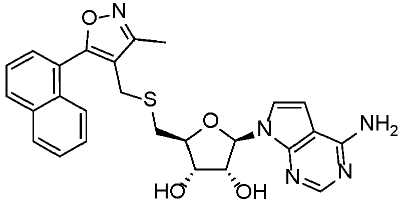 | **100c** | **121** | **+++++** |
| 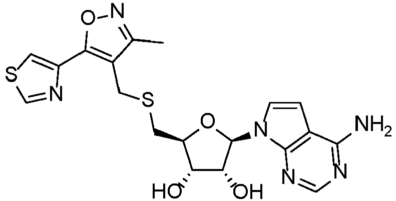 | **101c** | **122** | **++++** |
| 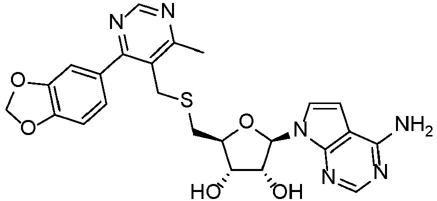 | **102c** | **123** | **++++** |
| 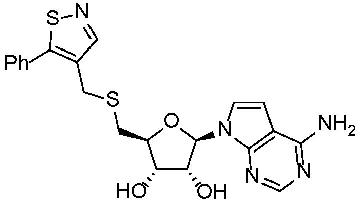 | **103c** | **124** | **++** |
| 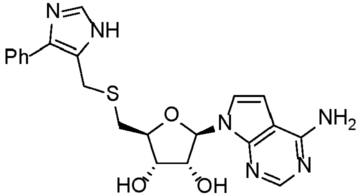 | **104c** | **125** | **++** |
| 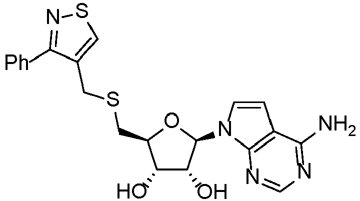 | **105c** | **126** | **++** |
| 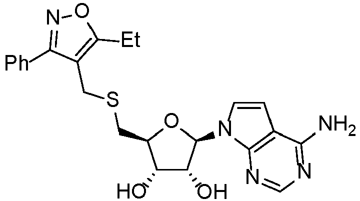 | **106c** | **127** | **+++** |
| 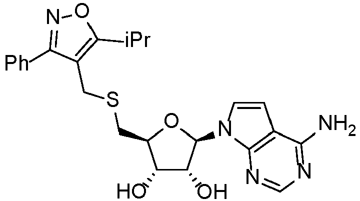 | **107c** | **128** | **++** |
| 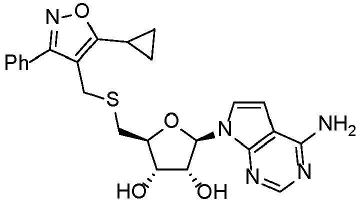 | **108c** | **129** | **+++** |
| 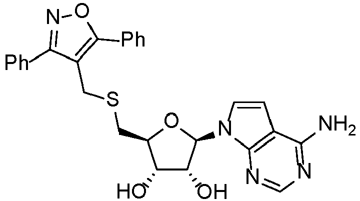 | **109c** | **130** | **+++** |
| 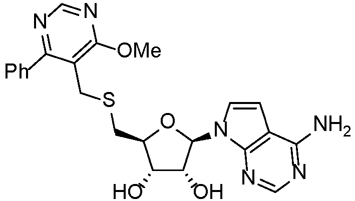 | **110c** | **131** | **++** |
| 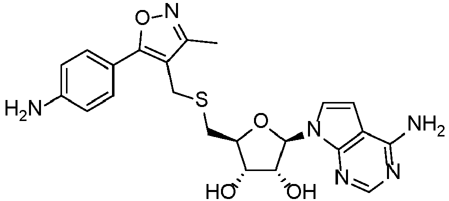 | **111** | **132** | **++++** |
| 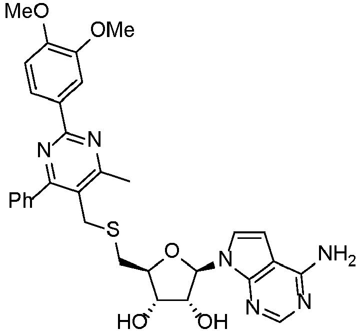 | **119c** | **140** | **++++** |
| 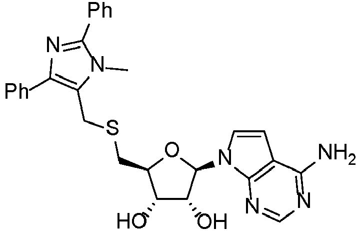 | **120c** | **141** | **++++** |
| 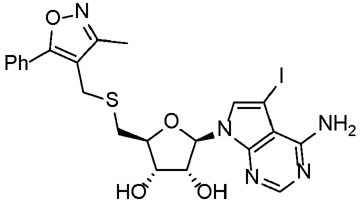 | **123** | **144** | **++++** |
| 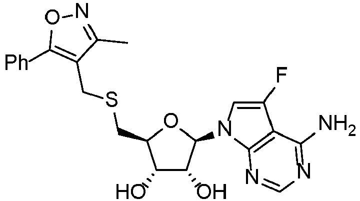 | **128** | **144** | **+++** |
| 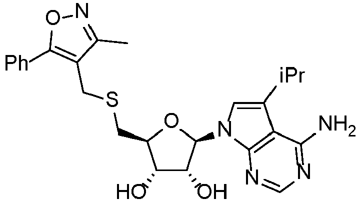 | **129b** | **162** | **+++** |
| 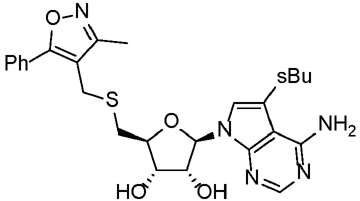 | **130b** | **163** | **+++** |
| 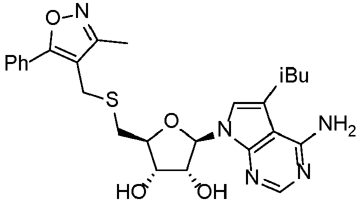 | **131b** | **164** | **+++** |
| 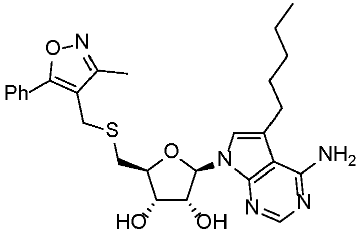 | **132b** | **165** | **++** |
| 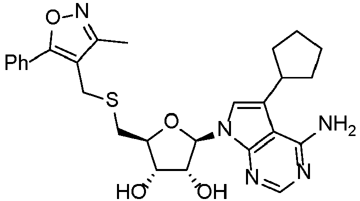 | **133b** | **166** | **+++** |
| 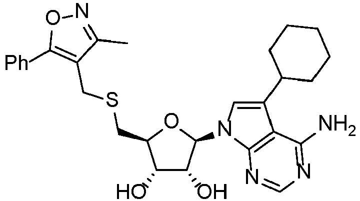 | **134b** | **167** | **+++** |
| 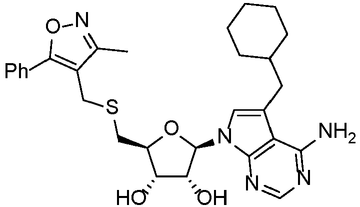 | **135b** | **168** | **++** |
| 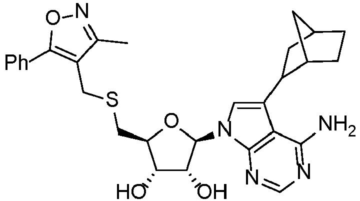 | **136b** | **169** | **+++** |
| 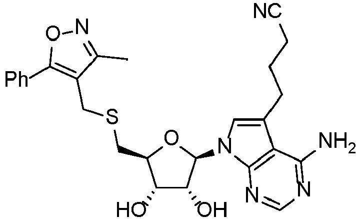 | **137b** | **170** | **+++++** |
| 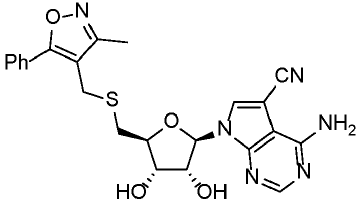 | **138b** | **171** | **++++** |
| 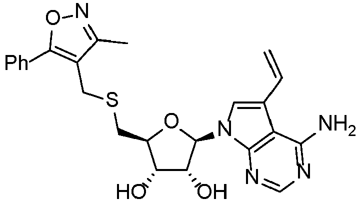 | **139b** | **172** | **++** |
| 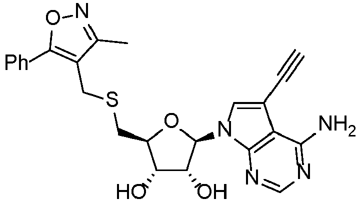 | **140b** | **173** | **++++** |
| 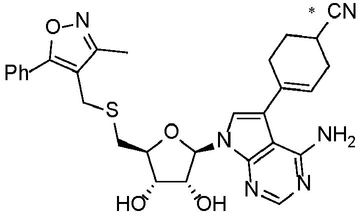 | **141b** | **174** | **+++++** |
| 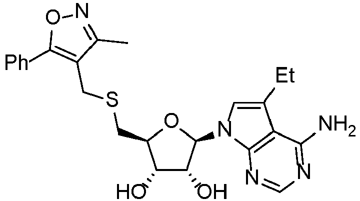 | **142** | **175** | **++++** |
| 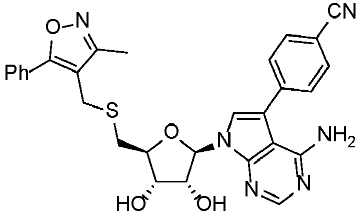 | **143b** | **182** | **+++++** |
| 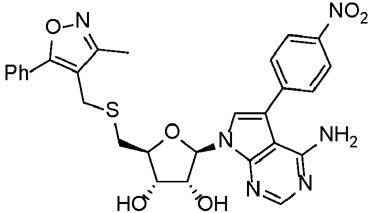 | **144b** | **183** | **++++** |
| 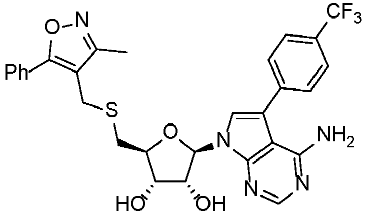 | **145b** | **184** | **+++** |
| 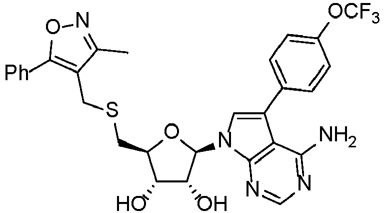 | **146b** | **185** | **+++** |
| 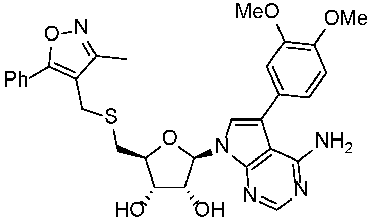 | **147b** | **186** | **+++++** |
| 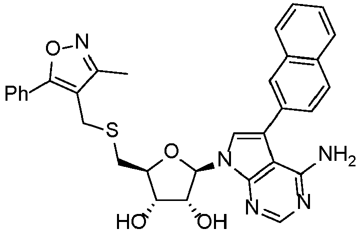 | **148b** | **187** | **+++** |
| 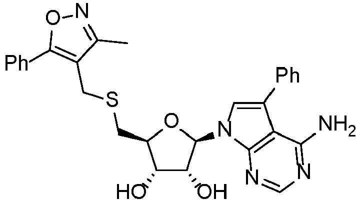 | **149** | **188** | **++++** |
| 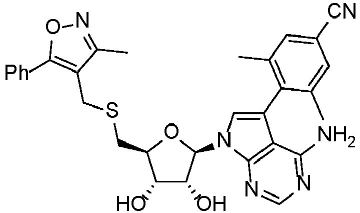 | **150** | **189** | **+++++** |
| 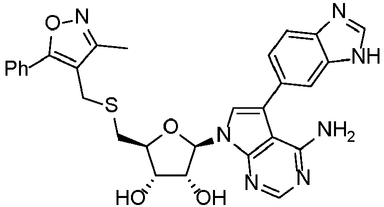 | **151** | **190** | **++++** |
| 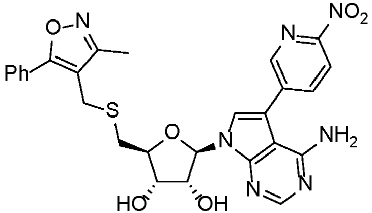 | **152** | **191** | **++++** |
| 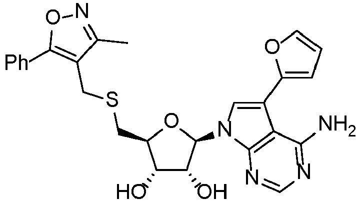 | **153** | **192** | **++++** |
| 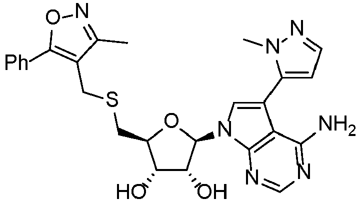 | **154** | **193** | **++++** |
| 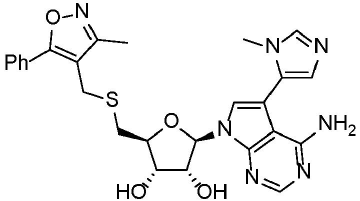 | **155** | **194** | **++++** |
| 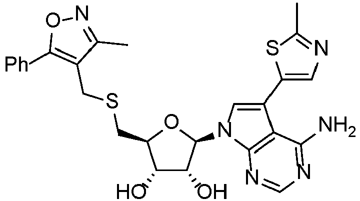 | **156** | **195** | **+++++** |
| 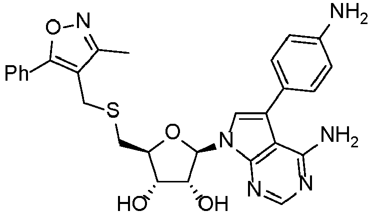 | **157** | **196** | **++++** |
| 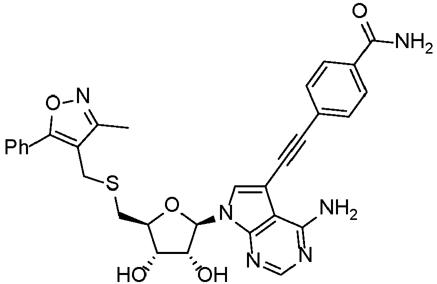 | **169b** | **219** | **++++** |
| 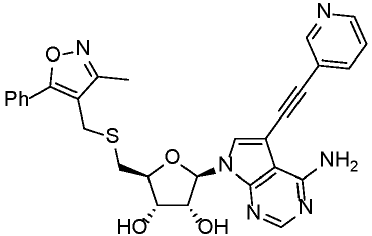 | **170b** | **220** | **+++++** |
| 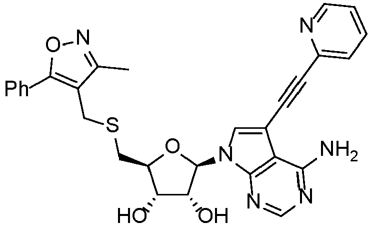 | **171b** | **221** | **+++++** |
| 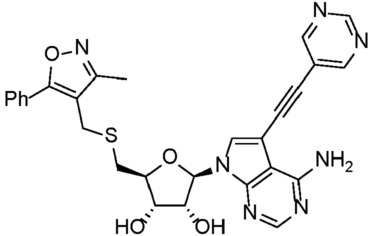 | **172b** | **222** | **+++++** |
| 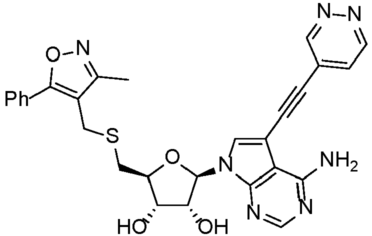 | **173b** | **223** | **+++++** |
| 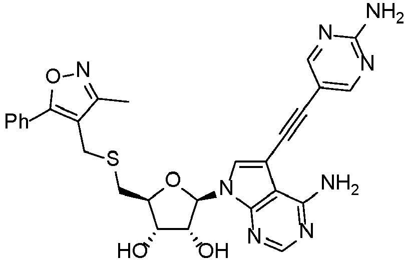 | **174b** | **224** | **++++** |
| 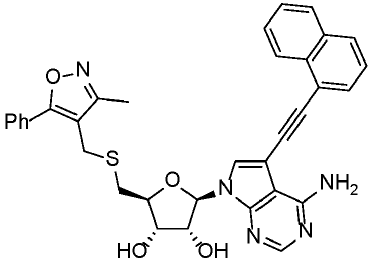 | **175b** | **225** | **++** |
| 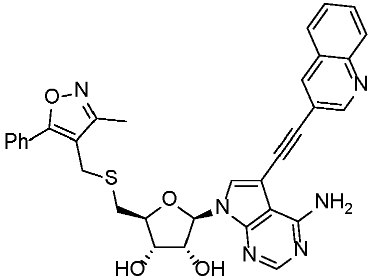 | **176b** | **226** | **+++** |
| 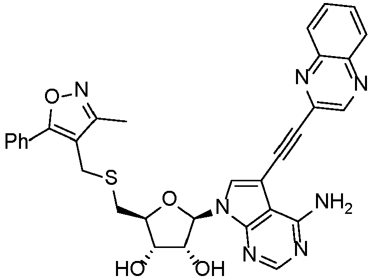 | **177b** | **227** | **++++** |
| 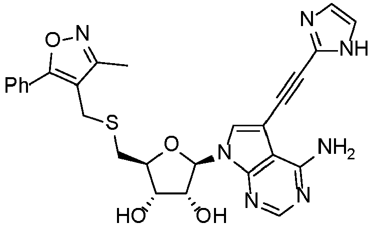 | **178b** | **228** | **++++** |
| 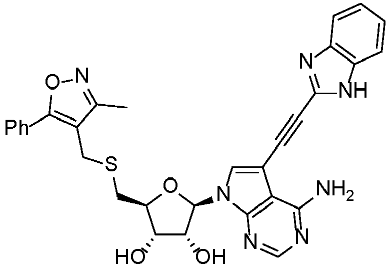 | **179b** | **229** | **+++** |
| 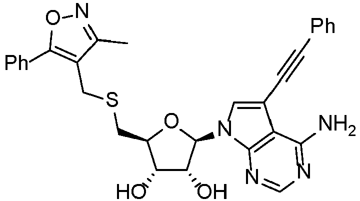 | **180** | **230** | **++++** |
| 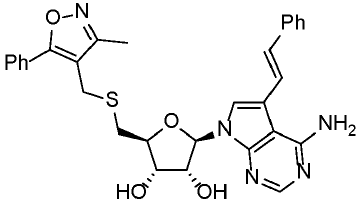 | **181b** | **233** | **+++** |
| 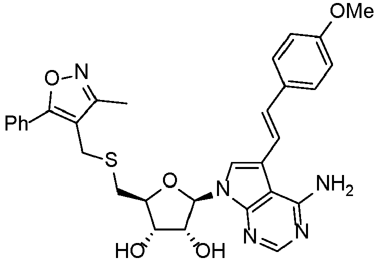 | **182b** | **234** | **++++** |
| 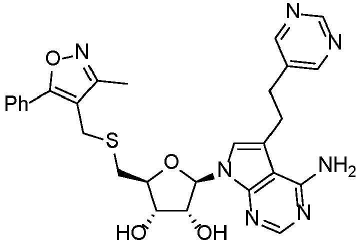 | **183** | **235** | **++++** |
| 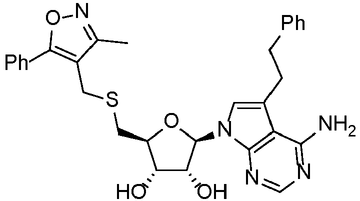 | **184** | **236** | **+++** |
| 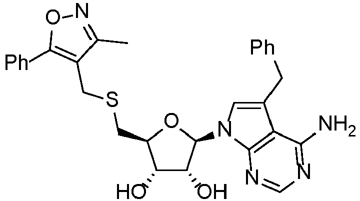 | **185b** | **239** | **+++** |
| 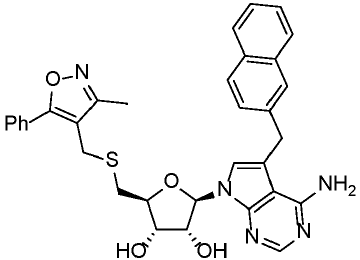 | **186b** | **240** | **++** |
| 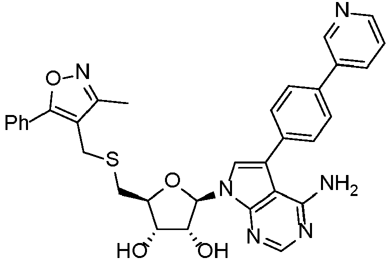 | **187** | **241** | **++++** |
| 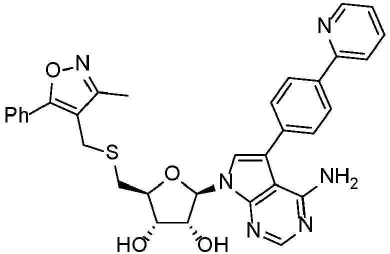 | **188** | **242** | **++++** |
| 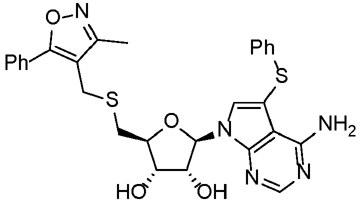 | **189b** | **245** | **++++** |
| 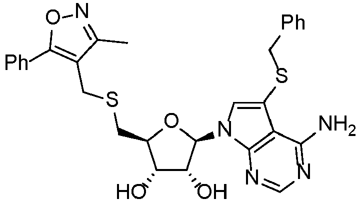 | **190b** | **246** | **+++** |
| 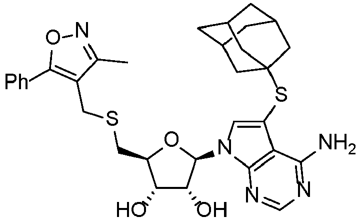 | **191** | **247** | **+++** |
| 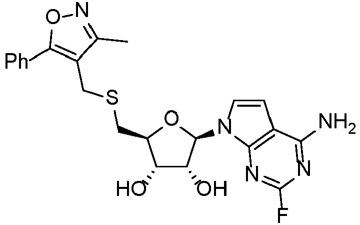 | **198** | **253** | **++++** |
| 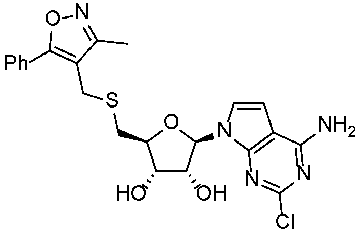 | **204** | **258** | **+++++** |
| 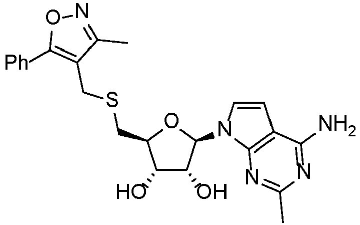 | **208** | **261** | **++++** |
| 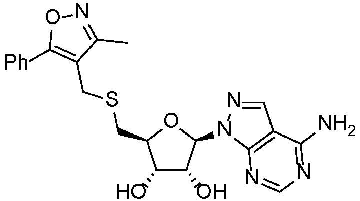 | **213** | **265** | **++++** |
| 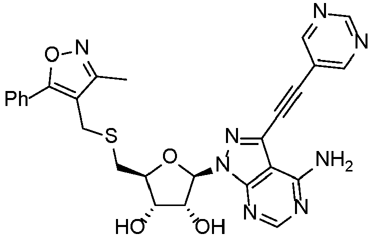 | **218b** | **275** | **++++** |
| 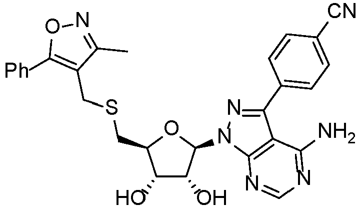 | **219b** | **276** | **++++** |
| 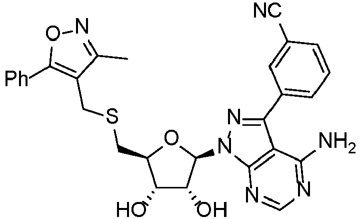 | **220b** | **277** | **+++** |
| 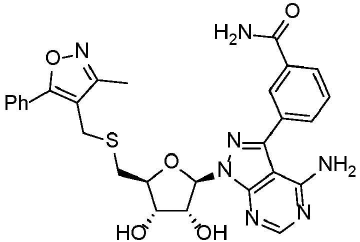 | **221b** | **278** | **++++** |
| 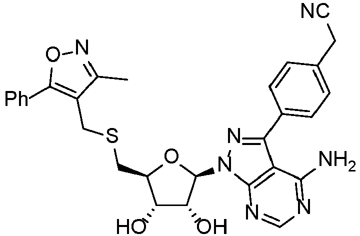 | **222b** | **279** | **+++++** |
| 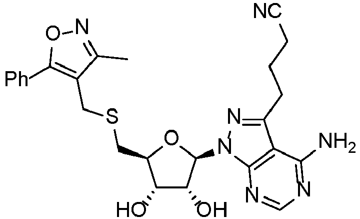 | **223b** | **280** | **++++** |
| 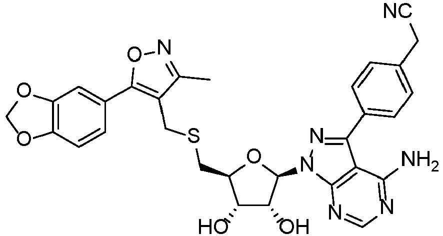 | **226** | **283** | **+++++** |
| 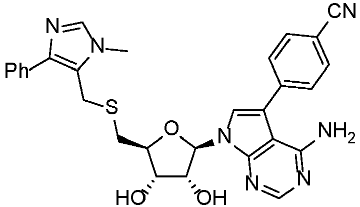 | **230b** | **294** | **+++** |
| 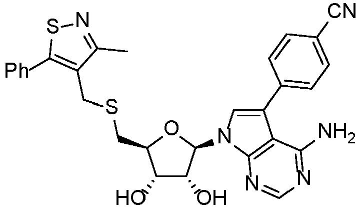 | **231b** | **295** | **+++** |
| 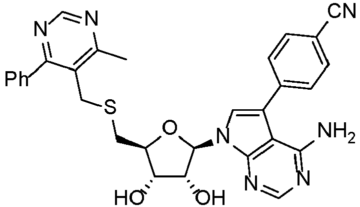 | **232b** | **296** | **++++** |
| 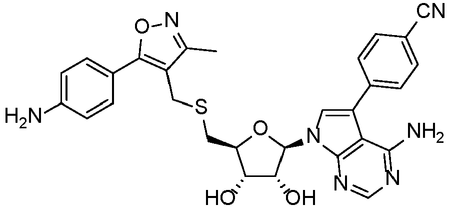 | **234b** | **297** | **+++++** |
| 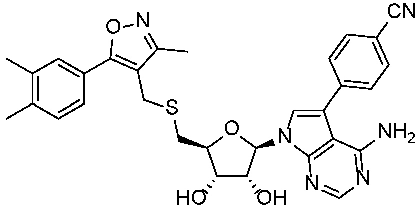 | **235b** | **298** | **++++** |
| 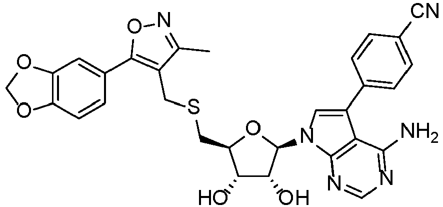 | **236b** | **299** | **+++++** |
| 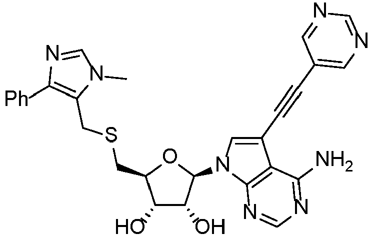 | **237c** | **313** | **++++** |
| 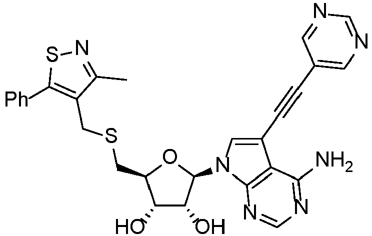 | **238c** | **314** | **++++** |
| 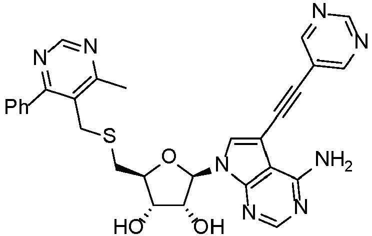 | **239c** | **315** | **++++** |
| 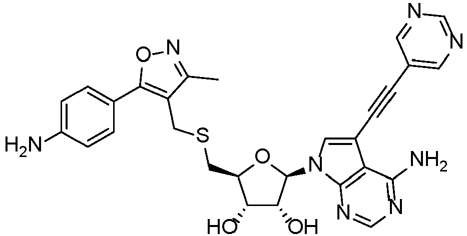 | **241c** | **316** | **+++++** |
| 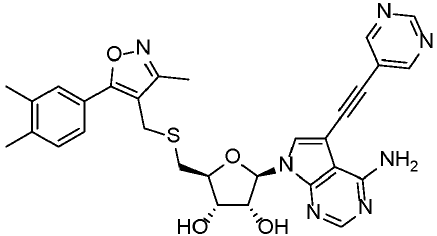 | **242c** | **317** | **+++++** |
| 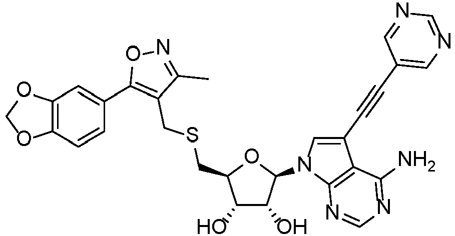 | **243c** | **318** | **+++++** |
| 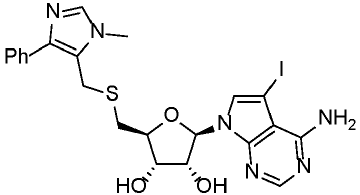 | **244** | **319** | **+++++** |
| 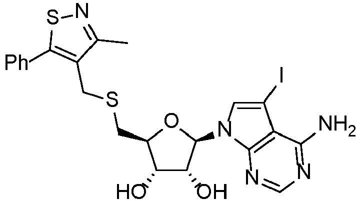 | **245** | **320** | **++++** |
| 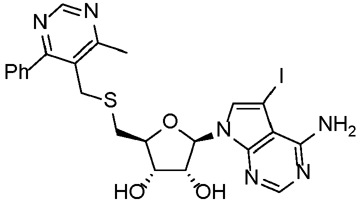 | **246** | **321** | **+++++** |

### Industrial application

The invention is applicable in the pharmaceutical industry and clinical use, in preparation and manufacture of medicaments and medical therapy for treatment or prevention of human or animal diseases caused by viruses from Orthocoronavirinae subfamily including but not limited to SARS-CoV, MERS-CoV, SARS-CoV-2, and FIPV.

## Claims

1. Compound of general formula (**I**): 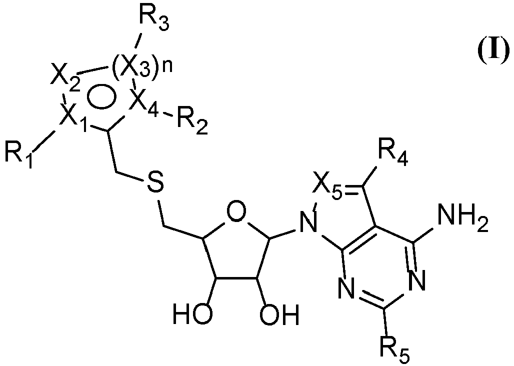 wherein:
**n** is 1 or 2,
**X₁** and **X₄** are independently selected from C and N; wherein one or both of **X₁** and **X₄** are carbon atoms;
and wherein when **X₁** and/or **X₄** is N, n is 1;
**X₂** and **X₃** are independently selected from C, CH, N, NH, O and S,
wherein preferably at least one of **X₂**, **X₃** is a heteroatom selected from N, O, S,
and wherein when **X₃** is oxygen or sulphur, **R₃** is not present;
**X₅** is CorN;
wherein **R₁** is selected from (C3-C6)cycloalkyl, benzyl, (C6-C12)aryl and heteroaryl containing 2-10 carbon atoms and at least one heteroatom selected from N, O, S;
wherein the cycloalkyl, benzyl, aryl and heteroaryl group in **R₁** may optionally be substituted by one or
more substituents, which may be the same or different, selected from halogeno, nitro, amino, (C1-C8)alkyl, (C1-C8)alkoxy;
**R₂** is selected from hydrogen, (C1-C8)alkyl, (C3-C6)cycloalkyl, (C6-C12)aryl, (C1-C8)alkoxy,
R**₃** is selected from hydrogen and phenyl, wherein phenyl can be substituted by one or more substituents selected from (C1-C4)alkyl, (C1-C4)alkoxy and halogen;,
**R₄** is a substituent group of the formula: 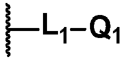
wherein **L₁** is selected from a bond, -(CH₂)_{1 to 4}-, -CH=CH-, -C≡C-, -S-, -O-, -NH-, (C6-C12)aryl and heteroaryl containing 2-10 carbon atoms and at least one heteroatom selected from N, O, S,
and **Q₁** is selected from hydrogen, halogen, trifluoromethyl, trifluoromethoxy, cyano, nitro, amino, (C1-C8)alkyl, (C2-C8)alkenyl, (C2-C8)alkynyl, (C3-C6)cycloalkyl, (C7-C10)polycycloalkyl, (C5-C6)cycloalkenyl, benzyl, (C1-C8)alkoxy, (C6-C10)aryl, and heteroaryl containing 2-10 carbon atoms and at least one heteroatom selected from N, O, S,
and wherein **Q₁** is optionally substituted by one or more substituents, which may be the same or different, selected from cyano, cyano(C1-C4)alkyl, amino, carbamoyl, (C1-C8)alkoxy,
**R₅** is selected from hydrogen, halogeno, trifluoromethyl, (C1-C6)alkyl, and (C3-C6) cycloalkyl, and/or hydrates and/or solvates, and/or pharmaceutically accetable salts thereof.

2. Compound according to claim 1, wherein **R₁** is benzo[d][1,3]dioxolyl, naphthyl or phenyl, preferably, **R₁** is phenyl further substituted by one or more of amino and (C1-C8)alkyl.

3. Compound according to claim 1 or 2, wherein **R₂** is (C1-C3)alkyl or (C3-C6)cycloalkyl, more preferably **R₂** is methyl or cyclopropyl.

4. Compound according to any one of claims 1 to 3, wherein **R₃** is hydrogen, phenyl or 3,4-dimethoxyphenyl.

5. Compound according to any one of claims 1 to 4, wherein **L₁** is selected from a bond, -CH₂-, -CH₂-CH₂-, -CH₂-CH₂-CH₂-, -CH=CH-, -C≡C-, -S-, wherein the configuration of the -CH=CH- is "trans"; preferably, **L₁** is selected from a bond and -C≡C-.

6. Compound according to any one of claims 1 to 5, wherein **Q₁** is selected from halogen, cyano, (C1-C12)aryl and heteroaryl containing 2-5 carbon atoms and at least one heteroatom selected from N, O, S.

7. Compound according to any one of claims 1 to 6, wherein **R₅** is selected from hydrogen, halogen and (C1-C8)alkyl.

8. Compound according to any one of claims 1 to 7, wherein
- both **X₁** and **X₄** are carbon atoms, n = 1, and **X₂** and **X₃** are N and O, or N and S, or C and S; or
- two or three of **X₁**, **X₂**, **X₃** and **X₄** are a nitrogen atom and the remaining instances of these substituents are carbon atoms, and n = 1; or
- two of **X₁**, **X₂**, **X₃** and **X₄** are a nitrogen atom and the remaining instances of these substituents are carbon atoms, and n = 2; or
- one of **X₂**, **X₃** is a nitrogen atom and the remaining instances of these substituents are carbon atoms, and n = 2; or
- two of **X₁**, **X₂**, **X₃** and **X₄** are a nitrogen atom and the remaining instances of these substituents are carbon atoms, and n = 2.

9. Compound according to any one of claims 1 to 8 and/or hydrate and/or solvate, and/or pharmaceutically accetable salt thereof for use as a medicament.

10. Compound according to any one of claims 1 to 8 and/or hydrate and/or solvate, and/or pharmaceutically accetable salt thereof for use in the treatment or prevention of human or animal diseases caused by viruses from Orthocoronavirinae subfamily.

11. Compound according to any one of claims 1 to 8 and/or hydrate and/or solvate, and/or pharmaceutically accetable salt thereof for use in the treatment or prevention of human or animal diseases caused by viruses selected from SARS-CoV, MERS-CoV, SARS-CoV-2, and FIPV.

12. A pharmaceutical composition comprising at least one compound according to any one of claims 1 to 8 and/or hydrate and/or solvate, and/or pharmaceutically accetable salt thereof and at least one pharmaceutically acceptable carrier, filler and/or diluent and/or adjuvant.
